(19) Europäisches Patentamt
European Patent Office
Office européen des brevets

(11) EP 4 438 601 A1

(12) EUROPEAN PATENT APPLICATION
published in accordance with Art. 153(4) EPC

(43) Date of publication:
02.10.2024 Bulletin 2024/40

(21) Application number: 22897914.2

(22) Date of filing: 25.11.2022

(51) International Patent Classification (IPC):
C07D 417/14 (2006.01) A61K 31/4353 (2006.01)
A61P 11/00 (2006.01)

(52) Cooperative Patent Classification (CPC):
A61K 31/4353; A61P 11/00; C07D 417/14

(86) International application number:
PCT/CN2022/134258

(87) International publication number:
WO 2023/093832 (01.06.2023 Gazette 2023/22)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 25.11.2021 CN 202111416045
29.09.2022 CN 202211205107
17.11.2022 CN 202211457401

(71) Applicant: Shanghai Jemincare Pharmaceutical Co., Ltd.
Shanghai 201315 (CN)

(72) Inventors:
- DENG, Gang
Shanghai 201203 (CN)
- GUO, Shuchun
Shanghai 201203 (CN)
- FAN, Jun
Shanghai 201203 (CN)
- ZHANG, Zhitao
Shanghai 201203 (CN)
- WU, Nan
Shanghai 201203 (CN)
- SHI, Wenqiang
Shanghai 201203 (CN)
- FANG, Zhihua
Shanghai 201203 (CN)
- FENG, Jianbo
Shanghai 201203 (CN)
- PENG, Jianbiao
Shanghai 201203 (CN)
- GUO, Haibing
Shanghai 201203 (CN)

(74) Representative: Dehns
St. Bride's House
10 Salisbury Square
London EC4Y 8JD (GB)

# (54) TRICYCLIC DERIVATIVE AND PREPARATION METHOD THEREFOR AND APPLICATION THEREOF

(57) Disclosed are a tricyclic derivative and a preparation method therefor and an application thereof. Specifically, disclosed are a compound shown in formula (I) and an optical isomer thereof or a pharmaceutically acceptable salt thereof, and an application of the compound as an Autotaxin inhibitor.

EP 4 438 601 A1

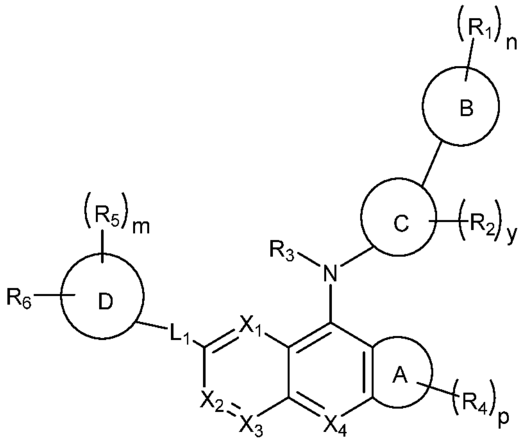

(I)

## Description

The present application claims the right of the following priorities:

CN202111416045.3, application date: November 25, 2021;
CN202211205107.0, application date: September 29, 2022;
CN202211457401.0, application date: November 17, 2022.

TECHNICAL FIELD

The present disclosure relates to a compound of formula (I), an optical isomer thereof, and a pharmaceutically acceptable salt thereof, and a use of the compound as an autotaxin inhibitor.

BACKGROUND

The present disclosure involves a small molecule inhibitor of autotaxin (ATX). Autotaxin, also known as ENPP2 (ectonucleotide pyrophosphatase/phosphodiesterase 2), possesses both phosphodiesterase (PDE) and lysophospholipase (LysoPLD) activities, and is capable of catalyzing the production of lysophosphatidic acid (LPA) using lysophosphatidylcholine (LPC) as a substrate. LPA is a signaling molecule that can activate cell surface G protein-coupled receptors $LPAR_{1-6}$, mediate a variety of signal transduction pathways and cause a wide range of biological effects, regulate cell proliferation, survival, and migration, and participate in a variety of physiological and pathological processes. Autotaxin is widely expressed in the human body, with the highest mRNA levels in the brain, lymph nodes, kidneys, and testicles, and is a key enzyme for the production of LPA in plasma and tissues. Autotaxin is localized on the cell surface by binding to cell surface molecules such as integrins, allowing LPA to be generated in the vicinity of LPAR receptors and rapidly activate downstream signaling pathways. The specific biological activity of the autotaxin-LPA-LPAR axis is affected by factors such as cell type, receptor subtype and expression level, and LPC/LPA concentration, and has a wide range of physiological and cellular effects.

There is evidence that the autotaxin-LPA-LPAR axis is associated with a variety of diseases, including fibrotic disease (*e.g.*, pulmonary fibrosis, renal fibrosis, hepatic fibrosis, non-alcoholic steatohepatitis (NASH)), proliferative disease (*e.g.*, tumors), inflammatory disease (*e.g.*, enteritis, inflammatory pain), autoimmune disease (*e.g.*, rheumatoid arthritis, multiple sclerosis), respiratory disease (*e.g.*, interstitial lung disease, asthma, chronic obstructive pulmonary disease (COPD)), cardiovascular disease (*e.g.*, vascular injury, atherosclerosis, coagulation), neuropathic pain, myelodysplastic syndrome, obesity and metabolic disease, and disease related to abnormal angiogenesis, *etc.* The LPA levels in the plasma of mutant mice heterozygous for knockout of autotaxin are approximately half those in normal mice, indicating that autotaxin is the predominant source for the production of LPA. Therefore, if autotaxin enzyme activity can be effectively inhibited, the effect of alleviating or treating a related disease may be achieved by down-regulating the LPA-LPAR signaling pathway related to the disease. There is an urgent need in the art for the development of a small molecule inhibitor with a novel structure, good druggability, and the ability to effectively inhibit autotaxin enzyme activity.

CONTENT OF THE PRESENT INVENTION

In the first aspect of the present disclosure, the present disclosure provides a compound of formula (I), an optical isomer thereof, or a pharmaceutically acceptable salt thereof,

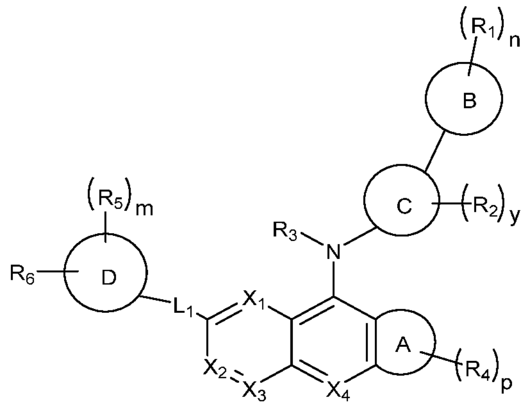

(I)

,

wherein

ring A is selected from cycloalkyl, heterocyclyl, and heteroaryl;
ring B is selected from cycloalkyl, heterocyclyl, aryl, and heteroaryl;
ring C is selected from aryl and heteroaryl;
ring D is selected from aryl, heteroaryl, cycloalkyl, and heterocyclyl;
$X_1$ is selected from $C(R_{7a})$ and N;
$X_2$ is selected from $C(R_{7b})$ and N;
$X_3$ is selected from $C(R_{7c})$ and N;
$X_4$ is selected from $C(R_{7d})$ and N;
Li is selected from a single bond, NRs, O, S, and $C_{1-6}$ alkyl;
each $R_1$ is independently selected from H, halogen, alkyl, haloalkyl, alkoxy, OH, hydroxyalkyl, cyano, amino, nitro, carboxyl, aldehyde, cycloalkyl, heterocyclyl, aryl, and heteroaryl;
each $R_2$ is independently selected from H, halogen, alkyl, haloalkyl, alkoxy, cyano, amino, nitro, carboxyl, aldehyde, OH, hydroxyalkyl, cycloalkyl, heterocyclyl, aryl, and heteroaryl;
$R_3$ is selected from H, alkyl, cycloalkyl, and heterocyclyl, wherein the alkyl, cycloalkyl, and heterocyclyl are each independently and optionally substituted by one or more than one substituent selected from halogen, alkyl, alkoxy, cyano, amino, nitro, OH, hydroxyalkyl, carboxyl, cycloalkyl, heterocyclyl, aryl, and heteroaryl;
$R_4$ is selected from H, halogen, alkyl, haloalkyl, heteroalkyl, cyano, amino, nitro, OH, hydroxyalkyl, cycloalkyl, heterocyclyl, $-COOR_9$, aryl, and heteroaryl, wherein the alkyl, heteroalkyl, cycloalkyl, heterocyclyl, aryl, and heteroaryl are each independently and optionally substituted by one or more than one substituent selected from halogen, alkyl, alkoxy, cyano, amino, nitro, OH, hydroxyalkyl, cycloalkyl, heterocyclyl, aryl, and heteroaryl;
each $R_5$ is independently selected from H, halogen, alkyl, haloalkyl, alkoxy, cyano, amino, nitro, carboxyl, aldehyde, OH, hydroxyalkyl, oxo, cycloalkyl, heterocyclyl, aryl, and heteroaryl, wherein the alkyl, alkoxy, cycloalkyl, heterocyclyl, aryl, and heteroaryl are each independently and optionally substituted by one or more than one substituent selected from halogen, alkyl, alkoxy, cyano, amino, nitro, carboxyl, OH, hydroxyalkyl, cycloalkyl, heterocyclyl, aryl, and heteroaryl;
$R_6$ is -M-L2-Ra;
M is selected from a single bond or alkyl, wherein the alkyl is optionally substituted by one or more than one substituent selected from halogen, alkyl, alkoxy, cyano, amino, nitro, carboxyl, OH, hydroxyalkyl, cycloalkyl, heterocyclyl, aryl, and heteroaryl;
$L_2$ is selected from a single bond, -C(=O)-, -C(=O)O-, $-C(=O)NR_b-$, $-NR_bC(=O)-$, $- NR_bC(=O)O-$, -O-, -OC(=O)-, -C(=O)-C(=O)-, $-C(=O)-C(=O)NR_b-$, $-NR_b-$, $-S(=O)_2-$, $- S(=O)_2NR_b-$, and $-NR_bS(=O)_2-$;
$R_a$ is selected from H, $-S(O)_2R_c$, alkyl, $-NR_bR_c$, cycloalkyl, heterocyclyl, aryl, and heteroaryl, wherein the alkyl, cycloalkyl, heterocyclyl, aryl, and heteroaryl are each independently and optionally substituted by one or more than one substituent selected from halogen, alkyl, alkoxy, oxo, cyano, amino, nitro, carboxyl, OH, hydroxyalkyl, cycloalkyl, heterocyclyl, aryl, and heteroaryl;
$R_b$ is selected from H, OH, alkyl, haloalkyl, hydroxyalkyl, and cycloalkyl;
$R_c$ is selected from H and alkyl;
$R_{7a}$, $R_{7b}$, $R_{7c}$, and $R_{7d}$ are each independently selected from H, halogen, alkyl, haloalkyl, alkoxy, cyano, amino, nitro, OH, hydroxyalkyl, cycloalkyl, heterocyclyl, aryl, and heteroaryl;
$R_8$ is selected from H, alkyl, haloalkyl, hydroxyalkyl, and cycloalkyl;

$R_9$ is selected from H, alkyl, haloalkyl, hydroxyalkyl, and cycloalkyl;
n is 0, 1, 2, 3, or 4;
y is 0, 1, 2, or 3;
m is 0, 1, 2, 3, or 4;
p is 0, 1, 2, or 3.

In another aspect of the present disclosure, the present disclosure also provides a compound of formula (II), an optical isomer thereof, or a pharmaceutically acceptable salt thereof,

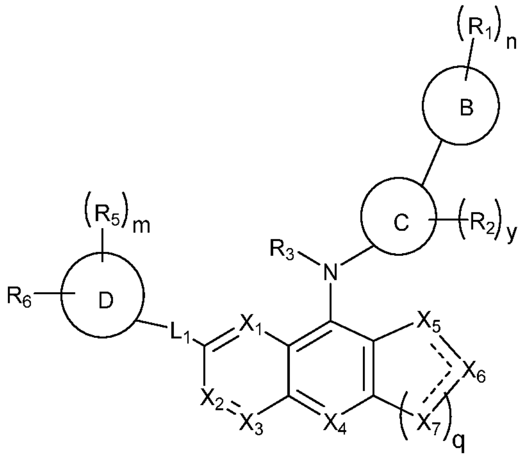

( II )

,

wherein

q is 0, 1, 2, or 3;
$X_5$ is selected from O, S, $N(R_{4a})$, $C(R_{4a})_2$, and C=O;
$X_6$ is selected from O, S, $N(R_{4b})$, $C(R_{4b})_2$, and C=O;
each $X_7$ is independently selected from $N(R_{4c})$, $C(R_{4c})_2$, and C=O;

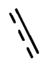

represents a double bond or a single bond;
and, when

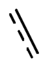

between $X_5$ and $X_6$ represents a double bond, $X_5$ is selected from N and $C(R_{4a})$, and $X_6$ is selected from N and $C(R_{4b})$; and, $X_6$ is not attached to two double bonds simultaneously;
$R_{4a}$, $R_{4b}$, and $R_{4c}$ are each independently selected from H, halogen, alkyl, haloalkyl, heteroalkyl, cyano, amino, nitro, OH, hydroxyalkyl, cycloalkyl, heterocyclyl, $-COOR^9$, aryl, and heteroaryl, wherein the alkyl, heteroalkyl, cycloalkyl, heterocyclyl, aryl, and heteroaryl are each independently and optionally substituted by one or more than one substituent selected from halogen, alkyl, alkoxy, cyano, amino, nitro, OH, hydroxyalkyl, cycloalkyl, heterocyclyl, aryl, and heteroaryl;
ring B, ring C, ring D, $R_1$, $R_2$, $R_3$, $R_5$, $R_6$, $X_1$, $X_2$, $X_3$, $X_4$, n, m, and y are as previously defined.

In some embodiments of the present disclosure, the compound of formula (II), the optical isomer thereof, or the pharmaceutically acceptable salt thereof is selected from

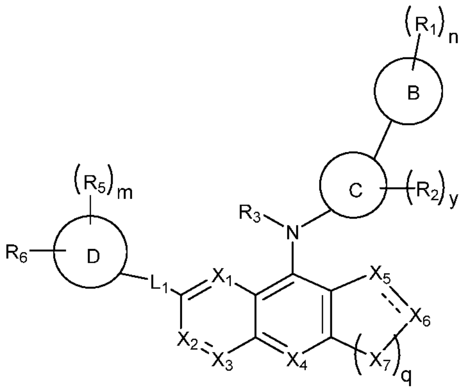

( II-A)

and

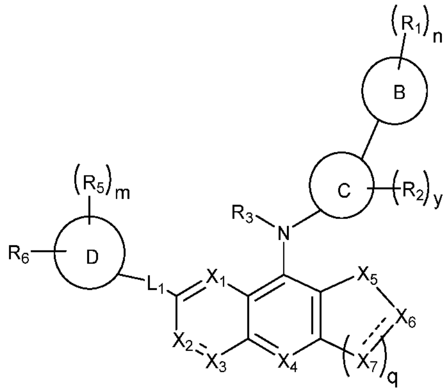

( II-B ) ,

wherein each variable is as previously described.

In some embodiments of the present disclosure, the compound of formula (II), the optical isomer thereof, or the pharmaceutically acceptable salt thereof is selected from

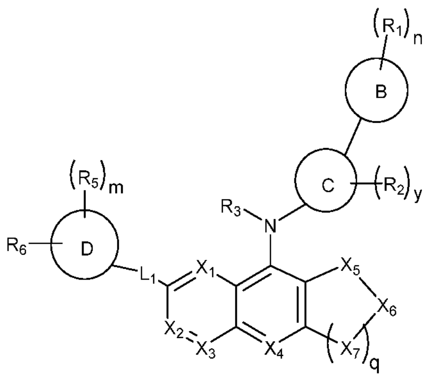

( II-1) ,

wherein $X_5$ is selected from O, S, $N(R_{4a})$, $C(R_{4a})_2$, and C=O; $X_6$ is selected from O, S, $N(R_{4b})$, $C(R_{4b})_2$, and C=O; each $X_7$ is independently selected from $N(R_{4c})$, $C(R_{4c})_2$, and C=O, and the rest of the variables are as defined in the present disclosure.

In some embodiments of the present disclosure, the compound of formula (II), the optical isomer thereof, or the pharmaceutically acceptable salt thereof is selected from

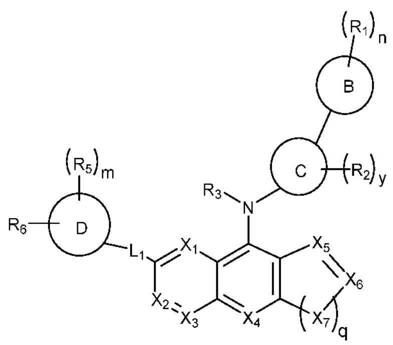

( II-2) ,

wherein $X_5$ is selected from N and $C(R_{4a})$, $X_6$ is selected from N and $C(R_{4b})$, each $X_7$ is independently selected from $N(R_{4c})$, $C(R_{4c})_2$, and C=O, and the rest of the variables are as defined in the present disclosure.

In some embodiments of the present disclosure, the compound of formula (II), the optical isomer thereof, or the pharmaceutically acceptable salt thereof is selected from

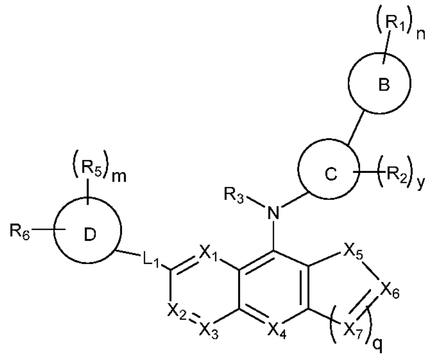

( II-3) ;

it should be noted that in formula (II-3), when q is selected from 2, the structure of formula (II-3) is

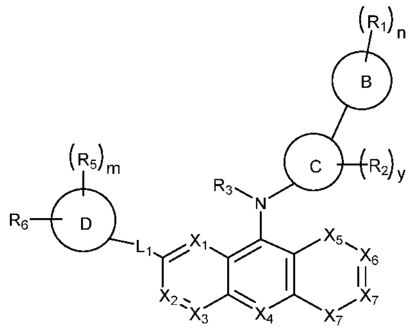

( II-3a) ;

when q is selected from 3, the structure of formula (II-3) is

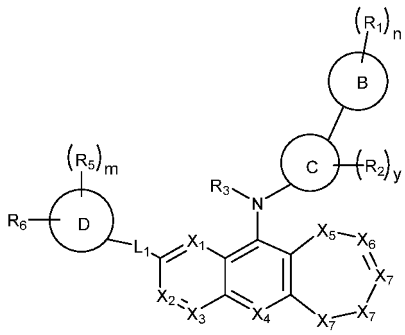

( II-3a) ;

wherein $X_5$ is selected from O, S, $N(R_{4a})$, $C(R_{4a})_2$, and C=O, and $X_6$ is selected from N and $C(R_{4b})$; when the bond attached to $X_7$ is a double bond, each $X_7$ is independently selected from N and $C(R_{4b})$; when the bond attached to $X_7$ is a single bond, each $X_7$ is independently selected from $N(R_{4c})$, $C(R_{4c})_2$, and C=O, and the rest of the variables are as defined in the present disclosure.

In another aspect of the present disclosure, the present disclosure also provides a compound of formula (III), an optical isomer thereof, or a pharmaceutically acceptable salt thereof,

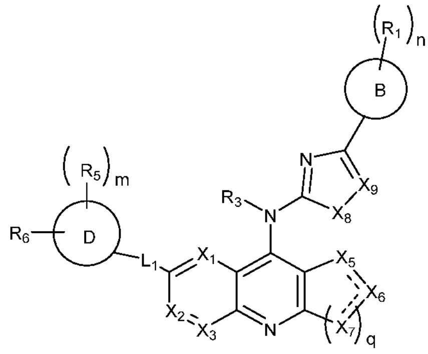

(III) ,

wherein

each $X_8$ is independently selected from O, S, N($R_{2a}$), -N=CH-, -CH=N-, and -CH=CH;
each $X_9$ is independently selected from C($R_{2b}$) and N;
$R_{2a}$ and $R_{2b}$ are each independently selected from H, halogen, alkyl, haloalkyl, alkoxy, cyano, amino, nitro, carboxyl, aldehyde, OH, hydroxyalkyl, cycloalkyl, heterocyclyl, aryl, and heteroaryl;
ring B, ring D, $R_1$, $R_3$, $R_5$, $R_6$, $X_1$, $X_2$, $X_3$, $X_4$, n, and m are as previously defined;

$X_5$, $X_6$, $X_7$, q, and 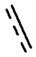 are as previously defined.

In some embodiments of the present disclosure, the compound of formula (III), the optical isomer thereof, or the pharmaceutically acceptable salt thereof is selected from

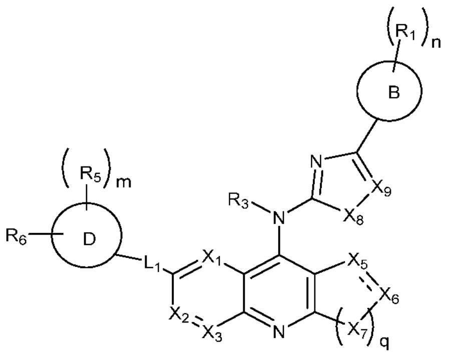

(III-A) and

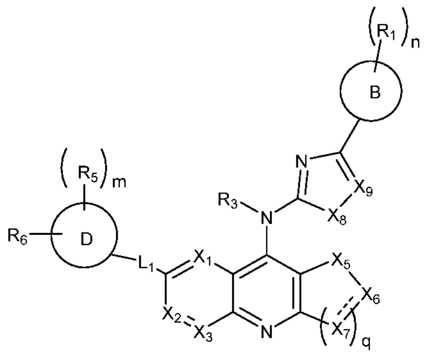

(III-B) ,

wherein each variable is as previously described.

In some embodiments of the present disclosure, the compound of formula (III), the optical isomer thereof, or the pharmaceutically acceptable salt thereof is selected from

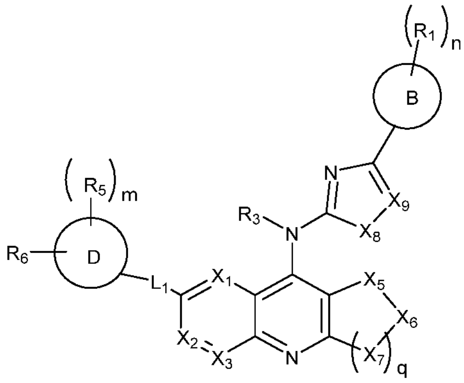

(III-1)

,

wherein $X_5$ is selected from O, S, $N(R_{4a})$, $C(R_{4a})_2$, and C=O; $X_6$ is selected from O, S, $N(R_{4b})$, $C(R_{4b})_2$, and C=O; each $X_7$ is independently selected from $N(R_{4c})$, $C(R_{4c})_2$, and C=O, and the rest of the variables are as defined in the present disclosure.

In some embodiments of the present disclosure, the compound of formula (III), the optical isomer thereof, or the pharmaceutically acceptable salt thereof is selected from

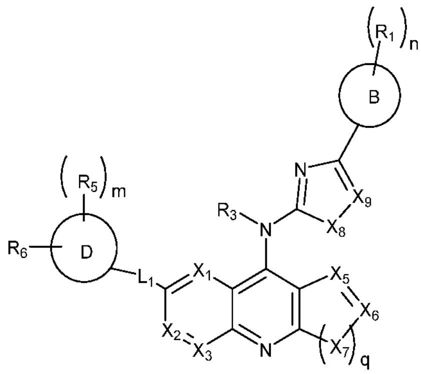

(III-2)

,

wherein $X_5$ is selected from N and $C(R_{4a})$, $X_6$ is selected from N and $C(R_{4b})$, each $X_7$ is independently selected from $N(R_{4c})$, $C(R_{4c})_2$, and C=O, and the rest of the variables are as defined in the present disclosure.

In some embodiments of the present disclosure, the compound of formula (III), the optical isomer thereof, or the pharmaceutically acceptable salt thereof is selected from

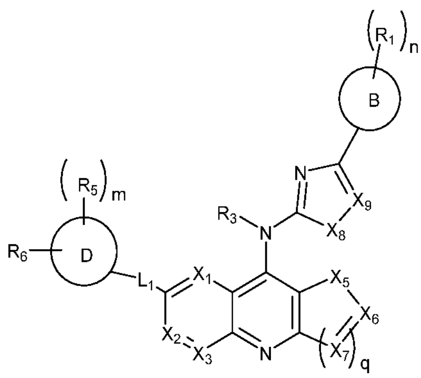

(III-3)

;

it should be noted that in formula (III-3), when q is selected from 2, the structure of formula (III-3) is

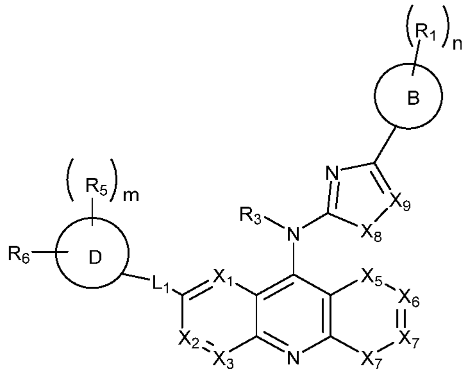

(III-3a) ;

when q is selected from 3, the structure of formula (III-3) is

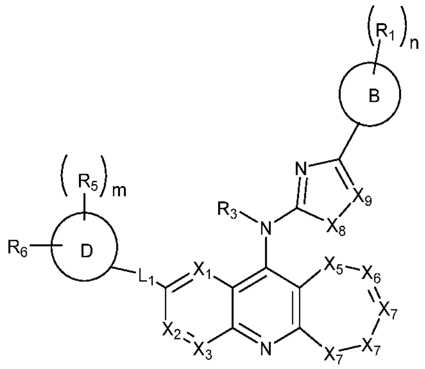

(III-3b) ;

wherein $X_5$ is selected from O, S, $N(R_{4a})$, $C(R_{4a})_2$, and C=O, and $X_6$ is selected from N and $C(R_{4b})$; when the bond attached to $X_7$ is a double bond, each $X_7$ is independently selected from N and $C(R_{4b})$; when the bond attached to $X_7$ is a single bond, each $X_7$ is independently selected from $N(R_{4c})$, $C(R_{4c})_2$, and C=O, and the rest of the variables are as defined in the present disclosure.

In some embodiments of the present disclosure, the ring A is selected from $C_{4-8}$ cycloalkyl, 4- to 8-membered heterocyclyl, and 5- to 6-membered heteroaryl, and other variables are as defined in the present disclosure.

In some embodiments of the present disclosure, the ring A is selected from $C_{4-6}$ cycloalkyl, 5- to 6-membered heterocyclyl, and 5- to 6-membered heteroaryl, and other variables are as defined in the present disclosure.

In some embodiments of the present disclosure, the ring A is selected from cyclobutyl, cyclopentyl, tetrahydrofuranyl, pyrrolidinyl, cyclopentanone, dihydrofuran-2(3*H*)-one, pyrrolidin-2-one, imidazolyl, pyrazolyl, oxazolyl, isoxazolyl, and 1,2,3-oxadiazolyl, and other variables are as defined in the present disclosure.

In some embodiments of the present disclosure, each $R_4$ is independently selected from H, OH, $C_{1-6}$ alkyl, and $C_{1-6}$ heteroalkyl, wherein the $C_{1-6}$ alkyl and $C_{1-6}$ heteroalkyl are optionally substituted by one or more than one of OH, amino, and halogen, and other variables are as defined in the present disclosure.

In some embodiments of the present disclosure, each $R_4$ is independently selected from H, OH, $C_{1-6}$ alkyl, and $C_{1-6}$ alkyl-C(=O)O-, wherein the $C_{1-6}$ alkyl and $C_{1-6}$ alkyl-C(=O)O-are optionally substituted by one or more than one of OH, amino, and halogen, and other variables are as defined in the present disclosure.

In some embodiments of the present disclosure, each $R_4$ is independently selected from H, OH, methyl, ethyl,

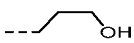,

and $CF_3$, and other variables are as defined in the present disclosure.

In some embodiments of the present disclosure, the $R_{4a}$, $R_{4b}$, and $R_{4c}$ are each independently selected from H, OH, $C_{1-6}$ alkyl, and $C_{1-6}$ heteroalkyl, wherein the $C_{1-6}$ alkyl and $C_{1-6}$ heteroalkyl are optionally substituted by one or more than one of OH, amino, and halogen, and other variables are as defined in the present disclosure.

In some embodiments of the present disclosure, the $R_{4a}$, $R_{4b}$, and $R_{4c}$ are each independently selected from H, OH, $C_{1-6}$ alkyl, and $C_{1-6}$ alkyl-C(=O)O-, wherein the $C_{1-6}$ alkyl and $C_{1-6}$ alkyl-C(=O)O- are optionally substituted by one or more than one of OH, amino, and halogen, and other variables are as defined in the present disclosure.

In some embodiments of the present disclosure, the $R_{4a}$, $R_{4b}$, and $R_{4c}$ are each independently selected from H, OH, methyl, ethyl,

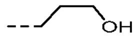,

and $CF_3$, and other variables are as defined in the present disclosure.

In some embodiments of the present disclosure, the structural moiety

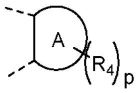

is selected from

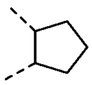, 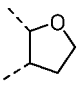, 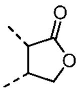, 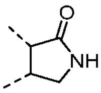, 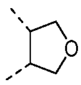, 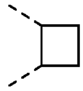, 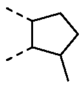, 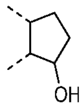, 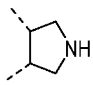,

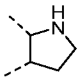, 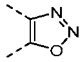, 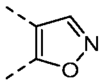, 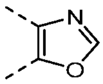, 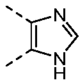, 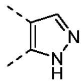, 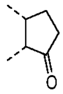, 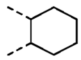, 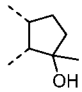, 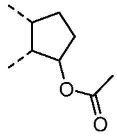,

and

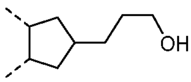,

and other variables are as defined in the present disclosure.

In some embodiments of the present disclosure, the ring B is selected from phenyl, 5-to 6-membered heteroaryl, $C_{3-6}$ cycloalkyl, 5- to 6-membered heterocyclyl, benzo-5- to 6-membered heterocyclyl, and 5- to 9-membered bicycloalkyl, and other variables are as defined in the present disclosure.

In some embodiments of the present disclosure, the ring B is selected from phenyl, pyridyl, $C_{3-6}$ cycloalkyl, 5- to 6-membered heterocycloalkyl, benzo-5- to 6-membered heterocycloalkyl, and 5- to 9-membered bicycloalkyl, and other variables are as defined in the present disclosure.

In some embodiments of the present disclosure, the ring B is selected from phenyl, pyridyl, cyclohexyl, tetrahydro-2*H*-pyranyl, benzo[*d*][1,3]dioxazolyl, and bicyclo[1.1.1]pentyl, and other variables are as defined in the present disclosure.

In some embodiments of the present disclosure, the structural moiety

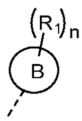

is selected from

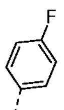, 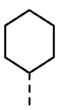, 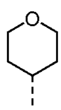, 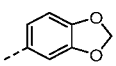, 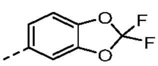, and 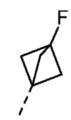,

and other variables are as defined in the present disclosure.

In some embodiments of the present disclosure, the ring D is selected from phenyl, 5-to 6-membered heteroaryl, $C_{3-6}$ cycloalkyl, and 6- to 10-membered heterocyclyl, and other variables are as defined in the present disclosure.

In some embodiments of the present disclosure, the ring D is selected from piperazinyl, 2,6-diazaspiro[3.3]heptyl, 2,7-diazaspiro[4.4]nonyl, 2,8-diazaspiro[4.5]decyl, 2,7-diazaspiro[3.5]nonyl, 2,5-diazabicyclo[2.2.1]heptyl, and octahydropyrrolo[3,4-c]pyrrolyl, and other variables are as defined in the present disclosure.

In some embodiments of the present disclosure, the ring D is selected from

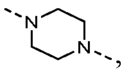,

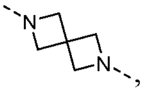, 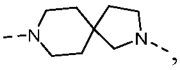, 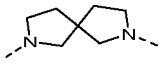, 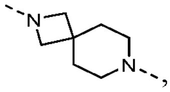, 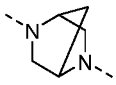, and 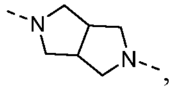,

and other variables are as defined in the present disclosure.

In some embodiments of the present disclosure, the $R_a$ is selected from -H, $C_{1-6}$ alkyl, NHOH, $-N(CH_3)_2$, $-NHCH_3$, 4- to 9-membered heterocyclyl, and 4- to 9-membered cycloalkyl, wherein the 4- to 9-membered heterocyclyl or 4- to 9-membered cycloalkyl is optionally substituted by 1, 2, or 3 OH, methyl, ethyl, or halogens, and other variables are as defined in the present disclosure.

In some embodiments of the present disclosure, the $R_6$ is selected from $-C_{1-3}$ alkyl-C(=O)-3- to 9-membered heterocyclyl, $-C_{1-3}$ alkyl-C(=O)-3- to 9-membered heterocyclyl-$C_{1-6}$ alkyl, -C(=O)-3- to 9-membered heterocyclyl, -C(=O)-$C_{3-9}$ cycloalkyl, -C(=O)-$C_{1-6}$ alkyl, $-C_{1-3}$ alkyl-C(=O)-NH-$C_{1-6}$ alkyl, $-S(=O)_2$-$C_{1-3}$ alkyl, and $-C_{1-3}$ alkyl-C(=O)NH-OH, wherein the - $C_{1-3}$ alkyl-C(=O)-3- to 9-membered heterocyclyl, $-C_{1-3}$ alkyl-C(=O)-3- to 9-membered heterocyclyl-$C_{1-6}$ alkyl, -C(=O)-5- to 6-membered heterocyclyl, -C(=O)-$C_{3-9}$ cycloalkyl, - C(=O)-$C_{1-6}$ alkyl, $-C_{1-3}$ alkyl-C(=O)-NH-$C_{1-6}$ alkyl, $-S(=O)_2$-$C_{1-3}$ alkyl, or $-C_{1-3}$ alkyl-C(=O)NH-OH is optionally substituted by 1, 2, or 3 OH, amino, methyl, ethyl, hydroxymethyl, trifluoromethyl, methoxy, or halogens, and other variables are as defined in the present disclosure.

In some embodiments of the present disclosure, the $R_6$ is selected from

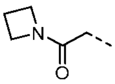,

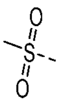, 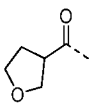, 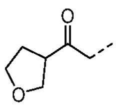, 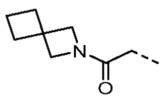, 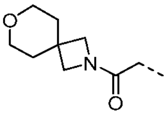, 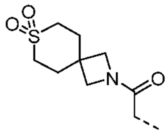, 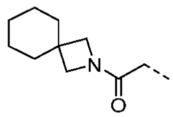, 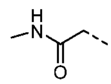,

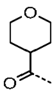, 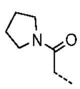, 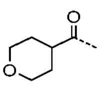, 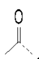, 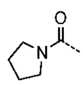, 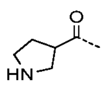, 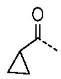, 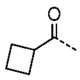, 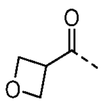, 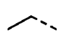, 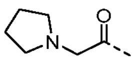, 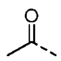,

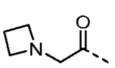, 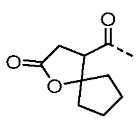, 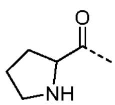, 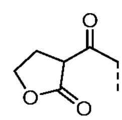, 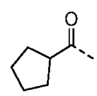, 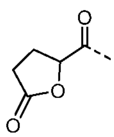, and 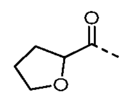,

wherein

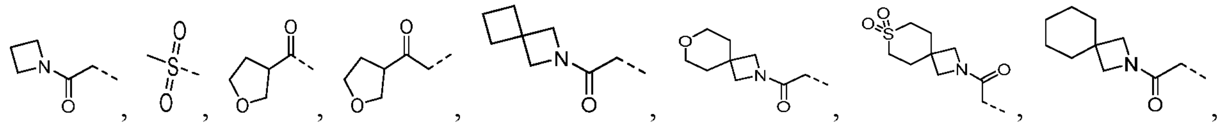,

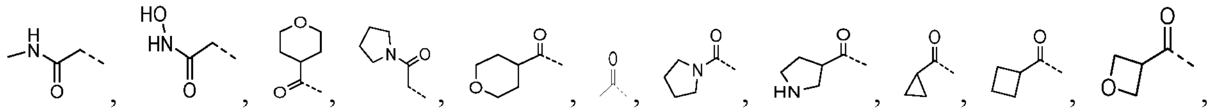,

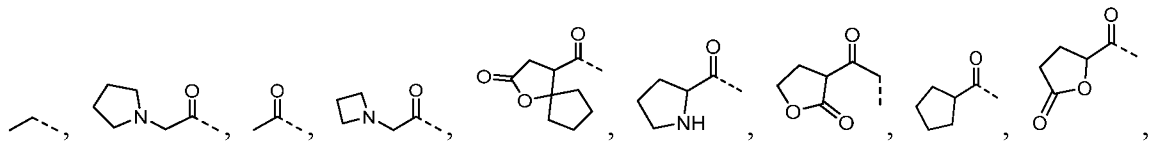,

and

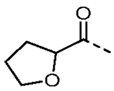

are optionally substituted by 1, 2, or 3 OH, methyl, ethyl, hydroxymethyl, or halogens, and other variables are as defined in the present disclosure.

In some embodiments of the present disclosure, the $R_6$ is selected from

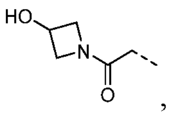,

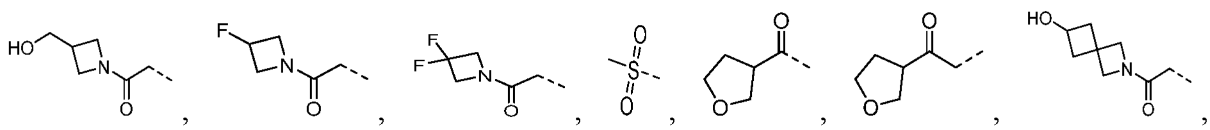,

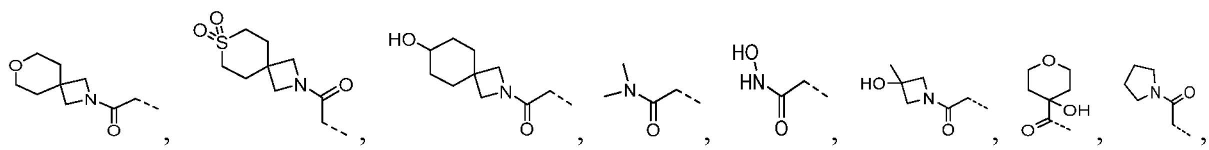,

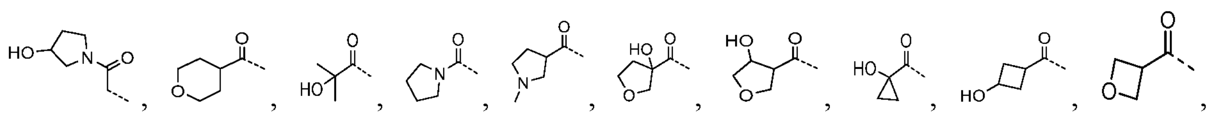,

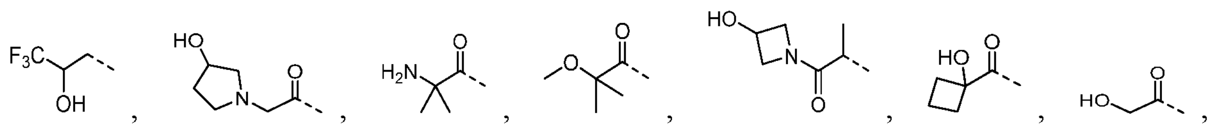,

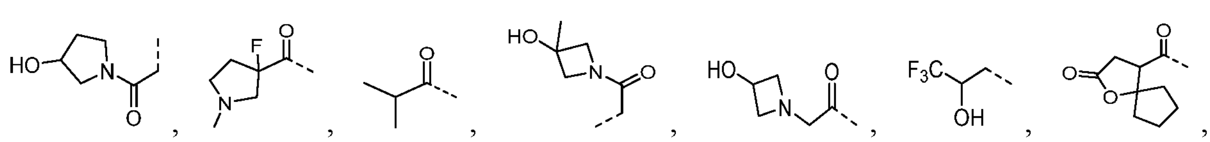,

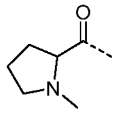, 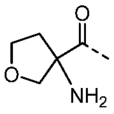, 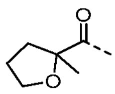, 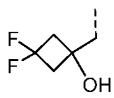, 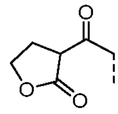, 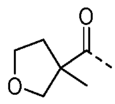, 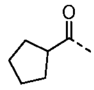, 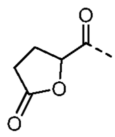,

and

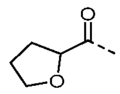,

and other variables are as defined in the present disclosure.

In some embodiments of the present disclosure, the structural moiety

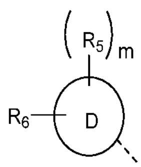

is selected from

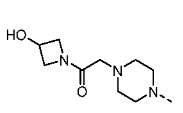, 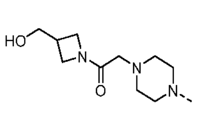, 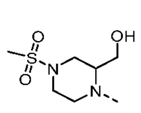, 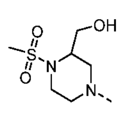, 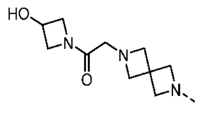,

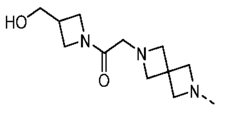, 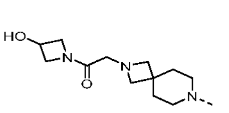, 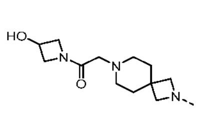, 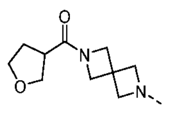, 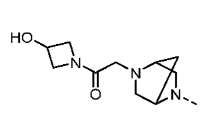,

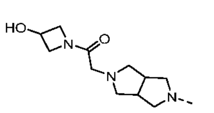, 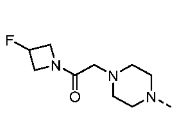, 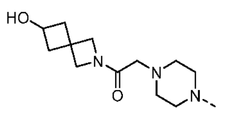, 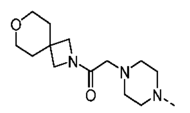, 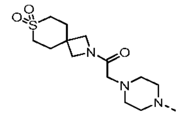,

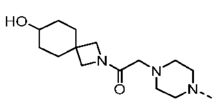, 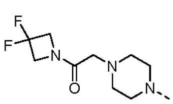, 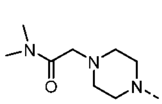, 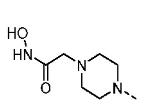, 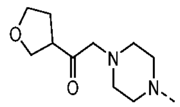, 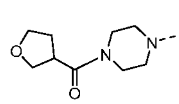,

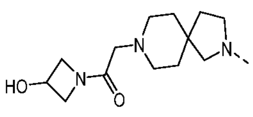, 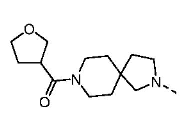, 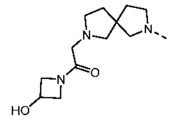, 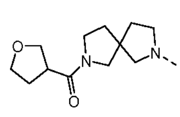, 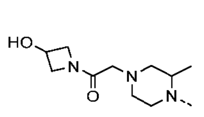,

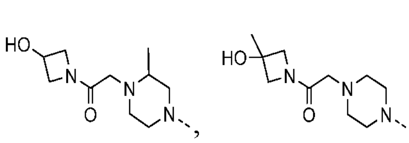, 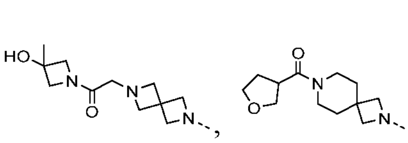, 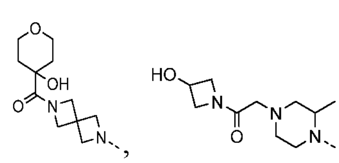,

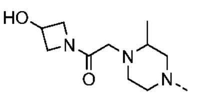, 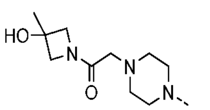, 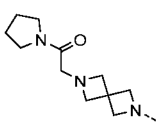, 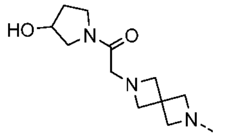, 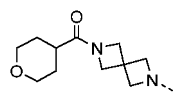,

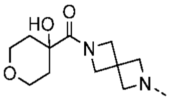, 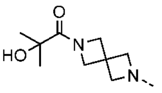, 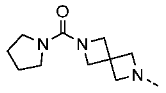, 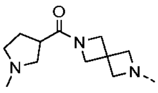, 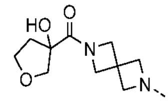, 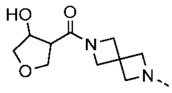,

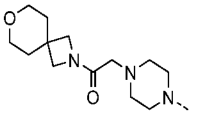, 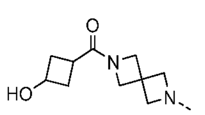, 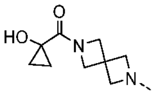, 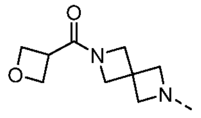, 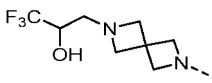,

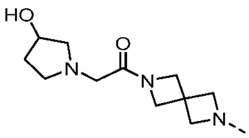, 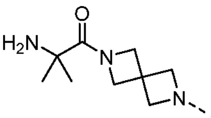, 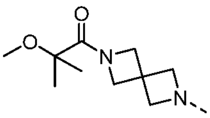, 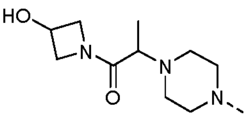, 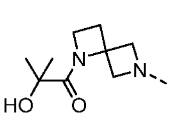,

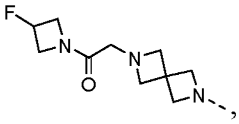, 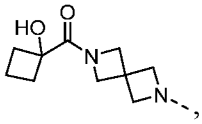, 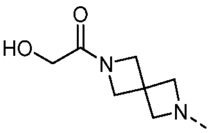, 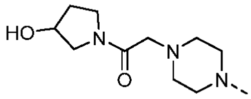, 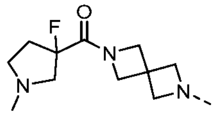,

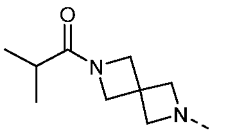, 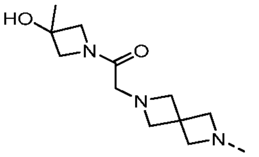, 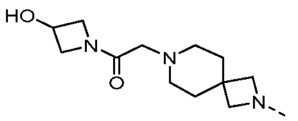, 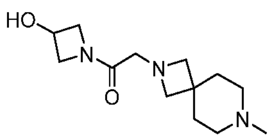,

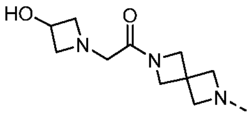, 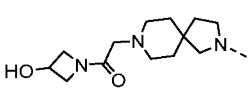, 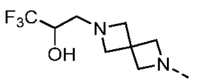, 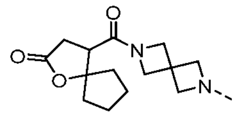,

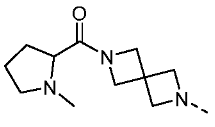, 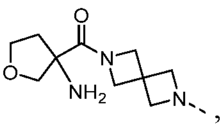, 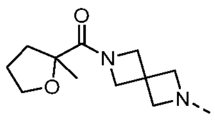, 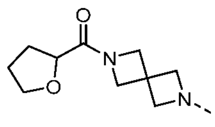, 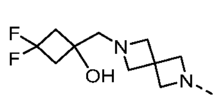,

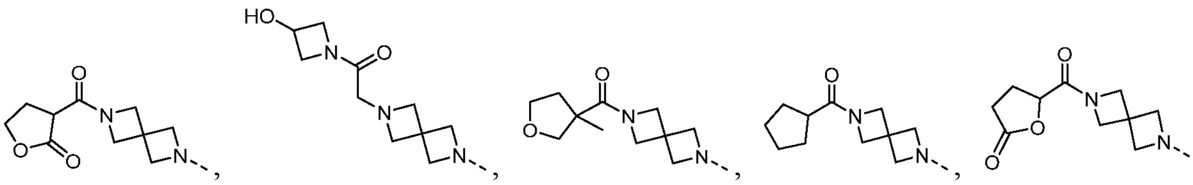, , , , ,

and

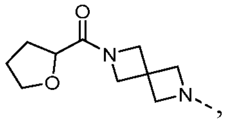,

and other variables are as defined in the present disclosure.

In some embodiments of the present disclosure, the $R_3$ is selected from H, $C_{1-6}$ alkyl, $C_{3-6}$ cycloalkyl, and 4- to 6-membered heterocyclyl, wherein the $C_{1-6}$ alkyl, $C_{3-6}$ cycloalkyl, or 4- to 6-membered heterocyclyl is optionally substituted by 1, 2, or 3 halogens, cyano, amino, or $C_{1-6}$ alkyl, and other variables are as defined in the present disclosure.

In some embodiments of the present disclosure, the $R_3$ is selected from H, methyl, ethyl,

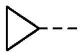, 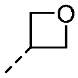, 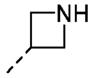, 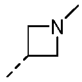, and 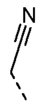,

and other variables are as defined in the present disclosure.

In some embodiments of the present disclosure, the ring C is selected from 5- to 6-membered heteroaryl, wherein the heteroaryl comprises 1 to 3 heteroatoms selected from N atom, O atom, or S atom, and other variables are as defined in the present disclosure.

In some embodiments of the present disclosure, the $R_{7a}$, $R_{7b}$, $R_{7c}$, and $R_{7d}$ are each independently selected from H, halogen, $C_{1-6}$ alkyl, $C_{1-6}$ alkoxy, cyano, amino, nitro, OH, $C_{3-6}$ cycloalkyl, 5- to 6-membered heterocyclyl, phenyl, and 5- to 6-membered heteroaryl, wherein the $C_{1-6}$ alkyl is optionally substituted by 1, 2, or 3 halogens, and other variables are as defined in the present disclosure.

In some embodiments of the present disclosure, the structural moiety

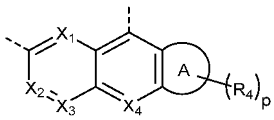

is selected from

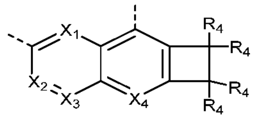, 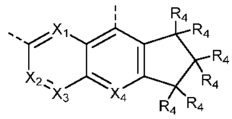,

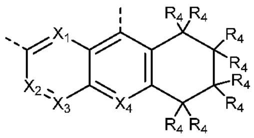, 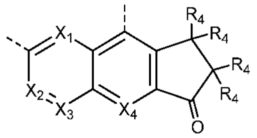, 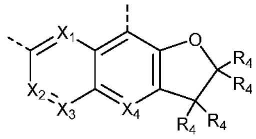, 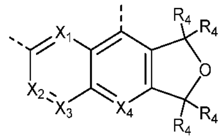,

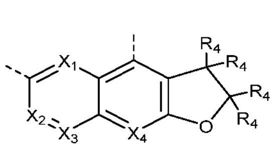, 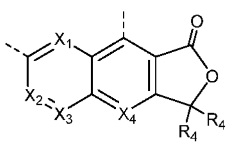, 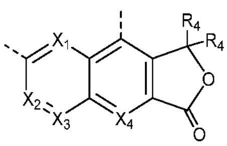, 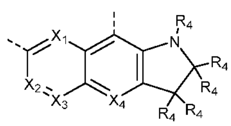,

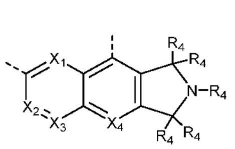, 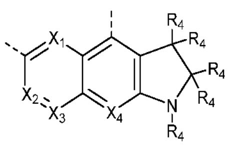, 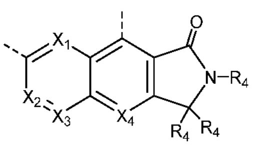, 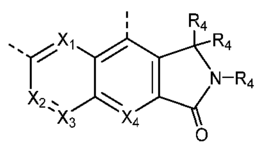,

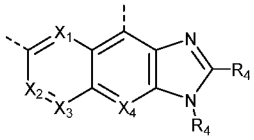, 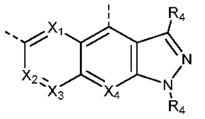, 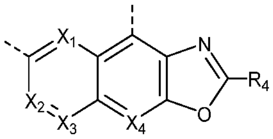, 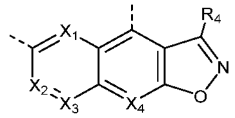,

and

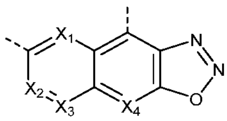,

and other variables are as defined in the present disclosure.

In some embodiments of the present disclosure, the structural moiety

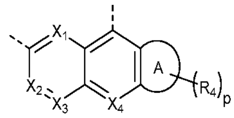

is selected from

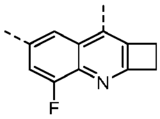, 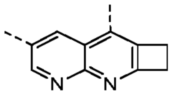, 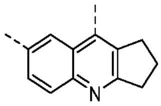, 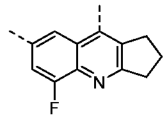,

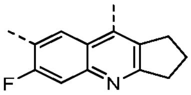, 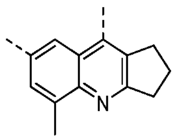, 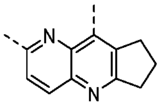, 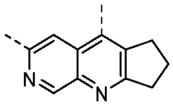, 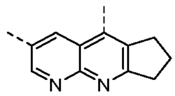, 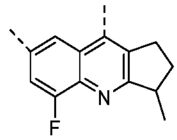,

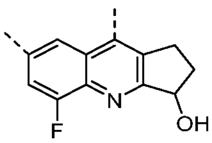, 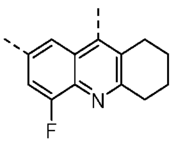, 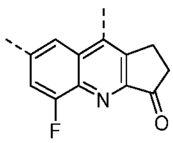, 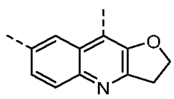, 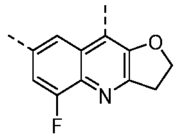, 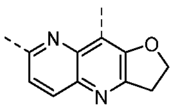,

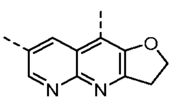, 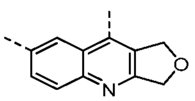, 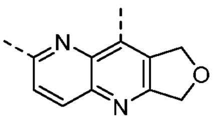, 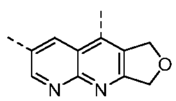, 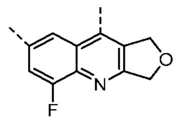,

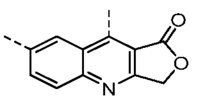, 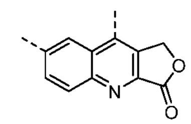, 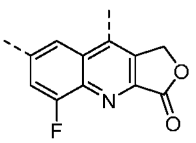, 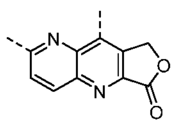, 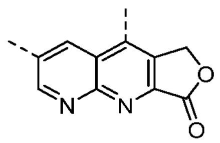,

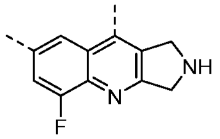, 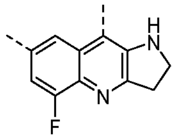, 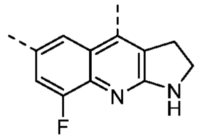, 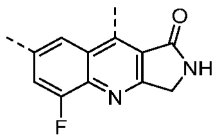, 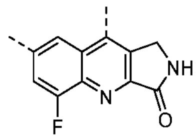,

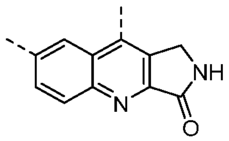, 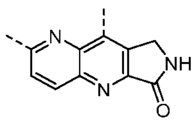, 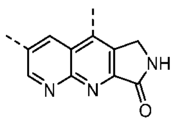, 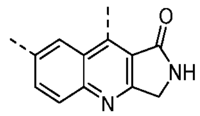, 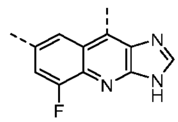,

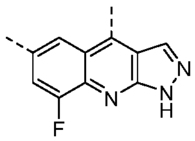, 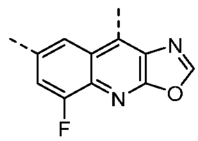, 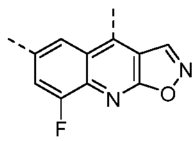, 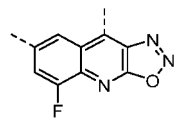, 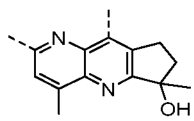,

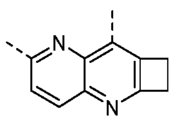, 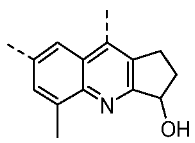, 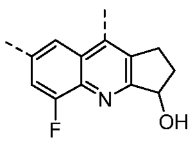, 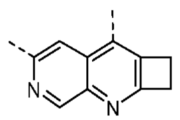, 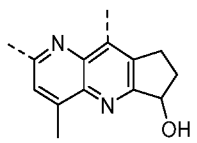,

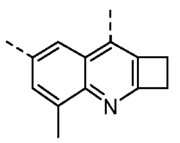, 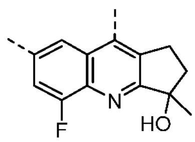, 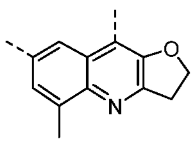, 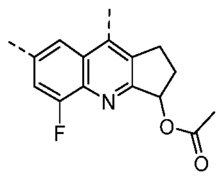, 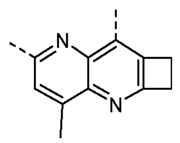,

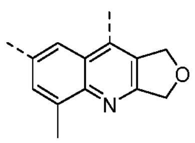, 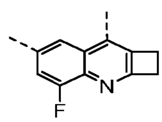, 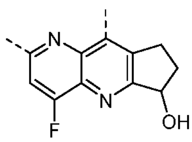, 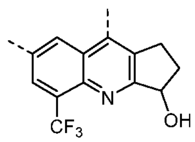, 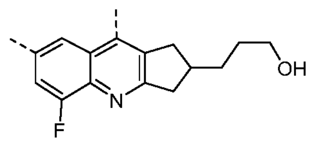,

and

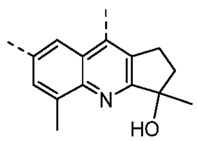,

and other variables are as defined in the present disclosure.

In yet another aspect of the present disclosure, the present disclosure also provides a compound of the following formulas, an optical isomer thereof, or a pharmaceutically acceptable salt thereof, selected from:

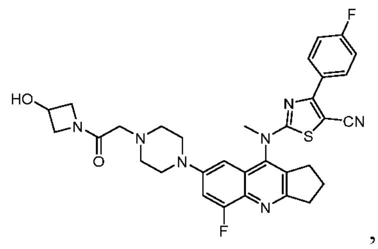, 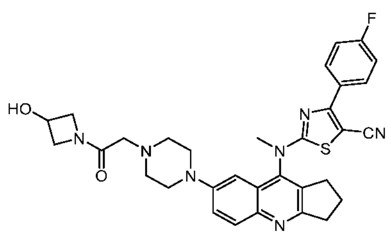, 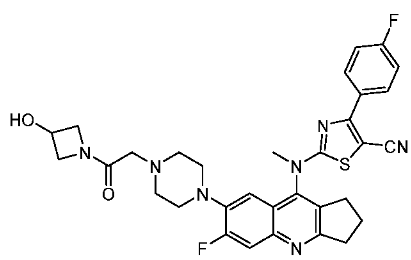,

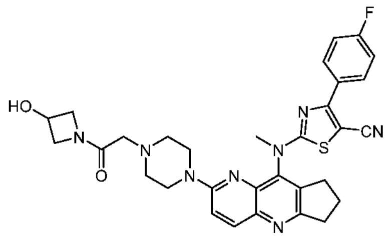,
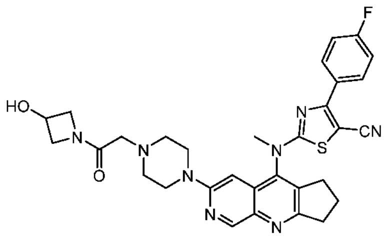,
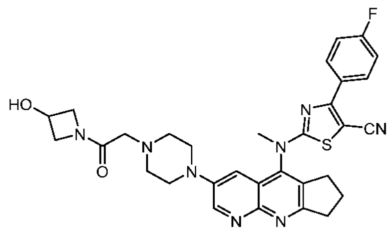,

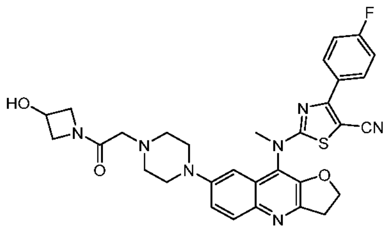,
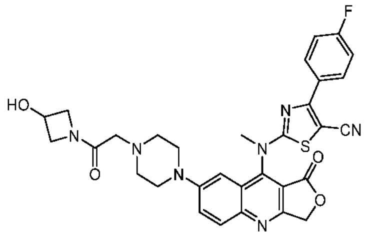,
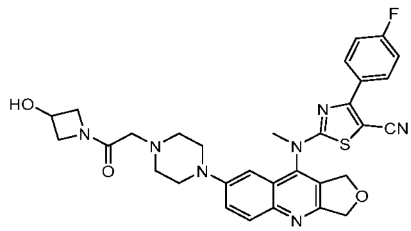,

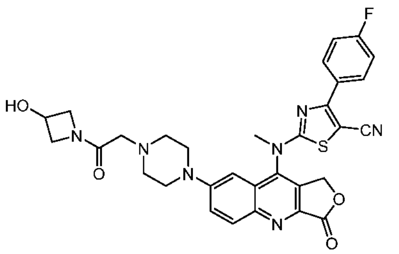,
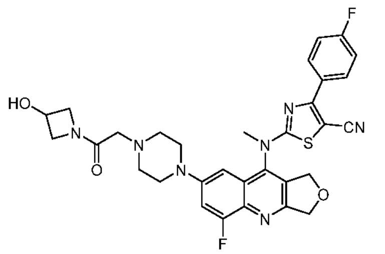,
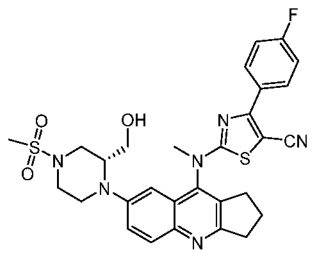,

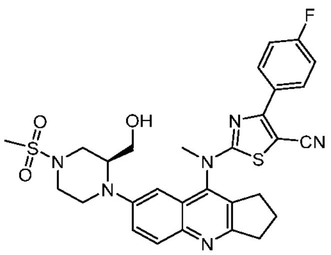,
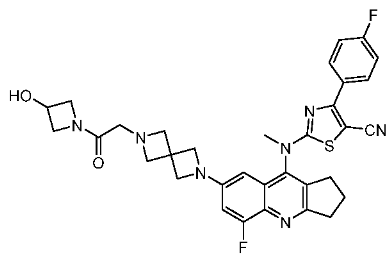,
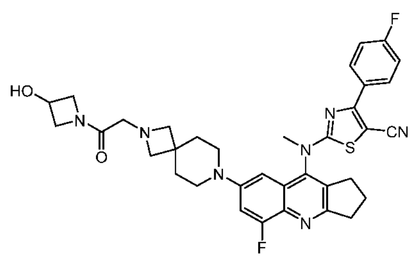,

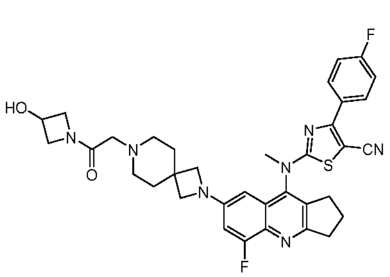,
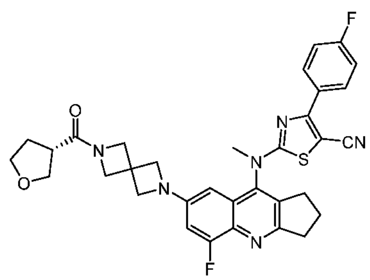,
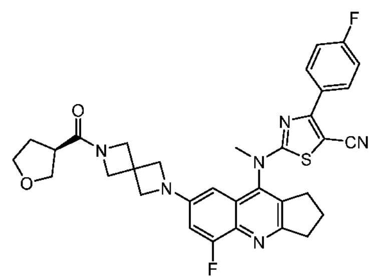,

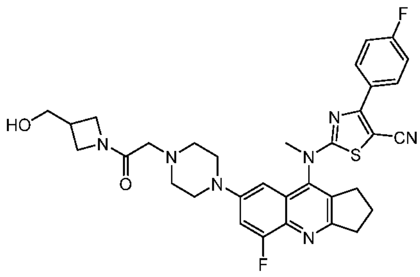
,
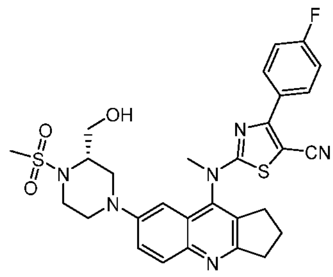
,
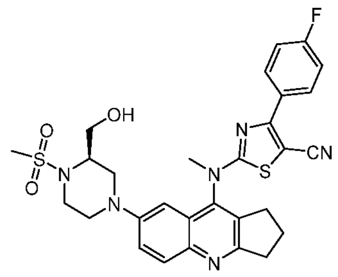
,
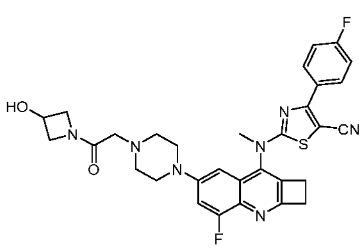
,
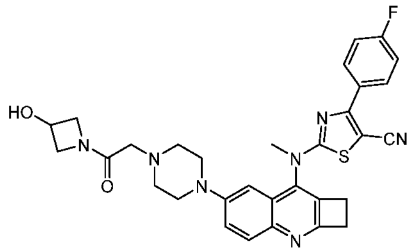
,
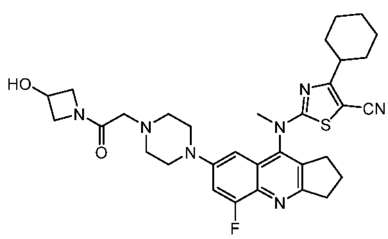
,
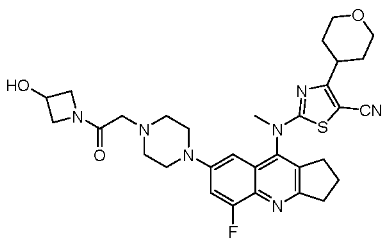
,
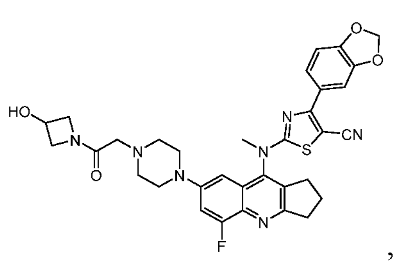
,
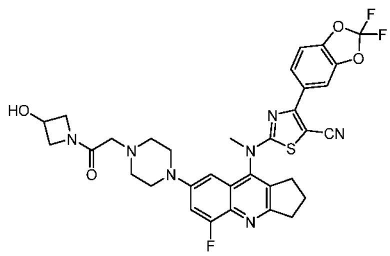
,
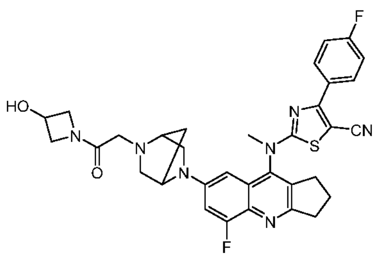
,
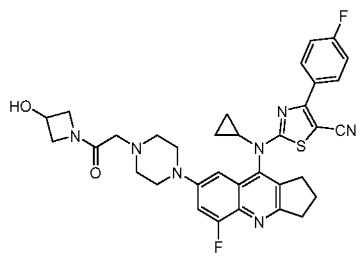
,
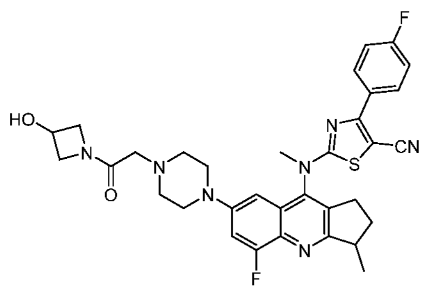
,
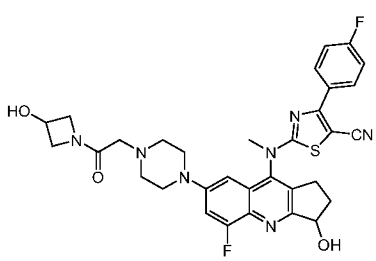
,
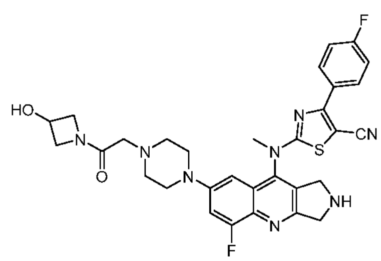
,
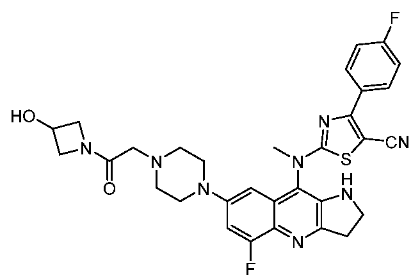
,

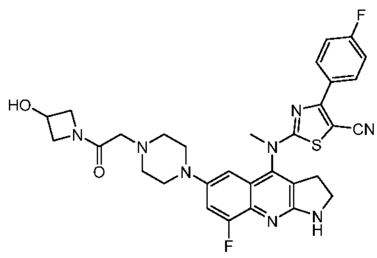 , 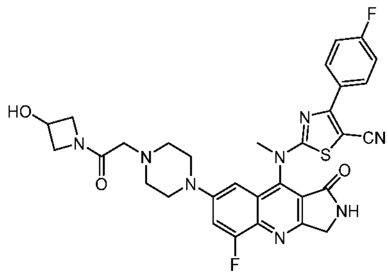 , 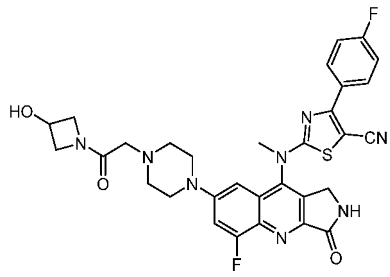 ,

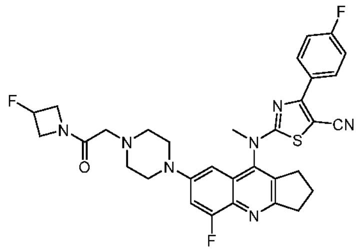 , 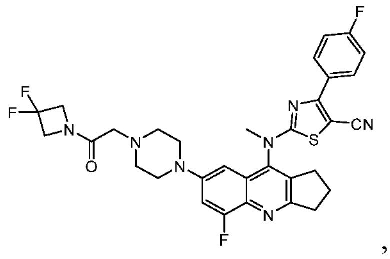 , 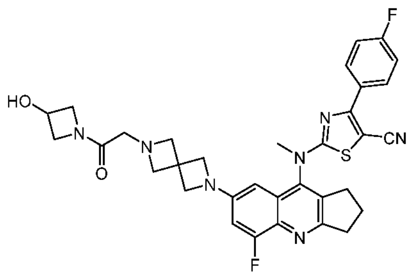 ,

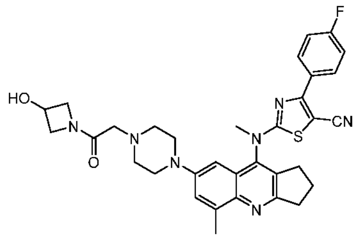 , 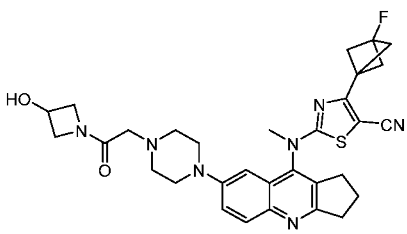 , 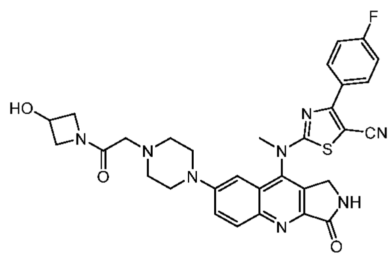 ,

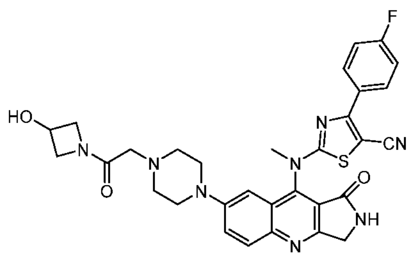 , 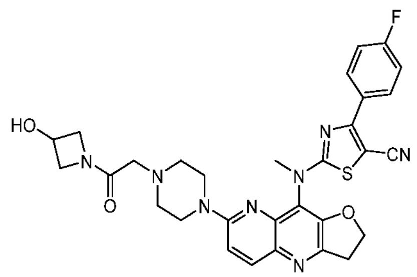 , 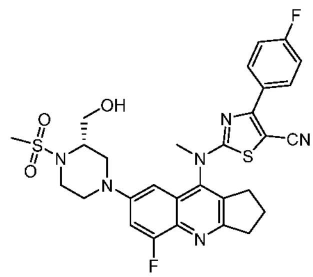 ,

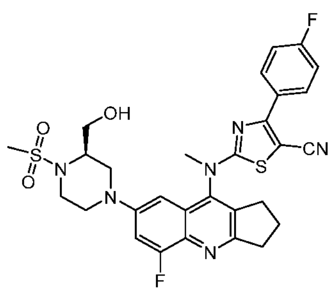 , 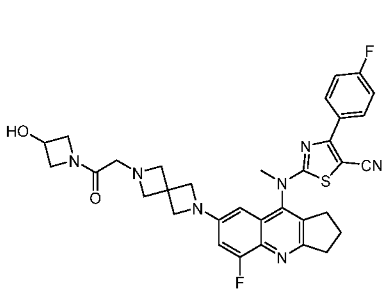 , 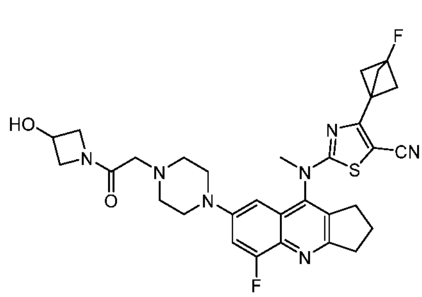 ,

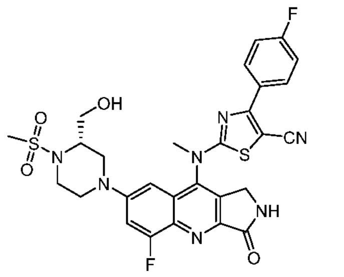 , 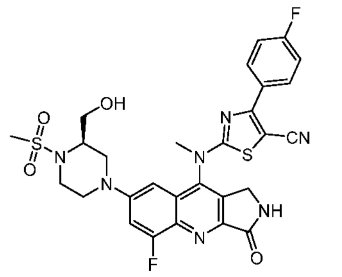 , 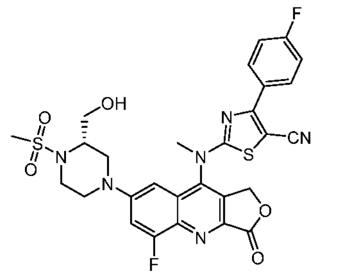 ,

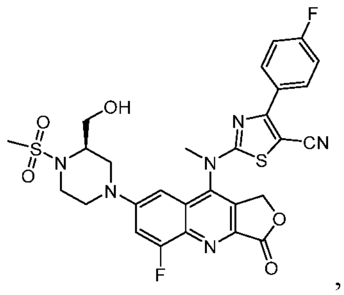 , 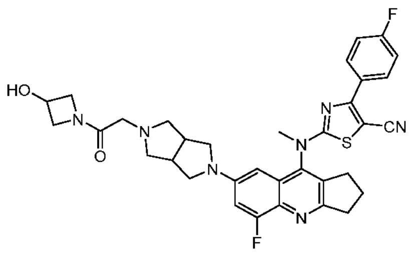 , 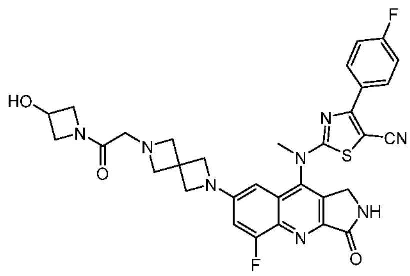 ,

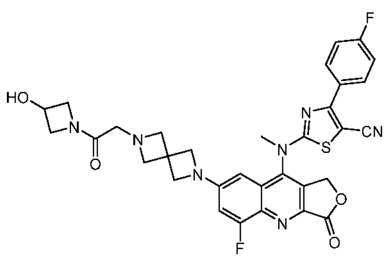 , 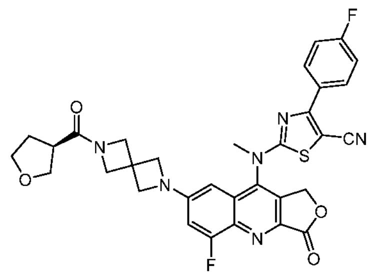 , 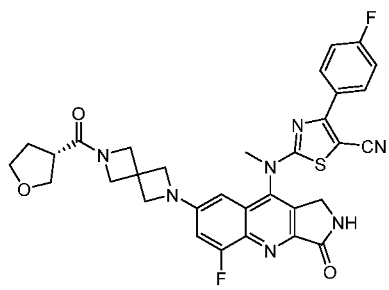 ,

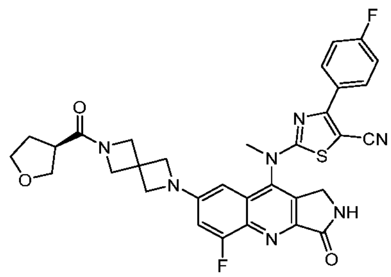 , 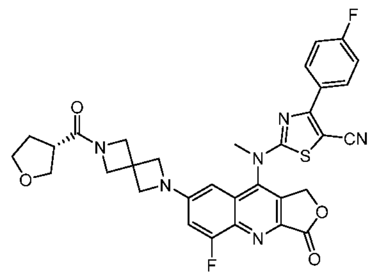 , 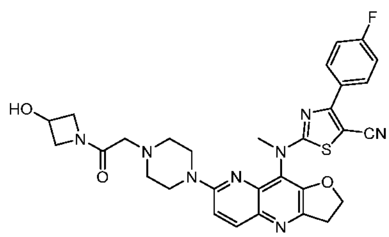 ,

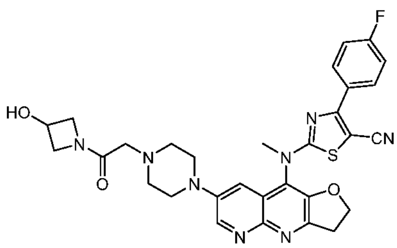 , 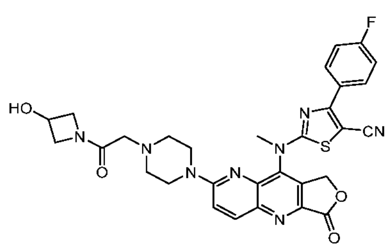 , 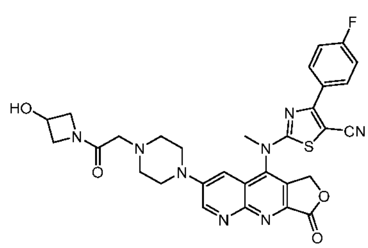 ,

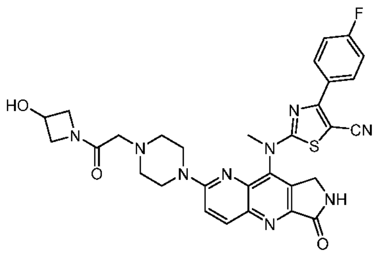
,
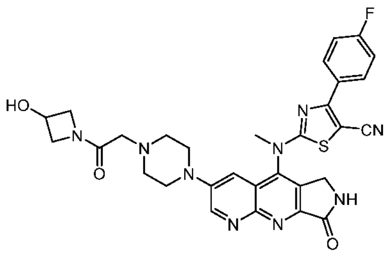
,
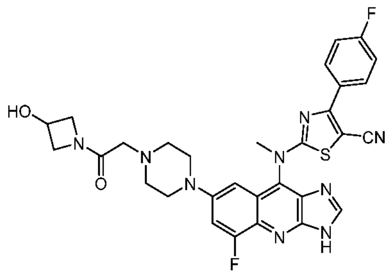
,

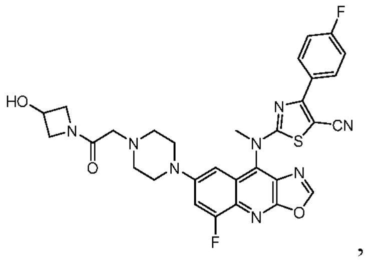
,
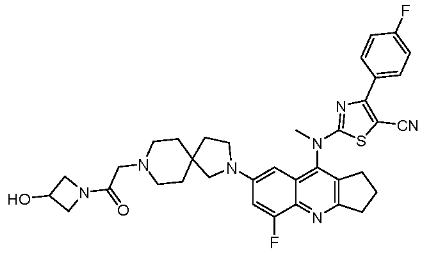
,
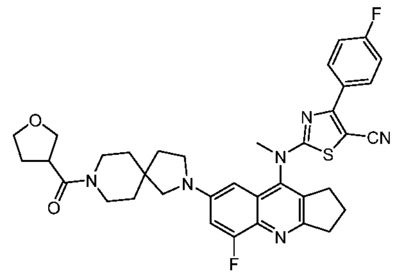
,

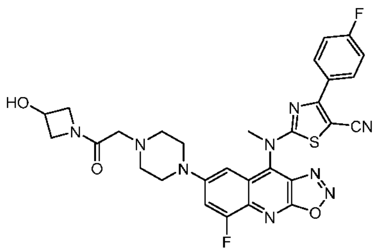
,
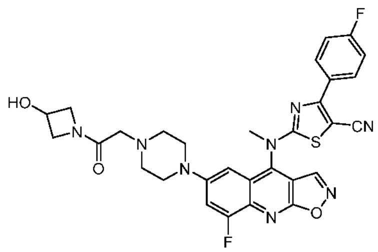
,
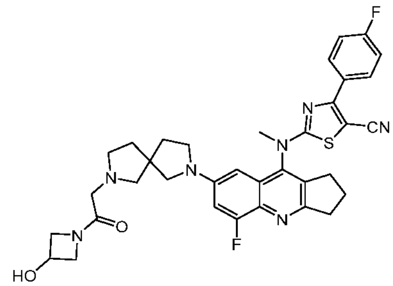
,

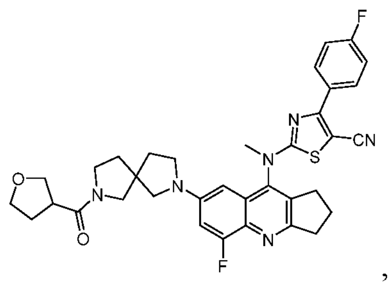
,
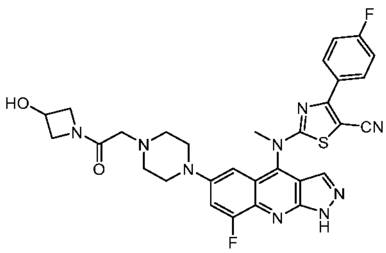
,
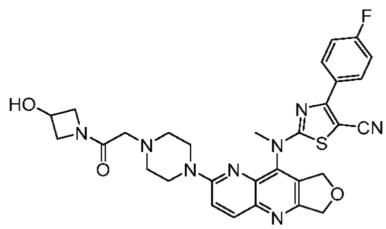
,

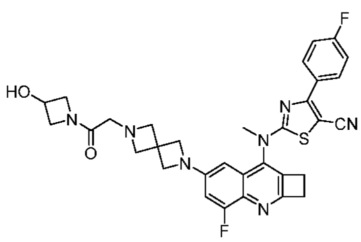
,
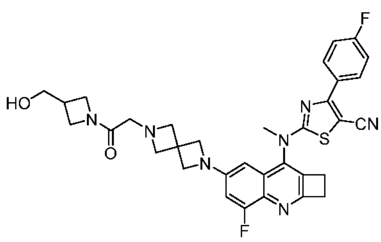
,
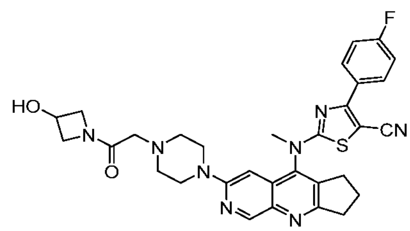
,

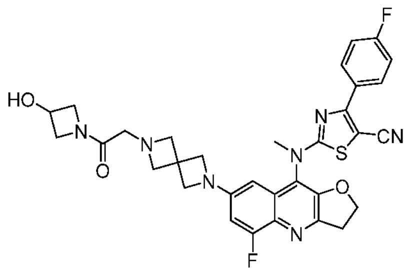 , 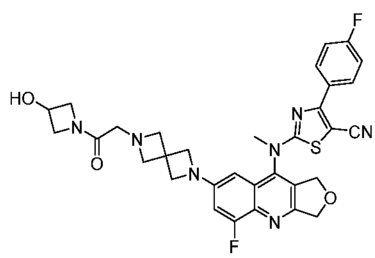 , 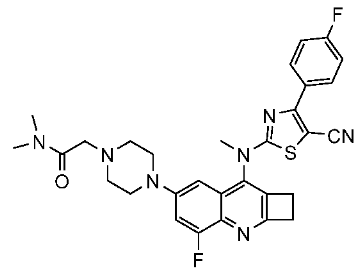 ,

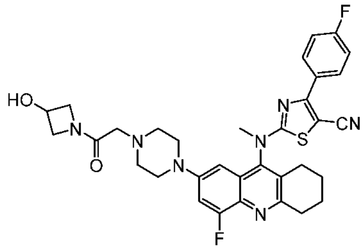 , 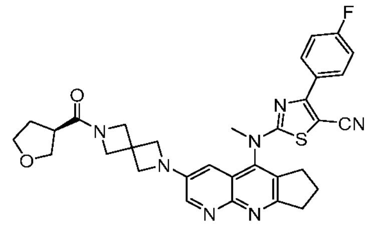 , 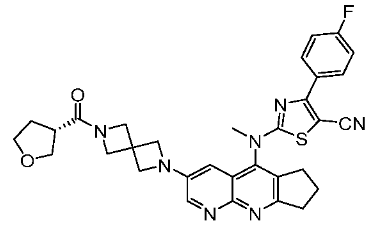 ,

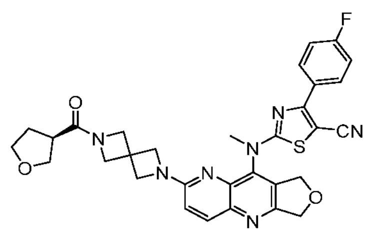 , 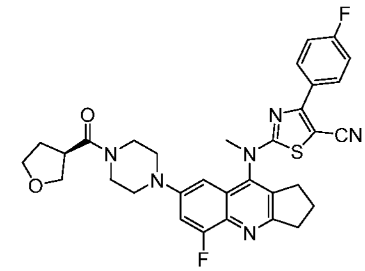 , 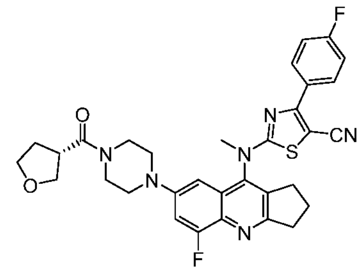 ,

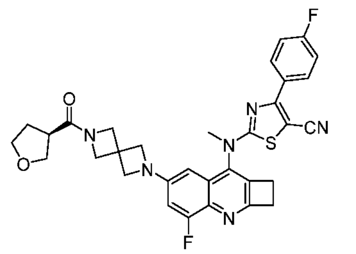 , 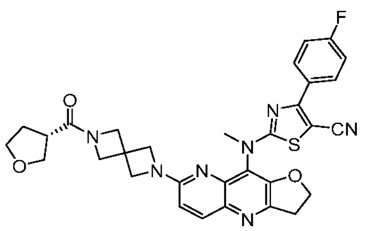 , 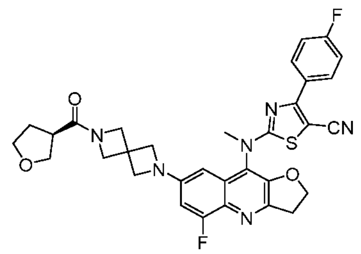 ,

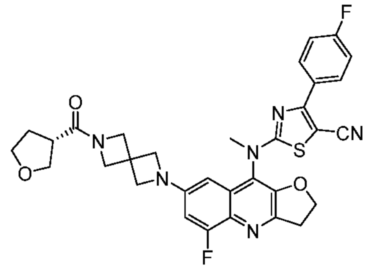 , 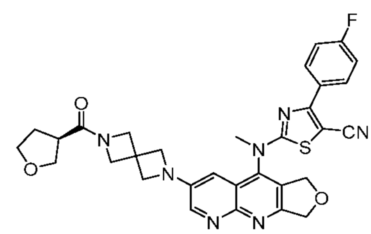 , 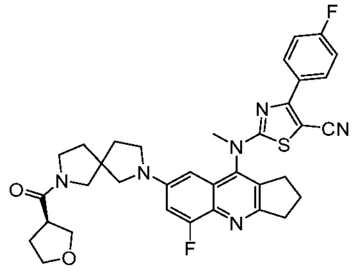 ,

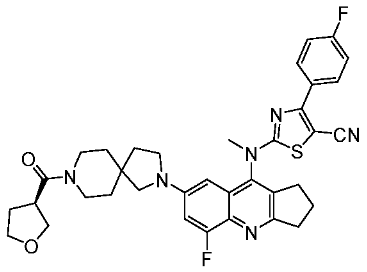
,
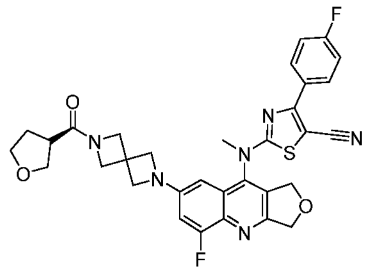
,
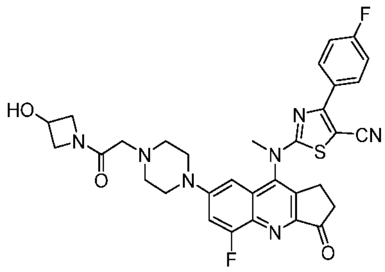
,
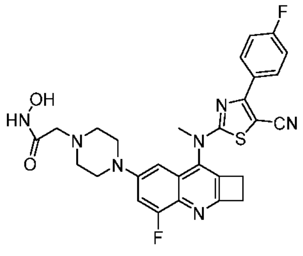
,
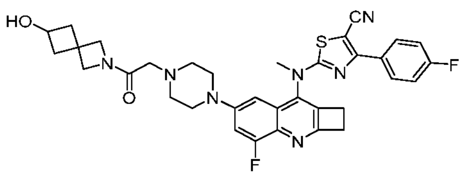
,
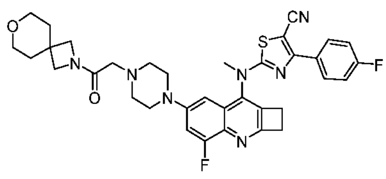
,
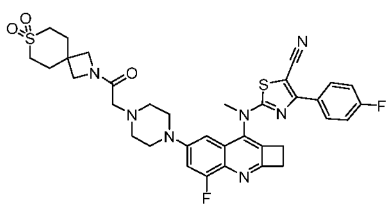
,
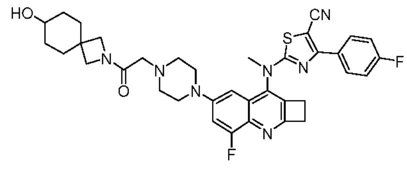
,
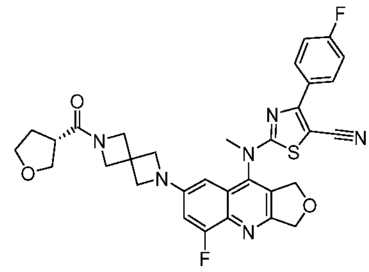
,
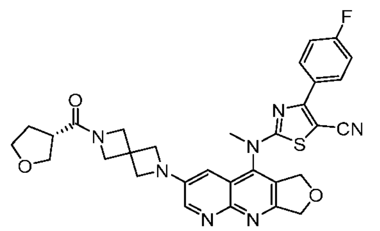
,
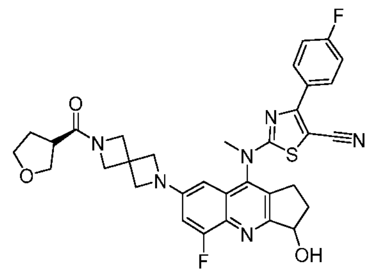
,
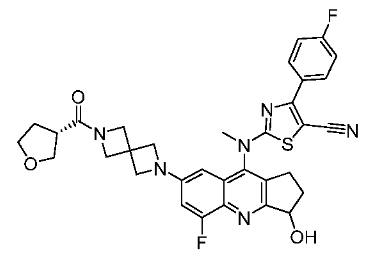
,
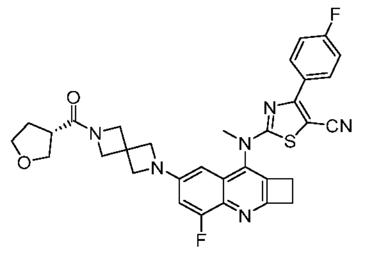
,
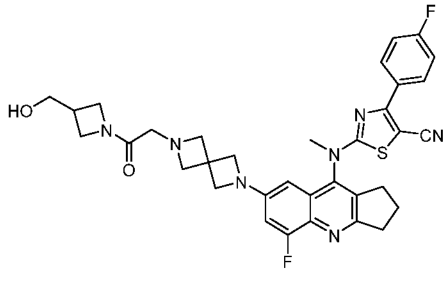
,

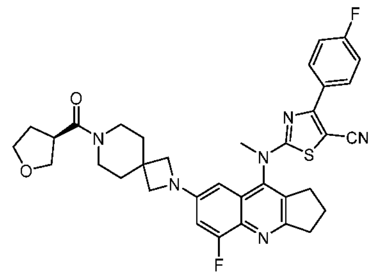,
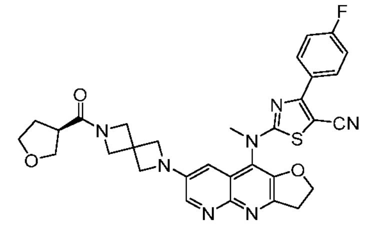,
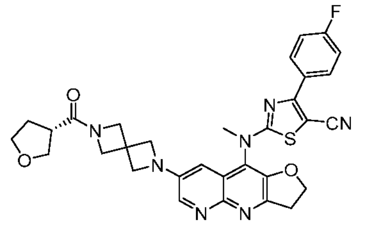,

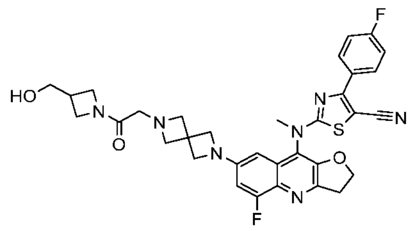,
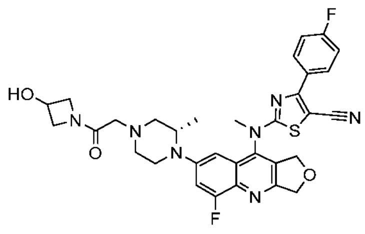,
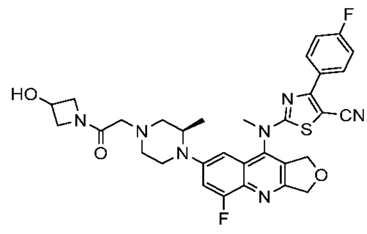,

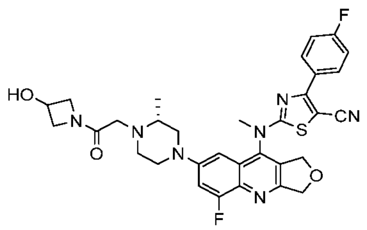,
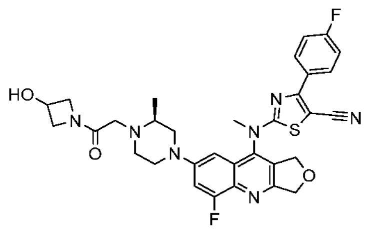,
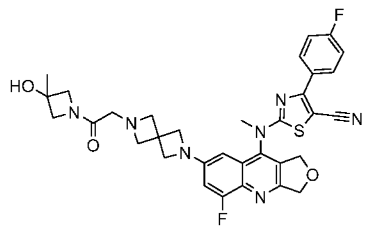,

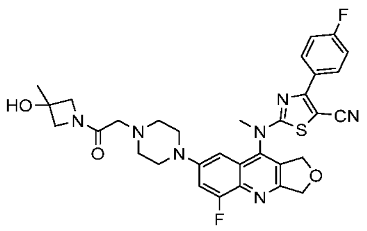,
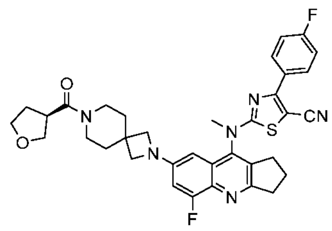,
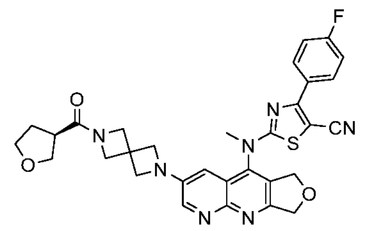,

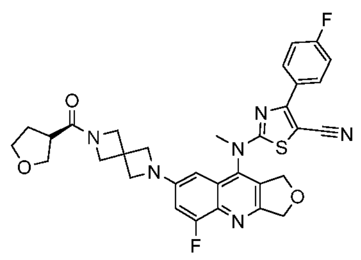,
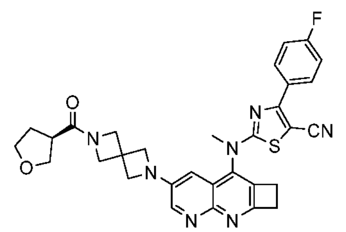,
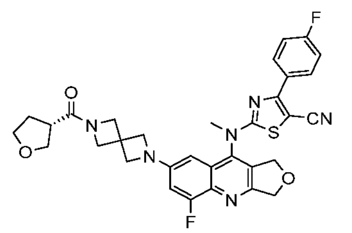,

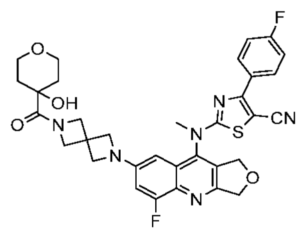,
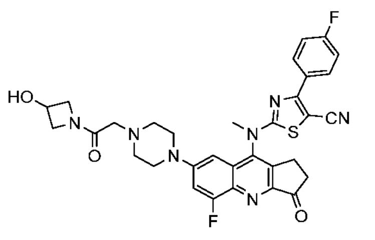,
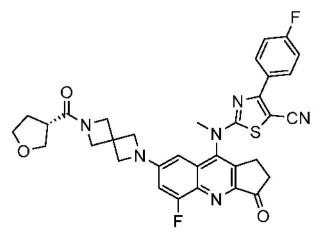,

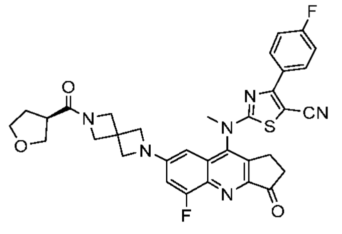,
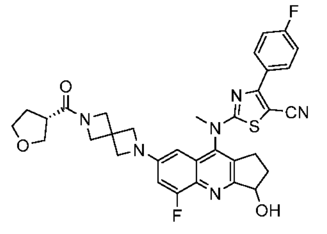,
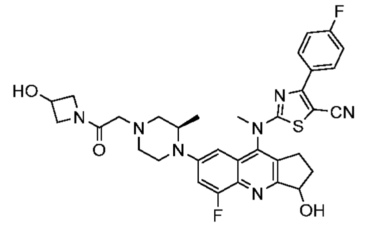,

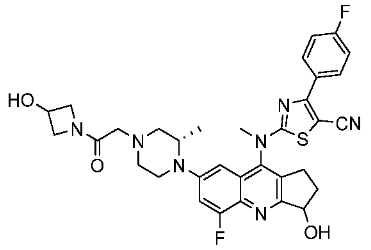,
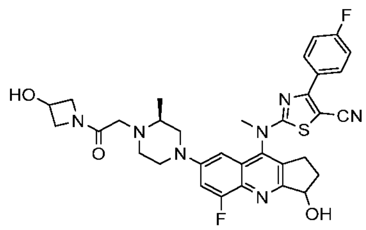,
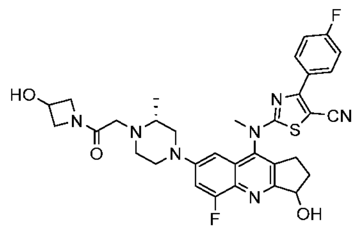,

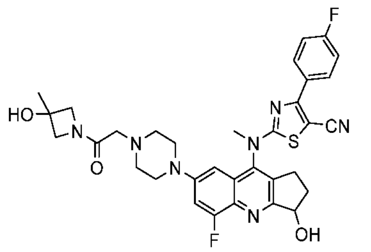,
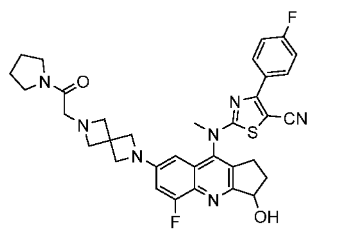,
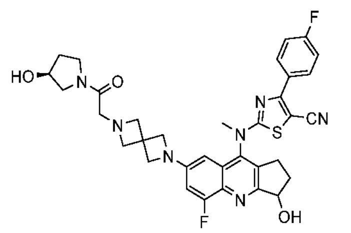,

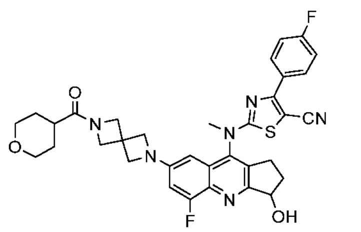,
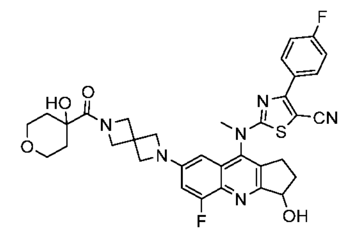,
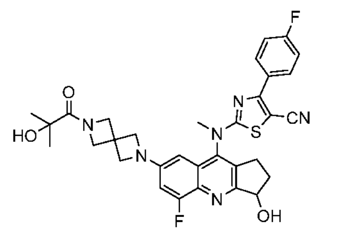,

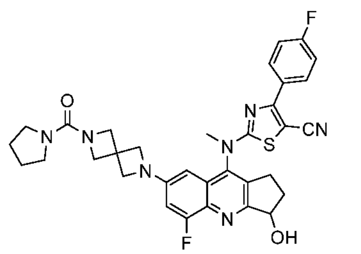,
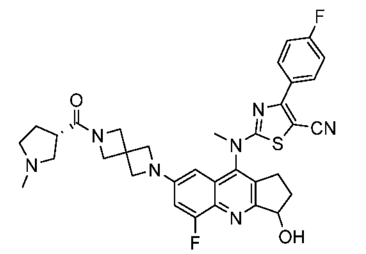,
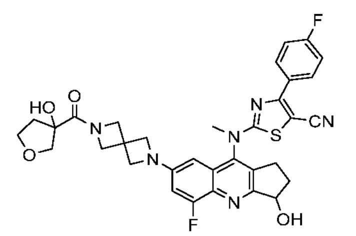,

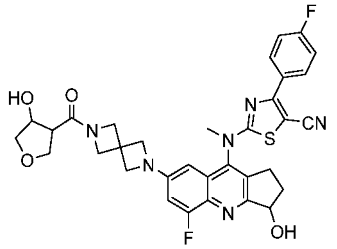,
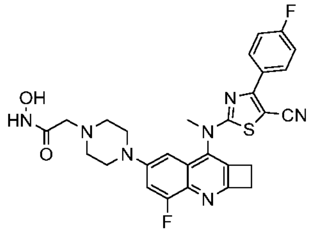,
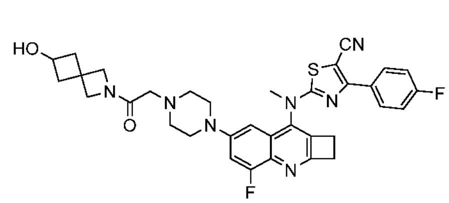,

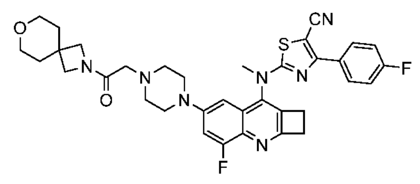 , 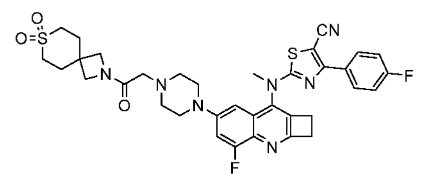 ,

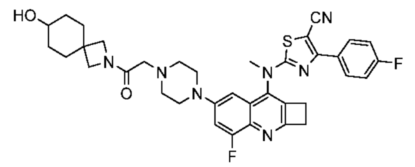 , 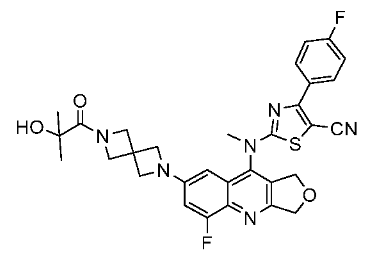 , 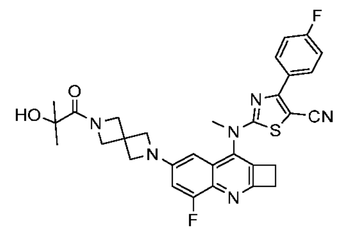 ,

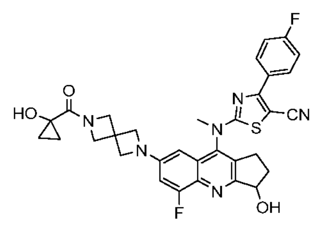 , 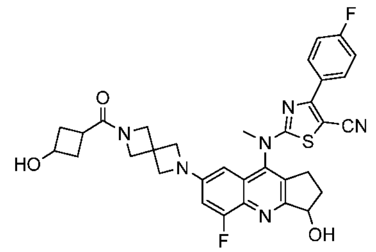 , 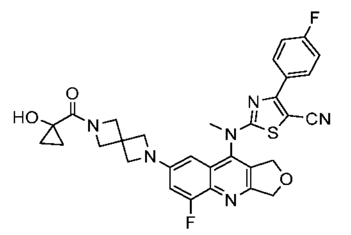 ,

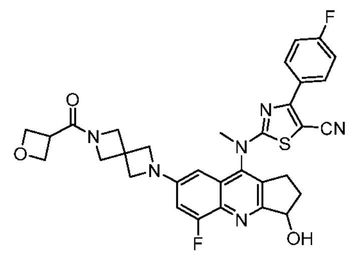 , 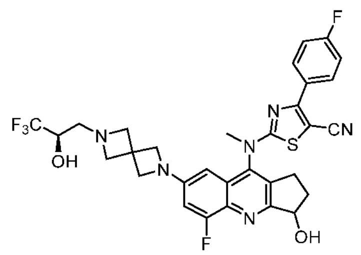 , 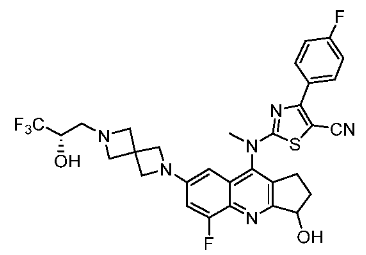 ,

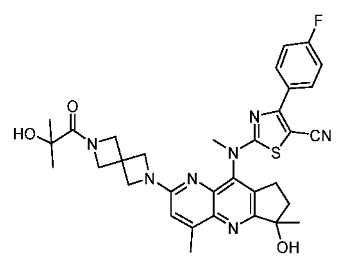 , 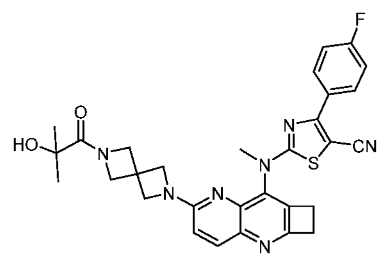 , 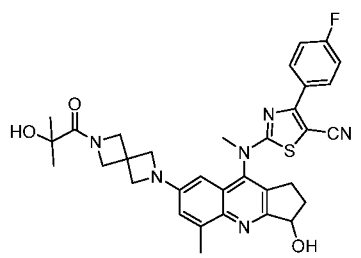 ,

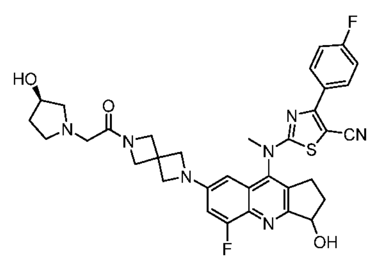 , 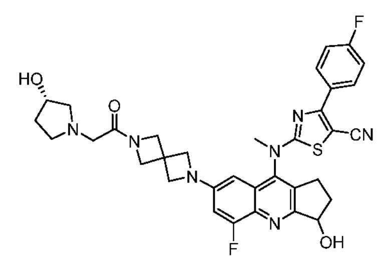 , 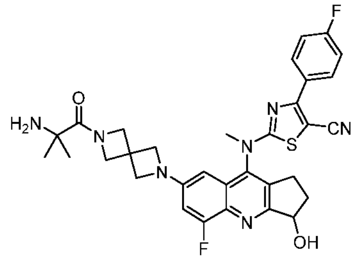 ,

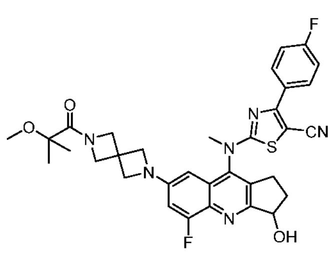 , 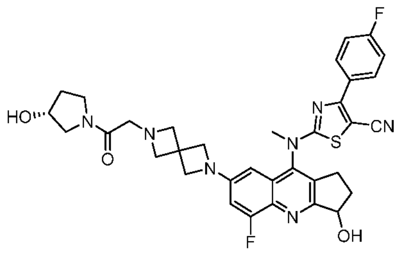 , 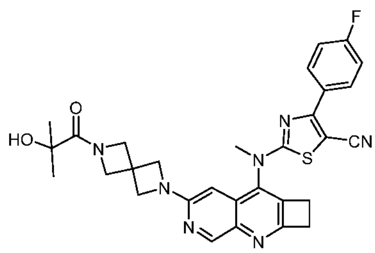 ,

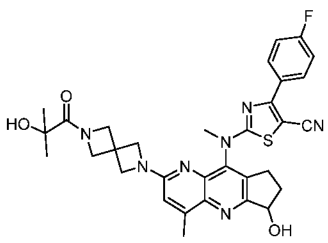 , 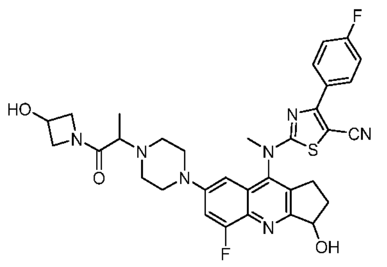 , 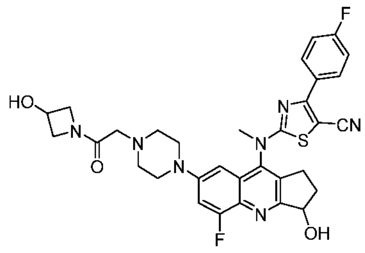 ,

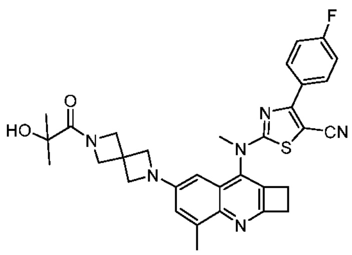 , 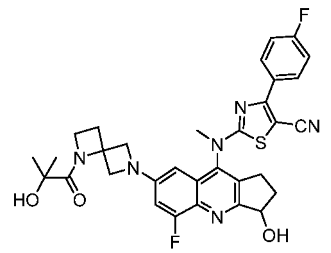 , 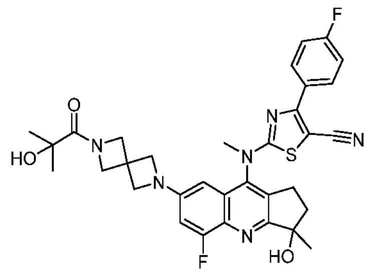 ,

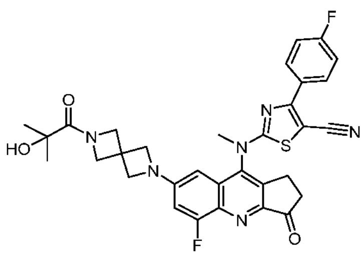 , 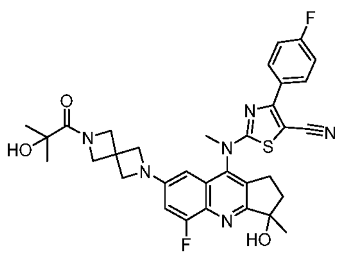 , 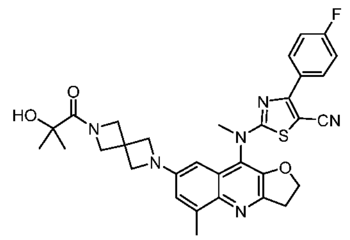 ,

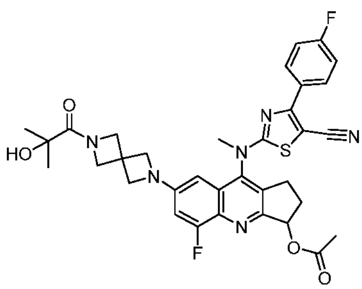 , 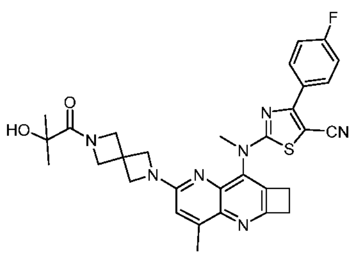 , 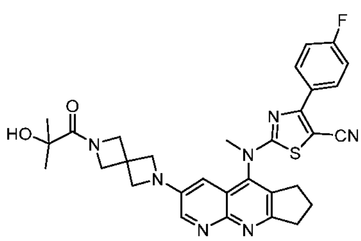 ,

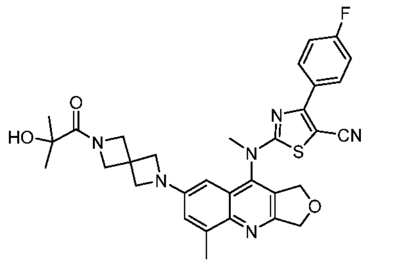

,

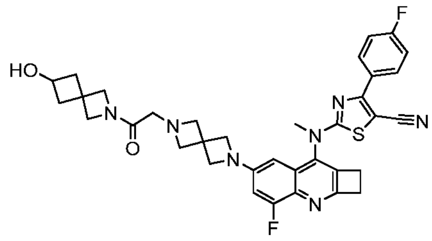

,

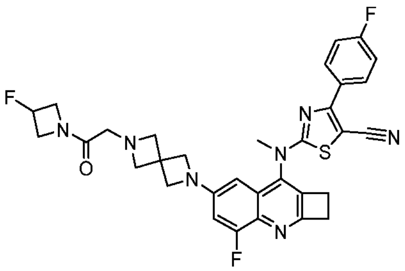

,

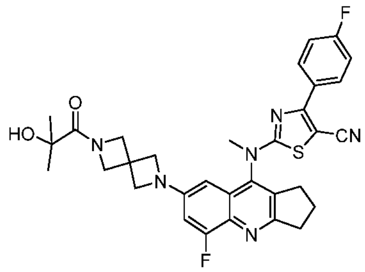

,

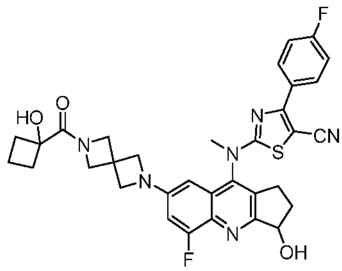

,

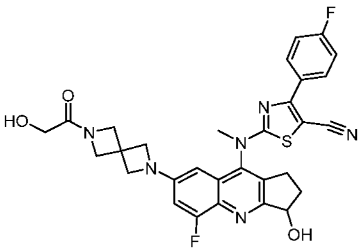

,

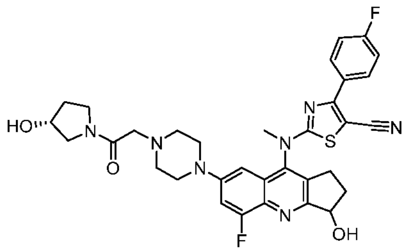

,

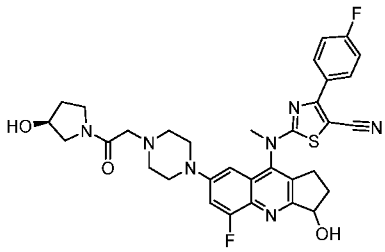

,

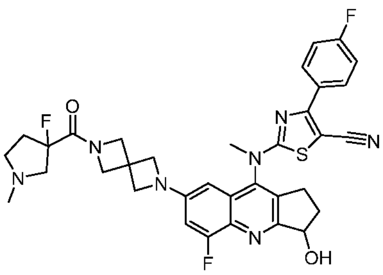

,

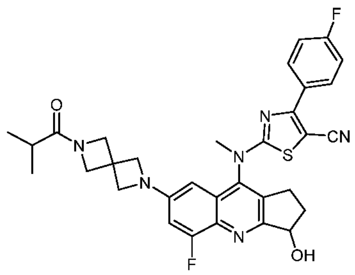

,

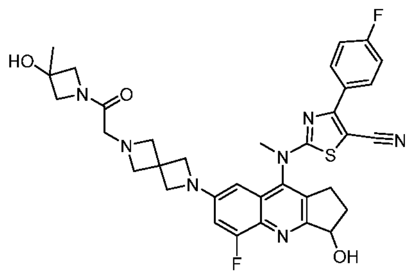

,

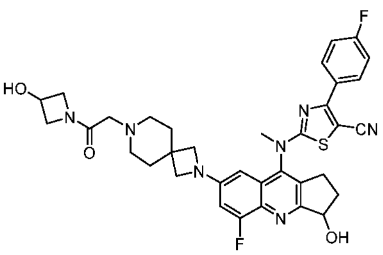

,

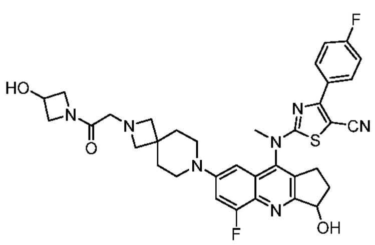

,

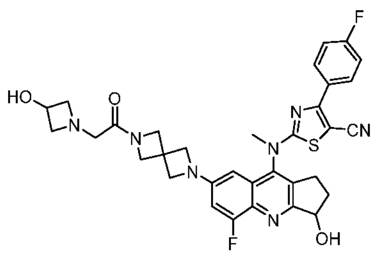

,

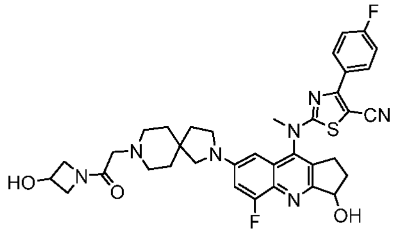,

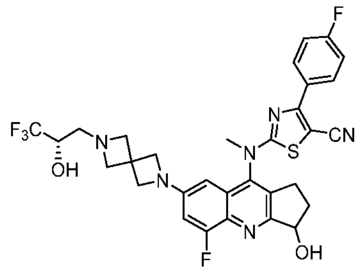,

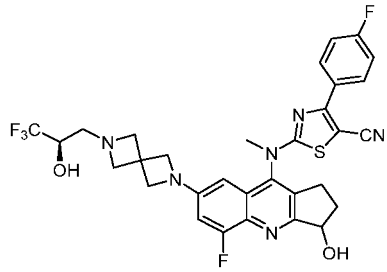,

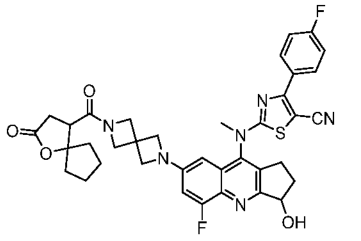,

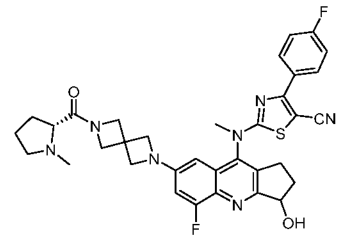,

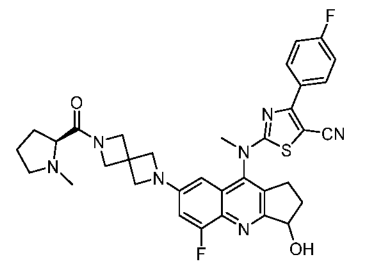,

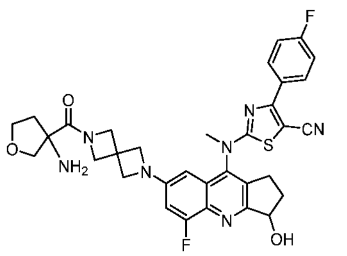,

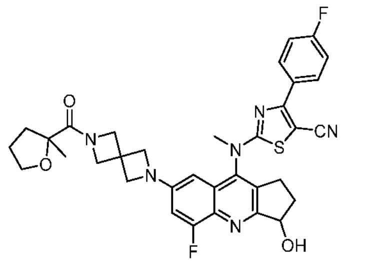,

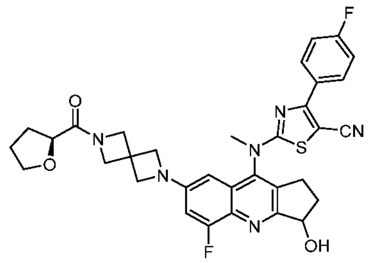,

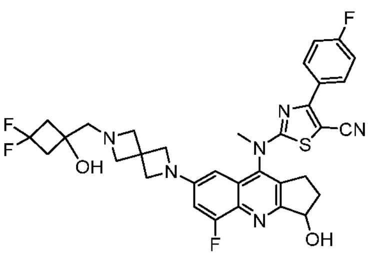,

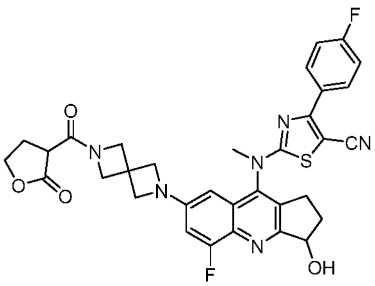,

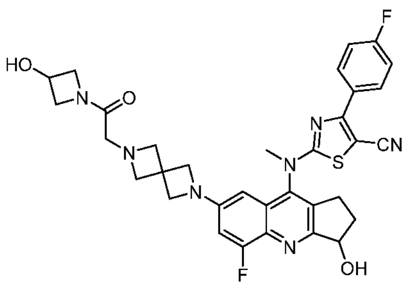,

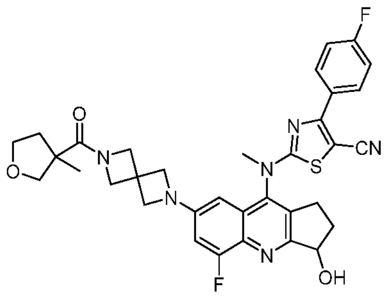,

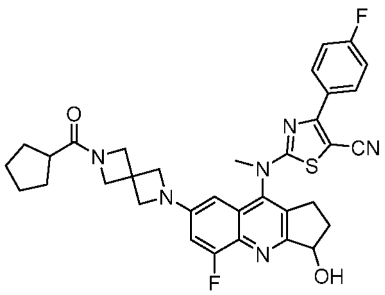,

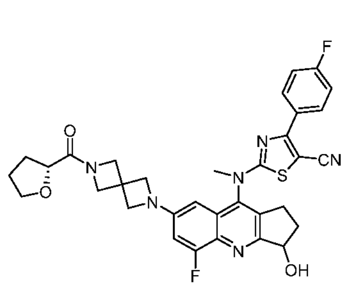,

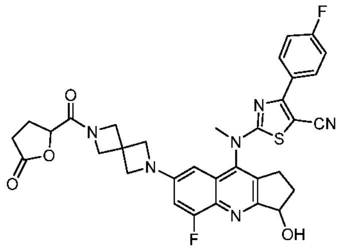, 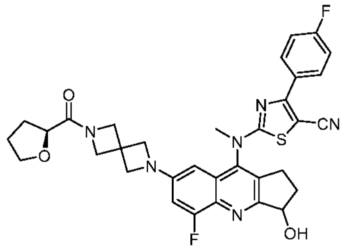, 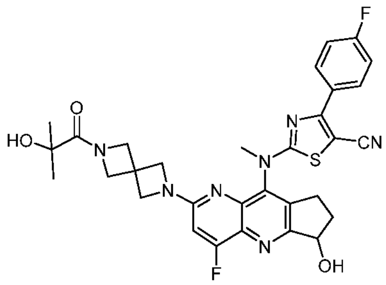,

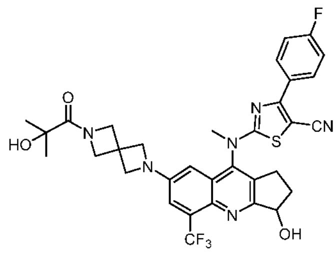, 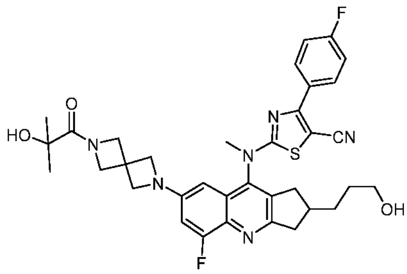, and 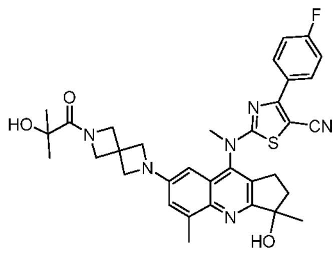.

Other aspects of the present disclosure also provide a compound of the following formulas, an optical isomer thereof, or a pharmaceutically acceptable salt thereof, selected from:

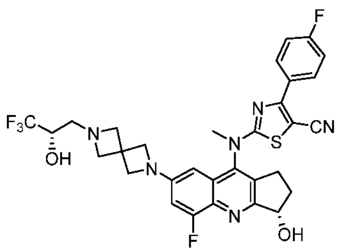, 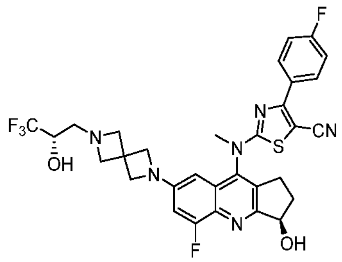, 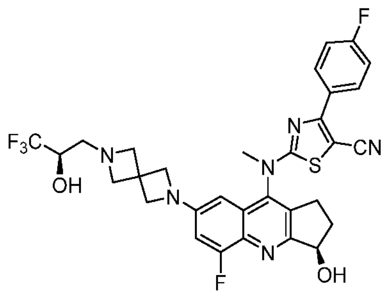,

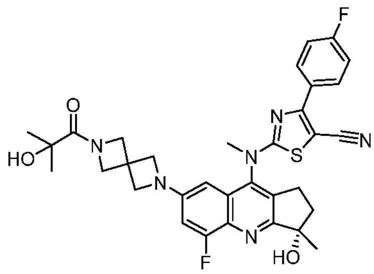, 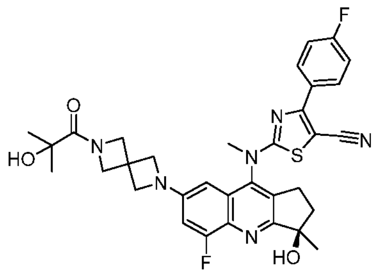, 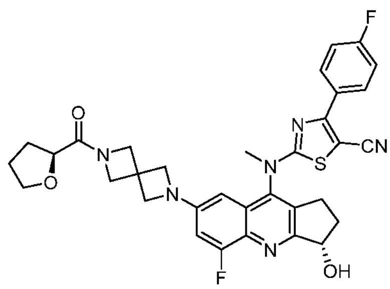,

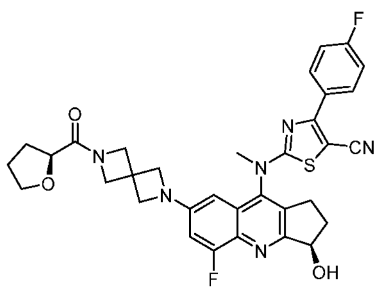, 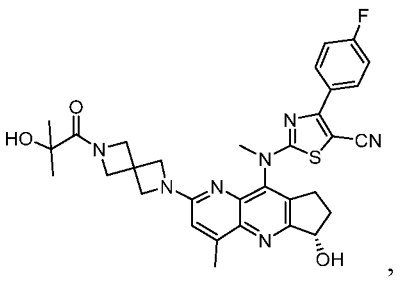, 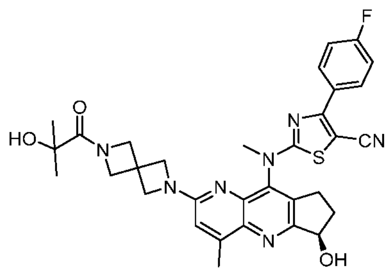,

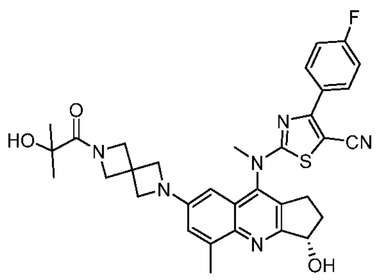, 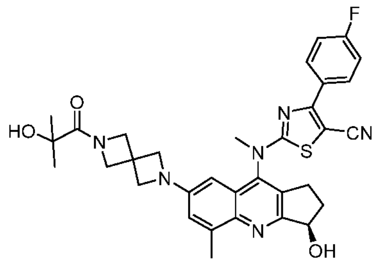, 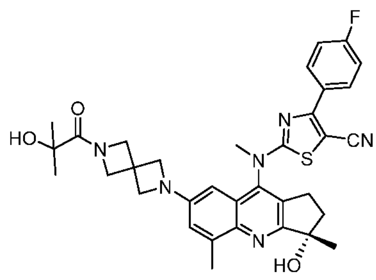,

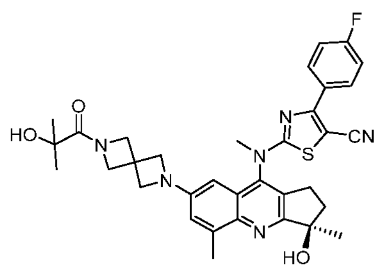, 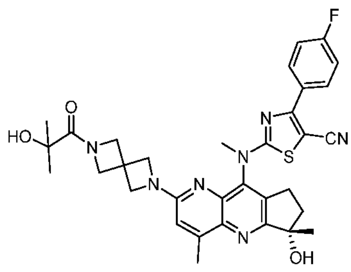, and 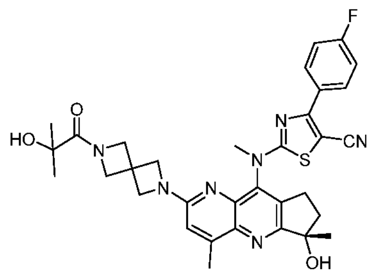.

In yet another aspect of the present disclosure, the present disclosure also provides a use of the compound, the optical isomer thereof, or the pharmaceutically acceptable salt thereof in the manufacture of a medicament for treating a disease related to ATX.

In some embodiments of the present disclosure, the disease related to ATX is selected from cancer, metabolic disease, renal disease, liver disease, fibrotic disease, inflammatory disease, pain, autoimmune disease, respiratory disease, cardiovascular disease, neurodegenerative disease, myelodysplastic syndrome, obesity, dermatological disorder, and/or disease related to abnormal angiogenesis.

In some embodiments of the present disclosure, the fibrotic disease is selected from pulmonary fibrosis, renal fibrosis, hepatic fibrosis, *etc.*

In some embodiments of the present disclosure, the pulmonary fibrosis is selected from idiopathic pulmonary fibrosis, non-idiopathic pulmonary fibrosis, *etc.*

In some embodiments of the present disclosure, the liver disease is selected from non-alcoholic steatohepatitis, *etc.*

In some embodiments of the present disclosure, the inflammatory disease is selected from enteritis, osteoarthritis, *etc.*

In some embodiments of the present disclosure, the autoimmune disease is selected from rheumatoid arthritis, multiple sclerosis, *etc.*

In some embodiments of the present disclosure, the respiratory disease is selected from interstitial lung disease, asthma, COPD, *etc.*

In some embodiments of the present disclosure, the cardiovascular disease is selected from vascular injury, atherosclerosis, coagulation, *etc.*

**Definition and description**

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, other problems, or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, and methanesulfonic acid; and a salt of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereomers isomers, (*D*)-isomers, (*L*)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are comprised within the scope of the present disclosure.

The compounds of the present disclosure may exist in specific forms. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) comprises interconversion through proton migration, such as keto-enol isomerization and imine-enamine isomerization. Valence tautomer comprises some recombination of bonding electrons for mutual transformation. A specific example of keto-enol tautomerization is the tautomerism between two tautomers of pentane-2,4-dione and 4-hydroxypent-3-en-2-one.

The compound of the present disclosure may contain an unnatural proportion of atomic isotope at one or more than one atom that constitute the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure. "Optional" or "optionally" means that the subsequent event or condition may occur but not requisite, and the description includes the instance in which the event or condition occurs and the instance in which the event or condition does not occur.

The term "substituted by..." means that one or more than one H on a specific atom is substituted by the substituent, including deuterium and hydrogen variables, as long as the valence of the specific atom is normal and the substituted compound is stable. The term "optionally substituted by..." means that an atom may or may not be substituted, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound. For example,

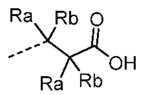

can be selected from

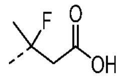,

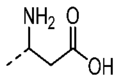, 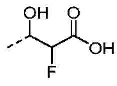, and 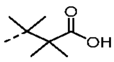.

A hyphen ("-") being not between two letters or symbols indicates the linkage site of a substituent. For example, $C_{1-6}$ alkylcarbonyl- refers to a $C_{1-6}$ alkyl group connected to the rest of the molecule through a carbonyl group. However, when the linkage site of a substituent is obvious to those skilled in the art, for example, a halogen substituent, "-" can be omitted.

When one of the variables is selected from a single bond, it means that the two groups linked by the single bond are linked directly. For example, when Li in

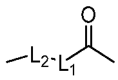

represents a single bond, the structure is actually

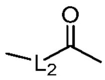.

Unless otherwise specified, when the valence bond of a group has a dashed line

"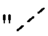",

such as in

"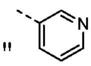",

the dashed line indicates the linkage site of the group to the rest of the molecule.

When the listed substituent does not indicate via which atom it is linked to the substituted group, such substituent can be bonded via any atom thereof, for example, pyridyl, as a substituent, can be linked to the substituted group via any carbon atom on the pyridine ring.

When the listed linking group does not indicate the direction for linking, the direction for linking is arbitrary, for example, the linking group L contained in

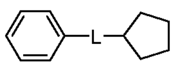

is

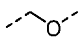,

then

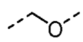

can link phenyl and cyclopentyl to form

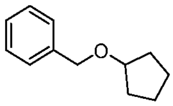

in the direction same as left-to-right reading order, and can link phenyl and cyclopentyl to form

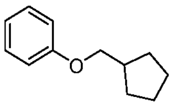

in the direction contrary to left-to-right reading order. A combination of the linking groups, substituents and/or variables thereof is allowed only when such combination can result in a stable compound.

Unless otherwise specified, the number of atoms on a ring is usually defined as the number of ring members, for example, "4- to 6-membered ring" refers to a "ring" in which 4 to 6 atoms are arranged around.

Unless otherwise specified, the term "alkyl" refers to a saturated aliphatic hydrocarbon group, which is a linear or branched group containing 1 to 20 carbon atoms, preferably an alkyl group containing 1 to 12 carbon atoms, more preferably an alkyl group containing 1 to 6 carbon atoms. It can be monovalent (such as methyl), divalent (such as methylene), or multivalent (such as methine). Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylpentyl, 2-methyl-3-ethylhexyl, 2,2-diethylpentyl, *n*-decyl, 3,3-diethylhexyl, 2,2-diethylhexyl, methylene ($-CH_2-$), 1,1-ethylidene ($-CH(CH_3)-$), 1,2-ethylidene ($-CH_2CH_2-$), 1,1-propylidene ($-CH(CH_2CH_3)-$), 1,2-propylidene ($-CH_2CH(CH_3)-$), 1,3-propylidene ($-CH_2CH_2CH_2-$), 1,4-butylidene ($-CH_2CH_2CH_2CH_2-$), and various branched isomers thereof. More preferred is a lower alkyl group containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *etc.* An alkyl group may be substituted or unsubstituted, and when substituted, a substituent may be substituted at any available linkage site. The substituent is preferably one or more than one of the following groups. The alkyl group is substituted by one or more than one substituent independently selected from alkyl, alkenyl, alkynyl, alkoxy, alkylthio, alkylamino, halogen, mercapto, OH, nitro, cyano, cycloalkyl, heterocycloalkyl, aryl, heteroaryl, cycloalkoxy, heterocycloalkoxy, cycloalkylthio, heterocycloalkylthio, and oxo.

The term "heteroalkyl" by itself or in combination with another term means a stable linear or branched alkyl atomic group consisting of a certain number of carbon atoms and at least one heteroatom or heteroatom group, or a combination thereof. In some embodiments, the heteroatom is selected from B, O, N, and S, wherein the nitrogen and sulfur atoms are optionally oxidized, and the nitrogen heteroatom is optionally quaternized. In other embodiments, the heteroatom group is selected from -C(=O)O-, -C(=O)-, -C(=S)-, -S(=O), - S(=O)z-, -C(=O)N(H)-, -N(H)-, -C(=NH)-, $-S(=O)_2N(H)-$, and -S(=O)N(H)-. In some embodiments, the heteroalkyl is $C_{1-6}$ heteroalkyl; in other embodiments, the heteroalkyl is $C_{1-3}$ heteroalkyl. A heteroatom or heteroatom group may be located at any internal position of heteroalkyl, including the position where the alkyl group is connected to the rest of the molecule. However, the term "alkoxy" is a conventional expression and refers to those alkyl groups connected to the rest of the molecule through an oxygen atom. Examples of heteroalkyl include, but are not limited to, $-OCH_3$, $-OCH_2CH_3$, $-OCH_2CH_2CH_3$, $-OCH_2(CH_3)_2$, $-CH_2-CH_2-O-CH_3$, $-NHCH_3$, $-N(CH_3)_2$, $-NHCH_2CH_3$, $-N(CH_3)(CH_2CH_3)$, $-CH_2-CH_2-NH-CH_3$, $-CH_2-CH_2-N(CH_3)-CH_3$, $-SCH_3$, $-SCH_2CH_3$, $-SCH_2CH_2CH_3$, $-SCH_2(CH_3)_2$, $-CH_2-S-CH_2-CH_3$, $-CH_2-CH_2$, $-S(=O)-CH_3$, $-CH_2-CH_2-S(=O)_2-CH_3$, and up to two heteroatoms may be consecutive, such as $-CH_2-NH-OCH_3$.

Unless otherwise specified, the term "$C_{1-6}$ alkoxy" refers to an alkyl group containing 1 to 6 carbon atoms that are connected to the rest of the molecule through an oxygen atom. The $C_{1-6}$ alkoxy includes $C_{1-4}$ alkoxy, $C_{1-3}$ alkoxy, $C_{1-2}$ alkoxy, $C_{2-6}$ alkoxy, $C_{2-4}$ alkoxy, $C_6$ alkoxy, $C_5$ alkoxy, $C_4$ alkoxy, $C_3$ alkoxy, *etc.* Examples of $C_{1-6}$ alkoxy include, but are not limited to, methoxy, ethoxy, propoxy (including *n*-propoxy and isopropoxy), butoxy (including *n*-butoxy, isobutoxy, *s*-butoxy, and *t*-butoxy), pentyloxy (including *n*-pentyloxy, isopentyloxy, and neopentyloxy), hexyloxy, *etc.*

Unless otherwise specified, the term "$C_{1-3}$ alkoxy" refers to an alkyl group containing 1 to 3 carbon atoms that

are connected to the rest of the molecule through an oxygen atom. The $C_{1-3}$ alkoxy includes $C_{1-3}$ alkoxy, $C_{1-2}$ alkoxy, $C_{2-3}$ alkoxy, $C_1$ alkoxy, $C_2$ alkoxy, $C_3$ alkoxy, *etc.* Examples of $C_{1-3}$ alkoxy include, but are not limited to, methoxy, ethoxy, propoxy (including *n*-propoxy and isopropoxy), *etc.*

Unless otherwise specified, the term "$C_{1-6}$ alkylamino" refers to an alkyl group containing 1 to 6 carbon atoms that are connected to the rest of the molecule through an amino group. The $C_{1-6}$ alkylamino includes $C_{1-4}$ alkylamino, $C_{1-3}$ alkylamino, $C_{1-2}$ alkylamino, $C_{2-6}$ alkylamino, $C_{2-4}$ alkylamino, $C_6$ alkylamino, $C_5$ alkylamino, $C_4$ alkylamino, $C_3$ alkylamino, $C_2$ alkylamino, *etc.* Examples of $C_{1-6}$ alkylamino include, but are not limited to, $-NHCH_3$, - $N(CH_3)_2$, $-NHCH_2CH_3$, $-N(CH_3)CH_2CH_3$, $-N(CH_2CH_3)(CH_2CH_3)$, $-NHCH_2CH_2CH_3$, - $NHCH_2(CH_3)_2$, $-NHCH_2CH_2CH_2CH_3$, *etc.*

Unless otherwise specified, the term "$C_{1-3}$ alkylamino" refers to an alkyl group containing 1 to 3 carbon atoms that are connected to the rest of the molecule through an amino group. The $C_{1-3}$ alkylamino includes $C_{1-3}$ alkylamino, $C_{1-2}$ alkylamino, $C_{2-3}$ alkylamino, $C_1$ alkylamino, $C_2$ alkylamino, $C_3$ alkylamino, *etc.* Examples of $C_{1-3}$ alkylamino include, but are not limited to, $-NHCH_3$, $-N(CH_3)_2$, $-NHCH_2CH_3$, $-N(CH_3)CH_2CH_3$, $-NHCH_2CH_2CH_3$, - $NHCH_2(CH_3)_2$, *etc.*

Unless otherwise specified, the term "$C_{1-6}$ alkylthio" refers to an alkyl group containing 1 to 6 carbon atoms that are connected to the rest of the molecule through a sulfur atom. The $C_{1-6}$ alkylthio includes $C_{1-4}$ alkylthio, $C_{1-3}$ alkylthio, $C_{1-2}$ alkylthio, $C_{2-6}$ alkylthio, $C_{2-4}$ alkylthio, $C_6$ alkylthio, $C_5$ alkylthio, $C_4$ alkylthio, $C_3$ alkylthio, $C_2$ alkylthio, *etc.* Examples of $C_{1-6}$ alkylthio include, but are not limited to, $-SCH_3$, $-SCH_2CH_3$, $-SCH_2CH_2CH_3$, $-SCH_2(CH_3)_2$, *etc.*

Unless otherwise specified, the term "$C_{1-3}$ alkylthio" refers to an alkyl group containing 1 to 3 carbon atoms that are connected to the rest of the molecule through a sulfur atom. The $C_{1-3}$ alkylthio includes $C_{1-3}$ alkylthio, $C_{1-2}$ alkylthio, $C_{2-3}$ alkylthio, Ci alkylthio, $C_2$ alkylthio, $C_3$ alkylthio, *etc.* Examples of $C_{1-3}$ alkylthio include, but are not limited to, - $SCH_3$, $-SCH_2CH_3$, $-SCH_2CH_2CH_3$, $-SCH_2(CH_3)_2$, *etc.*

Unless otherwise specified, the term "cycloalkyl" refers to a saturated or partially unsaturated monocyclic or polycyclic hydrocarbon substituent, and the cycloalkyl ring contains 3 to 20 carbon atoms, preferably contains 3 to 12 carbon atoms (it can be a specific point or an interval between any two points, such as 3, 4, 5, 6 ring atoms, 4 to 11 ring atoms, 6 to 12 ring atoms, *etc.*), more preferably contains 3 to 8 carbon atoms, most preferably contains 3 to 6 (such as 3, 4, 5, or 6) carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, *etc.,* preferably cycloalkyl; polycyclic cycloalkyl includes spirocycloalkyl, fused cycloalkyl, and bridged cycloalkyl.

Unless otherwise specified, the term "spirocycloalkyl" refers to a 5- to 20-membered polycyclic group that shares one carbon atom (called a spiro atom) between monocyclic rings, which may contain one or more than one double bond, but none of the rings has a fully conjugated π-electron system. It is preferably 6- to 14-membered, more preferably 7- to 10-membered. Spirocycloalkyl is divided into monospirocycloalkyl, bispirocycloalkyl, or polyspirocycloalkyl according to the number of spiro atoms shared between rings, preferably monospirocycloalkyl and bispirocycloalkyl. It is more preferably 4-membered/4-membered, 4-membered/5-membered, 4-membered/6-membered, 5-membered/5-membered, or 5-membered/6-membered monospirocycloalkyl. Non-limiting examples of spirocycloalkyl include:

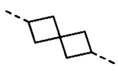, 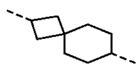,

*etc.*

Unless otherwise specified, the term "fused cycloalkyl" refers to a 5- to 20-membered full-carbon polycyclic group, in which each ring in the system shares an adjacent pair of carbon atoms with the other ring in the system, wherein one or more than one of the rings may contain one or more than one double bond, but none of the rings has a fully conjugated π-electron system. It is preferably 6- to 14-membered, more preferably 7- to 10-membered. According to the number of constituent rings, it can be divided into bicyclic, tricyclic, tetracyclic, or polycyclic fused cycloalkyl, preferably bicyclic or tricyclic, more preferably 5-membered/5-membered or 5-membered/6-membered bicycloalkyl. Non-limiting examples of fused cycloalkyl include:

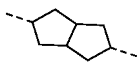,

*etc.*

Unless otherwise specified, the term "bridged cycloalkyl" refers to a 5- to 20-membered full-carbon polycyclic group, in which any two rings share two non-directly attached carbon atoms, and which may contain one or more than one double bond, but none of the rings has a fully conjugated π-electron system. It is preferably 6- to 14-membered, more preferably 7- to 10-membered. According to the number of constituent rings, it can be divided into bicyclic, tricyclic, tetracyclic, or polycyclic bridged cycloalkyl, preferably bicyclic, tricyclic, or tetracyclic, more preferably bicyclic or tricyclic.

Non-limiting examples of bridged cycloalkyl include:

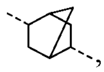

*etc.*

The cycloalkyl ring includes the cycloalkyl (*e.g.*, monocyclic cycloalkyl, fused cycloalkyl, spirocycloalkyl, and bridged cycloalkyl) fused to an aryl ring, a heteroaryl ring, or a heterocycloalkyl ring, wherein the ring attached to the parent structure is a cycloalkyl ring, non-limiting examples of which include indanyl, tetrahydronaphthyl, benzocycloheptyl, *etc*; preferably benzocyclopentyl, tetrahydronaphthyl.

The cycloalkyl may be optionally substituted or unsubstituted, and when substituted, the substituent is preferably one or more than one of the following groups independently selected from alkyl, alkenyl, alkynyl, alkoxy, alkylthio, alkylamino, halogen, mercapto, OH, nitro, cyano, cycloalkyl, heterocycloalkyl, aryl, heteroaryl, cycloalkoxy, heterocycloalkoxy, cycloalkylthio, heterocycloalkylthio, and oxo.

Unless otherwise specified, the term "heterocyclyl" refers to a saturated or partially unsaturated monocyclic or polycyclic hydrocarbon substituent containing 3 to 20 ring atoms, wherein one or more than one of the ring atoms are heteroatoms selected from nitrogen, oxygen, or, $S(O)_m$ (where m is an integer of 0 to 2), but excluding the ring portion of -O-O-, -O-S-, or - S-S-, and the remaining ring atoms are carbon. Preferably, it contains 3 to 12 ring atoms (it can be a specific point or an interval between any two points, such as 3, 4, 5, 6 ring atoms, 4 to 11 ring atoms, 6 to 12 ring atoms, *etc*.), 1 to 4 of which are heteroatoms; preferably, it contains 3 to 8 ring atoms, 1 to 3 of which are heteroatoms; more preferably, it contains 3 to 6 ring atoms, 1 to 3 of which are heteroatoms. Non-limiting examples of monocyclic heterocyclyl include azetidinyl, pyrrolidinyl, imidazolidinyl, tetrahydrofuranyl, tetrahydropyranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, homopiperazinyl, *etc.,* preferably tetrahydropyranyl, piperidinyl, pyrrolidinyl. Polycyclic heterocyclyl includes spiroheterocyclyl, fused heterocyclyl, and bridged heterocyclyl.

Unless otherwise specified, the term "spiroheterocyclyl" refers to a 5- to 20-membered polycyclic heterocyclic group that shares one atom (called a spiro atom) between monocyclic rings, wherein one or more than one of the ring atoms are heteroatoms selected from nitrogen, oxygen, or $S(O)_m$ (where m is an integer of 0 to 2), and the remaining ring atoms are carbon. It may contain one or more than one double bond, but none of the rings has a fully conjugated $\pi$-electron system. It is preferably 6- to 14-membered, more preferably 7- to 11-membered. Spiroheterocyclyl is divided into monospiroheterocyclyl, bispiroheterocyclyl, or polyspiroheterocyclyl according to the number of spiro atoms shared between rings, preferably monospiroheterocyclyl and bispiroheterocyclyl. It is more preferably 4-membered/4-membered, 4-membered/5-membered, 4-membered/6-membered, 5-membered/5-membered, or 5-membered/6-membered monospiroheterocyclyl. Non-limiting examples of spiroheterocyclyl include:

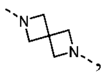
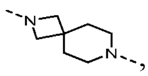

*etc.*

Unless otherwise specified, the term "fused heterocyclyl" refers to a 5- to 20-membered polycyclic heterocyclic group, in which each ring in the system shares an adjacent pair of atoms with the other ring in the system, one or more than one of the rings may contain one or more than one double bond, but none of the rings has a fully conjugated $\pi$-electron system, wherein one or more than one of the ring atoms are heteroatoms selected from nitrogen, oxygen, or $S(O)_m$ (where m is an integer of 0 to 2), and the remaining ring atoms are carbon. It is preferably 6- to 14-membered, more preferably 7- to 11-membered. According to the number of constituent rings, it can be divided into bicyclic, tricyclic, tetracyclic, or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include:

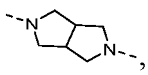

*etc.*

Unless otherwise specified, the term "bridged heterocyclyl" refers to a 5- to 14-membered polycyclic heterocyclic group, in which any two rings share two non-directly attached atoms, and which may contain one or more than one double bond, but none of the rings has a fully conjugated $\pi$-electron system, wherein one or more than one of the ring

atoms are heteroatoms selected from nitrogen, oxygen, or $S(O)_m$ (where m is an integer of 0 to 2), and the remaining ring atoms are carbon. It is preferably 6- to 14-membered, more preferably 7-to 11-membered. According to the number of constituent rings, it can be divided into bicyclic, tricyclic, tetracyclic, or polycyclic bridged heterocyclyl, preferably bicyclic, tricyclic, or tetracyclic, more preferably bicyclic or tricyclic. Non-limiting examples of bridged heterocyclyl include:

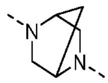,

*etc.*

The heterocyclyl ring includes the heterocyclyl (*e.g.*, monocyclic heterocyclyl, fused heterocyclyl, spiroheterocyclyl, and bridged heterocyclyl) fused to an aryl ring, a heteroaryl ring, or a cycloalkyl ring, wherein the ring attached to the parent structure is a heterocyclyl ring, non-limiting examples of which include:

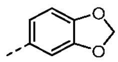,

*etc.*

The heterocyclyl may be optionally substituted or unsubstituted, and when substituted, the substituent is preferably one or more than one of the following groups independently selected from alkyl, alkenyl, alkynyl, alkoxy, alkylthio, alkylamino, halogen, mercapto, OH, nitro, cyano, cycloalkyl, heterocycloalkyl, aryl, heteroaryl, cycloalkoxy, heterocycloalkoxy, cycloalkylthio, heterocycloalkylthio, and oxo.

Unless otherwise specified, the term "aryl" refers to a 6- to 20-membered full-carbon monocyclic or fused polycyclic (*i.e.*, a ring sharing an adjacent pair of carbon atoms) group having a conjugated $\pi$-electron system, preferably 6- to 10-membered, more preferably 6-membered, such as phenyl and naphthyl. The aryl ring includes the aryl fused to a heteroaryl ring, a heterocyclyl ring, or a cycloalkyl ring, wherein the ring attached to the parent structure is an aryl ring, non-limiting examples of which include:

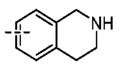, 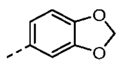, 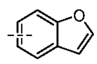,

*etc.*

The aryl may be substituted or unsubstituted, and when substituted, the substituent is preferably one or more than one of the following groups independently selected from alkyl, alkenyl, alkynyl, alkoxy, alkylthio, alkylamino, halogen, mercapto, OH, nitro, cyano, cycloalkyl, heterocycloalkyl, aryl, heteroaryl, cycloalkoxy, heterocycloalkoxy, cycloalkylthio, and heterocycloalkylthio.

Unless otherwise specified, the term "heteroaryl" refers to a heteroaromatic system containing 1 to 4 heteroatoms and 5 to 20 ring atoms, wherein the heteroatom is selected from oxygen, sulfur, and nitrogen. The heteroaryl is preferably 5- to 10-membered, containing 1 to 3 heteroatoms; more preferably 5- or 6-membered, containing 1 to 3 heteroatoms; non-limiting examples are pyrazolyl, imidazolyl, furyl, thienyl, thiazolyl, oxazolyl, pyrrolyl, triazolyl, tetrazolyl, pyridinyl, pyrimidinyl, thiadiazolyl, pyrazinyl, *etc.* The heteroaryl ring may be fused to an aryl ring, a heterocyclyl ring, or a cycloalkyl ring, wherein the ring attached to the parent structure is a heteroaryl ring, non-limiting examples of which include:

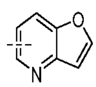,

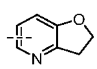,

*etc.*

The heteroaryl may be optionally substituted or unsubstituted, and when substituted, the substituent is preferably one or more than one of the following groups independently selected from alkyl, alkenyl, alkynyl, alkoxy, alkylthio, alkylamino, halogen, mercapto, OH, nitro, cyano, cycloalkyl, heterocycloalkyl, aryl, heteroaryl, cycloalkoxy, heterocycloalkoxy, cycloalkylthio, and heterocycloalkylthio.

Unless otherwise specified, the term "alkylthio" refers to -S-(alkyl) and -S-(unsubstituted cycloalkyl), wherein the alkyl or cycloalkyl is as defined above. Non-limiting examples of alkylthio include: methylthio, ethylthio, propylthio, butylthio, cyclopropylthio, cyclobutylthio, cyclopentylthio, cyclohexylthio. The alkylthio may be optionally substituted or unsubstituted, and when substituted, the substituent is preferably one or more than one of the following groups independently selected from alkyl, alkenyl, alkynyl, alkoxy, alkylthio, alkylamino, halogen, mercapto, OH, nitro, cyano, cycloalkyl, heterocycloalkyl, aryl, heteroaryl, cycloalkoxy, heterocycloalkoxy, cycloalkylthio, and heterocycloalkylthio.

Unless otherwise specified, the term "amino protecting group" is used to protect an amino group with a group that can be easily removed so that the amino group remains unchanged when other parts of the molecule are subject to a reaction. Non-limiting examples include *tert*-butoxycarbonyl, acetyl, benzyl, allyl, *p*-methoxybenzyl, *etc.* These groups may be optionally substituted by 1 to 3 substituents selected from halogen, alkoxy, or nitro. The amino protecting group is preferably *tert*-butoxycarbonyl.

Unless otherwise specified, the term "cycloalkyloxy" refers to -O-cycloalkyl, wherein the cycloalkyl is as defined above.

Unless otherwise specified, the term "haloalkyl" refers to an alkyl group substituted by halogen, wherein the alkyl is as defined above.

Unless otherwise specified, the term "haloalkoxy" refers to an alkoxy group substituted by halogen, wherein the alkoxy is as defined above.

Unless otherwise specified, the term "hydroxyalkyl" refers to an alkyl group substituted by OH, wherein the alkyl is as defined above.

Unless otherwise specified, the term "hydroxy" refers to an -OH group.

Unless otherwise specified, the term "halogen" refers to fluorine, chlorine, bromine, or iodine.

Unless otherwise specified, the term "aldehyde" refers to -C(O)H.

Unless otherwise specified, the term "carboxyl" refers to -C(O)OH.

Unless otherwise specified, the term "carboxylate group" refers to -C(O)O(alkyl) or - C(O)O(cycloalkyl), wherein the alkyl or cycloalkyl is as defined above.

Unless otherwise specified, the term "$C_{1-6}$ alkyl" refers to a linear or branched saturated hydrocarbon group consisting of 1 to 6 carbon atoms. The $C_{1-6}$ alkyl includes $C_{1-5}$ alkyl, $C_{1-4}$ alkyl, $C_{2-6}$ alkyl, *etc.*; it can be monovalent (such as methyl), divalent (such as methylene), or multivalent (such as methine). Examples of $C_{1-5}$ alkyl include, but are not limited to, methyl ("Me"), ethyl ("Et"), propyl such as *n*-propyl ("n-Pr") or isopropyl ("i-Pr"), butyl such as *n*-butyl ("n-Bu"), isobutyl ("i-Bu"), *sec*-butyl ("s-Bu"), or *tert*-butyl ("t-Bu"), pentyl, hexyl, *etc.*

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$ alkyl, $C_{2-3}$ alkyl, *etc.*; it can be monovalent (such as methyl), divalent (such as methylene), or multivalent (such as methine). Examples of $C_{1-3}$ alkyl include, but are not limited to, methyl (Me), ethyl (Et), propyl (including *n*-propyl and isopropyl), *etc.*

Unless otherwise specified, "$C_{2-6}$ alkenyl" refers to a linear or branched hydrocarbon group consisting of 2 to 6 carbon atoms containing at least one carbon-carbon double bond, and the carbon-carbon double bond may be located at any position of the group. The $C_{2-6}$ alkenyl includes $C_{2-4}$ alkenyl, $C_{2-3}$ alkenyl, $C_4$ alkenyl, $C_3$ alkenyl, $C_2$ alkenyl, *etc*; it can be monovalent, divalent, or multivalent. Examples of $C_{2-6}$ alkenyl include, but are not limited to, vinyl, propenyl, butenyl, pentenyl, hexenyl, butadienyl, pentadienyl, hexadienyl, *etc.*

Unless otherwise specified, "$C_{2-3}$ alkenyl" refers to a linear or branched hydrocarbon group consisting of 2 to 3 carbon atoms containing at least one carbon-carbon double bond, and the carbon-carbon double bond may be located at any position of the group. The $C_{2-3}$ alkenyl includes $C_3$ alkenyl and $C_2$ alkenyl; the $C_{2-3}$ alkenyl can be monovalent, divalent, or multivalent. Examples of $C_{2-3}$ alkenyl include, but are not limited to, vinyl, propenyl, *etc.*

Unless otherwise specified, "$C_{4-8}$ cycloalkyl" refers to a saturated monovalent monocyclic or bicyclic hydrocarbon group having 4 to 8 ring carbon atoms, such as 4 to 7 ring carbon atoms, such as 4 to 6 cyclic carbon atoms, such as 4 to 5 ring carbon atoms. For example, "$C_{4-8}$ cycloalkyl" means a cycloalkyl group having 4 to 8 ring carbon atoms. Similarly, "$C_{4-7}$ cycloalkyl" means a cycloalkyl group having 4 to 7 ring carbon atoms; "$C_{4-6}$ cycloalkyl" means a cycloalkyl group having 4 to 6 ring carbon atoms; "$C_{4-5}$ cycloalkyl" means a cycloalkyl group having 4 to 5 ring carbon atoms. Examples of cycloalkyl include, but are not limited to, cyclopropyl, cyclobutyl, cyclopentyl, cyclohexyl, cycloheptyl, cyclooctyl, *etc.*

Unless otherwise specified, "$C_{4-6}$ cycloalkyl" refers to a saturated cyclic hydrocarbon group consisting of 4 to 6 carbon atoms in monocyclic and bicyclic systems, and the $C_{4-6}$ cycloalkyl includes $C_{4-5}$ cycloalkyl, $C_{5-6}$ cycloalkyl, $C_4$ cycloalkyl, $C_5$ cycloalkyl, $C_6$ cycloalkyl, *etc*; it may be monovalent, divalent, or multivalent. Examples of $C_{4-6}$ cycloalkyl include, but are not limited to, cyclobutyl, cyclopentyl, cyclohexyl, *etc.*

Unless otherwise specified, the term "3- to 10-membered heterocyclyl" by itself or in combination with other terms refers to a saturated cyclic group consisting of 3 to 10 ring atoms, wherein 1, 2, 3, or 4 ring atoms are heteroatoms independently selected from O, S, and N, and the rest are carbon atoms, wherein the nitrogen atom is optionally quaternized, and the nitrogen and sulfur heteroatoms can be optionally oxidized (*i.e.*, NO and $S(O)_p$, p is 1 or 2). It includes

monocyclic and bicyclic systems, wherein the bicyclic system includes a spiro ring, a fused ring, and a bridged ring. In addition, with regard to the "3- to 10-membered heterocycloalkyl", a heteroatom may occupy the position where the heterocycloalkyl is connected to the rest of the molecule. The 3- to 10-membered heterocyclyl includes 6- to 9-membered heterocyclyl, 3- to 6-membered heterocyclyl, 3- to 5-membered heterocyclyl, 4- to 6-membered heterocyclyl, 5- to 6-membered heterocyclyl, 4-membered heterocyclyl, 5-membered heterocyclyl, 6-membered heterocyclyl, 7-membered heterocyclyl, 8-membered heterocyclyl, 9-membered heterocyclyl, 10-membered heterocyclyl, *etc.* Examples of 3- to 10-membered heterocyclyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, *etc.*), tetrahydrofuranyl (including tetrahydrofuran-2-yl, *etc.*), tetrahydropyranyl, piperidinyl (including 1-piperidinyl, 2-piperidinyl, 3-piperidinyl, *etc.*), piperazinyl (including 1-piperazinyl, 2-piperazinyl, *etc.*), morpholinyl (including 3-morpholinyl, 4-morpholinyl, *etc.*), dioxinyl, dithianyl, isoxazolidinyl, isothiazolidinyl, 1,2-oxazinyl, 1,2-thiazinyl, hexahydropyridazinyl, homopiperazinyl, homopiperidinyl, dioxacycloheptyl, 2,6-diazaspiro[3.3]heptyl, octahydropyrrolo[3,4-*c*]pyrrolyl, hexahydro-1*H*-furan[3,4-*c*]pyrrolyl, 2,7-diazaspiro[3.5]nonyl, 2,5-diazabicyclo[2.2.1]heptyl, *etc.* The term "5- to 9-membered bicycloalkyl" is also used herein to represent a fused ring having 5 to 9 ring atoms in a bicyclic system, such as bicyclo[1.1.1]pentyl, 2,5-diazabicyclo[2.2.1]heptyl, *etc.*

Unless otherwise specified, the term "3- to 6-membered heterocyclyl" by itself or in combination with other terms refers to a saturated cyclic group consisting of 3 to 6 ring atoms, wherein 1, 2, 3, or 4 ring atoms are heteroatoms independently selected from O, S, and N, and the rest are carbon atoms, wherein the nitrogen atom is optionally quaternized, and the nitrogen and sulfur heteroatoms can be optionally oxidized (*i.e.*, NO and $S(O)_p$, p is 1 or 2). It includes monocyclic and bicyclic systems, wherein the bicyclic system includes a spiro ring, a fused ring, and a bridged ring. In addition, with regard to the "3- to 6-membered heterocyclyl", a heteroatom may occupy the position where the heterocycloalkyl is connected to the rest of the molecule. The 3- to 6-membered heterocycloalkyl includes 5- to 6-membered heterocycloalkyl, 4-membered heterocycloalkyl, 5-membered heterocycloalkyl, 6-membered heterocycloalkyl, *etc.* Examples of 4- to 6-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, *etc.*), tetrahydrofuranyl (including tetrahydrofuran-2-yl, *etc.*), tetrahydropyranyl, piperidinyl (including 1-piperidinyl, 2-piperidinyl, 3-piperidinyl, *etc.*), piperazinyl (including 1-piperazinyl, 2-piperazinyl, *etc.*), morpholinyl (including 3-morpholinyl, 4-morpholinyl, *etc.*), dioxolyl, dithianyl, isoxazolidinyl, isothiazolidinyl, 1,2-oxazinyl, 1,2-thiazinyl, hexahydropyridazinyl, homopiperazinyl, homopiperidinyl, *etc.*

Unless otherwise specified, $C_{n-n+m}$ or $C_n$-$C_{n+m}$ includes any specific instance of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, and any range from n to n+m is also included, for example, $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, $C_{9-12}$, *etc.*; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, *etc.*

"Optional" or "optionally" means that the subsequently described event or circumstance may, but need not occur, and the description includes instances where the event or circumstance does or does not occur. For example, "a heterocyclic group optionally substituted by an alkyl group" means that an alkyl group may be, but need not be present, and the description includes instances where the heterocyclic group is substituted by an alkyl group and instances where the heterocyclic group is not substituted by an alkyl group.

"Substituted" means that one or more than one H in a group, preferably up to 5, more preferably 1 to 3 H, are independently substituted by a corresponding number of substituents, wherein each substituent has an independent option (*i.e.,* the substituents may be the same or different). It is self-evident that the substituents are only at their possible chemical positions, and that the possible or impossible substitutions can be determined (either experimentally or theoretically) by those skilled in the art without undue effort. For example, the combination of an amino group or OH having free hydrogen with a carbon atom having an unsaturated (such as olefinic) bond may be unstable.

It should be understood by those skilled in the art that some compounds of formula (I) can contain one or more than one chiral center. Therefore, the compound has two or more stereoisomers. Therefore, the compounds of the present disclosure can be present in the form of individual stereoisomers (*e.g.*, enantiomers, diastereomers) and mixtures thereof in arbitrary proportions, such as racemates, and under the proper condition, they can be present in the form of the tautomers and geometric isomers thereof.

As used herein, the term "stereoisomer" refers to compounds that have the same chemical constitution, but differ in the arrangement of the atoms or groups in space. Stereoisomer includes enantiomer, diastereomer, conformer, *etc.*

As used herein, the term "enantiomer" refers to two stereoisomers of a compound that are non-superimposable mirror images of each other.

As used herein, the term "diastereomer" refers to a stereoisomer in which a molecule has two or more chiral centers and whose molecules are not mirror images of each other. Diastereomers have different physical properties such as melting points, boiling points, spectral properties, or biological activities. Diastereomeric mixtures can be separated by high resolution analytical methods such as electrophoresis and chromatography (such as HPLC separation).

Stereochemical definitions and conventions can be followed in S. P. Parker ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds are present in optically active forms, *i.e.,* they have the ability to rotate the plane of plane polarized light. When optically active compounds are described, the prefixes D and L or R and S are used to denote the absolute configuration of the molecule with regard to its chiral centers. The prefixes d and l or (+) and (-) are used to denote the symbols of the compound's rotationally planar polarized light, wherein (-) or 1 indicates that the compound is levorotatory. Compounds with a prefix of (+) or d are dextrorotatory. For a given chemical structure, these stereoisomers are identical except that they are mirror images of each other. Specific stereoisomers can also be referred to as enantiomers, and mixtures of such isomers are often referred to as enantiomeric mixtures. A mixture of enantiomers in a ratio of 50 to 50 is known as a racemic mixture or racemate, which can be present in a chemical reaction or process without stereoselectivity or stereospecificity. The terms "racemic mixture" and "racemate" refer to an equimolar mixture of two enantiomers that is not optically active.

Racemic mixture can be used in its own form or after it is resolved into individual isomers. Resolution may yield stereochemically pure compounds or a mixture enriched in one or more isomers. Methods for separating isomers are well known (see Allinger N. L. and Eliel E. L., "Topics in Stereochemistry", Vol. 6, Wiley Interscience, 1971), including physical methods such as chromatography using chiral adsorbents. Individual isomers in a chiral form can be prepared from chiral precursors. Alternatively, a diastereomer salt can be formed with a chiral acid (such as a single enantiomer of 10-camphorsulfonic acid, camphoric acid, $\alpha$-bromocamphoric acid, tartaric acid, diacetyltartaric acid, malic acid, pyrrolidone-5-carboxylic acid), and then the mixture was chemically separated to obtain a single isomer. The salt is then graded and crystallized, and one or both of the split bases are freed. This process can be optionally repeated to obtain one or two isomers that essentially do not contain the other isomer, *i.e.*, the desired stereoisomer with an optical purity of at least 91%, 92%, 93%, 94%, 95%, 96%, 97%, 98%, 99%, or 99.5% by weight. Alternatively, the racemate can be covalently linked to a chiral compound (auxiliary) to obtain diastereomers, as is well known to those skilled in the art.

As used herein, the term "tautomer" or "tautomeric form" refers to structural isomers of different energies that are interconvertible via a low energy barrier. For example, proton tautomers (also called prototropic tautomers) include interconversion through proton migration, such as keto-enol isomerization and imine-enamine isomerization. Valence tautomers include interconversions by recombination of some bonding electrons.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The technical and scientific terms used herein that are not specifically defined have the meanings commonly understood by those skilled in the art to which the present disclosure belongs.

DETAILED DESCRIPTION OF THE PREFERRED EMBODIMENT

The present disclosure is further described in detail by the examples below. But it should be understood that these examples are only used to illustrate the present disclosure and are not intended to limit the scope of the present disclosure. Experimental methods in the following examples in which specific conditions are not indicated are usually in accordance with the conventional conditions for this type of reaction, or in accordance with the conditions recommended by the manufacturer. Unless otherwise specified, percentages and parts are by weight. Unless otherwise specified, ratios of liquids are by volume.

The experimental materials and reagents used in the following examples can be obtained from commercially available sources unless otherwise specified.

**Synthesis of example A1**

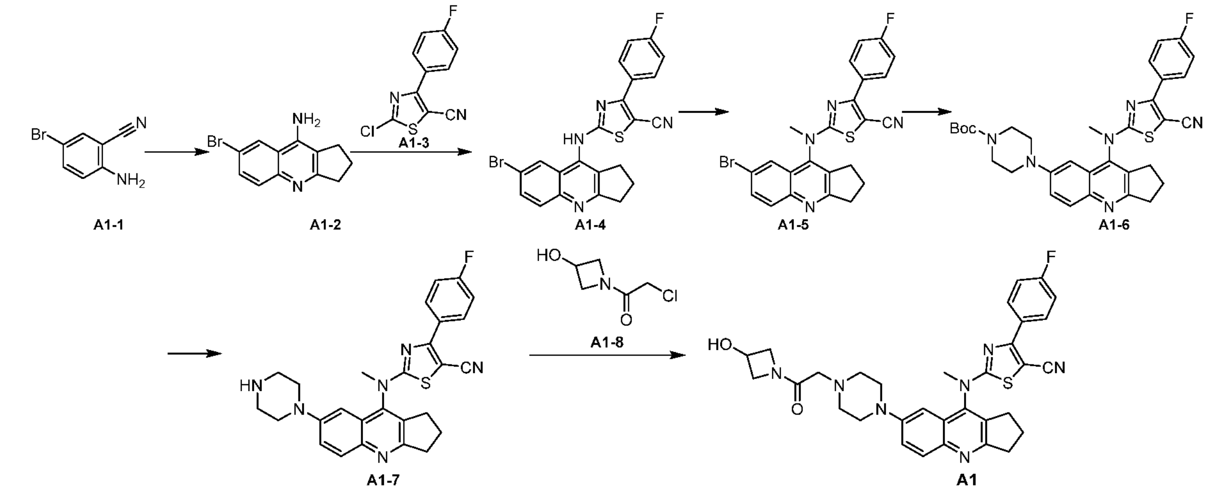

A1-1 A1-2 A1-3 A1-4 A1-5 A1-6 A1-7 A1-8 A1

**Step 1:** Preparation of compound **A1-2**

To a solution of compound **A1-1** (3 g, 15.23 mmol) and cyclopentanone (1.41 g, 16.75 mmol) in toluene (150 mL) was added indium chloride (4.04 g, 18.27 mmol) at room temperature. The reaction mixture was heated to 120°C and stirred for 24 hours. The system was cooled to room temperature (25°C), then added with sodium hydroxide aqueous solution (2 M, 180.00 mL), and heated to 120°C and stirred for 24 hours under argon atmosphere. The system was cooled to room temperature (25°C) and extracted with a mixed solvent (200 mL × 5) of chloroform/isopropanol (v/v = 3:1). The organic phases were combined, dried over anhydrous sodium sulfate, filtered, and the filtrate was concentrated to obtain a crude product. The crude product was slurried with a mixed solvent (20 mL) of petroleum ether/ethyl acetate (v/v = 2:1), filtered, and the filter cake was dried to obtain compound **A1-2,** which was directly used in the next reaction step without further purification.

$^1$H NMR (400 MHz, DMSO-*d6*) δ 8.40 (s, 1H), 7.70 - 7.48 (m, 2H), 6.55 (s, 2H), 2.88 (t, *J* = 7.6 Hz, 2H), 2.80 (t, *J* = 7.3 Hz, 2H), 2.13 - 1.91 (m, 2H). MS (ESI) *m/z* $(M+H)^+$ = 263.0.

**Step 2:** Preparation of compound **A1-3**

To a solution of compound 4-fluorobenzoylacetonitrile (25 g, 153.23 mmol) in ethanol (1000 mL) was added iodine (38.89 g, 153.23 mmol). The reaction mixture was heated to 70°C and stirred for 15 minutes before the system was cooled to room temperature (25°C). Pyridine (12.12 g, 153.23 mmol, 12.37 mL) and thiourea (23.33 g, 306.47 mmol) were dissolved in ethanol (300 mL), then slowly added to the above reaction system, and stirred at room temperature (25°C) for 1 hour. After the reaction was completed, the reaction system was added with ice-saturated sodium thiosulfate (500 mL) and filtered. The filter cake was washed with water and dried to obtain compound 2-amino-4-(4-fluorophenyl)-5-cyanothiazole, which was directly used in the next reaction step without further purification. $^1$H NMR (400 MHz, DMSO-*d6*) δ 8.25 (s, 2H), 8.11 - 7.86 (m, 2H), 7.50 - 7.30 (m, 2H).

Compound 2-amino-4-(4-fluorophenyl)-5-cyanothiazole (52 g, 237.19 mmol) and cuprous chloride (38.27 g, 284.62 mmol) were dissolved in acetonitrile (500 mL), and *tert*-butyl nitrite (36.69 g, 355.78 mmol, 42.32 mL) was added thereto. The system was stirred at room temperature (25°C) for 0.5 hours under nitrogen atmosphere, and 1 *N* HCl (300 mL), ethyl acetate (500 mL), and water (100 mL) were added thereto for phase separation and extraction. The organic phases were combined, washed with saturated brine (100 mL × 3), dried over anhydrous sodium sulfate, and filtered. The filtrate was concentrated to obtain a crude product, which was purified by medium-pressure column chromatography (ethyl acetate/petroleum ether (v/v) = 0 to 1%) to obtain compound **A1-3**.

**$^1$H NMR** (400 MHz, DMSO-*d6*) δ 8.24 - 8.00 (m, 2H), 7.57 - 7.23 (m, 2H).

**Step 3:** Preparation of compound **A1-4**

To a solution of compound **A1-2** (300 mg, 1.14 mmol) in tetrahydrofuran (10 mL) were added sodium hydride (90 mg, 2.25 mmol, purity: 60%) and compound **A1-3** (280 mg, 1.17 mmol), and the reaction mixture was stirred at room temperature (25°C) for 16 hours. After the reaction was completed, the reaction mixture was added with saturated ammonium chloride aqueous solution (1 mL) and water (20 mL) to quench, and extracted with ethyl acetate (40 mL). The organic phases were combined, washed with saturated brine (30 mL), dried over anhydrous sodium sulfate, and filtered. The filtrate was concentrated to obtain a crude product, which was purified by silica gel column chromatography

(ethyl acetate/petroleum ether (v/v) = 0 to 60%) to obtain compound **A1-4.**

$^1$H NMR (400 MHz, CHLOROFORM-*d*) δ 8.48 (br s, 1H), 8.07 (d, *J* = 2.0 Hz, 1H), 7.99 - 7.92 (m, 3H), 7.78 (dd, *J* = 2.1, 8.9 Hz, 1H), 7.08 - 6.98 (m, 2H), 3.23 (t, *J* = 7.5 Hz, 2H), 3.05 (t, *J* = 7.4 Hz, 2H), 2.34 - 2.20 (m, 2H). MS (ESI) *m*/*z* $(M+H)^+$ = 464.9.

**Step 4:** Preparation of compound **A1-5**

To a solution of compound **A1-4** (250 mg, 537.24 μmol) in *N,N*-dimethylformamide (5 mL) were added iodomethane (228.00 mg, 1.61 mmol) and potassium carbonate (150 mg, 1.09 mmol) at room temperature (25°C), and the reaction mixture was stirred at room temperature (25°C) for 5 hours under nitrogen atmosphere. After the reaction was completed, the system was added with ice water (50 mL) to precipitate, and filtered to collect a filter cake. The filter cake was dried to obtain compound **A1-5**, which was directly used in the next reaction step without further purification.

$^1$H NMR (400 MHz, CHLOROFORM-*d*) δ 8.18 - 8.08 (m, 2H), 8.01 (d, *J* = 8.9 Hz, 1H), 7.91 (d, *J* = 2.0 Hz, 1H), 7.81 (dd, *J* = 2.1, 8.9 Hz, 1H), 7.23 - 7.11 (m, 2H), 3.66 (s, 3H), 3.28 - 3.23 (m, 2H), 3.06 - 3.02 (m, 2H), 2.33 - 2.26 (m, 2H). MS (ESI) *m*/*z* $(M+H)^+$ = 479.1.

**Step 5:** Preparation of compound **A1-6**

1-(*tert*-Butoxycarbonyl)piperazine (250 mg, 1.34 mmol), compound **A1-5** (250 mg, 521.52 μmol), sodium tert-butoxide (150.00 mg, 1.56 mmol), tris(dibenzylideneacetone)dipalladium (100.00 mg, 109.20 μmol), and 2-(di-*tert*-butylphosphino)biphenyl (50.00 mg, 167.56 μmol) were dissolved in toluene (12 mL). The system was heated to 110°C and stirred for 1 hour under nitrogen atmosphere. The system was filtered and the filtrate was concentrated to obtain a crude product. The crude product was purified by medium-pressure column chromatography (ethyl acetate/petroleum ether (v/v) = 0 to 60%) to obtain compound **A1-6**. MS (ESI) *m*/*z* $(M+H)^+$ = 585.1.

**Step 6:** Preparation of compound **A1-7**

To a solution of compound **A1-6** (100 mg, 171.03 μmol) in dioxane (2 mL) was added a solution of hydrochloride acid in dioxane (4 M, 2 mL), and the reaction mixture was stirred at room temperature (25°C) for 1 hour. After the reaction was completed, the reaction mixture was concentrated under reduced pressure, added with ethyl acetate (20 mL) and water (10 mL), then added with saturated sodium bicarbonate aqueous solution to adjust the pH to 9, and extracted with ethyl acetate (20 mL × 2). The organic phases were combined, dried over anhydrous sodium sulfate, and filtered. The filtrate was concentrated to obtain compound **A1-7,** which was directly used in the next reaction step without further purification. MS (ESI) *m*/*z* $(M+H)^+$ = 485.1.

**Step 7:** Preparation of compound **A1-8**

3-Hydroxycyclobutylamine (5.0 g, 45.64 mmol, HCl salt) and potassium carbonate (13.88 g, 100.41 mmol) were dissolved in water (32 mL) and dichloromethane (35 mL), and chloroacetyl chloride (5.15 g, 45.64 mmol) was added thereto at 0°C. After the addition was completed, the system was stirred at room temperature (25°C) for 16 hours. The system was extracted with ethyl acetate/*n*-butanol (v/v = 1/1) (40 mL × 9). The organic phases were combined, dried over anhydrous sodium sulfate, and filtered. The filtrate was concentrated to obtain compound **A1-8,** which was directly used in the next reaction step without further purification.

$^1$H NMR (400 MHz, CHLOROFORM-*d*) δ 4.73 (br s, 1H), 4.55 - 4.47 (m, 1H), 4.38 - 4.27 (m, 1H), 4.23 - 4.11 (m, 1H), 3.96 (dd, *J* = 3.8, 11.0 Hz, 1H), 3.92 (s, 2H), 3.21 (br s, 1H).

**Step 8:** Preparation of compound **A1**

Compound **A1-7** (83 mg, 171.28 μmol), compound **A1-8** (51 mg, 340.97 μmol), and potassium carbonate (71 mg, 513.73 μmol) were dissolved in acetonitrile (3 mL), and the reaction mixture was stirred at room temperature (25°C) for 16 hours. The reaction mixture was diluted with water (20 mL) and ethyl acetate (50 mL), and then the phases were separated. The organic phases were combined, washed with saturated brine (20 mL), dried over anhydrous sodium sulfate, and filtered. The filtrate was concentrated to obtain a crude product, which was purified by preparative high performance liquid chromatography (separation conditions: chromatographic column: Phenomenex Gemini-NX 80 × 30 mm × 3 μm; column temperature: 25°C; mobile phase: water (10 mM ammonium bicarbonate)-acetonitrile; acetonitrile: 40% to 70% 9 min) to obtain compound **A1**.

$^1$H NMR (400 MHz, CHLOROFORM-*d*) δ 8.16 (br s, 2H), 8.00 (d, *J* = 9.3 Hz, 1H), 7.48 (dd, *J* = 2.3, 9.4 Hz, 1H), 7.18 (t, *J* = 8.5 Hz, 2H), 6.88 (d, *J* = 2.2 Hz, 1H), 4.73 - 4.64 (m, 1H), 4.51 - 4.40 (m, 1H), 4.33 - 4.25 (m, 1H), 4.11

(br dd, *J* = 3.6, 9.8 Hz, 1H), 3.90 (br dd, *J* = 3.9, 10.9 Hz, 1H), 3.66 (s, 3H), 3.31 (br s, 4H), 3.21 (dt, *J* = 4.1, 7.6 Hz, 2H), 3.10 (s, 2H), 3.04 - 2.93 (m, 2H), 2.69 (br d, *J* = 4.4 Hz, 4H), 2.29 - 2.22 (m, 2H). MS (ESI) *m/z* $(M+H)^+$ = 598.2. HPLC retention time: 6.39 min.

Separation conditions: chromatographic column: WELCH Ultimate LP-C18 150 * 4.6 mm 5 μm; column temperature: 40°C; mobile phase: water (2.75 mL/4 L trifluoroacetic acid)-acetonitrile (2.5 mL/4 L trifluoroacetic acid); acetonitrile: 10% to 80% 10 min, 80% 5 min; flow rate: 1.5 mL/min.

Similar to the synthesis of example **A1**, the following examples **A2** to **A38** were synthesized as shown in Table 1 below.

Table 1: Structural formulas and analytical data of examples **A2 to A38**

| Example | Structural formula | Analytical data |
|---|---|---|
| A2 | 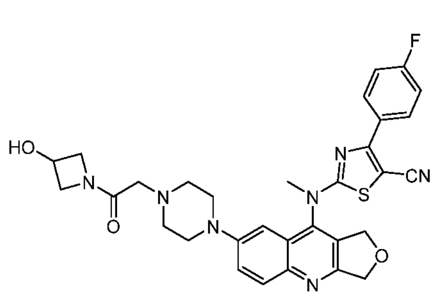 | $^1$H NMR (400 MHz, Chloroform-*d*) δ 8.14 (br dd, *J* = 5.4, 8.4 Hz, 2H), 8.04 (d, *J* = 9.3 Hz, 1H), 7.55 (dd, *J* = 2.5, 9.3 Hz, 1H), 7.17 (t, *J* = 8.7 Hz, 2H), 6.90 (d, *J* = 2.5 Hz, 1H), 5.29 - 5.14 (m, 4H), 4.74 - 4.64 (m, 1H), 4.46 (br t, *J* = 8.2 Hz, 1H), 4.29 (dd, *J* = 7.0, 10.8 Hz, 1H), 4.11 (dd, *J* = 4.1, 9.9 Hz, 1H), 3.90 (dd, *J* = 4.0, 10.3 Hz, 1H), 3.65 (s, 3H), 3.35 (br s, 4H), 3.14 - 3.03 (m, 2H), 2.70 (br t, *J* = 4.8 Hz, 4H), 2.40 (br s, 1H). MS (ESI) *m/z* $(M+H)^+$ = 600.1.<br>HPLC retention time: 3.527 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A3 | 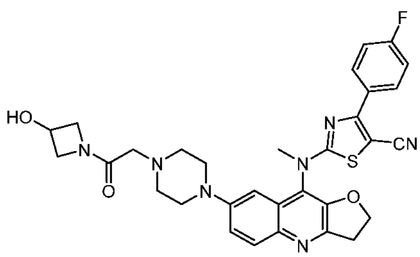 | $^1$H NMR (400 MHz, CHLOROFORM-*d*) δ 8.15 (br dd, *J* = 5.5, 8.3 Hz, 2H), 7.92 (d, *J* = 9.2 Hz, 1H), 7.33 (dd, *J* = 2.6, 9.3 Hz, 1H), 7.16 (t, *J* = 8.6 Hz, 2H), 6.84 (d, *J* = 2.5 Hz, 1H), 4.83 (t, *J* = 8.4 Hz, 2H), 4.74 - 4.62 (m, 1H), 4.45 (br t, *J* = 7.4 Hz, 1H), 4.28 (br dd, *J* = 6.8, 10.7 Hz, 1H), 4.10 (dd, *J* = 4.1, 9.9 Hz, 1H), 3.89 (br dd, *J* = 4.1, 10.8 Hz, 1H), 3.65 (s, 3H), 3.57 - 3.42 (m, 2H), 3.31 (br d, *J* = 3.3 Hz, 4H), 3.14 - 2.97 (m, 2H), 2.67 (t, *J* = 4.6 Hz, 4H). MS (ESI) *m/z* $(M+H)^+$ = 600.1.<br>HPLC retention time: 3.70 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A4 | 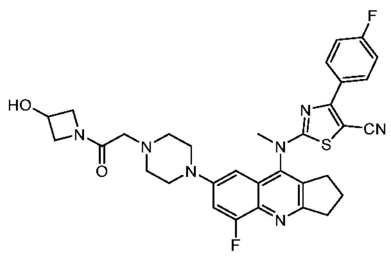 | $^1$H NMR (400 MHz, Chloroform-*d*) δ 8.03 (br s, 2H), 7.51 (br d, *J* = 14.3 Hz, 1H), 7.39 (br t, *J* = 8.2 Hz, 2H), 6.70 (s, 1H), 5.68 (br d, *J* = 5.8 Hz, 1H), 4.41 (br d, *J* = 5.8 Hz, 1H), 4.37 - 4.30 (m, 1H), 4.06 - 3.98 (m, 1H), 3.90 (dd, *J* = 4.1, 9.2 Hz, 1H), 3.63 - 3.53 (m, 4H), 3.30 - 3.24 (m, 4H), 3.14 - 2.94 (m, 5H), 2.92 - 2.76 (m, 1H), 2.54 (br s, 4H), 2.22 - 2.05 (m, 2H). MS (ESI) *m/z* $(M+H)^+$ = 616.1.<br>HPLC retention time: 4.017 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| A5 | 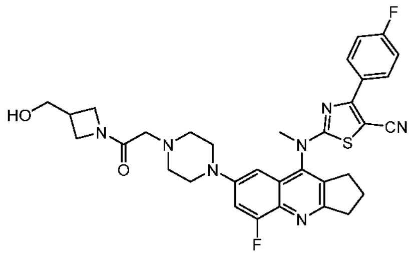 | $^1$H NMR (400 MHz, Chloroform-*d*) δ 8.22 - 8.09 (m, 2H), 7.24 - 7.12 (m, 3H), 6.65 (d, *J* = 2.0 Hz, 1H), 4.28 (br t, *J* = 8.3 Hz, 1H), 4.13 - 4.02 (m, 2H), 3.88 - 3.72 (m, 3H), 3.64 (s, 3H), 3.37 - 3.19 (m, 6H), 3.13 (s, 2H), 3.05 - 2.94 (m, 2H), 2.87 - 2.70 (m, 5H), 2.29 - 2.22 (m, 2H). MS (ESI) m/z $(M+H)^+$ = 630.1.<br>HPLC retention time: 4.025 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A6 | 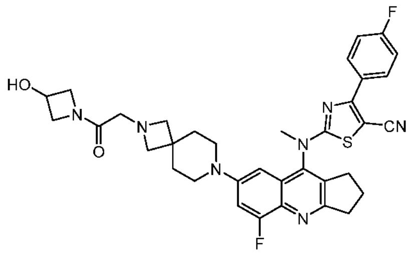 | $^1$H NMR (400 MHz, Chloroform-*d*) δ 8.16 (br s, 2H), 7.24 - 7.14 (m, 3H), 6.64 (s, 1H), 4.71 - 4.61 (m, 1H), 4.43 - 4.33 (m, 1H), 4.28 - 4.20 (m, 1H), 4.03 (br dd, *J* = 3.9, 9.4 Hz, 1H), 3.85 (br dd, *J* = 3.8, 10.5 Hz, 1H), 3.64 (s, 3H), 3.25 - 3.16 (m, 9H), 3.06 - 2.93 (m, 2H), 2.30 - 2.22 (m, 4H), 1.91 (br d, *J* = 5.0 Hz, 4H). MS (ESI) *m/z* $(M+H)^+$ = 656.1.<br>HPLC retention time: 4.215 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A7 | 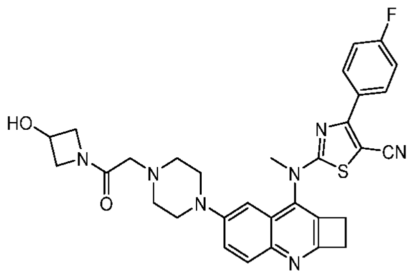 | $^1$H NMR (400 MHz, CHLOROFORM-*d*) δ 8.18 (dd, *J* = 5.3, 8.8 Hz, 2H), 8.04 (d, *J* = 9.3 Hz, 1H), 7.49 (dd, *J* = 2.5, 9.3 Hz, 1H), 7.19 (t, *J* = 8.7 Hz, 2H), 6.98 (d, *J* = 2.5 Hz, 1H), 4.70 (s, 1H), 4.54 - 4.44 (m, 1H), 4.31 (dd, *J* = 6.8, 10.8 Hz, 1H), 4.13 (dd, *J* = 4.0, 9.8 Hz, 1H), 3.92 (dd, *J* = 3.8, 11.0 Hz, 1H), 3.71 (s, 3H), 3.68 - 3.61 (m, 2H), 3.36 - 3.23 (m, 6H), 3.12 (s, 2H), 2.74 - 2.68 (m, 4H), 2.27 (s, 1H). MS (ESI) *m/z* $(M+H)^+$ = 584.2.<br>HPLC retention time: 2.943 min.<br>Separation conditions: chromatographic column: Ultimate C18 3 * 50 mm 3 μm; column temperature: 50°C; mobile phase: water (1.5 mL/4 L trifluoroacetic acid)-acetonitrile; acetonitrile: 10% to 80% 6 min, 80% 2 min; flow rate: 1.2 mL/min. |
| A8 | 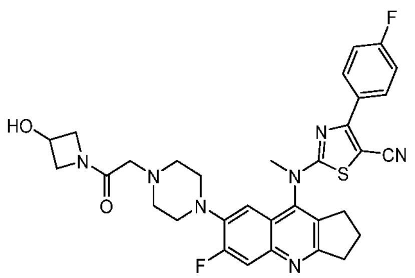 | $^1$H NMR (400 MHz, Chloroform-*d*) δ 8.15 (br s, 2H), 7.74 (d, *J* = 13.6 Hz, 1H), 7.17 (br t, *J* = 8.5 Hz, 2H), 7.05 (d, *J* = 8.8 Hz, 1H), 4.65 (br s, 1H), 4.50 - 4.40 (m, 1H), 4.34 - 4.23 (m, 1H), 4.21 (br d, *J* = 4.5 Hz, 1H), 4.01 - 3.90 (m, 1H), 3.65 (s, 3H), 3.50 - 3.26 (m, 6H), 3.26 - 3.18 (m, 2H), 3.15 - 2.94 (m, 6H), 2.26 (quin, *J* = 7.5 Hz, 2H). MS (ESI) *m/z* $(M+H)^+$ = 616.3.<br>HPLC retention time: 4.111 min.<br>Separation conditions: chromatographic column: Ultimate C18 3 * 50 mm 3 μm; column temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| A9 | 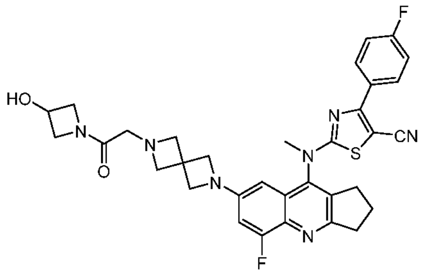 | $^1$H NMR (400 MHz, Chloroform-*d*) δ 8.16 (br s, 2H), 7.18 (t, *J* = 8.4 Hz, 2H), 6.67 (br d, *J* = 12.3 Hz, 1H), 6.14 (s, 1H), 4.67 (br s, 1H), 4.40 - 4.31 (m, 1H), 4.29 - 4.20 (m, 1H), 4.04 (s, 5H), 3.86 (br d, *J* = 10.6 Hz, 1H), 3.59 (br d, *J* = 19.0 Hz, 7H), 3.27 - 3.18 (m, 2H), 3.12 (br d, *J* = 5.3 Hz, 2H), 2.98 (br s, 2H), 2.30 - 2.16 (m, 2H). MS (ESI) *m/z* $(M+H)^+$ = 628.1.<br>HPLC retention time: 4.000 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A10 | 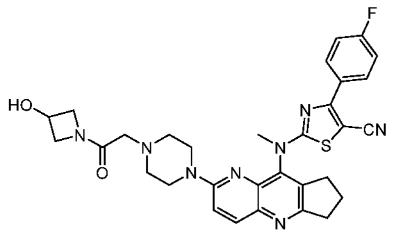 | $^1$H NMR (400 MHz, CHLOROFORM-*d*) δ 8.21 - 8.02 (m, 3H), 7.23 - 7.12 (m, 3H), 4.62 (br s, 1H), 4.44 - 4.36 (m, 1H), 4.25 (br s, 1H), 4.13 (br d, *J* = 5.8 Hz, 1H), 4.03 - 3.77 (m, 5H), 3.66 (s, 3H), 3.34 (br s, 1H), 3.23 (br t, *J* = 7.8 Hz, 3H), 3.09 (br t, *J* = 6.5 Hz, 2H), 2.92 (br s, 3H), 2.30 (td, *J* = 7.4, 14.8 Hz, 3H). MS (ESI) *m/z* $(M+H)^+$ = 599.1.<br>HPLC retention time: 3.942 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 0.5 min; flow rate: 0.8 mL/min. |
| A11 | 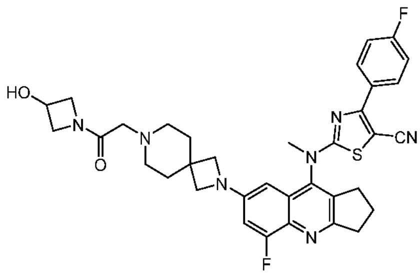 | $^1$H NMR (400 MHz, CHLOROFORM-*d*) δ 8.16 (br s, 2H), 7.18 (t, *J* = 8.7 Hz, 2H), 6.86 - 6.85 (m, 1H), 6.75 - 6.62 (m, 1H), 6.12 (d, *J* = 2.3 Hz, 1H), 4.76 - 4.62 (m, 1H), 4.45 (br t, *J* = 8.2 Hz, 1H), 4.29 (dd, *J* = 6.8, 11.3 Hz, 1H), 4.09 (br d, *J* = 9.8 Hz, 1H), 3.89 (dd, *J* = 4.5, 11.0 Hz, 1H), 3.67 (s, 4H), 3.62 (s, 3H), 3.27 - 3.17 (m, 2H), 3.04 - 2.93 (m, 4H), 2.48 (br s, 4H), 2.24 (quin, *J* = 7.5 Hz, 2H), 1.87 (br t, *J* = 5.3 Hz, 4H). MS (ESI) *m/z* $(M+H)^+$ = 656.3.<br>HPLC retention time: 4.350 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A12 | 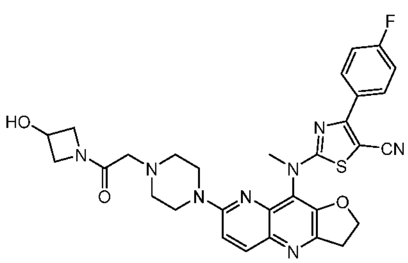 | $^1$H NMR (400 MHz, CHLOROFORM-*d*) δ 8.19 - 8.00 (m, 3H), 7.17 (br t, *J* = 8.5 Hz, 2H), 7.02 (d, *J* = 9.3 Hz, 1H), 4.90 (t, *J* = 8.4 Hz, 2H), 4.59 (br s, 1H), 4.44 - 4.34 (m, 1H), 4.23 (br s, 1H), 4.12 (br d, *J* = 7.0 Hz, 1H), 4.06 - 3.73 (m, 5H), 3.65 (s, 3H), 3.54 (br t, *J* = 8.5 Hz, 2H), 3.47 - 3.13 (m, 3H), 2.94 (br s, 3H). MS (ESI) *m/z* $(M+H)^+$ = 601.1.<br>HPLC retention time: 3.68 min.<br>Separation conditions: chromatographic column: Ultimate C18 3 * 50 mm 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |

(continued)

| Example | Structural formula | Analytical data |
|---|---|---|
| A13 | 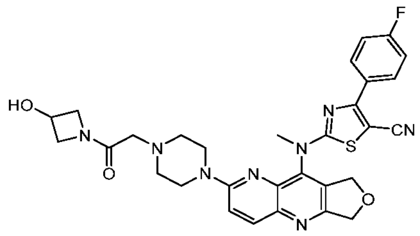 | $^1$H NMR (400 MHz, Chloroform-*d*) δ 8.16 (d, *J* = 9.4 Hz, 1H), 8.06 (br dd, *J* = 5.5, 8.3 Hz, 2H), 7.22 (d, *J* = 9.3 Hz, 1H), 7.16 (t, *J* = 8.6 Hz, 2H), 5.26 (s, 2H), 5.20 (s, 2H), 4.68 - 4.54 (m, 1H), 4.43 - 4.35 (m, 1H), 4.29 - 4.21 (m, 1H), 4.13 (br dd, *J* = 3.2, 9.2 Hz, 1H), 4.04 - 3.74 (m, 5H), 3.64 (s, 3H), 3.44 (br d, *J* = 14.1 Hz, 1H), 3.28 (brd, *J* = 14.5 Hz, 1H), 3.01 (br s, 4H). MS (ESI) *m/z* (M+H)$^+$ = 601.1.<br>HPLC retention time: 3.464 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A14 | 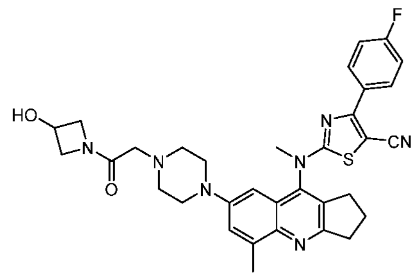 | $^1$H NMR (400 MHz, CHLOROFORM-*d*) δ 8.16 (br s, 2H), 7.34 (br s, 1H), 7.18 (br t, *J* = 8.4 Hz, 2H), 6.74 (s, 1H), 4.68 (br s, 1H), 4.51 - 4.41 (m, 1H), 4.33 - 4.24 (m, 1H), 4.11 (br d, *J* = 8.2 Hz, 1H), 3.90 (br d, *J* = 7.7 Hz, 1H), 3.64 (s, 3H), 3.29 (br s, 4H), 3.26 - 3.17 (m, 2H), 3.14 - 3.05 (m, 2H), 2.99 (br s, 2H), 2.79 (s, 3H), 2.68 (br s, 4H), 2.51 (br s, 1H), 2.28 - 2.21 (m, 2H). MS (ESI) *m/z* (M+H)$^+$ = 612.1.<br>HPLC retention time: 4.345 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A15 | 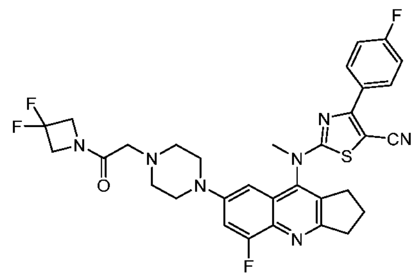 | $^1$H NMR (400 MHz, CHLOROFORM-*d*) δ 8.24 - 8.04 (m, 2H), 7.24 - 7.09 (m, 3H), 6.66 (d, *J* = 2.3 Hz, 1H), 4.59 (br t, *J* = 11.9 Hz, 2H), 4.36 (br t, *J* = 12.3 Hz, 2H), 3.65 (s, 3H), 3.39 - 3.22 (m, 6H), 3.19 (s, 2H), 3.06 - 2.89 (m, 2H), 2.69 (t, *J* = 4.9 Hz, 4H), 2.30 - 2.24 (m, 2H). MS (ESI) *m/z* (M+H)$^+$ = 636.1.<br>HPLC retention time: 4.733 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A16 | 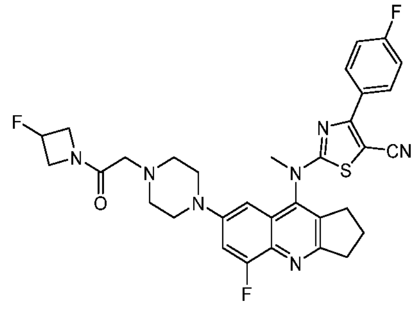 | $^1$H NMR (400 MHz, CHLOROFORM-*d*) δ 8.26 - 8.04 (m, 2H), 7.24 - 7.09 (m, 3H), 6.66 (d, *J* = 2.0 Hz, 1H), 5.49 - 5.18 (m, 1H), 4.65 - 4.45 (m, 1H), 4.43 - 4.25 (m, 2H), 4.23 - 4.07 (m, 1H), 3.64 (s, 3H), 3.43 - 3.21 (m, 6H), 3.19 - 3.08 (m, 2H), 3.07 - 2.86 (m, 2H), 2.68 (br t, *J* = 4.8 Hz, 4H), 2.29 - 2.24 (m, 2H). MS (ESI) *m/z* (M+H)$^+$ = 618.1.<br>HPLC retention time: 4.450 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| A17 | 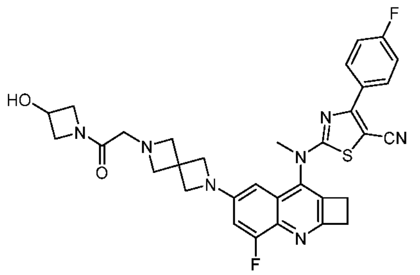 | $^1$H NMR (400 MHz, Chloroform-*d*) δ 8.16 (dd, *J* = 5.4, 8.8 Hz, 2H), 7.18 (t, *J* = 8.6 Hz, 2H), 6.68 (dd, *J* = 2.3, 12.0 Hz, 1H), 6.22 (d, *J* = 1.9 Hz, 1H), 4.74 - 4.65 (m, 1H), 4.41 - 4.33 (m, 1H), 4.26 (br dd, *J* = 7.0, 10.8 Hz, 1H), 4.08 - 3.97 (m, 5H), 3.87 (br dd, *J* = 4.0, 10.9 Hz, 1H), 3.71 - 3.60 (m, 6H), 3.56 (s, 3H), 3.26 - 3.19 (m, 2H), 3.16 - 3.06 (m, 2H). MS (ESI) *m/z* $(M+H)^+$ = 614.1.<br>HPLC retention time: 3.93 min.<br>Separation conditions: chromatographic column: Ultimate C18 3 * 50 mm 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| A18 | 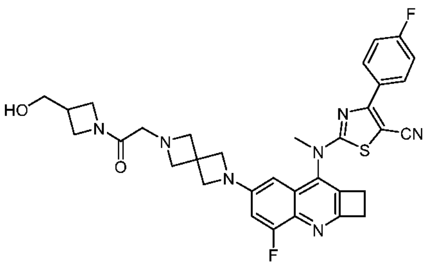 | $^1$H NMR (400 MHz, Chloroform-*d*) δ 8.16 (dd, *J* = 5.3, 8.8 Hz, 2H), 7.18 (t, *J* = 8.7 Hz, 2H), 6.67 (br d, *J* = 12.0 Hz, 1H), 6.24 (s, 1H), 4.24 - 4.15 (m, 1H), 4.12 (br s, 1H), 4.14 - 3.97 (m, 5H), 3.86 - 3.74 (m, 6H), 3.68 - 3.61 (m, 4H), 3.68 - 3.58 (m, 1H), 3.36 - 3.17 (m, 4H), 2.81 (br s, 1H), 2.90 - 2.76 (m, 1H), 2.57 (br s, 2H). MS (ESI) *m/z* $(M+H)^+$ = 628.1.<br>HPLC retention time: 3.305 min.<br>Separation conditions: chromatographic column: Xtimate C18 2.1 * 30 mm, 3 μm; column temperature: 50°C; mobile phase: water (1.5 mL/4 L trifluoroacetic acid)-acetonitrile; acetonitrile: 10% to 80% 6 min, 80% 0.5 min; flow rate: 0.8 mL/min. |
| A19 | 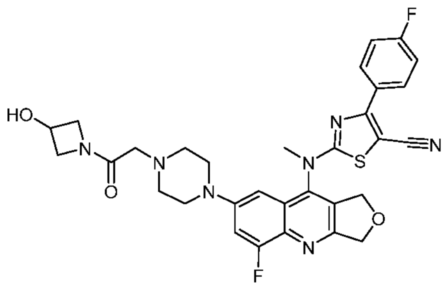 | $^1$H NMR (400 MHz, CHLOROFORM-*d*) δ 8.11 (br dd, *J* = 5.3, 8.4 Hz, 2H), 7.23 (br d, *J* = 2.3 Hz, 1H), 7.16 (t, *J* = 8.6 Hz, 2H), 6.71 (d, *J* = 1.9 Hz, 1H), 5.27 - 5.18 (m, 4H), 4.66 (br s, 1H), 4.47 - 4.40 (m, 1H), 4.31 - 4.24 (m, 1H), 4.18 (br d, *J* = 7.0 Hz, 1H), 3.95 (br d, *J* = 10.7 Hz, 1H), 3.63 (s, 3H), 3.55 - 3.41 (m, 5H), 3.28 (br d, *J* = 15.3 Hz, 2H), 3.04 (br s, 3H). MS (ESI) *m/z* $(M+H)^+$ = 618.1.<br>HPLC retention time: 3.585 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| A20 | 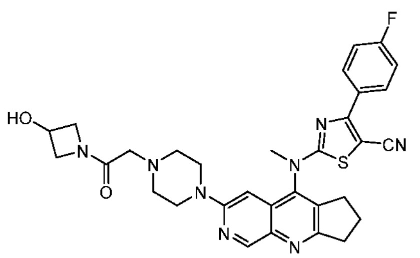 | $^1$H NMR (400 MHz, CHLOROFORM-*d*) δ = 9.08 (s, 1H), 8.13 - 7.99 (m, 2H), 7.09 (t, *J* = 8.6 Hz, 2H), 6.44 (s, 1H), 4.63 - 4.54 (m, 1H), 4.38 (br t, *J* = 7.8 Hz, 1H), 4.25 - 4.16 (m, 1H), 4.08 (br dd, *J* = 3.8, 9.8 Hz, 1H), 3.85 (br d, *J* = 10.7 Hz, 1H), 3.74 - 3.59 (m, 4H), 3.56 (s, 3H), 3.20 - 3.04 (m, 4H), 2.96 - 2.87 (m, 2H), 2.77 (br s, 4H), 2.25 - 2.15 (m, 2H). MS (ESI) *m/z* $(M+1)^+$ = 599.1.<br>HPLC retention time: 3.90 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |

(continued)

| Example | Structural formula | Analytical data |
|---|---|---|
| A21 | 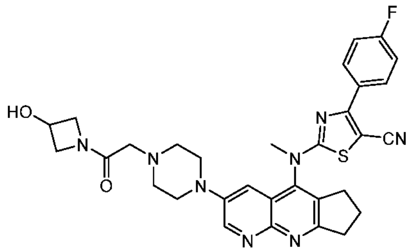 | $^1$H NMR (400 MHz, CHLOROFORM-*d*) δ 8.94 (br d, *J* = 2.2 Hz, 1H), 8.25 - 8.05 (m, 2H), 7.24 - 7.08 (m, 3H), 4.71 (br s, 1H), 4.47 (br d, *J* = 7.9 Hz, 1H), 4.37 - 4.25 (m, 1H), 4.20 - 4.06 (m, 1H), 3.93 (br d, *J* = 9.9 Hz, 1H), 3.65 (s, 4H), 3.47 - 3.21 (m, 4H), 3.29 - 3.23 (m, 2H), 3.17 - 3.08 (m, 2H), 3.02 - 2.95 (m, 2H), 2.75 (br s, 4H), 2.28 (quin, *J* = 7.3 Hz, 2H). MS (ESI) *m*/*z* $(M+H)^+$ = 599.1.<br>HPLC retention time: 6.73 min.<br>Separation conditions: chromatographic column: WELCH Ultimate LP-C18 150 * 4.6 mm 5 μm; column temperature: 40°C; mobile phase: water (2.75 mL/4 L trifluoroacetic acid)-acetonitrile (2.5 mL/4 L trifluoroacetic acid); acetonitrile: 10% to 80% 10 min, 80% 5 min; flow rate: 1.5 mL/min. |
| A23 | 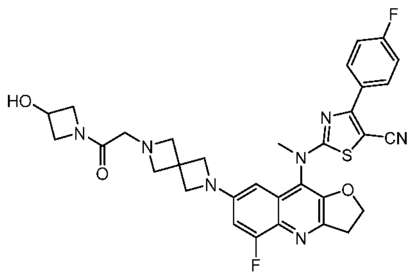 | $^1$H NMR (400 MHz, CHLOROFORM-*d*) δ 8.15 (br dd, *J* = 5.5, 8.5 Hz, 2H), 7.17 (t, *J* = 8.8 Hz, 2H), 6.54 (br d, *J* = 12.3 Hz, 1H), 6.10 (s, 1H), 4.83 (brt, *J* = 8.3 Hz, 2H), 4.67 (br s, 1H), 4.40 - 4.31 (m, 1H), 4.30 - 4.22 (m, 1H), 4.11 (br s, 1H), 4.04 (s, 4H), 3.87 (br d, *J* = 11.0 Hz, 1H), 3.63 - 3.51 (m, 9H), 3.20 - 3.05 (m, 2H). MS (ESI) *m*/*z* $(M+H)^+$ = 630.1.<br>HPLC retention time: 3.90 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| A24 | 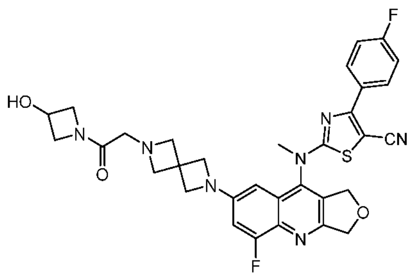 | $^1$H NMR (400 MHz, CHLOROFORM-*d*) δ 8.19 - 8.07 (m, 2H), 7.17 (brt, *J* = 8.5 Hz, 2H), 6.73 (br d, *J* = 11.8 Hz, 1H), 6.17 (s, 1H), 5.24 - 5.12 (m, 4H), 4.67 (br s, 1H), 4.34 (br t, *J* = 7.8 Hz, 1H), 4.29 - 4.21 (m, 1H), 4.08 (s, 4H), 4.01 (br d, *J* = 5.5 Hz, 1H), 3.88 (br d, *J* = 10.3 Hz, 1H), 3.67 (s, 4H), 3.61 (s, 3H), 3.24 - 3.10 (m, 2H). MS (ESI) *m*/*z* $(M+H)^+$ = 630.1.<br>HPLC retention time: 3.74 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| A25 | 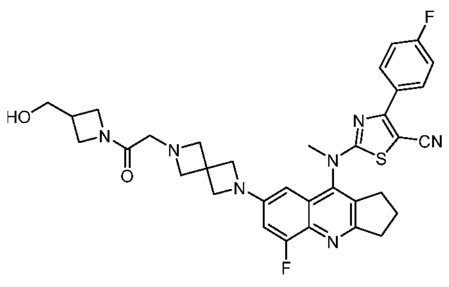 | $^1$H NMR (400 MHz, CHLOROFORM-*d*) *δ* = 8.17 (br s, 2H), 7.18 (br t, *J* = 8.5 Hz, 2H), 6.68 (br d, *J* = 12.0 Hz, 1H), 6.15 (s, 1H), 4.25 - 4.15 (m, 1H), 4.11 - 3.96 (m, 6H), 3.86 - 3.67 (m, 3H), 3.72 (s, 4H), 3.62 (s, 3H), 3.30 - 3.10 (m, 4H), 2.99 (br s, 2H), 2.82 (br d, *J* = 6.0 Hz, 1H), 2.29 - 2.16 (m, 2H). MS (ESI) *m*/*z* $(M+H)^+$ = 642.1.<br>HPLC retention time: 4.031 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| A26 | 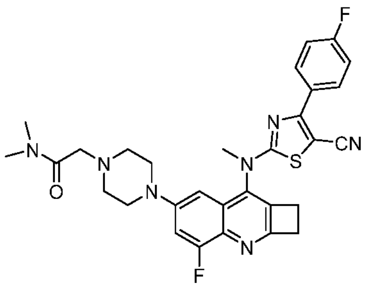 | $^1$H NMR (400 MHz, DMSO-*d6*) δ 8.05 - 7.87 (m, 2H), 7.44 (d, *J* = 14.2, 2.5 Hz, 1H), 7.33 (t, *J* = 8.8 Hz, 2H), 6.73 (d, *J* = 2.4 Hz, 1H), 3.56 (s, 3H), 3.52 - 3.42 (m, 2H), 3.20 (t, *J* = 4.8 Hz, 4H), 3.17 - 3.13 (m, 2H), 3.11 (s, 2H), 2.94 (s, 3H), 2.73 (s, 3H), 2.50 (t, *J* = 5.0 Hz, 4H). MS (ESI) *m/z* (M+H)$^+$ = 574.0.<br>HPLC purity: 98.8%; retention time: 6.582 min.<br>Separation conditions: chromatographic column: Waters XBridge 4.6 * 100 mm, 3.5 μm; column temperature: 40°C; mobile phase: water (10 mM $NH_4HCO_3$)-acetonitrile; acetonitrile: 5% to 95% 7 min; 95% 8 min; flow rate: 1.2 mL/min. |
| A27 | 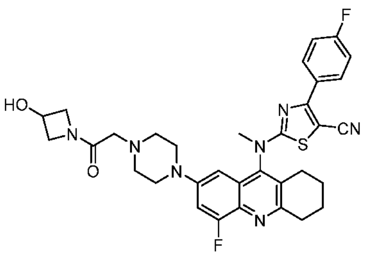 | $^1$H NMR (400 MHz, DMSO-*d6*) δ 8.05 (s, 2H), 7.47 (d, *J* = 13.9 Hz, 1H), 7.34 (s, 2H), 6.49 (d, *J* = 2.5 Hz, 1H), 5.61 (d, *J* = 6.1, 1.9 Hz, 1H), 4.40 - 4.31 (m, 1H), 4.27 (t, *J* = 8.1 Hz, 1H), 3.96 (t, *J* = 10.1, 6.8 Hz, 1H), 3.83 (dd, *J* = 9.3, 4.2 Hz, 1H), 3.50 (d, *J* = 15.3 Hz, 4H), 3.19 (q, *J* = 5.8 Hz, 4H), 3.02 - 2.89 (m, 4H), 2.65 - 2.55 (m, 2H), 2.47 (s, 4H), 1.90 - 1.64 (m, 4H). MS (ESI) *m/z* (M+H)$^+$ = 630.0.<br>HPLC purity: 98.8%; retention time: 6.224 min.<br>Separation conditions: chromatographic column: Waters XBridge 4.6 * 100 mm, 3.5 μm; column temperature: 40°C; mobile phase: water (10 mM $NH_4HCO_3$)-acetonitrile; acetonitrile: 5% to 95% 7 min; 95% 8 min; flow rate: 1.2 mL/min. |
| A28 | 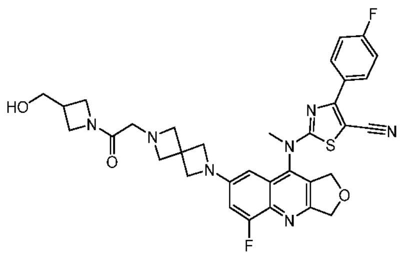 | $^1$H NMR (400 MHz, CHLOROFORM-*d*) δ = 8.13 (br dd, *J* = 5.3, 8.3 Hz, 2H), 7.17 (br t, *J* = 8.7 Hz, 2H), 6.73 (br d, *J* = 11.5 Hz, 1H), 6.17 (s, 1H), 5.22 - 5.07 (m, 4H), 4.35 - 4.12 (m, 2H), 4.12 - 4.05 (m, 4H), 4.00 - 3.94 (m, 1H), 3.87 - 3.80 (m, 1H), 3.80 - 3.74 (m, 2H), 3.69 (s, 4H), 3.60 (s, 3H), 3.27 - 3.08 (m, 2H), 2.81 (br s, 1H). MS (ESI) *m/z* (M+H)$^+$ = 644.2.<br>HPLC retention time: 3.83 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| A29 | 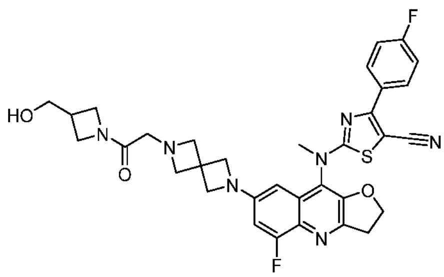 | $^1$H NMR (400 MHz, DMSO-*d6*) δ = 8.06 (br s, 2H), 7.41 (br t, *J* = 8.7 Hz, 2H), 6.81 - 6.69 (m, 1H), 6.11 (br d, *J* = 2.3 Hz, 1H), 4.82 (br t, *J* = 7.8 Hz, 2H), 4.12 - 3.88 (m, 6H), 3.83 - 3.75 (m, 1H), 3.83 - 3.75 (m, 1H), 3.83 - 3.71 (m, 1H), 3.55 (br d, *J* = 1.5 Hz, 6H), 3.47 (br s, 1H), 3.33 (br s, 8H), 2.97 (s, 1H), 2.66 - 2.54 (m, 1H). MS (ESI) *m/z* (M+H)$^+$ = 644.1.<br>HPLC retention time: 2.972 min.<br>Separation conditions: chromatographic column: Boston Green ODS 150 * 30 mm * 5 μm; column temperature: 40°C; mobile phase: water (2.75 mL/4 L trifluoroacetic acid)-acetonitrile (2.5 mL/4 L trifluoroacetic acid); acetonitrile: 10% to 80% 7 min, 80% 2 min; flow rate: 1.2 mL/min. |

(continued)

| Example | Structural formula | Analytical data |
|---|---|---|
| A30 | 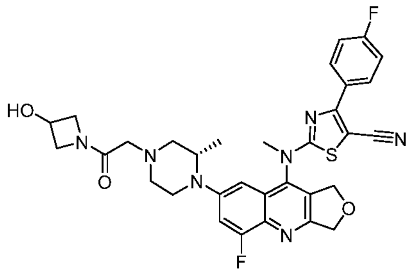 | $^1$H NMR (400 MHz, CHLOROFORM-*d*) δ = 8.20 - 8.06 (m, 2H), 7.23 (s, 1H), 7.17 (t, *J* = 8.5 Hz, 2H), 6.64 (s, 1H), 5.24 - 5.16 (m, 4H), 4.70 (br s, 1H), 4.54 - 4.45 (m, 1H), 4.35 - 4.26 (m, 1H), 4.19 - 4.11 (m, 1H), 4.05 (br s, 1H), 3.95 - 3.88 (m, 1H), 3.63 (br d, *J* = 5.0 Hz, 3H), 3.35 (br d, *J* = 11.8 Hz, 1H), 3.28 - 3.19 (m, 1H), 3.16 - 3.04 (m, 2H), 3.00 (br d, *J* = 10.5 Hz, 1H), 2.89 - 2.78 (m, 1H), 2.58 (br d, *J* = 10.0 Hz, 1H), 2.51 - 2.32 (m, 2H), 1.24 - 1.15 (m, 3H). MS (ESI) *m*/*z* (M+H)$^+$ = 632.1.<br>HPLC retention time: 3.84 min.<br>Separation conditions: chromatographic column: Boston Green ODS 150 * 30 mm * 5 μm; column temperature: 40°C; mobile phase: water (2.75 mL/4 L trifluoroacetic acid)-acetonitrile (2.5 mL/4 L trifluoroacetic acid); acetonitrile: 10% to 80% 7 min, 80% 2 min; flow rate: 1.2 mL/min. |
| A31 | 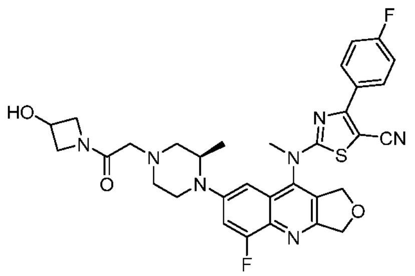 | $^1$H NMR (400 MHz, CHLOROFORM-*d*) δ = 8.19 - 8.04 (m, 2H), 7.23 (s, 1H), 7.17 (t, *J* = 8.5 Hz, 2H), 6.62 (s, 1H), 5.21 (br dd, *J* = 3.9, 9.4 Hz, 4H), 4.70 (br s, 1H), 4.55 - 4.43 (m, 1H), 4.35 - 4.25 (m, 1H), 4.19 - 4.10 (m, 1H), 4.05 (br s, 1H), 3.90 (br d, *J* = 10.5 Hz, 1H), 3.63 (br d, *J* = 4.8 Hz, 3H), 3.40 - 3.30 (m, 1H), 3.27 - 3.16 (m, 1H), 3.14 - 3.02 (m, 2H), 2.96 (br s, 1H), 2.84 - 2.74 (m, 1H), 2.54 (br s, 1H), 2.34 (br s, 1H), 1.19 (td, *J* = 5.5, 17.6 Hz, 3H). MS (ESI) *m*/*z* (M+H)$^+$ = 632.1.<br>HPLC retention time: 3.818 min.<br>Separation conditions: chromatographic column: Xbridge Shield RP-18, 5 μm, 2.1 * 50 mm; column temperature: 40°C; mobile phase: water (2 mL/4 L ammonia water)-acetonitrile; acetonitrile: 10% to 80% 6 min, 80% 2 min; flow rate: 1.2 mL/min. |
| A32 | 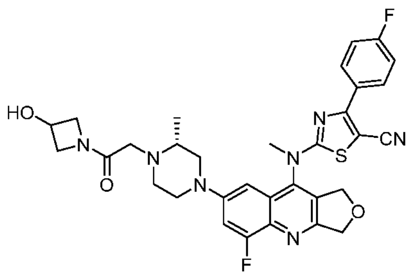 | $^1$H NMR (400 MHz, CHLOROFORM-*d*) δ = 8.16 - 8.06 (m, 2H), 7.23 (s, 1H), 7.17 (t, *J* = 8.5 Hz, 2H), 6.66 (s, 1H), 5.28 - 5.13 (m, 4H), 4.68 (d, *J* = 4.0 Hz, 1H), 4.52 - 4.41 (m, 1H), 4.33 - 4.23 (m, 1H), 4.20 - 4.06 (m, 1H), 3.90 (d, *J* = 10.5 Hz, 1H), 3.63 (s, 3H), 3.55 - 3.40 (m, 3H), 3.12 (s, 1H), 2.99 (d, *J* = 13.1 Hz, 2H), 2.81 (d, *J* = 6.0 Hz, 2H), 2.64 (s, 1H), 1.16 (s, 3H). MS (ESI) *m*/*z* (M+H)$^+$ = 632.0.<br>HPLC retention time: 3.81 min.<br>Separation conditions: chromatographic column: Boston Green ODS 150 * 30 mm * 5 μm; column temperature: 40°C; mobile phase: water (2.75 mL/4 L trifluoroacetic acid)-acetonitrile (2.5 mL/4 L trifluoroacetic acid); acetonitrile: 10% to 80% 7 min, 80% 2 min; flow rate: 1.2 mL/min. |

(continued)

| Example | Structural formula | Analytical data |
|---|---|---|
| A33 | 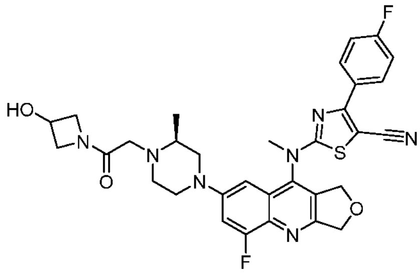 | $^1$H NMR (400 MHz, CHLOROFORM-*d*) δ = 8.12 (dd, *J* = 5.4, 8.4 Hz, 2H), 7.26 - 7.22 (m, 1H), 7.17 (t, *J* = 8.6 Hz, 2H), 6.66 (s, 1H), 5.26 - 5.16 (m, 4H), 4.69 (s, 1H), 4.54 - 4.41 (m, 1H), 4.34 - 4.25 (m, 1H), 4.21 - 4.06 (m, 1H), 3.91 (d, *J* = 10.6 Hz, 1H), 3.63 (s, 3H), 3.57 - 3.43 (m, 3H), 3.21 - 2.58 (m, 6H), 2.37 (s, 1H), 1.18 (s, 3H). MS (ESI) *m/z* $(M+H)^+$ = 632.3.<br>HPLC retention time: 3.81 min.<br>Separation conditions: chromatographic column: Boston Green ODS 150 * 30 mm * 5 μm; column temperature: 40°C; mobile phase: water (2.75 mL/4 L trifluoroacetic acid)-acetonitrile (2.5 mL/4 L trifluoroacetic acid); acetonitrile: 10% to 80% 7 min, 80% 2 min; flow rate: 1.2 mL/min. |
| A34 | 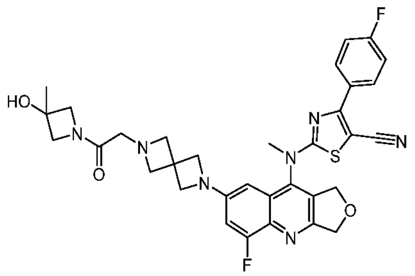 | $^1$H NMR (400 MHz, CHLOROFORM-*d*) δ = 8.13 (br dd, *J* = 5.5, 8.1 Hz, 2H), 7.17 (br t, *J* = 8.6 Hz, 2H), 6.78 - 6.68 (m, 1H), 6.17 (d, *J* = 1.5 Hz, 1H), 5.22 - 5.15 (m, 4H), 4.29 - 4.14 (m, 1H), 4.08 (s, 4H), 4.00 (br d, *J* = 9.1 Hz, 1H), 3.96 - 3.84 (m, 2H), 3.75 - 3.54 (m, 1H), 3.70 - 3.52 (m, 6H), 3.18 (br d, *J* = 19.7 Hz, 2H), 1.54 (s,3H). MS (ESI) *m/z* $(M+H)^+$ = 644.1.<br>HPLC retention time: 3.88 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| A35 | 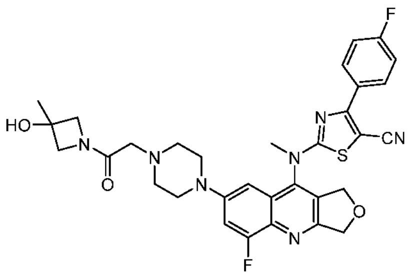 | $^1$H NMR (400 MHz, CHLOROFORM-*d*) δ = 8.12 (dd, *J* = 5.4, 8.6 Hz, 2H), 7.23 (br d, *J* = 2.3 Hz, 1H), 7.17 (t, *J* = 8.6 Hz, 2H), 6.71 (d, *J* = 2.0 Hz, 1H), 5.26 - 5.19 (m, 4H), 4.24 (br d, *J* = 9.2 Hz, 1H), 4.10 (d, *J* = 9.1 Hz, 1H), 4.03 - 3.94 (m, 2H), 3.63 (s, 3H), 3.46 (br s, 4H), 3.36 (br d, *J* = 12.3 Hz, 1H), 3.24 - 3.18 (m, 1H), 2.97 (br d, *J* = 11.7 Hz, 4H), 2.73 (br s, 1H), 1.55 (s, 3H). MS (ESI) *m/z* $(M+H)^+$ = 632.3.<br>HPLC retention time: 3.73 min.<br>Separation conditions: chromatographic column: Xbridge Shield RP-18, 5 μm, 2.1 * 50 mm; column temperature: 40°C; mobile phase: water (2 mL/4 L ammonia water)-acetonitrile; acetonitrile: 10% to 80% 6 min, 80% 2 min; flow rate: 1.2 mL/min. |
| A37 | 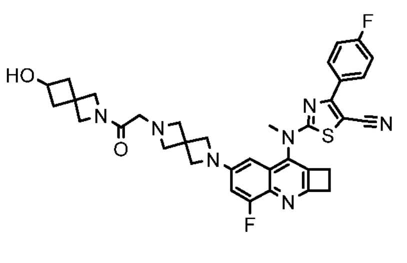 | $^1$H NMR (400 MHz, $CDCl_3$) δ 8.20 - 8.10 (m, 2H), 7.17 (t, *J* = 8.5 Hz, 2H), 6.67 (d, *J* = 10.8 Hz, 1H), 6.22 (s, 1H), 4.24 (dd, *J* = 15.9, 9.5 Hz, 1H), 4.04 (dd, *J* = 35.4, 16.3 Hz, 9H), 3.66 - 3.56 (m, 9H), 3.22 (s, 2H), 3.08 (s, 2H), 2.56 (s, 2H), 2.11 (d, *J* = 9.2 Hz, 2H). MS (ESI): *m/z* $[M+H]^+$ = 654.3.<br>HPLC retention time: 8.36 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |

(continued)

| Example | Structural formula | Analytical data |
|---|---|---|
| A38 | 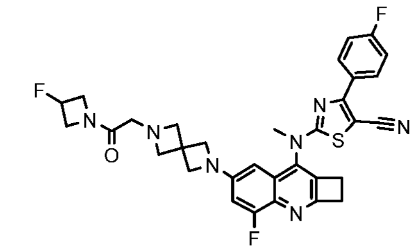 | $^1$H NMR (400 MHz, $CDCl_3$) δ 8.15 (dd, *J* = 8.8, 5.4 Hz, 2H), 7.17 (t, *J* = 8.7 Hz, 2H), 6.67 (dd, *J* = 11.8,2.3 Hz, 1H), 6.23 (d, *J* = 1.9 Hz, 1H), 5.46 - 5.17 (m, 1H), 4.41 (dd, *J* = 19.3, 5.6 Hz, 1H), 4.26 (dd, *J* = 21.7, 10.6 Hz, 2H), 4.12 (dd, *J* = 24.8, 12.7 Hz, 1H), 4.04 (s, 4H), 3.69 - 3.58 (m, 9H), 3.26 - 3.20 (m, 2H), 3.17 (s, 2H). MS (ESI) *m/z* $(M+H)^+$ = 616.4. HPLC retention time: 8.72 min. Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |

**Synthesis of A20 intermediate 5-amino-2-chloroisonicotinonitrile**

To a solution of 3-amino-4-cyanopyridine (1.5 g, 12.59 mmol) in *N,N*-dimethylformamide (15 mL) was added N-chlorosuccinimide (1.68 g, 12.59 mmol) under argon atmosphere, and the reaction mixture was stirred at room temperature (20°C) for 18 hours. After the reaction was completed, the system was poured into water (20 mL) and extracted with ethyl acetate (50 mL × 3). The organic phases were combined, dried over anhydrous sodium sulfate, filtered, and the filtrate was concentrated to obtain a crude product. The crude product was purified by medium-pressure column chromatography (ethyl acetate/petroleum ether (v/v) = 0 to 50%) to obtain compound 5-amino-2-chloroisonicotinonitrile. MS (ESI) *m/z* $(M+1)^+$ = 153.8.

**Synthesis of example A22**

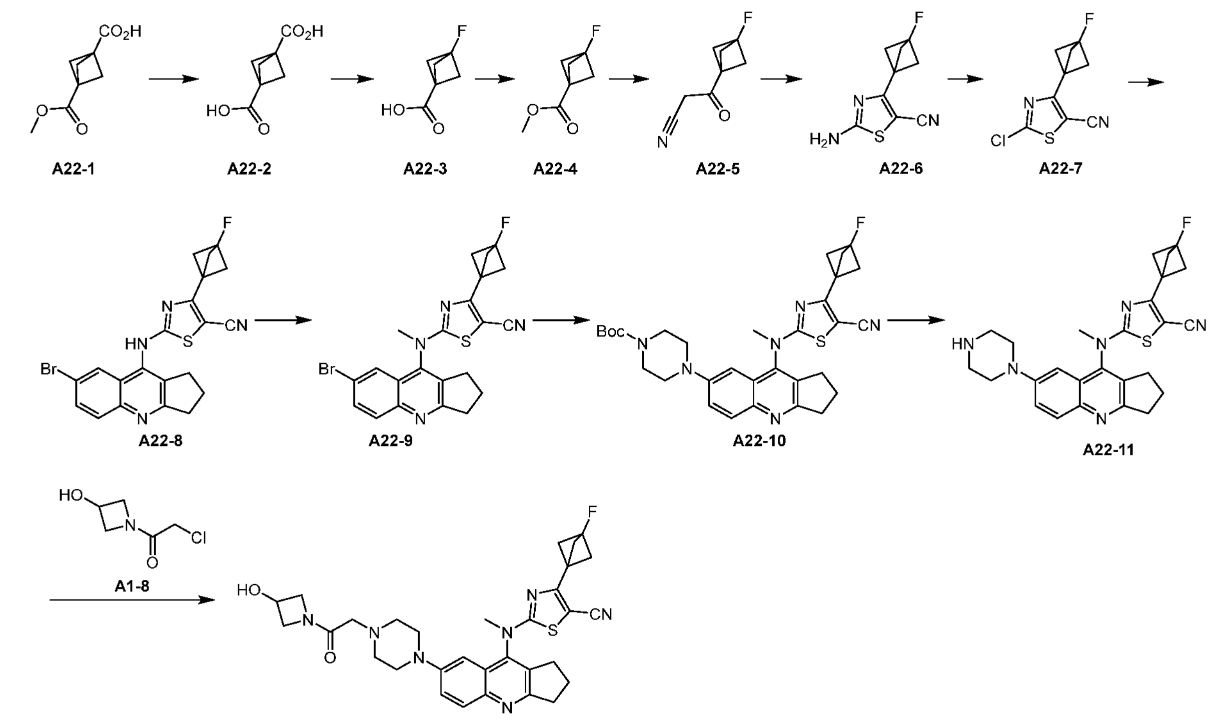
A22-1 A22-2 A22-3 A22-4 A22-5 A22-6 A22-7
A22-8 A22-9 A22-10 A22-11
A1-8
A22

**Step 1:** Preparation of compound **A22-2**

To a solution of compound bicyclo[1.1.1]pentane-1,3-dicarboxylic acid and 1-methyl ester (2 g, 11.75 mmol) in water (20 mL) was added sodium hydroxide (611.14 mg, 15.28 mmol). The reaction mixture was heated to 100°C and stirred for 1 hour. The reaction mixture was washed with *tert*-butyl methyl ether (5 mL), then added with concentrated hydrochloric acid to adjust the pH to 3, and extracted with ethyl acetate (50 mL × 6). The organic phases were combined, dried over anhydrous sodium sulfate, and filtered. The filtrate was concentrated to obtain **A22-2,** which was directly used in the next reaction step without further purification.

$^1$H NMR (400 MHz, DEUTERIUM OXIDE) δ = 2.15 (s, 1H).

**Step 2:** Preparation of compound **A22-3**

**A22-2** (1.4 g, 8.97 mmol) was dissolved in water (15 mL), and selectfluor (7.94 g, 22.42 mmol) and silver nitrate (152.31 mg, 896.66 μmol) were added thereto. After the addition was completed, the system was heated to 65°C and stirred for 16 hours. The system was filtered and extracted with *tert*-butyl methyl ether (3 × 30 mL). The organic phases were combined, dried over anhydrous sodium sulfate, and filtered. The filtrate was concentrated to obtain compound **A22-3,** which was directly used in the next reaction step without further purification.

$^1$H NMR (400 MHz, CHLOROFORM-*d*) δ = 2.42 (d, *J* = 2.3 Hz, 1H); $^{19}$F NMR (376 MHz, CHLOROFORM-*d*) δ = -149.78 (s, 1F).

**Step 3:** Preparation of compound **A22-4**

To a solution of **A22-3** (800 mg, 6.15 mmol) in anhydrous dichloromethane (25 mL) and methanol (1 mL) was added (trimethylsilyl)diazomethane (2 M, 4.61 mL), and the reaction mixture was stirred at room temperature (25°C) for 24 hours. After the reaction was completed, the reaction mixture was concentrated under reduced pressure to obtain compound **A22-4,** which was directly used in the next reaction step without further purification.

**Step 4:** Preparation of compound **A22-5**

To a solution of *n*-butyllithium (2.5 M, 2.78 mL) in tetrahydrofuran (10 mL) was added acetonitrile (284.80 mg, 6.94 mmol) at -70°C. After the addition was completed, the system was stirred at -30°C for 0.5 hours. To the system was added dropwise **A22-4** (0.5 g, 3.47 mmol). After the dropwise addition was completed, the system was stirred at room temperature (25°C) for 4 hours. The system was added with saturated ammonium chloride aqueous solution (1 mL) to quench, and extracted with ethyl acetate (3 × 30 mL). The organic phases were combined, dried over anhydrous sodium sulfate, and filtered. The filtrate was concentrated to obtain compound **A22-5,** which was directly used in the next reaction step without further purification.

$^1$H NMR (400 MHz, CHLOROFORM-*d*) δ = 3.56 (s, 2H), 2.50 (d, *J* = 2.3 Hz, 6H).

**Step 5:** Preparation of compound **A22-6**

To a solution of **A22-5** (500 mg, 3.26 mmol) in ethanol (10 mL) was added pyridine (258.24 mg, 3.26 mmol). The reaction mixture was stirred at 70°C for 15 minutes, and then cooled to room temperature (25°C). A solution of iodine (828.61 mg, 3.26 mmol, 657.63 μL) and thiourea (497.02 mg, 6.53 mmol) in ethanol (3 mL) was slowly added thereto. After the addition was completed, the reaction system was stirred at room temperature (25°C) for 1 hour. The system was added with saturated ammonium chloride aqueous solution (1 mL) and saturated sodium thiosulfate (1 mL) to quench. The system was concentrated to remove most of the solvent, then diluted with water (10 mL), and extracted with ethyl acetate (3 × 30 mL). The organic phases were combined, dried over anhydrous sodium sulfate, filtered, and the filtrate was concentrated to obtain a crude product. The crude product was purified by medium-pressure column chromatography (ethyl acetate/petroleum ether (v/v) = 0 to 60%) to obtain **A22-6.**

$^1$H NMR (400 MHz, CHLOROFORM-*d*) δ = 5.55 (br s, 2H), 2.56 (d, *J* = 2.4 Hz, 6H); $^{19}$F NMR (376 MHz, CHLOROFORM-*d*) δ = -148.50 (s, 1F). MS (ESI) *m/z* $(M+1)^+$ = 209.9.

**Step 6:** Preparation of compound **A22-7**

To a solution of **A22-6** (0.12 g, 573.50 μmol) in acetonitrile (5 mL) were added *tert*-butyl nitrite (88.71 mg, 860.24 μmol) and copper chloride (92.53 mg, 688.20 μmol), and the reaction mixture was stirred at room temperature (20°C) for 1 hour. The reaction mixture was added with 1 *N* HCl (1 mL) to quench, diluted with water (10 mL), and extracted with ethyl acetate (20 mL × 3). The organic phases were combined, washed with saturated brine, dried over

anhydrous sodium sulfate, and filtered. The filtrate was concentrated to obtain a crude product, which was purified by medium-pressure column chromatography (ethyl acetate/petroleum ether (v/v) = 0 to 10%) to obtain **A22-7.**

**Steps 7 to 10: Preparation of compound A22**

Similar to the synthesis of example **A1,** A22 was synthesized.

$^1$H NMR (400 MHz, CHLOROFORM-*d*) δ = 7.19 (dd, *J* = 2.4, 13.2 Hz, 1H), 6.63 (d, *J* = 1.8 Hz, 1H), 4.73 (br s, 1H), 4.54 - 4.43 (m, 1H), 4.33 (dd, *J* = 6.6, 11.0 Hz, 1H), 4.14 (br d, *J* = 9.8 Hz, 1H), 3.94 (dd, *J* = 4.1, 10.8 Hz, 1H), 3.56 (s, 3 H), 3.37 - 3.29 (m, 4H), 3.25 (dt, *J* = 2.2, 7.6 Hz, 2H), 3.13 (s, 2H), 2.96 (t, *J* = 7.3 Hz, 2H), 2.72 (br d, *J* = 4.1 Hz, 4H), 2.59 (d, *J* = 2.4 Hz, 6H), 2.39 (br s, 1H), 2.25 (quin, *J* = 7.6 Hz, 2H).

MS (ESI) *m/z* $(M+1)^+$ = 606.3.

HPLC retention time: 3.56 min.

Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min.

**Synthesis of example A36**

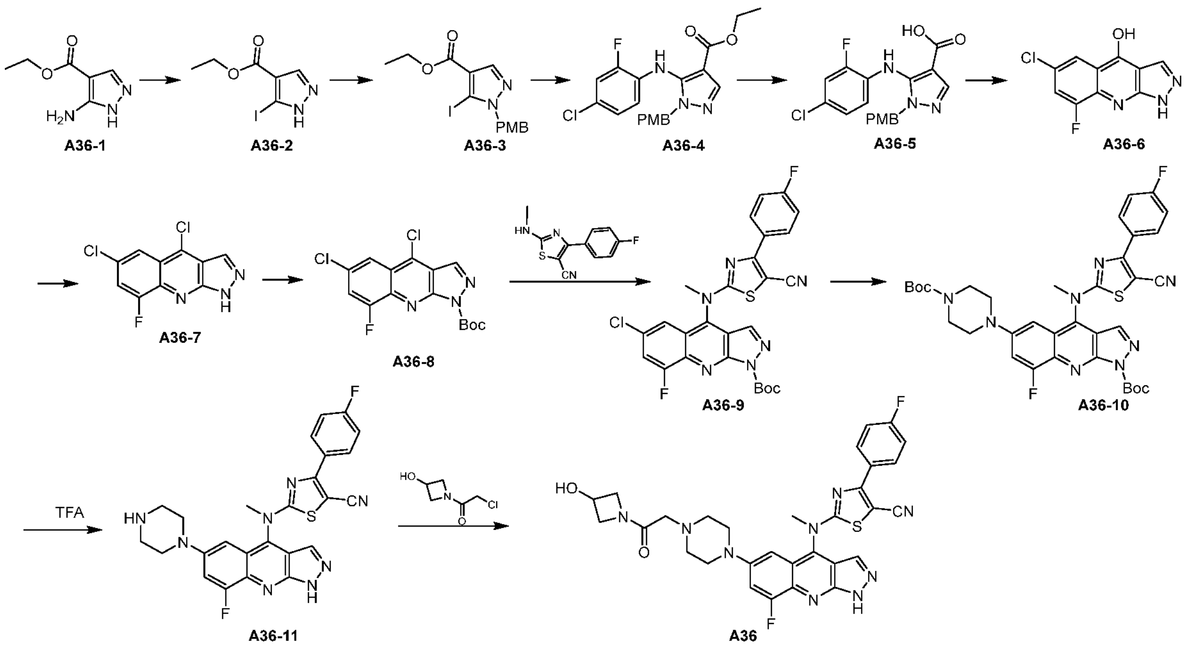


A36-1, A36-2, A36-3, A36-4, A36-5, A36-6, A36-7, A36-8, A36-9, A36-10, A36-11, A36

**Step 1:** Preparation of compound **A36-2**

Compound **A36-1** (10 g, 64.45 mmol) was dissolved in concentrated hydrochloric acid (50 mL), and sodium nitrite (8.89 g, 128.90 mmol) was added thereto at 0°C. The reaction mixture was stirred at the same temperature for 10 minutes, then added with potassium iodide (26.75 g, 161.13 mmol), and stirred at 0°C for another 1 hour. After the reaction was completed, the reaction mixture was added with saturated sodium bicarbonate aqueous solution (150 mL) and saturated sodium thiosulfate (50 mL) to quench, extracted with ethyl acetate (150 mL × 3), and washed with saturated brine (150 mL × 3). The organic phases were combined, dried over anhydrous sodium sulfate, filtered, and concentrated to obtain a crude product, which was purified by silica gel column chromatography (ethyl acetate/petroleum ether = 25%) to obtain 7.52 g of compound **A36-2** with a yield of 42%. MS (ESI) *m/z* $(M+H)^+$ = 266.8.

**Step 2:** Preparation of compound **A36-3**

Compound **A36-2** (5.5 g, 20.67 mmol) and *p*-methoxybenzyl chloride (4.86 g, 31.01 mmol, 4.22 mL) were dissolved in anhydrous *N,N*-dimethylformamide (50 mL), then potassium carbonate (8.57 g, 62.02 mmol) was added thereto at 0°C, and the reaction mixture was stirred at room temperature (25°C) for 18 hours. After the reaction was

completed, the reaction mixture was diluted with water (100 mL), extracted with ethyl acetate (100 mL × 3), and washed with saturated brine (100 mL × 3). The organic phases were combined, dried over anhydrous sodium sulfate, filtered, and concentrated to obtain a crude product, which was purified by silica gel column chromatography (ethyl acetate/petroleum ether = 10%) to obtain 5.9 g of compound **A36-3** with a yield of 67%. MS (ESI) *m/z* $(M+H)^+$ = 386.9.

**Step 3:** Preparation of compound **A36-4**

To a 100 mL single-necked flask were sequentially added compound **A36-3** (3.9 g, 10.10 mmol), 4-chloro-2-fluoroaniline (2.21 g, 15.15 mmol), and 1,4-dioxane (40 mL). The system was replaced with argon three times, then tris(dibenzylideneacetone)dipalladium (924.77 mg, 1.01 mmol), 4,5-bis(diphenylphosphino)-9,9-dimethylxanthene (1.17 g, 2.02 mmol), and cesium carbonate (6.58 g, 20.20 mmol) were sequentially added thereto under argon atmosphere, and the reaction was carried out at 100°C for 2 hours. After the reaction was completed, the reaction mixture was cooled to room temperature (25°C) and then filtered through diatomite. The filtrate was added with water (50 mL), extracted with ethyl acetate (50 mL × 3), and washed with saturated brine. The organic phases were combined, dried over anhydrous sodium sulfate, filtered, and concentrated to obtain a crude product, which was purified by silica gel column chromatography (ethyl acetate/petroleum ether = 5%) to obtain 2.90 g of compound **A36-4** with a yield of 71%.

$^1H$ NMR (400 MHz, $CDCl_3$) δ = 8.38 - 8.28 (m, 2H), 7.59 (s, 1H), 7.46 - 7.39 (m, 1H), 7.26 - 7.23 (m, 1H), 7.14 - 7.05 (m, 2H), 6.95 - 6.86 (m, 2H), 5.13 (s, 2H), 4.29 (q, *J* = 7.2 Hz, 2H), 3.81 (s, 3H), 1.34 (t, *J* = 7.2 Hz, 3H).

**Step 4:** Preparation of compound **A36-5**

Compound **A36-4** (2.8 g, 6.93 mmol) was dissolved in a mixed solvent of methanol (10 mL) and tetrahydrofuran (10 mL), and sodium hydroxide aqueous solution (8 *M*, 2.60 mL) was slowly added dropwise thereto at 0°C under nitrogen atmosphere. After the dropwise addition was completed, the reaction mixture was stirred at 70°C for 3 hours. After the reaction was completed, the reaction mixture was diluted with water (20 mL), then added with 1 N hydrochloric acid to adjust the pH to 3 to 5, and extracted with ethyl acetate (30 mL × 3). The organic phases were combined, washed with saturated brine (100 mL), dried over anhydrous sodium sulfate, filtered, and concentrated to obtain crude compound **A36-5,** which was directly used in the next reaction step without purification. MS (ESI) *m/z* $(M+H)^+$ = 375.8.

**Step 5:** Preparation of compound **A36-6**

To a 100 mL single-necked flask were sequentially added crude compound **A36-5** (2.6 g, 6.93 mmol) and Eaton's reagent (50 mL), and the system was stirred at 80°C for 2 hours. After the system was returned to room temperature (25°C), the reaction mixture was slowly added dropwise into water (300 mL), then added with saturated sodium bicarbonate aqueous solution to adjust the pH to 9, and extracted with ethyl acetate (300 mL × 3). The organic phases were combined, dried over sodium sulfate, filtered, and concentrated to obtain a crude product, which was purified by silica gel column chromatography (methanol/ethyl acetate = 10%) to obtain 900 mg of compound **A36-6.** MS (ESI) *m/z* $(M+H)^+$ = 238.0.

**Step 6: Preparation of compound A36-7**

Compound **A36-6** (900 mg, 3.8 mmol) was dissolved in 1,4-dioxane (20 mL), then phosphorus oxychloride (1.41 mL, 15.2 mmol) was slowly added thereto at room temperature (25°C), and the system was reacted at 80°C for 1 hour. After the system was returned to room temperature (25°C), the reaction mixture was slowly poured into ice water (200 mL) and extracted with ethyl acetate (3 × 80 mL). The organic phases were combined, dried over anhydrous sodium sulfate, filtered, and concentrated to obtain crude compound **A36-7,** which was directly used in the next reaction step without purification. MS (ESI) *m/z* $(M+H)^+$ = 256.0.

**Step 7: Preparation of compound A36-8**

Compound **A36-7** (921 mg, 3.60 mmol) was dissolved in tetrahydrofuran (10 mL) solution, then 4-dimethylaminopyridine (43.94 mg, 359.68 μmol), di-*tert*-butyl dicarbonate (1.57 g, 7.19 mmol, 1.65 mL), and triethylamine (1.09 g, 10.79 mmol, 1.50 mL) were added thereto at 0°C. The system was reacted at room temperature (25°C) for 1 hour. The reaction mixture was concentrated, and the crude product was purified by silica gel column chromatography (ethyl acetate/petroleum ether = 10%) to obtain 680 mg of compound **A36-8.**

$^1H$ NMR (400 MHz, CHLOROFORM-*d*) δ = 8.49 (s, 1H), 8.18 (s, 1H), 7.59 (dd, *J* = 2.1, 9.5 Hz, 1H), 1.78 (s, 9H). MS (ESI) *m/z* $(M+H)^+$ = 256.0.

**Steps 8 to 12: Preparation of compound A36**

Similar to the synthesis of example **A1, A36** was synthesized.

$^1H$ NMR (400 MHz, METHANOL-$d_4$) δ = 8.29 (s, 1H), 8.34 - 8.23 (m, 1H), 8.08 (br s, 2H), 7.62 (br d, *J* = 16.3 Hz, 1H), 7.22 (t, *J* = 8.8 Hz, 2H), 6.87 (s, 1H), 4.60 (s, 5H), 4.57 (br s, 1H), 4.51 - 4.45 (m, 1H), 4.25 - 4.18 (m, 1H), 4.08 - 4.02 (m, 1H), 3.89 (s, 3H), 3.77 (br d, *J* = 10.0 Hz, 1H), 3.11 (d, *J* = 3.8 Hz, 2H), 2.67 (br d, *J* = 4.8 Hz, 4H). MS (ESI) *m/z* $(M+H)^+$ = 616.2.

HPLC retention time: 3.51 min.

Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min.

**Synthesis of example B1**

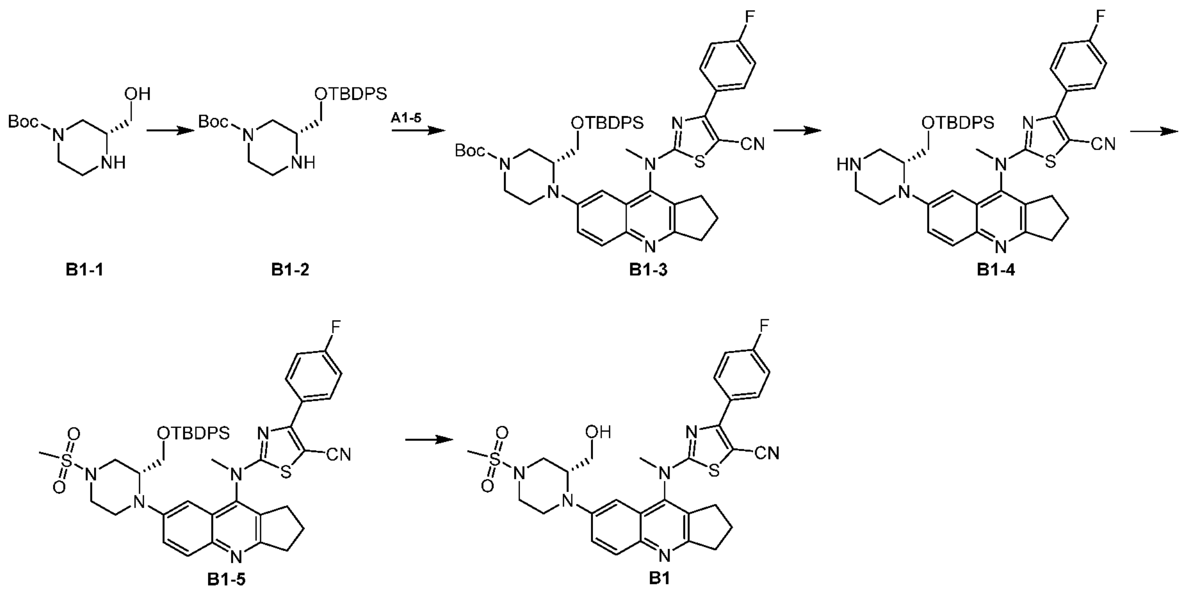


B1-1 B1-2 B1-3 B1-4

B1-5 B1

**Step 1:** Preparation of compound **B1-2**

Compound **B1-1** (1 g, 4.62 mmol) and imidazole (629.54 mg, 9.25 mmol) were dissolved in dichloromethane (20 mL), and *tert*-butyldiphenylchlorosilane (1.40 g, 5.09 mmol) was added dropwise thereto at 0°C. After the dropwise addition was completed, the system was warmed to room temperature (25°C) and stirred for 2 hours. The system was diluted with saturated sodium bicarbonate (25 mL) and extracted with dichloromethane (50 mL × 3). The organic phases were combined, washed with saturated brine (50 mL × 3), dried over anhydrous sodium sulfate, and filtered. The filtrate was concentrated to obtain a crude product, which was purified by silica gel column chromatography (ethyl acetate/petroleum ether (v/v) = 0 to 40%) to obtain compound **B1-2.**

$^1H$ NMR (400 MHz, CHLOROFORM-*d*) δ 7.72 - 7.57 (m, 4H), 7.49 - 7.35 (m, 6H), 3.94 (br s, 2H), 3.67 - 3.50 (m, 2H), 3.01 (br d, *J* = 11.2 Hz, 1H), 2.91 - 2.73 (m, 3H), 2.55 (br t, *J* = 11.2 Hz, 1H), 2.02 (br s, 1H), 1.46 (s, 9H), 1.07 (s, 9H). MS (ESI) *m/z* $(M+H)^+$ = 455.6.

**Step 2:** Preparation of compound **B1-3**

Compound **B1-2** (569.10 mg, 1.25 mmol), compound **A1-5** (300 mg, 625.83 μmol), sodium *tert*-butoxide (120.29 mg, 1.25 mmol), tris(dibenzylideneacetone)dipalladium (34.38 mg, 37.55 μmol), and 2-(di-*tert*-butylphosphino)biphenyl (18.67 mg, 62.58 μmol) were dissolved in toluene (1 mL). The system was heated to 115°C and stirred for 2 hours under nitrogen atmosphere. The system was filtered and the filtrate was concentrated to obtain a crude product. The crude product was purified by medium-pressure column chromatography (ethyl acetate/petroleum ether (v/v) = 0 to 25%) to

obtain compound **B1-3.** MS (ESI) *m*/*z* $(M+H)^+$ = 853.2.

**Step 3:** Preparation of compound **B1-4**

To a solution of compound **B1-3** (100 mg, 117.22 μmol) in dioxane (3 mL) was added a solution of hydrochloride acid in dioxane (4 M, 3 mL), and the reaction mixture was stirred at room temperature (25°C) for 2 hours. After the reaction was completed, the system was added with saturated sodium bicarbonate (20 mL) to quench, and extracted with ethyl acetate (20 mL × 3). The organic phases were combined, washed with saturated brine (20 mL × 3), dried over anhydrous sodium sulfate, and filtered. The filtrate was concentrated to obtain compound **B1-4,** which was directly used in the next reaction step without further purification. MS (ESI) *m*/*z* $(M+H)^+$ = 753.4.

**Step 4:** Preparation of compound **B1-5**

Compound **B1-4** (70 mg, 92.96 μmol) was dissolved in dichloromethane (10 mL), and triethylamine (32.70 mg, 323.19 μmol) and methanesulfonyl chloride (37.32 mg, 325.83 μmol) were added thereto at 0°C. After the addition was completed, the system was stirred at room temperature (25°C) for 1 hour under nitrogen atmosphere. The system was added with saturated sodium bicarbonate (10 mL) to quench at 0°C, and extracted with ethyl acetate (20 mL × 3). The organic phases were combined, washed with saturated brine (20 mL × 3), dried over anhydrous sodium sulfate, and filtered. The filtrate was concentrated to obtain compound **B1-5,** which was directly used in the next reaction step without further purification. MS (ESI) *m*/*z* $(M+H)^+$ = 831.3.

**Step 5:** Preparation of compound **B1**

A solution of compound **B1-5** (80 mg, 96.26 μmol) and tetra-*n*-butylammonium fluoride (1 M, 105.88 μL) in tetrahydrofuran (6 mL) was stirred at room temperature (25°C) for 1 hour under nitrogen atmosphere. After the reaction was completed, the reaction mixture was diluted with water (20 mL) and extracted with ethyl acetate (20 mL × 3). The organic phases were combined, washed with saturated brine (20 mL × 6), dried over anhydrous sodium sulfate, and filtered. The filtrate was concentrated to obtain a crude product, which was purified by preparative high performance liquid chromatography (separation conditions: chromatographic column: Phenomenex Gemini-NX 80 × 30 mm × 3 μm; column temperature: 25°C; mobile phase: water (10 mM ammonium bicarbonate)-acetonitrile; acetonitrile: 37% to 67% 9 min) to obtain compound **B1.**

$^1H$ NMR (400 MHz, DMSO-*d6*) δ 8.05 (br s, 2H), 7.91 (br d, *J* = 9.0 Hz, 1H), 7.62 (br d, *J* = 9.0 Hz, 1H), 7.40 (br s, 2H), 6.92 (br s, 1H), 4.82 (br d, *J* = 19.8 Hz, 1H), 4.07 (br s, 1H), 3.77 - 3.67 (m, 2H), 3.61 (br s, 5H), 3.08 (br d, *J* = 7.5 Hz, 4H), 2.99 (br d, *J* = 10.3 Hz, 2H), 2.91 (br d, *J* = 3.5 Hz, 5H), 2.15 (br d, *J* = 6.8 Hz, 2H). MS (ESI) *m*/*z* $(M+H)^+$ = 593.2. HPLC retention time: 3.90 min.

Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min.

Similar to the synthesis of example **B1,** the following examples **B2** to **B4** were synthesized as shown in Table 2 below.

Table 2: Structural formulas and analytical data of examples **B2** to **B4**

| Example | Structural formula | Analytical data |
|---|---|---|
| B2 | 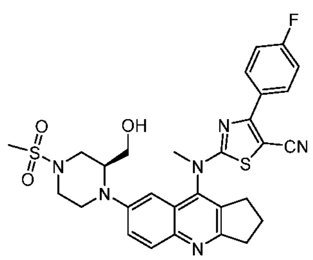 | $^1H$ NMR (400 MHz, DMSO-d6) *δ* 8.05 (br s, 2H), 7.91 (d, *J* = 9.3 Hz, 1H), 7.62 (br d, *J* = 9.0 Hz, 1H), 7.40 (br t, *J* = 8.2 Hz, 2H), 6.94 - 6.89 (m, 1H), 4.86 - 4.77 (m, 1H), 4.07 (br s, 1H), 3.78 - 3.66 (m, 2H), 3.64 - 3.52 (m, 6H), 3.14 - 3.05 (m, 3H), 3.04 - 2.96 (m, 2H), 2.91 (d, *J* = 4.3 Hz, 5H), 2.19 - 2.11 (m, 2H).<br>MS (ESI) *m*/*z* $(M+H)^+$ = 593.2.<br>HPLC retention time: 3.91 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |

(continued)

| Example | Structural formula | Analytical data |
|---|---|---|
| B3 | 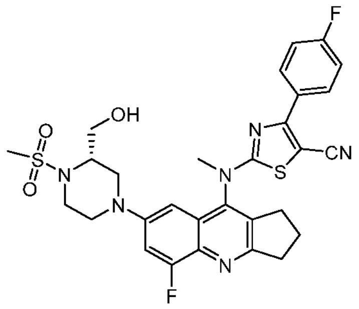 | $^1H$ NMR (400 MHz, CHLOROFORM-*d*) δ 8.23 - 8.11 (m, 2H), 7.25 - 7.10 (m, 3H), 6.70 (s, 1H), 4.14 (br s, 1H), 4.10 - 3.99 (m, 1H), 3.95 - 3.87 (m, 1H), 3.85 - 3.73 (m, 2H), 3.66 (s, 3H), 3.60 (br t, *J* = 12.8 Hz, 1H), 3.52 - 3.41 (m, 1H), 3.28 (dt, *J* = 2.9, 7.7 Hz, 2H), 3.14 (td, *J* = 4.4, 12.9 Hz, 1H), 3.08 - 2.97 (m, 6H), 2.33 - 2.23 (m, 2H), 1.94 (br s, 1H).<br>MS (ESI) *m/z* $(M+H)^+$ = 611.0.<br>HPLC retention time: 4.327 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| B4 | 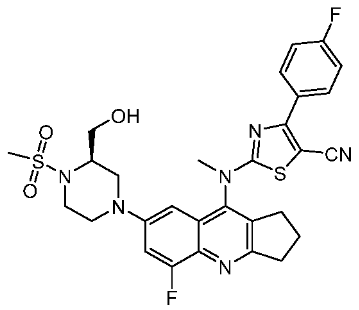 | $^1H$ NMR (400 MHz, CHLOROFORM-*d*) δ 8.15 (br d, *J* = 6.3 Hz, 2H), 7.23 - 7.14 (m, 3H), 6.69 (s, 1H), 4.12 (br s, 1H), 4.07 - 3.98 (m, 1H), 3.88 (br s, 1H), 3.84 - 3.73 (m, 2H), 3.65 (s, 3H), 3.62 - 3.54 (m, 1H), 3.50 - 3.40 (m, 1H), 3.27 (dt, *J* = 3.0, 7.7 Hz, 2H), 3.12 (td, *J* = 4.5, 12.7 Hz, 1H), 3.01 (s, 6H), 2.27 (m, *J* = 7.4 Hz, 2H), 1.91 (br s, 1H). MS (ESI) *m/z* $(M+H)^+$ = 611.0.<br>HPLC retention time: 4.63 min.<br>Separation conditions: chromatographic column: Ultimate C18 3 * 50 mm 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |

**Synthesis of example C1**

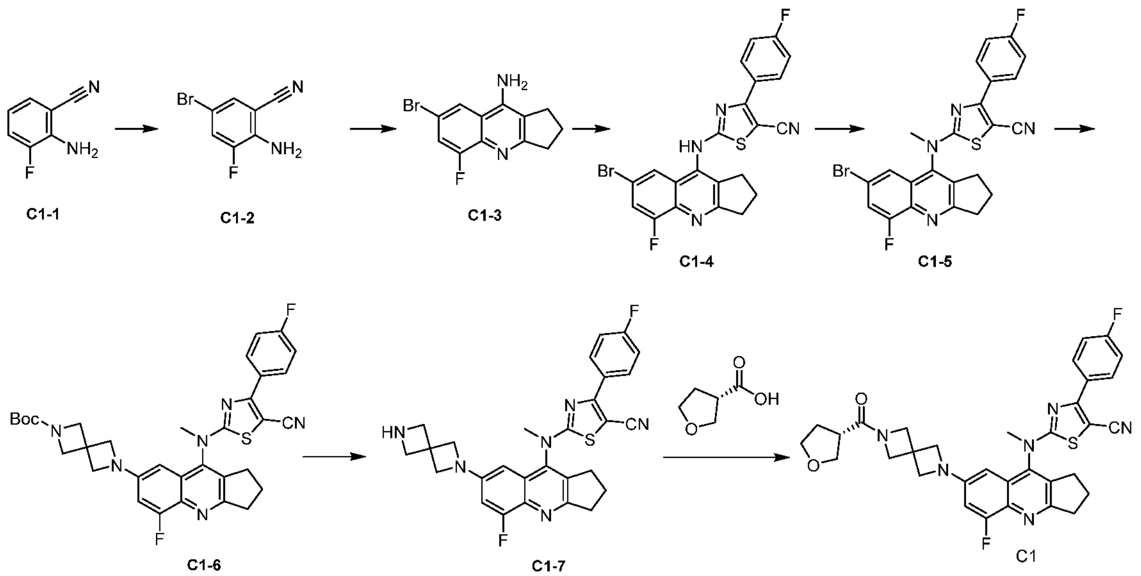

C1-1 C1-2 C1-3 C1-4 C1-5

C1-6 C1-7 C1

**Step 1:** Preparation of compound **C1-2**

To a solution of compound **C1-1** (5 g, 36.73 mmol) in *N,N*-dimethylformamide (100 mL) was added *N*-bromosuccinimide (6.54 g, 36.73 mmol), and the reaction mixture was stirred at room temperature (25°C) for 16 hours. After

the reaction was completed, the system was poured into ice water (500 mL) to precipitate, and filtered to collect a filter cake. The filter cake was dried to obtain crude compound **C1-2,** which was directly used in the next reaction step.

$^1$H NMR (400 MHz, DMSO-d6) δ 7.60 (dd, *J* = 2.2, 11.0 Hz, 1H), 7.53 (s, 1H), 6.41 (s, 2H).

**Step 2:** Preparation of compound **CI-3**

To a solution of compound **C1-2** (5 g, 23.25 mmol) and cyclopentanone (2.15 g, 25.58 mmol, 2.26 mL) in toluene (150 mL) was added indium chloride (6.17 g, 27.90 mmol), and the reaction mixture was stirred at 120°C for 24 hours. The system was cooled to room temperature (25°C), then added with sodium hydroxide aqueous solution (2 M, 275.00 mL), and heated to 120°C and stirred for 24 hours under argon atmosphere. The system was cooled to room temperature (25°C) and filtered. The filter cake was washed with ethyl acetate (100 mL), and the resulting filtrate was extracted with ethyl acetate (200 mL × 2). The organic phases were combined, dried over anhydrous sodium sulfate, filtered, and the filtrate was concentrated to obtain a crude product. The crude product was slurried with a mixed solvent (100 mL) of petroleum ether/ethyl acetate (v/v = 1:1), filtered, and the filter cake was dried to obtain crude compound **C1-3,** which was directly used in the next reaction step.

$^1$H NMR (400 MHz, DMSO-d6) δ 8.25 (s, 1H), 7.56 (dd, *J* = 1.9, 10.5 Hz, 1H), 6.68 (s, 2H), 2.90 (t, *J* = 7.7 Hz, 2H), 2.81 (t, *J* = 7.4 Hz, 2H), 2.06 (quin, *J* = 7.5 Hz, 2H). MS (ESI) *m/z* $(M+H)^+$ = 281.0.

**Step 3:** Preparation of compound **CI-4**

To a solution of compound **C1-3** (1.4 g, 4.98 mmol) in *N*,*N*-dimethylformamide (50 mL) was added sodium hydride (600.00 mg, 15.00 mmol) at 0°C, and the reaction mixture was stirred for 10 minutes. Compound **A1-3** (1.43 g, 5.98 mmol) was then added thereto. After the addition was completed, the system was warmed to room temperature (25°C) and stirred for 2 hours. The system was added with saturated ammonium chloride aqueous solution (10 mL) and water (200 mL) to quench, and extracted with ethyl acetate (300 mL). The organic phases were combined, washed with saturated brine (300 mL), dried over anhydrous sodium sulfate, filtered, and the filtrate was concentrated to obtain a crude product. The crude product was slurried with a mixed solvent (60 mL) of petroleum ether/ethyl acetate (v/v = 1:1), filtered, and the filter cake was dried to obtain crude compound **C1-4,** which was directly used in the next reaction step.

$^1$H NMR (400 MHz, DMSO-d6) δ 11.27 (br s, 1H), 8.11 (s, 1H), 7.99 - 7.89 (m, 2H), 7.84 (dd, *J* = 1.9, 10.0 Hz, 1H), 7.35 (t, *J* = 8.9 Hz, 2H), 3.11 (t, *J* = 7.5 Hz, 2H), 2.88 (brt, *J* = 7.2 Hz, 2H), 2.11 (quin, *J* = 7.4 Hz, 2H). MS (ESI) *m/z* $(M+H)^+$ = 483.0.

**Step 4:** Preparation of compound **C1-5**

To a solution of compound **C1-4** (2.29 g, 4.74 mmol) in *N*,*N*-dimethylformamide (30 mL) were added iodomethane (2.01 g, 14.17 mmol, 882.17 μL) and potassium carbonate (1.97 g, 14.22 mmol) at room temperature (25°C). After the addition was completed, the system was stirred at room temperature (25°C) for 3 hours under nitrogen atmosphere. The system was added with ice water (200 mL) to precipitate, and filtered to collect a filter cake. The filter cake was dried to obtain crude compound **C1-5,** which was directly used in the next reaction step.

$^1$H NMR (400 MHz, CHLOROFORM-*d*) δ 8.17 - 8.06 (m, 2H), 7.71 (d, *J* = 1.5 Hz, 1H), 7.61 - 7.52 (m, 1H), 7.16 (t, *J* = 8.5 Hz, 2H), 3.65 (s, 3H), 3.32 - 3.28 (m, 2H), 3.07 - 3.02 (m, 2H), 2.36 - 2.23 (m, 2H). MS (ESI) *m/z* $(M+H)^+$ = 479.1.

**Step 5:** Preparation of compound **CI-6**

A solution of compound **C1-5** (380 mg, 764.04 μmol), compound *tert*-butyl 2,6-diazaspiro[3.3]heptane-2-carboxylate (454.44 mg, 2.29 mmol), sodium *tert*-butoxide (220 mg, 2.29 mmol), tris(dibenzylideneacetone)dipalladium (140 mg, 152.81 μmol), and 2-(di-*tert*-butylphosphino)biphenyl (88 mg, 152.81 μmol) in toluene (6 mL) was heated to 100°C and stirred for 3 hours under argon atmosphere. The system was cooled to room temperature (25°C), then filtered, and the filtrate was concentrated. The residue was diluted with ethyl acetate (20 mL) and washed with saturated brine (10 mL × 2). The organic phases were combined, dried over anhydrous sodium sulfate, filtered, and the filtrate was concentrated to obtain a crude product. The crude product was purified by silica gel column chromatography (methanol/dichloromethane (v/v) = 0 to 5%) to obtain compound **C1-6.**

$^1$H NMR (400 MHz, Chloroform-*d*) δ 8.17 (br d, *J* = 5.5 Hz, 2H), 7.18 (br t, *J* = 8.6 Hz, 2H), 6.76 - 6.61 (m, 1H), 6.17 (s, 1H), 4.22 - 4.03 (m, 8H), 3.68 - 3.59 (m, 3H), 3.33 - 3.14 (m, 2H), 3.09 - 2.90 (m, 2H), 2.33 - 2.16 (m, 2H), 1.52 - 1.34 (m, 9H). MS (ESI) *m/z* $(M+H)^+$ = 615.3.

**Step 6:** Preparation of compound **Cl-7**

To a solution of compound **C1-6** (300 mg, 488.04 μmol) in dichloromethane (4 mL) was added trifluoroacetic acid (1.54 g, 13.51 mmol), and the reaction mixture was stirred at room temperature (20°C) for 2 hours. The reaction mixture was added with ethyl acetate (20 mL) and water (10 mL), then added with saturated sodium bicarbonate aqueous solution to adjust the pH to 9, and extracted with ethyl acetate (20 mL × 2). The organic phases were combined, dried over anhydrous sodium sulfate, and filtered. The filtrate was concentrated to obtain a crude product, which was purified by silica gel column chromatography (methanol/dichloromethane (v/v) = 0 to 7%) to obtain compound **C1-7.** MS (ESI) *m*/*z* $(M+H)^+$ = 515.1.

**Step 7:** Preparation of compound **C1**

To a solution of compound **C1-7** (25 mg, 48.58 μmol) and (*S*)-tetrahydrofuran-3-carboxylic acid (10 mg, 86.12 μmol) in *N*,*N*-dimethylformamide (1 mL) were added 2-(7-azabenzotriazol-1-yl)-*N*,*N*,*N'*,*N'*-tetramethyluronium hexafluorophosphate (33 mg, 86.79 μmol) and *N*,*N*-diisopropylethylamine (37.10 mg, 287.06 μmol). After the addition was completed, the system was stirred at room temperature (25°C) for 2 hours. Ethyl acetate (50 mL) and water (20 mL) was added thereto. The organic phase was washed with saturated brine (20 mL), then dried over anhydrous sodium sulfate, filtered, and concentrated under reduced pressure to obtain a crude product, which was purified by preparative high performance liquid chromatography (separation conditions: chromatographic column: Phenomenex Gemini-NX 80 × 30 mm × 3 μm; column temperature: 25°C; mobile phase: water (10 mM ammonium bicarbonate)-acetonitrile; acetonitrile: 46% to 76% 9 min) to obtain compound **C1.**

$^1H$ NMR (400 MHz, CHLOROFORM-*d*) δ 8.22 - 8.10 (m, 2H), 7.18 (t, *J* = 8.5 Hz, 2H), 6.70 (dd, *J* = 2.3, 11.8 Hz, 1H), 6.19 (s, 1H), 4.40 - 4.31 (m, 2H), 4.21 (s, 2H), 4.15 - 4.08 (m, 4H), 4.02 - 3.96 (m, 1H), 3.94 - 3.77 (m, 3H), 3.63 (s, 3H), 3.27 - 3.22 (m, 2H), 3.00 (br t, *J* = 7.0 Hz, 2H), 2.95 - 2.87 (m, 1H), 2.29 - 2.22 (m, 2H), 2.20 - 2.06 (m, 2H). MS (ESI) *m*/*z* $(M+H)^+$ = 613.1. HPLC retention time: 4.392 min.

Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.

Similar to the synthesis of example **C1,** the following examples **C2** to **C35** were synthesized as shown in Table 3 below.

Table 3: Structural formulas and analytical data of examples C2 to C35

| Example | Structural formula | Analytical data |
|---|---|---|
| C2 | 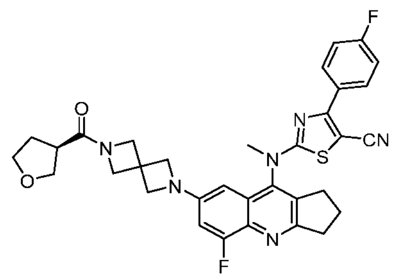 | $^1H$ NMR (400 MHz, CHLOROFORM-*d*) δ 8.17 (br d, *J* = 5.0 Hz, 2H), 7.18 (t, *J* = 8.7 Hz, 2H), 6.70 (dd, *J* = 2.1, 11.7 Hz, 1H), 6.19 (d, *J* = 1.8 Hz, 1H), 4.43 - 4.29 (m, 2H), 4.24 - 4.17 (m, 2H), 4.15 - 4.08 (m, 4H), 3.99 (dt, *J* = 2.0, 8.2 Hz, 1H), 3.94 - 3.77 (m, 3H), 3.63 (s, 3H), 3.27 - 3.22 (m, 2H), 3.00 (br t, *J* = 6.5 Hz, 2H), 2.95 - 2.87 (m, 1H), 2.29 - 2.22 (m, 2H), 2.20 - 2.12 (m, 1H), 2.11 - 2.01 (m, 1H). MS (ESI) *m*/*z* $(M+H)^+$ = 613.1.<br>HPLC retention time: 4.403 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| C3 | 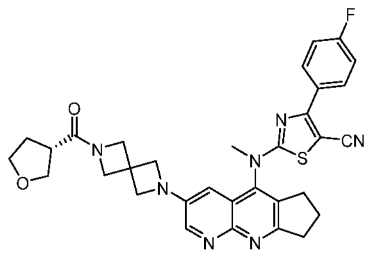 | $^1$H NMR (400 MHz, CHLOROFORM-*d*) $\delta$ = 8.47 (d, *J* = 2.9 Hz, 1H), 8.19 - 8.04 (m, 2H), 7.15 (br t, *J* = 8.6 Hz, 2H), 6.71 (d, *J* = 2.9 Hz, 1H), 4.43 - 4.30 (m, 2H), 4.26 - 4.11 (m, 6H), 3.97 (br t, *J* = 8.2 Hz, 1H), 3.92 - 3.74 (m, 3H), 3.61 (s, 3H), 3.26 (dt, *J* = 2.6, 7.6 Hz, 2H), 3.05 - 2.95 (m, 2H), 2.94 - 2.83 (m, 1H), 2.25 (quin, *J* = 7.5 Hz, 2H), 2.19 - 2.09 (m, 1H), 2.09 - 1.98 (m, 1H). MS (ESI) *m/z* $(M+H)^+$ = 596.1.<br>HPLC retention time: 3.871 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C4 | 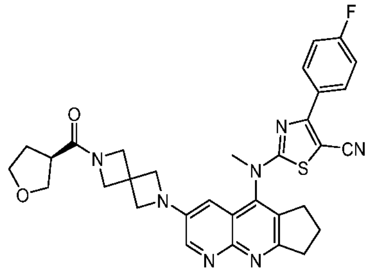 | $^1$H NMR (400 MHz, CHLOROFORM-*d*) $\delta$ = 8.47 (d, *J* = 2.4 Hz, 1H), 8.12 (br s, 2H), 7.15 (br t, *J* = 8.5 Hz, 2H), 6.71 (d, *J* = 2.4 Hz, 1H), 4.44 - 4.30 (m, 2H), 4.26 - 4.09 (m, 6H), 4.05 - 3.72 (m, 4H), 3.61 (s, 3H), 3.33 - 3.16 (m, 2H), 2.99 (br t, *J* = 7.3 Hz, 2H), 2.90 (td, *J* = 7.8, 15.9 Hz, 1H), 2.25 (quin, *J* = 7.3 Hz, 2H), 2.19 - 2.09 (m, 1H), 2.08 - 1.96 (m, 1H). MS (ESI) *m/z* $(M+H)^+$ = 596.1.<br>HPLC retention time: 3.867 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C5 | 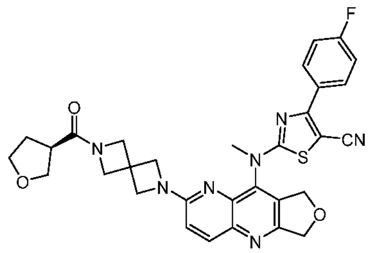 | $^1$H NMR (400 MHz, CHLOROFORM-*d*) δ 8.21 (br d, *J* = 9.1 Hz, 1H), 8.06 (dd, *J* = 5.4, 8.6 Hz, 2H), 7.14 (t, *J* = 8.6 Hz, 2H), 6.83 (d, *J* = 9.2 Hz, 1H), 5.23 (s, 2H), 5.21 (s, 2H), 4.42 - 4.32 (m, 3H), 4.30 (s, 3H), 4.22 (s, 2H), 4.01 - 3.97 (m, 1H), 3.94 - 3.88 (m, 1H), 3.88 - 3.84 (m, 1H), 3.78 - 3.78 (m, 1H), 3.82 - 3.78 (m, 1H), 3.68 (s, 3H), 2.97 - 2.87 (m, 1H), 2.21 - 2.13 (m, 1H), 2.10 - 2.01 (m, 1H). MS (ESI) *m/z* $(M+H)^+$ = 598.1.<br>HPLC retention time: 3.860 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C6 | 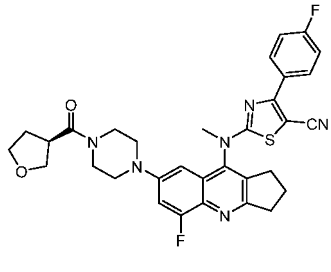 | $^1$H NMR (400 MHz, CHLOROFORM-*d*) δ 8.16 (br d, *J* = 5.0 Hz, 2H), 7.24 - 7.10 (m, 3H), 6.68 (s, 1H), 4.03 (br t, *J* = 8.2 Hz, 1H), 3.95 - 3.78 (m, 5H), 3.71 (br s, 2H), 3.65 (s, 3H), 3.28 (br d, *J* = 4.8 Hz, 7H), 3.01 (br t, *J* = 7.1 Hz, 2H), 2.37 - 2.20 (m, 3H), 2.19 - 2.04 (m, 1H). MS (ESI) *m/z* $(M+H)^+$ = 601.3.<br>HPLC retention time: 4.438 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| C7 | 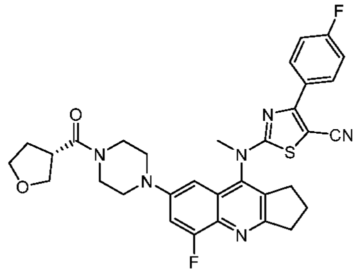 | $^1$H NMR (400 MHz, CHLOROFORM-*d*) δ 8.22 - 8.08 (m, 2H), 7.24 - 7.13 (m, 3H), 6.68 (d, *J* = 2.1 Hz, 1H), 4.03 (dt, *J* = 2.1, 8.3 Hz, 1H), 3.95 - 3.79 (m, 5H), 3.71 (br d, *J* = 3.9 Hz, 2H), 3.65 (s, 3H), 3.33 - 3.19 (m, 7H), 3.06 - 2.95 (m, 2H), 2.34 - 2.19 (m, 3H), 2.16 - 2.01 (m, 1H). MS (ESI) *m*/*z* $(M+H)^+$ = 601.3. HPLC retention time: 4.439 min. Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C8 | 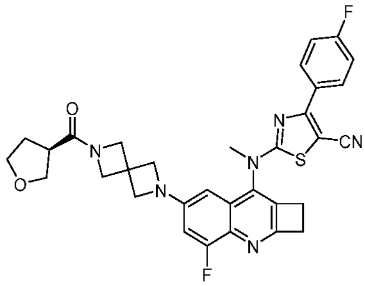 | $^1$H NMR (400 MHz, Chloroform-*d*) δ 8.15 (dd, *J* = 5.4, 8.8 Hz, 2H), 7.18 (t, *J* = 8.6 Hz, 2H), 6.71 (dd, *J* = 2.2, 11.7 Hz, 1H), 6.28 (d, *J* = 1.9 Hz, 1H), 4.43 - 4.30 (m, 2H), 4.25 - 4.17 (m, 2H), 4.12 (s, 4H), 3.99 (t, *J* = 8.2 Hz, 1H), 3.95 - 3.88 (m, 1H), 3.82 (td, *J* = 7.6, 15.3 Hz, 2H), 3.70 - 3.61 (m, 5H), 3.29 - 3.21 (m, 2H), 2.97 - 2.87 (m, 1H), 2.22 - 2.12 (m, 1H), 2.11 - 2.00 (m, 1H). MS (ESI) *m*/*z* $(M+H)^+$ = 599.1. HPLC retention time: 4.282 min. Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C9 | 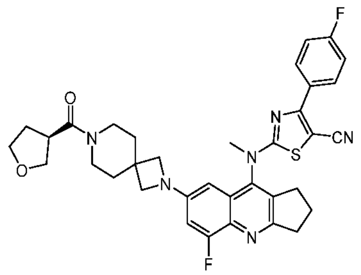 | $^1$H NMR (400 MHz, Chloroform-*d*) δ 8.23 - 8.08 (m, 2H), 7.18 (t, *J* = 8.5 Hz, 2H), 6.71 (br d, *J* = 11.5 Hz, 1H), 6.16 (s, 1H), 4.05 - 3.99 (m, 1H), 3.93 - 3.83 (m, 3H), 3.75 (br s, 4H), 3.63 (s, 5H), 3.49 (br s, 2H), 3.33 - 3.21 (m, 3H), 3.04 - 2.92 (m, 2H), 2.30 - 2.21 (m, 3H), 2.08 (br dd, *J* = 9.0, 12.0 Hz, 1H), 1.89 - 1.81 (m, 4H). MS (ESI) *m*/*z* $(M+H)^+$ = 641.1. HPLC retention time: 4.791 min. Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C10 | 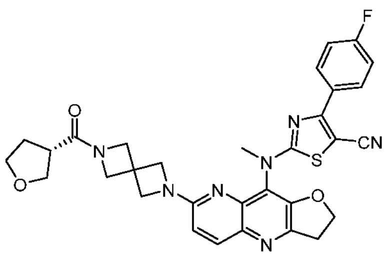 | $^1$H NMR (400 MHz, CHLOROFORM-*d*) δ 8.13 (br dd, *J* = 5.5, 8.5 Hz, 2H), 8.02 (d, *J* = 9.0 Hz, 1H), 7.15 (t, *J* = 8.7 Hz, 2H), 6.63 (d, *J* = 9.0 Hz, 1H), 4.88 (t, *J* = 8.4 Hz, 2H), 4.41 - 4.29 (m, 2H), 4.26 - 4.17 (m, 6H), 3.98 (t, *J* = 8.2 Hz, 1H), 3.94 - 3.77 (m, 3H), 3.68 (s, 3H), 3.52 (t, *J* = 8.5 Hz, 2H), 2.95 - 2.86 (m, 1H), 2.21 - 2.11 (m, 1H), 2.10 - 2.01 (m, 1H). MS (ESI) *m*/*z* $(M+H)^+$ = 598.2. HPLC retention time: 4.035 min. Separation conditions: chromatographic column: Xbridge Shield RP-18, 5 μm, 2.1 * 50 mm; column temperature: 40°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| C11 | 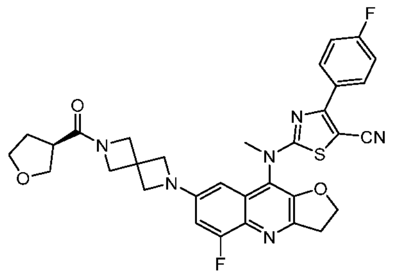 | $^1$H NMR (400 MHz, CHLOROFORM-*d*) δ 8.18 - 8.12 (m, 2H), 7.17 (t, *J* = 8.7 Hz, 2H), 6.56 (br d, *J* = 9.5 Hz, 1H), 6.15 (s, 1H), 4.85 (t, *J* = 8.4 Hz, 2H), 4.36 (br d, *J* = 7.0 Hz, 2H), 4.20 (br s, 2H), 4.12 (br s, 4H), 4.04 - 3.98 (m, 1H), 3.91 (br d, *J* = 6.3 Hz, 1H), 3.85 - 3.76 (m, 2H), 3.62 (s, 3H), 3.58 (br d, *J* = 8.5 Hz, 2H), 2.96 - 2.89 (m, 1H), 2.16 (br d, *J* = 7.3 Hz, 1H), 2.05 (br s, 1H). MS (ESI) *m/z* $(M+H)^+$ = 615.1.<br>HPLC retention time: 4.85 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| C12 | 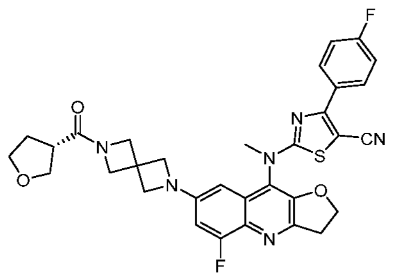 | $^1$H NMR (400 MHz, CHLOROFORM-*d*) δ 8.15 (dd, *J* = 5.3, 8.8 Hz, 2H), 7.17 (t, *J* = 8.7 Hz, 2H), 6.56 (dd, *J* = 2.1, 11.7 Hz, 1H), 6.15 (s, 1H), 4.85 (t, *J* = 8.4 Hz, 2H), 4.39 - 4.33 (m, 2H), 4.20 (br s, 2H), 4.12 (br s, 4H), 3.99 (t, *J* = 8.2 Hz, 1H), 3.91 (br d, *J* = 6.3 Hz, 1H), 3.85 - 3.78 (m, 2H), 3.62 (s, 3H), 3.57 (br t, *J* = 8.9 Hz, 2H), 2.96 - 2.88 (m, 1H), 2.17 (s, 1H), 2.06 (br s, 1H). MS (ESI) *m/z* $(M+H)^+$ = 615.1.<br>HPLC retention time: 4.85 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| C13 | 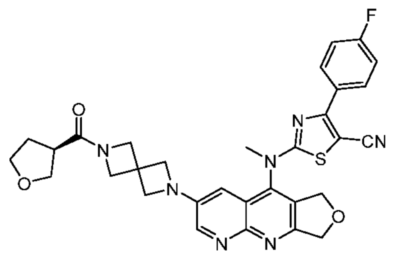 | $^1$H NMR (400 MHz, CHLOROFORM-*d*) δ = 8.19 - 8.05 (m, 3H), 7.86 (d, *J* = 2.5 Hz, 1H), 7.15 (t, *J* = 8.7 Hz, 2H), 5.16 (s, 2H), 4.90 (s, 2H), 4.41 (q, *J* = 8.9 Hz, 2H), 4.30 - 4.13 (m, 6H), 4.02 (t, *J* = 8.2 Hz, 1H), 3.98 - 3.73 (m, 6H), 3.03 - 2.87 (m, 1H), 2.28 - 2.00 (m, 2H). MS (ESI) *m/z* $(M+H)^+$ = 598.1.<br>HPLC retention time: 4.081 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C14 | 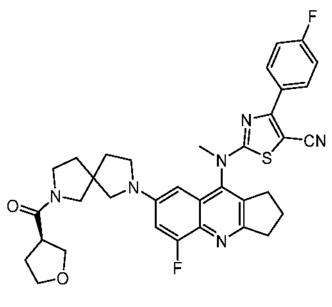 | $^1$H NMR (400 MHz, CHLOROFORM-*d*) δ = 8.17 (br s, 2H), 7.18 (t, *J* = 8.5 Hz, 2H), 6.94 - 6.81 (m, 1H), 6.28 - 6.17 (m, 1H), 4.11 - 3.96 (m, 1H), 3.95 - 3.80 (m, 3H), 3.65 (s, 5H), 3.57 - 3.41 (m, 5H), 3.32 (s, 1H), 3.30 - 3.21 (m, 2H), 3.18 - 3.07 (m, 1H), 3.03 - 2.92 (m, 2H), 2.33 - 2.16 (m, 3H), 2.15 - 1.93 (m, 5H). MS (ESI) *m/z* $(M+H)^+$ = 641.1.<br>HPLC retention time: 4.695 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| C15 | 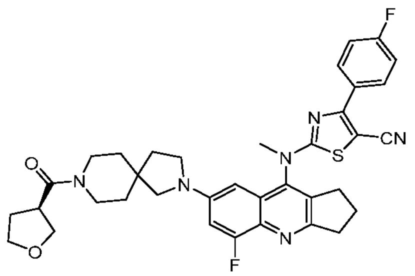 | $^1H$ NMR (400 MHz, CHLOROFORM-*d*) $\delta$ 8.23 - 8.00 (m, 2H), 7.18 (t, *J* = 8.7 Hz, 2H), 6.89 (dd, *J* = 2.0, 13.1 Hz, 1H), 6.22 (s, 1H), 4.07 - 3.96 (m, 1H), 3.88 (q, *J* = 6.8 Hz, 3H), 3.74 - 3.59 (m, 5H), 3.53 (br s, 2H), 3.45 (br d, *J* = 7.0 Hz, 2H), 3.30 - 3.20 (m, 5H), 3.05 - 2.93 (m, 2H), 2.29 - 2.20 (m, 3H), 2.13 - 2.04 (m, 1H), 2.02 - 1.96 (m, 2H), 1.72 - 1.60 (m, 4H). MS (ESI) *m/z* $(M+H)^+$ = 655.1.<br>HPLC retention time: 4.936 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C16 | 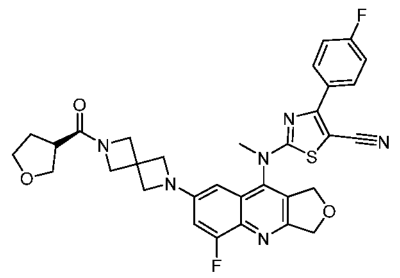 | $^1H$ NMR (400 MHz, CHLOROFORM-*d*) $\delta$ 8.13 (dd, *J* = 5.3, 8.5 Hz, 2H), 7.18 (t, *J* = 8.5 Hz, 2H), 6.77 (dd, *J* = 2.1, 11.7 Hz, 1H), 6.22 (d, *J* = 2.0 Hz, 1H), 5.22 (s, 2H), 5.19 (s, 2H), 4.37 (br d, *J* = 8.0 Hz, 2H), 4.26 - 4.08 (m, 6H), 4.03 - 3.96 (m, 1H), 3.94 - 3.87 (m, 1H), 3.86 - 3.76 (m, 2H), 3.62 (s, 3H), 2.92 (quin, *J* = 7.9 Hz, 1H), 2.23 - 2.12 (m, 1H), 2.11 - 2.02 (m, 1H). MS (ESI) *m/z* $(M+H)^+$ = 517.2.<br>HPLC retention time: 4.67 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| C17 | 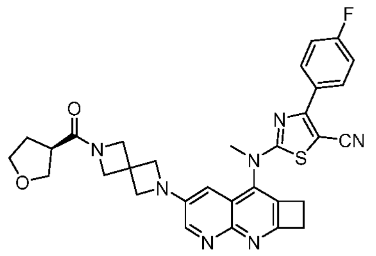 | $^1H$ NMR (400 MHz, CHLOROFORM-*d*) $\delta$ = 8.56 - 8.42 (m, 1H), 8.11 (dd, *J* = 5.3, 8.8 Hz, 2H), 7.17 (t, *J* = 8.6 Hz, 2H), 6.92 (br s, 1H), 4.43 - 4.33 (m, 1H), 4.22 (s, 5H), 3.99 (t, *J* = 8.2 Hz, 1H), 3.94 - 3.77 (m, 3H), 3.75 - 3.72 (m, 1H), 3.71 - 3.64 (m, 3H), 3.29 (br t, *J* = 5.0 Hz, 2H), 3.00 - 2.86 (m, 1H), 2.12 (br dd, *J* = 6.7, 15.5 Hz, 4H). MS (ESI) *m/z* $(M+H)^+$ = 582.2.<br>HPLC retention time: 3.97 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| C18 | 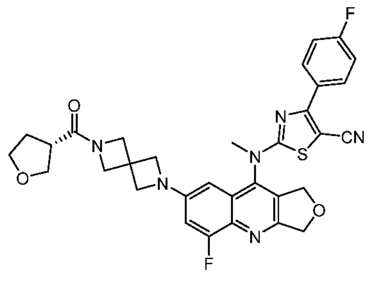 | $^1H$ NMR (400 MHz, CHLOROFORM-*d*) $\delta$ = 8.13 (dd, *J* = 5.4, 8.7 Hz, 2H), 7.18 (t, *J* = 8.6 Hz, 2H), 6.77 (dd, *J* = 2.3, 11.6 Hz, 1H), 6.22 (d, *J* = 2.0 Hz, 1H), 5.27 - 5.16 (m, 4H), 4.37 (br d, *J* = 7.6 Hz, 2H), 4.22 (br s, 2H), 4.15 (s, 4H), 3.99 (t, *J* = 8.2 Hz, 1H), 3.95 - 3.78 (m, 3H), 3.62 (s, 3H), 2.98 - 2.87 (m, 1H), 2.23 - 2.12 (m, 1H), 2.12 - 2.00 (m, 1H), 2.12 - 2.00 (m, 1H). MS (ESI) *m/z* $(M+H)^+$ = 615.1.<br>HPLC retention time: 3.88 min.<br>Separation conditions: chromatographic column: Xbridge Shield RP-18, 5 μm, 2.1 * 50 mm; column temperature: 40°C; mobile phase: water (2 mL/4 L ammonia water)-acetonitrile; acetonitrile: 10% to 80% 6 min, 80% 2 min; flow rate: 1.2 mL/min. |

(continued)

| Example | Structural formula | Analytical data |
|---|---|---|
| C19 | 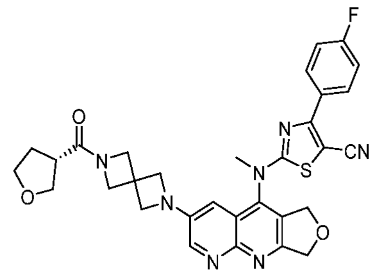 | $^1H$ NMR (400 MHz, CHLOROFORM-*d*) δ = 8.56 (d, *J* = 3.0 Hz, 1H), 8.12 (dd, *J* = 5.4, 8.7 Hz, 2H), 7.17 (t, *J* = 8.7 Hz, 3H), 6.75 (d, *J* = 3.0 Hz, 1H), 5.26 - 5.21 (m, 4H), 4.45 - 4.34 (m, 2H), 4.31 - 4.17 (m, 7H), 4.03 - 3.95 (m, 1H), 3.95 - 3.75 (m, 4H), 3.63 (s, 3H), 2.98 - 2.87 (m, 1H), 2.26 - 1.97 (m, 5H). MS (ESI) *m/z* $(M+H)^+$ = 598.1.<br>HPLC retention time: 3.469 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |
| C20 | 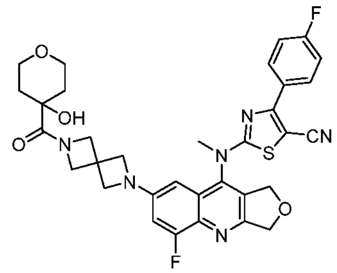 | $^1H$ NMR (400 MHz, DMSO-*d6*) δ = 8.14 - 7.87 (m, 2H), 7.50 - 7.29 (m, 2H), 7.03 (dd, *J* = 1.9, 12.4 Hz, 1H), 6.26 (d, *J* = 1.3 Hz, 1H), 5.31 - 5.18 (m, 2H), 5.14 - 4.99 (m, 3H), 4.55 (s, 2H), 4.17 - 4.05 (m, 4H), 4.05 - 4.00 (m, 2H), 3.67 - 3.53 (m, 7H), 1.86 (br d, *J* = 8.7 Hz, 2H), 1.42 (br d, *J* = 12.8 Hz, 2H). MS (ESI) *m/z* $(M+H)^+$ = 645.1.<br>HPLC retention time: 4.51 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| C21 | 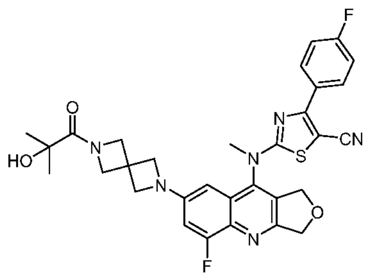 | $^1H$ NMR (400 MHz, Chloroform-*d*) δ 8.19 - 8.04 (m, 2H), 7.22 - 7.10 (m, 2H), 6.77 (dd, *J* = 11.6,2.3 Hz, 1H), 6.22 (d, *J*=2.2 Hz, 1H), 5.24 (s, 2H), 5.18 (s, 2H), 4.57 (s, 2H), 4.26 (s, 2H), 4.15 (s, 4H), 3.61 (s, 3H), 1.41 (s, 6H). MS (ESI) *m/z* $(M+H)^+$ = 602.8.<br>HPLC retention time: 5.91 min.<br>Separation conditions: chromatographic column: High Performance GOLD 50 g HP C18; mobile phase: [water (10 mM/L ammonium bicarbonate)-acetonitrile]; gradient: 0 to 60% acetonitrile/water; flow rate: 40 mL/min. |
| C22 | 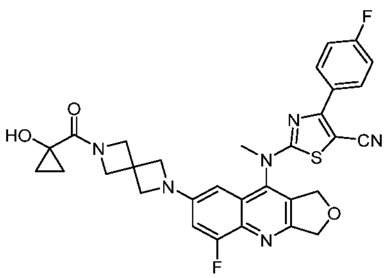 | $^1H$ NMR (400 MHz, Chloroform-*d*) δ 8.12 (dd, *J* = 8.6, 5.2 Hz, 2H), 7.17 (t, *J* = 8.6 Hz, 2H), 6.82 - 6.71 (m, 1H), 6.21 (d, *J* = 1.9 Hz, 1H), 5.20 (d, *J* = 15.3 Hz, 4H), 4.64 (s, 2H), 4.43 - 4.02 (m, 6H), 3.61 (s, 3H), 1.33 (q, *J* = 4.7 Hz, 2H), 1.00 (q, *J* = 4.7 Hz, 2H). MS (ESI) *m/z* $(M+H)^+$ = 600.8.<br>HPLC retention time: 5.85 min.<br>Separation conditions: chromatographic column: High Performance GOLD 50 g HP C18; mobile phase: [water (10 mM/L ammonium bicarbonate)-acetonitrile]; gradient: 0 to 60% acetonitrile/water; flow rate: 40 mL/min. |

(continued)

| Example | Structural formula | Analytical data |
|---|---|---|
| C23 | 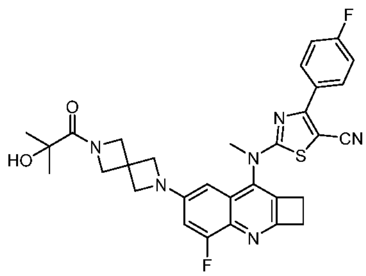 | $^1$H NMR (400 MHz, Chloroform-*d*) δ 8.17 - 8.09 (m, 2H), 7.21 - 7.13 (m, 2H), 6.73 (d, *J* = 10.9 Hz, 1H), 6.28 (d, *J* = 2.1 Hz, 1H), 4.56 (s, 2H), 4.25 (s, 2H), 4.13 (s, 4H), 3.70 (t, *J* = 5.2 Hz, 2H), 3.66 (s, 3H), 3.26 (t, *J* = 5.2 Hz, 2H), 1.41 (s, 6H). MS (ESI) *m/z* $(M+H)^+$ = 586.8. HPLC retention time: 6.25 min. Separation conditions: chromatographic column: High Performance GOLD 50 g HP C18; mobile phase: [water (10 mM/L ammonium bicarbonate)-acetonitrile]; gradient: 0 to 50% acetonitrile/water; flow rate: 50 mL/min. |
| C24 | 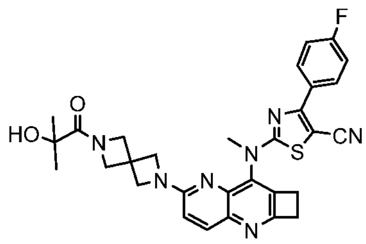 | $^1$H NMR (400 MHz, $CDCl_3$) δ 8.18 (s, 1H), 8.11 (dd, *J* = 8.7, 5.3 Hz, 2H), 7.15 (t, *J* = 8.6 Hz, 2H), 6.77 (d, *J* = 7.9 Hz, 1H), 4.55 (s, 2H), 4.27 (s, 6H), 3.77 (s, 3H), 3.59 (s, 2H), 3.24 (s, 2H), 1.41 (s, 6H). MS (ESI) *m/z* $(M+H)^+$ = 570.3. HPLC retention time: 8.94 min. Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| C25 | 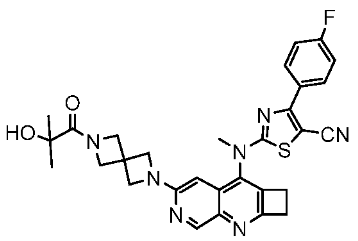 | $^1$H NMR (400 MHz, $CDCl_3$) δ 9.15 (s, 1H), 8.13 (dd, *J* = 8.9, 5.3 Hz, 2H), 7.17 (t, *J* = 8.7 Hz, 2H), 6.26 (s, 1H), 4.56 (s, 2H), 4.24 (d, *J* = 9.7 Hz, 6H), 3.67 - 3.62 (m, 5H), 3.34 (s, 1H), 3.28 - 3.22 (m, 2H), 1.41 (s, 6H). MS (ESI): *m/z* $[M+H]^+$ = 570.3. HPLC retention time: 8.97 min. Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| C26 | 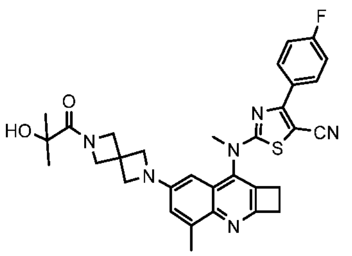 | $^1$H NMR (400 MHz, $CDCl_3$) δ 8.15 (dd, *J* = 8.8, 5.4 Hz, 2H), 7.17 (t, *J* = 8.6 Hz, 2H), 6.84 (s, 1H), 6.38 (d, *J* = 2.3 Hz, 1H), 4.55 (s, 2H), 4.24 (s, 2H), 4.11 (s, 4H), 3.67 (d, *J* = 9.5 Hz, 5H), 3.27 - 3.17 (m, 2H), 2.79 (s, 3H), 1.41 (s, 6H). MS (ESI) *m/z* $(M+H)^+$ = 583.3. HPLC retention time: 9.54 min. Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |

(continued)

| Example | Structural formula | Analytical data |
|---|---|---|
| C27 | 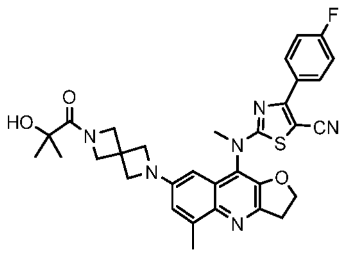 | $^1$H NMR (400 MHz, $CDCl_3$) δ 8.14 (dd, *J* = 8.7, 5.4 Hz, 2H), 7.16 (t, *J* = 8.7 Hz, 2H), 6.71 (s, 1H), 6.25 (d, *J* = 2.1 Hz, 1H), 4.83 (t, *J* = 7.9 Hz, 2H), 4.54 (s, 2H), 4.24 (s, 2H), 4.11 (s, 4H), 3.62 (s, 5H), 2.79 (s, 3H), 1.40 (s, 6H).<br>MS (ESI): *m/z* $[M+H]^+$ = 599.4.<br>HPLC retention time: 10.17 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| C28 | 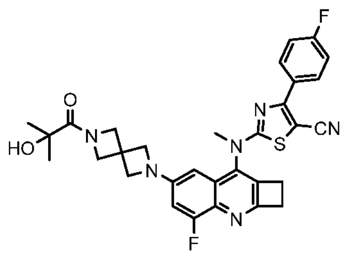 | $^1$H NMR (400 MHz, $CDCl_3$) δ 8.12 (dd, *J* = 8.6, 5.3 Hz, 2H), 7.17 (t, *J* = 8.6 Hz, 2H), 6.74 (d, *J* = 11.0 Hz, 1H), 6.28 (s, 1H), 4.57 (s, 2H), 4.25 (s, 2H), 4.13 (s, 4H), 3.68 (d, *J* = 12.5 Hz, 5H), 3.27 (s, 2H), 1.41 (s, 6H).<br>MS (ESI) *m/z* $(M+H)^+$ = 587.3.<br>HPLC retention time: 9.96 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| C29 | 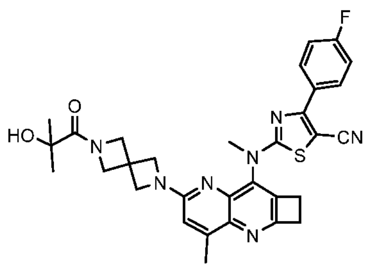 | $^1$H NMR (400 MHz, DMSO-d6) δ 8.05 (dd, *J* = 8.8, 5.5 Hz, 2H), 7.40 (t, *J* = 8.9 Hz, 2H), 6.82 (s, 1H), 4.53 (s, 2H), 4.17 (s, 4H), 4.02 (s, 2H), 3.69 (s, 3H), 3.53 (m, 2H), 3.21 (d, *J* = 5.3 Hz, 2H), 2.62 (s, 3H), 1.23 (s, 6H).<br>MS (ESI) *m/z* $(M+H)^+$ = 584.4.<br>HPLC retention time: 9.27 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| C30 | 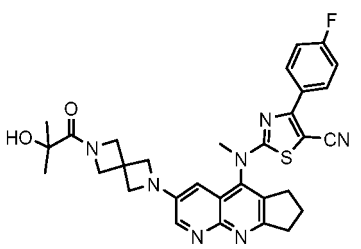 | $^1$H NMR (400 MHz, $CDCl_3$) δ 8.09 (d, *J* = 13.4 Hz, 3H), 7.93 (s, 1H), 7.12 (t, *J* = 8.6 Hz, 2H), 4.59 (s, 2H), 4.24 (d, *J* = 18.4 Hz, 6H), 4.03 (s, 3H), 3.16 (t, *J* = 7.3 Hz, 2H), 2.76 (t, *J* = 7.2 Hz, 2H), 2.18 - 2.12 (m, 2H), 1.43 (s, 6H). MS (ESI): *m/z* $[M+H]^+$ = 584.3.<br>HPLC retention time: 7.99 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |

(continued)

| Example | Structural formula | Analytical data |
|---|---|---|
| C31 | 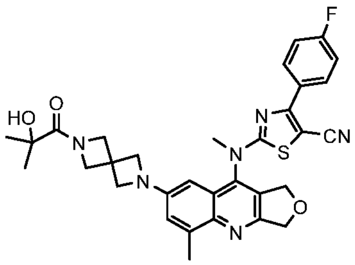 | $^1$H NMR (400 MHz, DMSO-*d6*) δ 8.03 (s, 2H), 7.40 (t, *J* = 8.8 Hz, 2H), 7.02 (s, 1H), 6.29 (d, *J* = 2.3 Hz, 1H), 5.24 (d, *J* = 14.0 Hz, 1H), 5.14 - 5.01 (m, 4H), 4.53 (s, 2H), 4.13 - 3.98 (m, 6H), 3.58 (s, 3H), 2.68 (s, 3H), 1.23 (s, 6H). MS (ESI): *m/z* [M+H]$^+$ = 599.4.<br>HPLC retention time: 10.04 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| C32 | 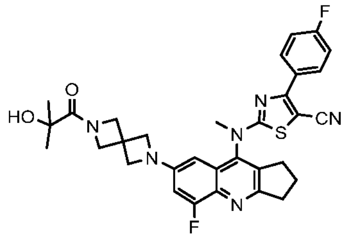 | $^1$H NMR (400 MHz, DMSO-*d6*) δ 8.06 (s, 2H), 7.40 (t, *J* = 8.3 Hz, 2H), 6.93 (d, *J* = 11.0 Hz, 1H), 6.20 (s, 1H), 5.06 (s, 1H), 4.53 (s, 2H), 4.19 - 3.97 (m, 6H), 3.59 (s, 3H), 3.06 (qd, *J* = 16.7, 9.0 Hz, 3H), 2.87 (dd, *J* = 13.2, 4.6 Hz, 1H), 2.25 - 2.05 (m, 2H), 1.23 (s, 6H).<br>MS (ESI) *m/z* (M+H)$^+$ = 601.4.<br>HPLC retention time: 9.82 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| C33 | 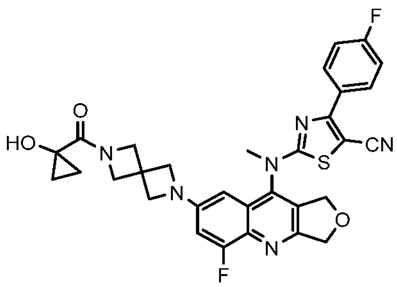 | $^1$H NMR (400 MHz, Chloroform-*d*) δ 8.12 (dd, *J* = 8.6, 5.2 Hz, 2H), 7.17 (t, *J* = 8.6 Hz, 2H), 6.76 (d, *J* = 11.0 Hz, 1H), 6.21 (s, 1H), 5.20 (d, *J* = 15.3 Hz, 4H), 4.64 (m, 2H), 4.30 (m, 2H), 4.14(m, 4H), 3.61 (s, 3H), 1.33 (q, *J* = 4.7 Hz, 2H), 1.00 (q, *J* = 4.7 Hz, 2H).<br>MS (ESI) *m/z* (M+H)$^+$ = 600.8.<br>HPLC retention time: 5.85 min.<br>Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min. |
| C34 | 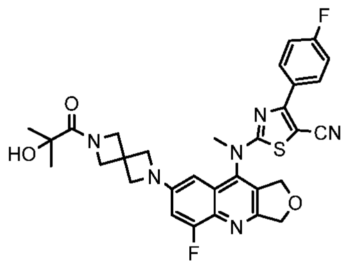 | $^1$H NMR (400 MHz, Chloroform-*d*) δ 8.11 (dd, *J* = 8.8, 5.4 Hz, 2H), 7.16 (dd, *J* = 9.5, 7.8 Hz, 2H), 6.77 (dd, *J* = 11.6, 2.3 Hz, 1H), 6.22 (d, *J* = 2.2 Hz, 1H), 5.21 (d, *J* = 27.4 Hz, 4H), 4.57 (s, 2H), 4.26 (s, 2H), 4.15 (s, 4H), 3.61 (s, 3H), 1.41 (s, 6H).<br>MS (ESI) *m/z* (M+H)$^+$ = 602.8.<br>HPLC retention time: 5.91 min.<br>Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min. |

(continued)

| Example | Structural formula | Analytical data |
|---|---|---|
| C35 | 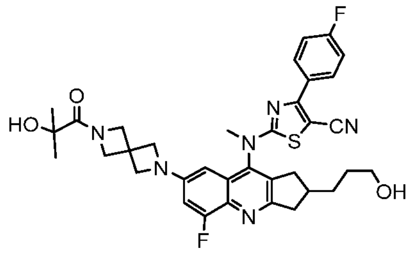 | $^1$H NMR (400 MHz, DMSO-*d6*) δ 8.26 - 7.72 (m, 2H), 7.40 (t, *J* = 7.7 Hz, 2H), 6.96 - 6.89 (m, 1H), 6.26 - 6.11 (m, 1H), 5.06 (d, *J* = 1.4 Hz, 1H), 4.53 (s, 2H), 4.37 (q, *J* = 5.3 Hz, 1H), 4.05 (dd, *J* = 13.0, 7.2 Hz, 6H), 3.58 (s, 3H), 3.45 - 3.35 (m, 3H), 3.26 - 2.96 (m, 2H), 2.84 - 2.65 (m, 2H), 1.49 (d, *J* = 5.0 Hz, 4H), 1.22 (s, 6H).<br>MS (ESI) *m/z* (M+H)$^+$ = 659.5.<br>HPLC retention time: 8.649 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |

**Synthesis of example D1**

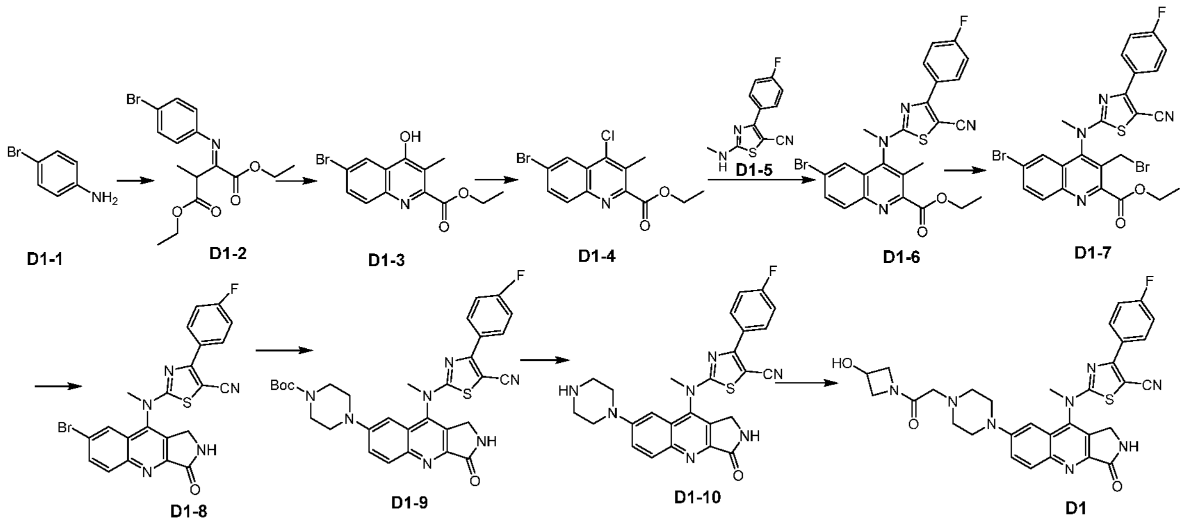
D1-1 D1-2 D1-3 D1-4 D1-5 D1-6 D1-7 D1-8 D1-9 D1-10 D1

**Step 1:** Preparation of compound **D1-2**

To a solution of compound **D1-1** (10 g, 58.13 mmol) and diethyl oxalpropionate (11.75 g, 58.13 mmol, 10.78 mL) in toluene (250 mL) was added *p*-toluenesulfonic acid (553 mg, 2.91 mmol). After the addition was completed, the system was heated to reflux (130°C) and stirred for 16 hours using a Dean-Stark trap. The system was cooled to room temperature (25°C), filtered, and the filter cake was washed with toluene. The filtrate was concentrated to obtain compound **D1-2,** which was directly used in the next reaction step without further purification.

**Step 2:** Preparation of compound **Dl-3**

Compound **D1-2** (10 g, 28.07 mmol) was dissolved in polyphosphoric acid (30 g). After the addition was completed, the system was heated to 120°C and stirred for 3 hours. After the reaction was completed, the system was added with sodium hydroxide aqueous solution (2 M) to quench to precipitate a yellow solid. After filtration, the filter cake was slurried with ethyl acetate (500 mL), filtered, and the filtrate was concentrated to obtain a crude product. The crude product was purified by medium-pressure column chromatography (ethyl acetate/petroleum ether (v/v) = 0 to 50%; methanol/dichloromethane (v/v) = 0 to 20%) to obtain compound **D1-3.**

$^1$H NMR (400 MHz, DMSO-d6) δ 11.89 (s, 1H), 8.16 (d, *J* = 1.9 Hz, 1H), 7.87 - 7.74 (m, 2H), 4.45 (q, *J* = 7.2

Hz, 2H), 2.20 (s, 3H), 1.38 (t, *J* = 7.1 Hz, 3H). MS (ESI) *m/z* $(M+H)^+$ = 309.7.

**Step 3:** Preparation of compound **D1-4**

Compound **D1-3** (2.09 g, 6.75 mmol) was dissolved in phosphorus oxychloride (33.00 g, 215.22 mmol, 20.00 mL). After the addition was completed, the system was heated to 110°C and stirred for 0.5 hours. The system was concentrated, diluted with water (30 mL), added with saturated sodium bicarbonate (30 mL) to adjust the pH to >7, and extracted with ethyl acetate (80 mL × 3). The organic phases were combined, dried over anhydrous sodium sulfate, filtered, and the filtrate was concentrated to obtain a crude product. The crude product was purified by medium-pressure column chromatography (ethyl acetate/petroleum ether (v/v) = 0 to 12%) to obtain compound **D1-4.**

$^1H$ NMR (400 MHz, CHLOROFORM-*d*) δ 8.30 (d, *J* = 2.1 Hz, 1H), 7.92 (d, *J* = 8.9 Hz, 1H), 7.72 (dd, *J* = 2.1, 8.9 Hz, 1H), 4.45 (q, *J* = 7.2 Hz, 2H), 2.67 - 2.49 (m, 3H), 1.39 (t, *J* = 7.2 Hz, 3H). MS (ESI) *m/z* $(M+H)^+$ = 327.7.

**Step 4:** Preparation of compound **D1-5**

Compound **A1-3** (3 g, 12.57 mmol) and triethylamine (6.54 g, 64.66 mmol, 9 mL) were dissolved in 1,4-dioxane (30 mL), then methylamine hydrochloride (2.55 g, 37.71 mmol) was added thereto at room temperature, and the reaction mixture was stirred and reacted at 80°C for 2 hours. The reaction mixture was evaporated to dryness by rotary evaporation, diluted with 100 mL of ethyl acetate, filtered, and the filtrate was washed with 100 mL of water. The organic phase was dried, filtered, and evaporated to dryness by rotary evaporation. The crude product was separated by silica gel column chromatography (petroleum ether/ethyl acetate = 30% to 50%). Compound **D1-5** (2.6 g, yield: 88.67%) was obtained as a yellow solid.

$^1H$ NMR (400 MHz, DMSO-*d6*) δ = 8.75 (br s, 1H), 8.12 - 7.93 (m, 2H), 7.37 (br t, *J* = 8.9 Hz, 2H), 2.95 (br d, *J* = 4.5 Hz, 3H).

**Step 5:** Preparation of compound **D1-6**

To a solution of compound **D1-4** (300 mg, 913.00 μmol) and compound **D1-5** (300 mg, 1.29 mmol) in *N*,*N*-dimethylformamide (10 mL) was added potassium carbonate (380 mg, 2.75 mmol), and the reaction mixture was heated to 130°C and stirred for 5 hours under microwave irradiation. After the reaction was completed, the system was diluted with water (50 mL) and extracted with ethyl acetate (30 mL × 3). The organic phases were combined, washed with saturated brine (50 mL), dried over anhydrous sodium sulfate, filtered, and the filtrate was concentrated to obtain a crude product. The crude product was purified by medium-pressure column chromatography (ethyl acetate/petroleum ether (v/v) = 0 to 20%) to obtain compound **D1-6.**

$^1H$ NMR (400 MHz, CHLOROFORM-*d*) δ 8.23 - 8.08 (m, 3H), 7.94 - 7.85 (m, 2H), 7.24 - 7.07 (m, 2H), 4.57 (q, *J* = 7.2 Hz, 2H), 3.67 (s, 3H), 2.54 (s, 3H), 1.51 (t, *J* = 7.1 Hz, 3H). MS (ESI) *m/z* $(M+H)^+$ = 525.0.

**Step 6:** Preparation of compound **D1-7**

Compound **D1-6** (130 mg, 247.43 μmol) was dissolved in carbon tetrachloride (5 mL), and N-bromosuccinimide (73.11 mg, 410.74 μmol) and azobisisobutyronitrile (20.33 mg, 123.78 μmol) were added thereto. After the addition was completed, the system was heated to 80°C and stirred for 16 hours under argon atmosphere. The system was concentrated, and the residue was diluted with water (50 mL) and extracted with ethyl acetate (50 mL). The organic phases were combined, washed with saturated brine (50 mL), dried over anhydrous sodium sulfate, filtered, and the filtrate was concentrated to obtain a crude product. The crude product was purified by medium-pressure column chromatography (ethyl acetate/petroleum ether (v/v) = 0 to 30%) to obtain compound **D1-7.**

$^1H$ NMR (400 MHz, CHLOROFORM-*d*) δ 8.21 (d, *J* = 8.8 Hz, 1H), 8.16 - 8.04 (m, 2H), 7.95 (dd, *J* = 1.8, 9.0 Hz, 1H), 7.86 (s, 1H), 7.16 (t, *J* = 8.4 Hz, 2H), 5.13 (d, *J* = 10.6 Hz, 1H), 4.72 (br d, *J* = 10.4 Hz, 1H), 4.68 - 4.54 (m, 2H), 3.75 (s, 3H), 1.56 - 1.50 (m, 3H). MS (ESI) *m/z* $(M+H)^+$ = 603.0.

**Step 7:** Preparation of compound **D1-8**

To a solution of compound **D1-7** (100 mg, 165.48 μmol) in acetonitrile (5 mL) were added *N,N*-diisopropylethylamine (148.40 mg, 1.15 mmol, 200 μL) and a solution of ammonia in methanol (7 M, 1.00 mL). After the addition was completed, the system was stirred at room temperature (20°C) for 16 hours. After the reaction was completed, the system was concentrated under reduced pressure to obtain a crude product. The crude product was slurried with a mixed solvent (10 mL) of petroleum ether/ethyl acetate (v/v = 3:1), filtered, and the filter cake was dried to obtain compound **D1-8,** which was directly used in the next reaction step without further purification.

$^1H$ NMR (400 MHz, DMSO-*d6*) δ 9.44 (br s, 1H), 8.35 - 8.25 (m, 2H), 8.09 (br d, *J* = 9.2 Hz, 1H), 7.91 (br s, 1H), 7.35 (br s, 2H), 4.70 - 4.59 (m, 1H), 4.50 - 4.38 (m, 1H), 3.68 (br s, 3H). MS (ESI) *m/z* $(M+H)^+$ = 494.0.

**Step 8:** Preparation of compound **D1-9**

A solution of compound **D1-8** (100 mg, 202.29 μmol), 1-(*tert*-butoxycarbonyl)piperazine (150.00 mg, 805.37 μmol), tris(dibenzylideneacetone)dipalladium (37.50 mg, 40.95 μmol), 2-dicyclohexylphosphino-2',6'-diisopropoxy-1,1'-biphenyl (25.00 mg, 53.57 μmol), and sodium *tert*-butoxide (71.43 mg, 743.27 μmol) in toluene (5 mL) was stirred at 100°C for 1 hour under argon atmosphere. After the reaction was completed, the system was filtered and concentrated. The residue was dissolved in ethyl acetate (20 mL) and washed with saturated brine (10 mL). The organic phases were combined, dried over anhydrous sodium sulfate, filtered, and the filtrate was concentrated to obtain a crude product. The crude product was slurried with a mixed solvent of petroleum ether/ethyl acetate (v/v = 5:1), filtered, and the filter cake was dried to obtain compound **D1-9,** which was directly used in the next reaction step without further purification. MS (ESI) *m/z* $(M+H)^+$ = 600.4.

**Step 9:** Preparation of compound **D1-10**

To a solution of compound **D1-9** (100 mg, 166.76 μmol) in dichloromethane (4 mL) was added trifluoroacetic acid (1.54 g, 13.51 mmol, 1 mL). After the addition was completed, the system was stirred at room temperature (20°C) for 1 hour. After the reaction was completed, the system was concentrated. The residue was dissolved in ethyl acetate (30 mL), and sequentially washed with saturated sodium bicarbonate (10 mL) and saturated brine (10 mL). The organic phases were combined, dried over anhydrous sodium sulfate, and filtered. The filtrate was concentrated under reduced pressure to obtain compound **D1-11,** which was directly used in the next reaction step without further purification. MS (ESI) *m/z* $(M+H)^+$ = 500.1.

**Step 10:** Preparation of compound **D1**

To a solution of compound **D1-10** (85 mg, 170.15 μmol) and compound **A1-8** (51.00 mg, 340.97 μmol) in acetonitrile (1 mL) and *N,N*-dimethylformamide (3 mL) was added potassium carbonate (69 mg, 499.24 μmol), and the reaction mixture was stirred at room temperature (25°C) for 24 hours. Saturated sodium bicarbonate aqueous solution (20 mL) and dichloromethane (20 mL) were added thereto. The organic phases were combined, washed with saturated brine (20 mL), dried over anhydrous sodium sulfate, and filtered. The filtrate was concentrated to obtain a crude product, which was purified by preparative high performance liquid chromatography (separation conditions: chromatographic column: Phenomenex Gemini-NX 80 × 40 mm × 3 μm; mobile phase: water (10 mM ammonium bicarbonate)-acetonitrile; acetonitrile: 30% to 60% 9 min) to obtain compound **D1.**

$^1H$ NMR (400 MHz, CHLOROFORM-*d*) δ 8.34 (d, *J* = 9.5 Hz, 1H), 8.15 - 8.10 (m, 2H), 7.67 - 7.62 (m, 1H), 7.17 (t, *J* = 8.5 Hz, 2H), 6.88 (d, *J* = 2.5 Hz, 1H), 6.65 (s, 1H), 4.71 (br s, 1H), 4.54 (s, 2H), 4.48 - 4.43 (m, 1H), 4.35 - 4.26 (m, 1H), 4.13 - 4.09 (m, 1H), 3.94 - 3.90 (m, 1H), 3.71 (s, 3H), 3.44 (br s, 4H), 3.12 (s, 2H), 2.72 (br s, 4H), 2.25 - 2.19 (m, 1H). MS (ESI) *m/z* $(M+H)^+$ = 613.1. HPLC retention time: 2.934 min.

Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.

**Synthesis of examples E1-a and E1-b**

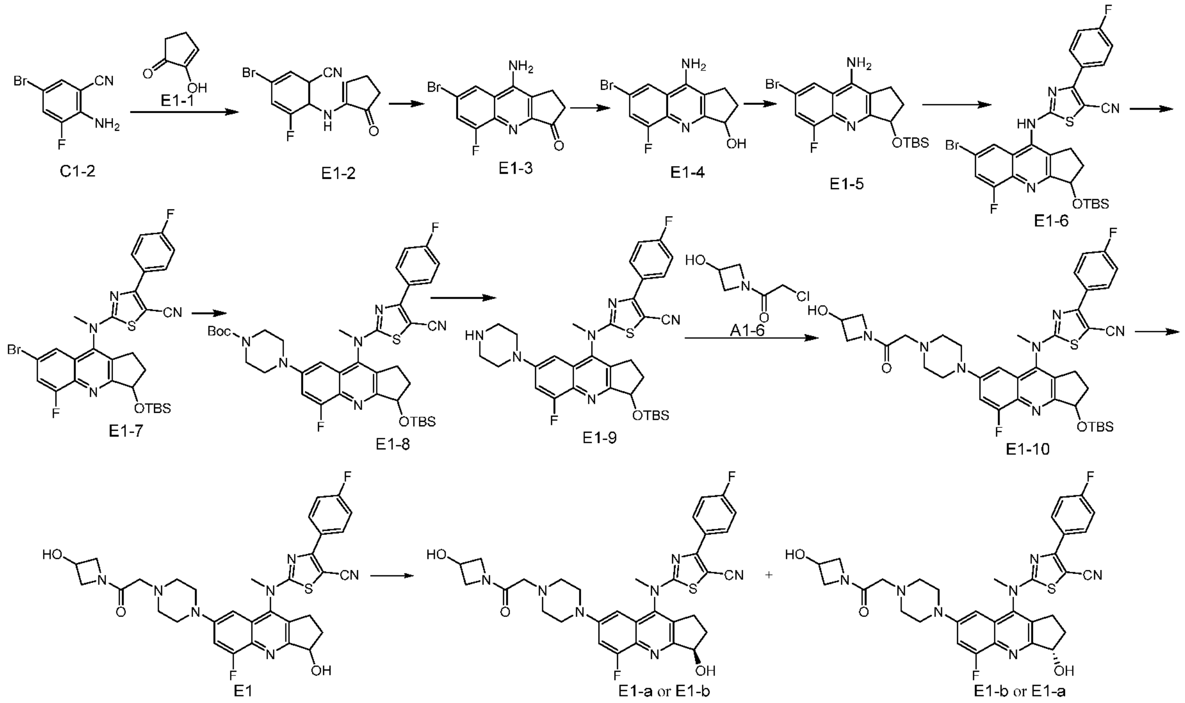


**Step 1:** Preparation of compound **E1-2**

To a solution of compound **C1-2** (1.8 g, 8.37 mmol) and compound **E1-1** (900.00 mg, 9.17 mmol) in toluene (20 mL) was added *p*-toluenesulfonic acid (50 mg, 262.85 μmol). The reaction mixture was heated to 140°C and stirred for 2 hours. The system was concentrated to obtain compound **E1-2,** which was directly used in the next reaction step without further purification.

$^1$H NMR (400 MHz, DMSO-d6) δ = 8.02 (s, 1H), 7.92 - 7.83 (m, 2H), 6.55 (br d, *J* = 1.8 Hz, 1H), 2.45 (br d, *J* = 4.2 Hz, 4H). MS (ESI) *m/z* $(M+1)^+$ = 295.0.

**Step 2:** Preparation of compound **E1-3**

Compound **E1-2** (2.5 g, 8.47 mmol) was dissolved in tetrahydrofuran (20 mL), and lithium diisopropylamide (2 M, 12.8 mL) was added thereto. After the addition was completed, the system was stirred at room temperature (25°C) for 30 minutes under argon atmosphere. The system was added with saturated ammonium chloride aqueous solution (10 mL) and extracted with ethyl acetate (50 mL × 2). The organic phases were combined, washed with saturated brine, dried over anhydrous sodium sulfate, filtered, and the filtrate was concentrated to obtain a crude product. The crude product was purified by medium-pressure column chromatography (methanol/dichloromethane (v/v) = 0 to 30%) to obtain compound **E1-3.** MS (ESI) *m/z* $(M+1)^+$ = 294.5.

**Step 3:** Preparation of compound **E1-4**

To a solution of compound **E1-3** (350 mg, 1.19 mmol) in tetrahydrofuran (5 mL) and methanol (5 mL) was added sodium borohydride (50.00 mg, 1.32 mmol) at room temperature, and the reaction mixture was stirred for 1 hour. After the reaction was completed, the reaction mixture was added with water (20 mL) to quench, and extracted with ethyl acetate (20 mL × 2). The organic phases were combined, washed with saturated brine, dried over anhydrous sodium sulfate, and filtered. The filtrate was concentrated to obtain a crude product, which was purified by medium-pressure column chromatography (methanol/dichloromethane (v/v) = 0 to 30%) to obtain compound **E1-4.**

$^1$H NMR (400 MHz, DMSO-d6) δ = 8.29 (s, 1H), 7.61 (br d, *J* = 10.8 Hz, 1H), 6.79 (s, 2H), 5.41 (d, *J* = 5.8 Hz, 1H), 4.90 (q, *J* = 5.9 Hz, 1H), 2.94 - 2.62 (m, 2H), 2.42 - 2.06 (m, 2H). MS (ESI) *m/z* $(M+1)^+$ = 299.0.

**Step 4:** Preparation of compound **E1-5**

To a solution of compound **E1-4** (130 mg, 437.53 μmol) in dichloromethane (5 mL) were added imidazole (65.00 mg, 954.80 μmol) and *tert*-butyldimethylsilyl chloride (78.00 mg, 517.52 μmol), and the reaction mixture was

stirred at room temperature for 18 hours. After the reaction was completed, the reaction mixture was added with water (20 mL) to quench, and extracted with dichloromethane (20 mL × 2). The organic phases were combined, washed with saturated brine, dried over anhydrous sodium sulfate, and filtered. The filtrate was concentrated to obtain a crude product, which was purified by medium-pressure column chromatography (ethyl acetate/petroleum ether (v/v) = 0 to 40%) to obtain compound **E1-5.**

$^1$H NMR (400 MHz, DMSO-*d6*) δ = 8.28 (s, 1H), 7.59 (dd, *J* = 1.5, 10.3 Hz, 1H), 6.80 (s, 2H), 5.11 (t, *J* = 6.3 Hz, 1H), 2.94 - 2.57 (m, 2H), 2.45 - 2.35 (m, 1H), 1.93 - 1.81 (m, 1H). MS (ESI) *m/z* $(M+1)^+$= 411.1.

**Step 5:** Preparation of compound **E1-10**

Similar to the synthesis of example **A1,** compound **E1-10** was synthesized. MS (ESI) *m/z* $(M+1)^+$ = 746.3.

**Step 6:** Preparation of compound **E1**

Compound **E1-10** (80 mg, 107.25 μmol) was dissolved in tetrahydrofuran (2 mL), and tetrabutylammonium fluoride (1 M, 160 μL) was added thereto. After the addition was completed, the system was stirred at room temperature (25°C) for 2 hours. The system was diluted with water (20 mL) and extracted with ethyl acetate (20 mL × 2). The organic phases were combined, washed with saturated brine, dried over anhydrous sodium sulfate, filtered, and the filtrate was concentrated to obtain a crude product. The crude product was purified by preparative high performance liquid chromatography (separation conditions: chromatographic column: Phenomenex Gemini-NX 80 × 30 mm × 3 μm; mobile phase: water (10 mM ammonium bicarbonate)-acetonitrile; acetonitrile: 42% to 72% 9 min) to obtain compound **E1.**

$^1$H NMR (400 MHz, Chloroform-*d*) δ = 8.14 (br s, 2H), 7.24 - 7.13 (m, 2H), 7.24 - 7.13 (m, 1H), 6.66 (s, 1H), 5.44 - 5.31 (m, 1H), 4.70 (br s, 1H), 4.51 - 4.39 (m, 1H), 4.35 - 4.25 (m, 1H), 4.13 (br dd, *J* = 4.1, 9.7 Hz, 1H), 3.92 (br d, *J* = 7.5 Hz, 1H), 3.64 (s, 3H), 3.54 - 3.43 (m, 1H), 3.35 (br s, 4H), 3.21 - 2.99 (m, 4H), 2.96 - 2.82 (m, 1H), 2.79 - 2.60 (m, 5H), 2.18 (qd, *J* = 6.9, 13.8 Hz, 1H). MS (ESI) *m/z* $(M+1)^+$ = 632.2.

HPLC retention time: 3.433 min.

Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.

**Step 7:** Preparation of compounds **E1-a** and **E1-b**

Compound **E1** was purified by SFC (separation conditions: chromatographic column: DAICEL CHIRALCEL OD H (250 mm * 30 mm, 5 μm); mobile phase: [$CO_2$-methanol (0.1% ammonia water)]; methanol%: 50%). After concentration, compound **E1-a** and compound **E1-b** were obtained.

**Compound E1-a:** $^1$H NMR (400 MHz, Chloroform-*d*) δ = 8.15 (br s, 2H), 7.18 (br t, *J* = 8.4 Hz, 2H), 6.66 (br s, 1H), 5.42 - 5.32 (m, 1H), 4.69 (br s, 1H), 4.46 (br s, 1H), 4.30 (br s, 1H), 4.11 (br d, *J* = 6.4 Hz, 1H), 3.91 (br d, *J* = 7.3 Hz, 1H), 3.65 (s, 2H), 3.33 (br s, 3H), 3.23 - 3.19 (m, 1H), 3.19 - 3.17 (m, 1H), 3.11 (br s, 1H), 3.22 - 3.01 (m, 1H), 2.98 - 2.81 (m, 2H), 2.69 (br s, 4H). MS (ESI) m/z $(M+H)^+$ = 632.6. SFC retention time: 7.004 min.

Separation conditions: chromatographic column: DAICEL CHIRALCEL OD-H (250 mm * 30 mm, 5 μm); column temperature: 35°C; mobile phase: $CO_2$-methanol (0.05% DEA); methanol: 5% to 40% 5 min, 40% 2.5 min, 5% 2.5 min; flow rate: 2.5 mL/min. HPLC retention time: 4.55 min.

Separation conditions: chromatographic column: Xbridge Shield RP-18, 5 μm, 2.1 * 50 mm; column temperature: 40°C; mobile phase: water-acetonitrile; acetonitrile: 0% to 60% 4 min, 60% 2 min; flow rate: 1.2 mL/min.

**Compound E1-b:** $^1$H NMR (400 MHz, Methanol-$d_4$) δ = 8.09 (br s, 2H), 7.45 (br d, *J* = 14.1 Hz, 1H), 7.22 (br t, *J* = 8.7 Hz, 2H), 6.79 (s, 1H), 5.33 - 5.17 (m, 1H), 4.58 (br s, 1H), 4.51 - 4.39 (m, 1H), 4.22 (dd, *J* = 7.0, 10.3 Hz, 1H), 4.04 (br d, *J* = 7.3 Hz, 1H), 3.85 - 3.74 (m, 1H), 3.78 (dd, *J* = 4.0, 10.5 Hz, 1H), 3.71 - 3.63 (m, 1H), 3.67 (d, *J* = 6.5 Hz, 2H), 3.40 (br s, 4H), 3.35 - 3.22 (m, 8H), 3.20 - 3.03 (m, 1H), 3.01 - 2.73 (m, 6H), 2.64 - 2.49 (m, 1H), 2.09 (qd, *J* = 6.4, 12.8 Hz, 1H). MS (ESI) *m/z* $(M+H)^+$ = 632.1. SFC retention time: 7.708 min.

Separation conditions: chromatographic column: DAICEL CHIRALCEL OD-H (250 mm * 30 mm, 5 μm); column temperature: 35°C; mobile phase: $CO_2$-methanol (0.05% DEA); methanol: 5% to 40% 5 min, 40% 2.5 min, 5% 2.5 min; flow rate: 2.5 mL/min. HPLC retention time: 4.54 min.

Separation conditions: chromatographic column: Xbridge Shield RP-18, 5 μm, 2.1 * 50 mm; column temperature: 40°C; mobile phase: water-acetonitrile; acetonitrile: 0% to 60% 4 min, 60% 2 min; flow rate: 1.2 mL/min.

**Synthesis of example E2**

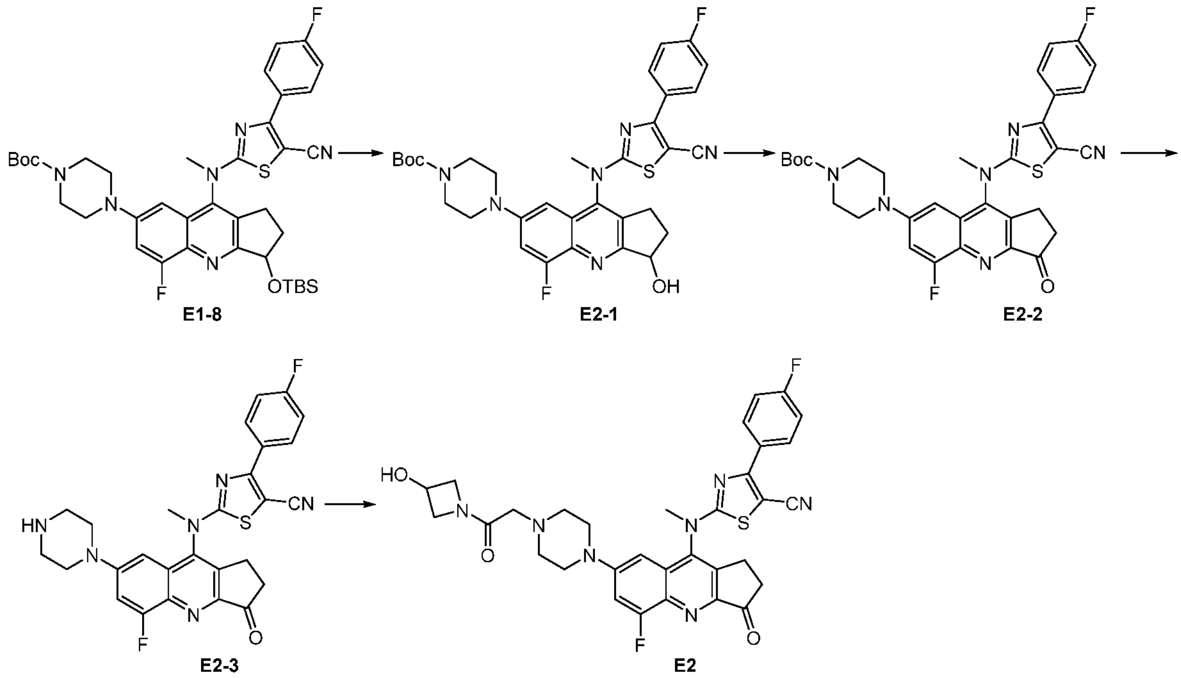

E1-8 E2-1 E2-2

E2-3 E2

**Step 1:** Preparation of compound **E2-1**

To a solution of compound **E1-8** (120 mg, 163.72 μmol) in tetrahydrofuran (5 mL) was added tetrabutylammonium fluoride (1 M, 0.2 mL), and the reaction mixture was stirred at room temperature (20°C) for 1 hour. After the reaction was completed, the reaction mixture was diluted with ethyl acetate (50 mL), and sequentially washed with saturated brine (30 mL × 2) and water (30 mL × 2). The organic phase was dried over anhydrous sodium sulfate, filtered, and the filtrate was concentrated to obtain compound **E2-1,** which was directly used in the next reaction step without further purification.

$^1$H NMR (400 MHz, Chloroform-*d*) δ = 8.15 (br s, 2H), 7.23 (br dd, *J* = 2.1, 13.1 Hz, 2H), 7.18 (br t, *J* = 8.6 Hz, 2H), 6.69 (s, 1H), 5.37 (td, *J* = 6.5, 12.9 Hz, 1H), 3.70 - 3.60 (m, 7H), 3.27 (br s, 4H), 3.18 - 2.99 (m, 2H), 2.94 - 2.84 (m, 1H), 2.65 (br dd, *J* = 4.2, 12.7 Hz, 1H), 2.25 - 2.10 (m, 1H), 1.49 - 1.46 (m, 9H). MS (ESI) *m/z* $(M+1)^+$ = 619.3.

**Step 2:** Preparation of compound **E2-2**

Compound **E2-1** (85 mg, 137.39 μmol) was dissolved in dichloromethane (5 mL), then manganese dioxide (85 mg, 977.69 μmol) was added thereto, and the reaction mixture was stirred at room temperature for 18 hours. The reaction mixture was filtered and concentrated under reduced pressure to obtain a crude product, which was purified by medium-pressure column chromatography (methanol/dichloromethane (v/v) = 0 to 5%) to obtain compound **E2-2.** MS (ESI) *m/z* $(M+1)^+$ = 617.3.

**Step 3:** Preparation of compound **E2**

Similar to the synthesis of example **A1,** the following example **E2** was synthesized.

$^1$H NMR (400 MHz, Chloroform-*d*) δ = 8.13 (br s, 2H), 7.32 (br s, 1H), 7.17 (br t, *J* = 8.5 Hz, 2H), 6.60 (s, 1H), 4.71 (br s, 1H), 4.44 (br s, 1H), 4.35 - 4.26 (m, 1H), 4.10 (br dd, *J* = 3.3, 9.5 Hz, 1H), 3.91 (br d, *J* = 14.6 Hz, 1H), 3.70 (s, 3H), 3.46 (br s, 4H), 3.20 (br t, *J* = 5.9 Hz, 2H), 3.11 (s, 2H), 2.93 - 2.84 (m, 2H), 2.71 (br s, 4H), 2.42 (br s, 1H). MS (ESI) *m/z* $(M+H)^+$ = 630.1. HPLC retention time: 3.7712 min.

Separation conditions: chromatographic column: Xbridge Shield RP-18, 5 μm, 2.1 * 50 mm; column temperature: 50°C; mobile phase: water (0.2 M/L ammonia water)-acetonitrile; acetonitrile: 10% to 80% 6 min, 80% 2 min; flow rate: 0.8 mL/min.

**Synthesis of example E3**

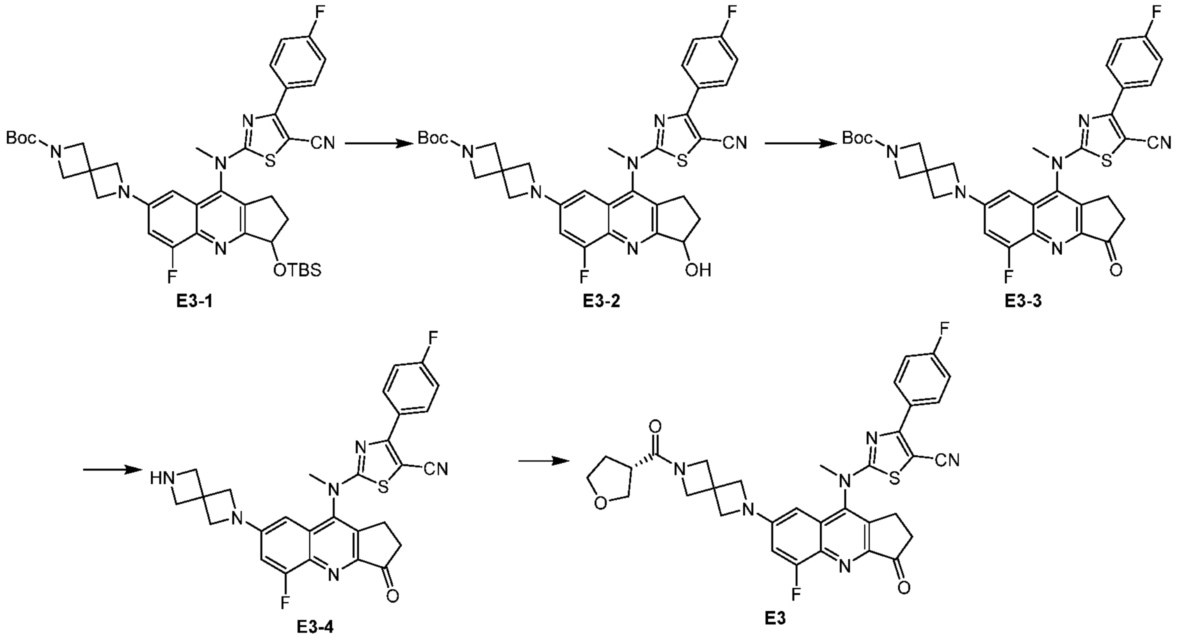

E3-1 E3-2 E3-3

E3-4 E3

Similar to the synthesis of compound **E2-3,** compound **E3-4** was synthesized from compound **E3-1.**

To a solution of compound **E3-4** (40 mg, 75.68 μmol) and (*S*)-tetrahydrofuran-3-carboxylic acid (13.33 mg, 114.83 μmol) in dichloromethane (3 mL) were added 2-(7-azabenzotriazol-1-yl)-*N*,*N*,*N'*,*N'*-tetramethyluronium hexafluorophosphate (46.67 mg, 122.73 μmol) and triethylamine (48.47 mg, 478.97 μmol, 66.67 μL), and the reaction mixture was stirred at room temperature (25°C) for 18 hours. After the reaction was completed, the reaction mixture was diluted with water (20 mL), extracted with ethyl acetate (20 mL × 2), and washed with saturated brine (20 mL × 2). The organic phases were combined, dried over anhydrous sodium sulfate, filtered, and concentrated to obtain a crude product, which was purified by preparative high performance liquid chromatography to obtain compound **E3.**

$^1H$ NMR (400 MHz, CHLOROFORM-*d*) δ = 8.16 (br s, 2H), 7.19 (t, *J* = 8.7 Hz, 2H), 6.82 (br d, *J* = 8.8 Hz, 1H), 6.19 - 6.11 (m, 1H), 4.40 (br s, 2H), 4.25 (br s, 6H), 4.00 - 3.80 (m, 4H), 3.70 (s, 3H), 3.26 - 3.17 (m, 2H), 2.96 - 2.89 (m, 3H), 2.17 (s, 1H), 2.08 (br s, 1H). MS (ESI) m/z $(M+1)^+$ = 627.1. HPLC retention time: 3.934 min.

Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.

Similar to the synthesis of example **E3,** example **E4** was synthesized as shown in Table 4 below.

Table 4: Structural formula and analytical data of example **E4**

| Example | Structural formula | Analytical data |
|---|---|---|
| E4 | 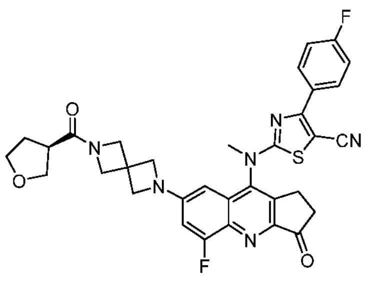 | $^1H$ NMR (400 MHz, CHLOROFORM-*d*) δ = 8.14 (br s, 1H), 8.25 - 8.00 (m, 1H), 7.22 - 7.13 (m, 1H), 7.16 (br s, 1H), 6.91 - 6.76 (m, 1H), 6.95 - 6.76 (m, 1H), 6.13 (s, 1H), 6.25 - 6.01 (m, 1H), 4.53 (br s, 2H), 4.41 - 4.28 (m, 3H), 4.24 (s, 2H), 4.27 - 4.16 (m, 1H), 3.95 - 3.77 (m, 4H), 3.71 - 3.59 (m, 3H), 3.38 (br s, 1H), 3.20 (br d, *J* = 5.8 Hz, 1H), 2.92 (br d, *J* = 6.8 Hz, 3H), 2.17 (br s, 1H), 2.07 (br s, 1H). MS (ESI) *m/z* $(M+1)^+$ = 627.1.<br>HPLC retention time: 3.926 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. |

**Synthesis of example E5**

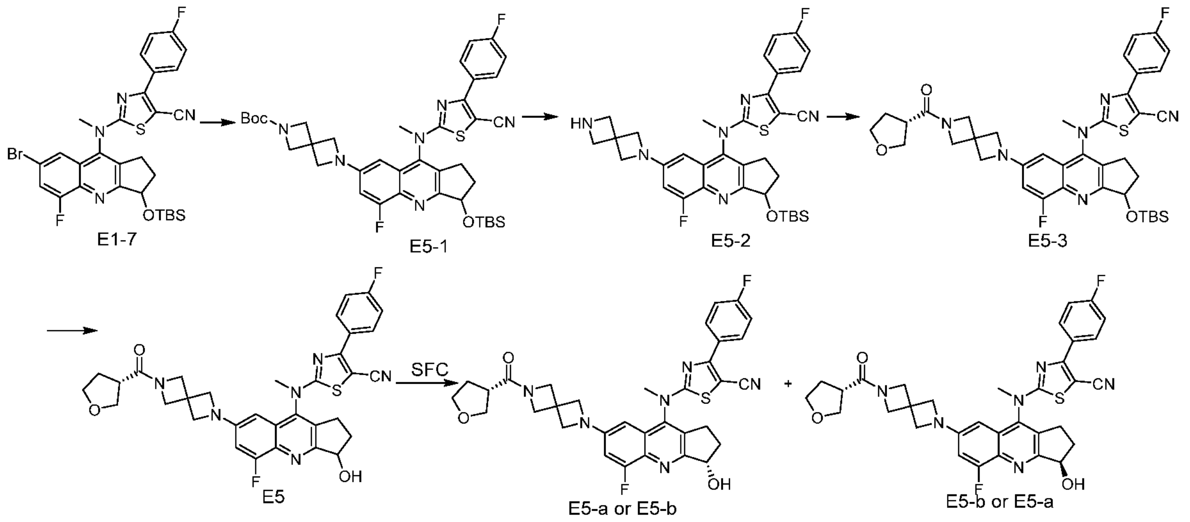


**Step 1:** Preparation of compound **E5-1**

Similar to the synthesis of compound **E1-8,** compound **E5-1** was synthesized from compound **EI-7.**

**Step 2:** Preparation of compound **E5-2**

Similar to the synthesis of compound **E1-9,** compound **E5-2** was synthesized from compound **E5-1.**

**Step 3:** Preparation of compound **E5-3**

Similar to the synthesis of compound **E3**

**Step 4:** Preparation of compound **E5**

Similar to the synthesis of compound **E1,** compound **E5** was synthesized from compound **E5-3.**

$^1$H NMR (400 MHz, CHLOROFORM-*d*) δ = 8.15 (br s, 2H), 7.18 (t, *J* = 8.5 Hz, 2H), 6.79 - 6.68 (m, 1H), 6.20 (s, 1H), 5.41 - 5.28 (m, 1H), 4.44 - 4.32 (m, 2H), 4.21 (s, 2H), 4.14 (br s, 4H), 4.02 - 3.95 (m, 1H), 3.94 - 3.88 (m, 1H), 3.87 - 3.76 (m, 2H), 3.63 (s, 3H), 3.35 (br s, 1H), 3.10 (dt, *J* = 4.5, 8.7 Hz, 1H), 2.97 - 2.84 (m, 2H), 2.69 - 2.60 (m, 1H), 2.24 - 2.13 (m, 2H), 2.11 - 2.01 (m, 1H). MS (ESI) *m/z* $(M+1)^+$ = 629.1. HPLC retention time: 3.79 min.

Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.

**Step 5:** Preparation of compound **E5-a** or **E5-b**

Compound **E5** was purified by SFC to obtain compound **E5-a** and compound **E5-b.**

**Compound E5-a:** $^1$H NMR (400 MHz, CHLOROFORM-*d*) δ = 8.14 (br s, 2H), 7.18 (t, *J* = 8.5 Hz, 2H), 6.73 (br d, *J* = 11.8 Hz, 1H), 6.20 (s, 1H), 5.39 (br d, *J* = 6.0 Hz, 1H), 4.37 (q, *J* = 8.6 Hz, 2H), 4.21 (s, 2H), 4.15 (br s, 4H), 3.99 (t, *J* = 8.2 Hz, 1H), 3.95 - 3.76 (m, 3H), 3.63 (s, 3H), 3.09 (br s, 1H), 2.98 - 2.83 (m, 2H), 2.72 - 2.58 (m, 1H), 2.25 - 2.12 (m, 2H), 2.11 - 2.02 (m, 1H). MS (ESI) *m/z* $(M+H)^+$ = 629.1. SFC retention time: 2.172 min.

Separation conditions: chromatographic column: Chiralpak AD-3 50 mm * 4.6 mm I.D., 3 μm; column temperature: 35°C; mobile phase: $CO_2$-ethanol (0.05% DEA); ethanol: 5% to 40% 2 min, 40% 1.2 min, 5% 0.8 min; flow rate: 0.8 mL/min. HPLC retention time: 3.769 min.

Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.

**Compound E5-b:** $^1$H NMR (400 MHz, CHLOROFORM-*d*) δ = 8.14 (br s, 2H), 7.18 (br t, *J* = 8.4 Hz, 2H), 6.73 (br d, *J* = 11.3 Hz, 1H), 6.20 (s, 1H), 5.45 - 5.31 (m, 1H), 4.46 - 4.28 (m, 2H), 4.21 (br s, 2H), 4.14 (br s, 4H), 3.99 (br t, *J* = 7.8 Hz, 1H), 3.94 - 3.76 (m, 3H), 3.63 (s, 3H), 3.09 (br s, 1H), 2.98 - 2.83 (m, 2H), 2.70 - 2.59 (m, 1H), 2.26 - 1.98 (m, 3H). MS (ESI) *m/z* $(M+H)^+$ = 629.2. SFC retention time: 2.566 min.

Separation conditions: chromatographic column: Chiralpak AD-3 50 mm * 4.6 mm I.D., 3 μm; column temperature: 35°C; mobile phase: $CO_2$-ethanol (0.05% DEA); ethanol: 5% to 40% 2 min, 40% 1.2 min, 5% 0.8 min; flow rate: 0.8 mL/min. HPLC retention time: 3.816 min.

Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.

**Synthesis of example E25**

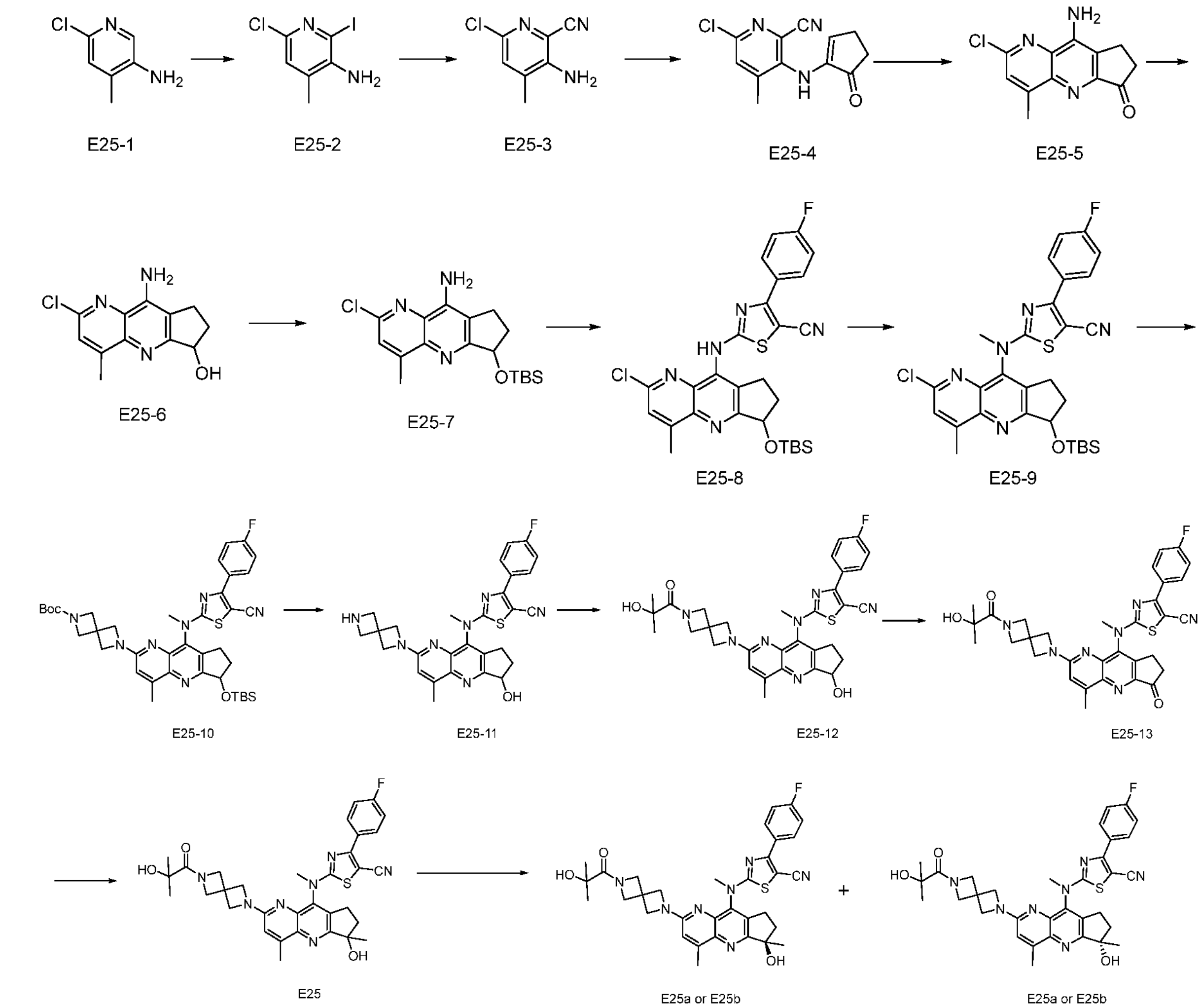

E25-1 E25-2 E25-3 E25-4 E25-5

E25-6 E25-7 E25-8 E25-9

E25-10 E25-11 E25-12 E25-13

E25 E25a or E25b + E25a or E25b

**Step 1:** Preparation of compound **E25-2**

Compound **E25-1** (8.0 g, 56.1 mmol) was dissolved in DMF (60 mL). The reaction mixture was cooled in an ice-water bath, then added with NIS (16.4 g, 72.9 mmol) in batches, and naturally warmed to room temperature and stirred overnight. The reaction mixture was poured into saturated sodium sulfite aqueous solution (300 mL) and extracted with EtOAc (300 mL * 2). The organic phase was washed with saturated brine (200 mL * 2), dried, concentrated, and subjected to normal phase column chromatography (EtOAc/PE = 0 to 25%) to obtain the title compound **E25-2** (8.2 g, light brown solid, yield: 54.3%). LC-MS (ESI): *m/z* $[M+H]^+$: 269.0.

**Step 2:** Preparation of compound **E25-3**

Compound **E25-2** (4.1 g, 15.3 mmol) was dissolved in DMF (30 mL) at room temperature, then zinc cyanide (1.97 g, 16.8 mmol) and $Pd(PPh_3)_4$ (0.88 g, 0.76 mmol) were added thereto, and the reaction mixture was stirred at 80°C for 4 hours under nitrogen atmosphere. LC-MS monitored that the reaction was completed. The reaction mixture

was cooled to room temperature, poured into cold sodium bicarbonate aqueous solution (100 mL), added with ethyl acetate (100 mL), filtered through diatomite, rinsed with EtOAc, and the filtrate was extracted with ethyl acetate (200 mL * 2). The organic phase was washed with saturated brine (150 mL * 2), dried over anhydrous sodium sulfate, concentrated, and subjected to normal phase column chromatography (EtOAc/PE = 0 to 30%) to obtain the title compound **E25-3** (2.25 g, yellow solid, yield: 87.9%). LC-MS (ESI): *m*/*z* $[M+H]^+$: 168.2.

**Step 3:** Preparation of compound **E25-4**

Compound **E25-3** (2.0 g, 11.9 mmol) was dissolved in toluene (30 mL) at room temperature, and *p*-toluenesulfonic acid (0.1 g, 0.6 mmol) and compound **E1-1** (1.17 g, 11.9 mmol) were added thereto. The reaction mixture was heated to 170°C, refluxed, subjected to water separation using a trap, and reacted for 4 hours. The reaction mixture was cooled, concentrated, and subjected to normal phase column chromatography (MeOH/DCM = 0 to 5%) to obtain the title compound **E25-4** (1.62 g, yellow solid, yield: 81.0%). LC-MS (ESI): *m*/*z* $[M+H]^+$: 248.1.

**Step 4:** Preparation of compound **E25-5**

To compound **E25-4** (2.1 g, 8.5 mmol) in THF (16 mL) under nitrogen atmosphere was slowly added dropwise LiHMDS (17 mL, 1.0 M, 17 mmol) at -78°C, and the temperature was controlled at -60°C or below. After the dropwise addition was completed, the reaction system was stirred at a maintained temperature of -60°C or below for 1 hour. LC-MS monitored that the reaction was completed. The reaction mixture was poured into ammonium chloride aqueous solution (20 mL) to precipitate a solid, and filtered. The filter cake was rinsed with water and naturally dried to obtain the title compound **E25-5** (4.2 g, crude product). LC-MS (ESI): *m*/*z* $[M+H]^+$: 248.1.

**Step 5:** Preparation of compound **E25-6**

To a mixed solvent of compound **E25-5** (4.2 g, crude product) in MeOH (25 mL) and THF (25 mL) was added $NaBH_4$ (0.32 g, 8.5 mmol) in batches at room temperature. After the addition was completed, the reaction mixture was stirred at room temperature for 1 hour. LC-MS monitored that the reaction was completed. The reaction mixture was concentrated to remove most of the solvent, added with half-saturated ammonium chloride aqueous solution, slurried at room temperature for about 10 minutes, and filtered. The filter cake was rinsed with water, added with acetonitrile, and concentrated with water to obtain the title compound **E25-6** (1.34 g, gray solid, two-step yield: 64.1%). LC-MS (ESI): *m*/*z* $[M+H]^+$: 250.2.

**Step 6:** Preparation of compound **E25-7**

To compound **E25-6** (2.6 g, 10.4 mmol) in DMF (18 mL) were added TBSCl (3.14 g, 20.82 mmol) and imidazole (1.77 g, 26.0 mmol) at room temperature, and the reaction mixture was stirred at room temperature overnight. LC-MS monitored that the reaction was completed. The reaction mixture was poured into water, extracted with EtOAc, washed with saturated brine, dried, concentrated, and purified by column chromatography (EtOAc/PE = 0 to 20%) to obtain the title compound **E25-7** (2.87 g, gray solid, yield: 75.9%). LC-MS (ESI): *m*/*z* $[M+H]^+$: 364.2.

**Step** 7: Preparation of compound **E25-8**

To a solvent of NaH (1.17 g, 29.25 mmol) in dry DMF (5 mL) was added dropwise a solution of compound **A1-3** (2.13 g, 5.85 mmol) in DMF (10 mL) in an ice bath. After the dropwise addition was completed, the reaction mixture was stirred in an ice-water bath for about 30 minutes. A solution of compound **E25-7** (1.82 g, 7.61 mmol) in DMF (10 mL) was then added dropwise thereto. After the dropwise addition was completed, the reaction mixture was naturally warmed to room temperature and stirred for 2 hours. LC-MS monitored that the reaction was completed. The reaction mixture was poured into water and extracted with EtOAc. The organic phase was concentrated and subjected to column chromatography (EtOAc/PE = 0 to 20%) to obtain the title compound **E25-8** (2.18 g, light yellow solid, yield: 65.9%). LC-MS (ESI): *m*/*z* $[M+H]^+$: 566.1.

**Step 8:** Preparation of compound **E25-9**

To a solution of compound **E25-8** (1.2 g, 2.12 mmol) in DMF (8.0 mL) were sequentially added potassium carbonate (1.17 g, 8.48 mmol) and MeI (0.6 g, 4.24 mmol) in an ice-water bath, and the reaction mixture was naturally warmed to room temperature and stirred for 2 hours. LC-MS monitored that the reaction was completed. The reaction mixture was added with water and extracted with EtOAc. The organic phase was evaporated to dryness by rotary

evaporation and purified by column chromatography (EtOAc/PE = 0 to 20%) to obtain the title compound **E25-9** (1.15 g, green solid, yield: 93.5%). LC-MS (ESI): *m/z* $[M+H]^+$: 580.1.

**Step 9:** Preparation of compound **E25-10**

To NMP (2.5 mL) were added compound **E25-9** (0.46 g, 0.79 mmol), compound *tert*-butyl 2,6-diazaspiro[3.3] heptane-2-carboxylate hemioxalate (0.46 g, 1.9 mmol), and DIEA (0.46 g, 3.56 mmol). The reaction mixture was heated to 140°C and reacted overnight. LC-MS monitored that the reaction was completed. The reaction mixture was poured into water (20 mL) to precipitate a solid and filtered. The filter cake was dissolved in ethyl acetate and washed once with saturated brine. The organic phase was dried over anhydrous sodium sulfate, concentrated, and purified by column chromatography (EtOAc/PE = 0 to 30%) to obtain the title compound **E25-10** (0.36 g, light yellow solid, yield: 61.0%). LC-MS (ESI): *m/z* $[M+H]^+$: 742.3.

**Step 10:** Preparation of compound **E25-11**

To a solution of **E25-10** (1.5 g, 2.02 mmol) in methanol (10 mL) was added 6 N hydrochloric acid (8.0 mL) at room temperature, and the reaction system was stirred at room temperature overnight. LC-MS monitored that the reaction was completed. The reaction mixture was slowly added with saturated sodium bicarbonate aqueous solution to adjust the pH to alkalinity, and extracted with DCM. The organic phase was concentrated and purified by column chromatography (MeOH/DCM = 0 to 10%) to obtain the title compound **E25-11** (0.74 g, light yellow solid, yield: 69.1%). LC-MS (ESI): *m/z* $[M+H]^+$: 528.1.

**Step 11:** Preparation of compound **E25-12**

To a reaction flask containing DMF (8.0 mL) were added compound **E25-11** (1.11 g, 2.1 mmol) and 2-methyl-2-hydroxypropionic acid (0.24 g, 2.31 mmol), then the reaction mixture was cooled in an ice-water bath, and HATU (0.96 g, 2.53 mmol) and DIEA (0.68 g, 5.25 mmol) were added thereto. The ice-water bath was then removed, and the reaction mixture was stirred at room temperature for about 3 hours. The reaction mixture was added with water, extracted twice with EtOAc, and washed with brine. The organic phase was dried, concentrated, and purified by column chromatography (MeOH/DCM = 0 to 5%) to obtain the title compound **E25-12** (1.03 g, light yellow solid, yield: 79.8%). LC-MS (ESI): *m/z* $[M+H]^+$. 614.7.

**Step 12:** Preparation of compound **E25-13**

Compound **E25-12** (1.14 g, 1.86 mmol) was dissolved in DCM (12 mL) under nitrogen atmosphere, then DMP (1.18 g, 2.79 mmol) was added thereto in batches after an ice-water bath, and the reaction system was warmed to room temperature and stirred for 4 hours. LC-MS monitored that the reaction was completed. The reaction mixture was slowly added with sodium bicarbonate aqueous solution (20 mL) to generate bubbles, and extracted with DCM. The organic phase was washed with brine, dried, concentrated, and purified by normal phase column chromatography (MeOH/DCM = 0 to 2%) to obtain the title compound **E25-13** (0.66 g, light yellow solid, yield: 58.4%). LC-MS (ESI): *m/z* $[M+H]^+$: 612.2.

**Step 13:** Preparation of compound **E25**

Compound **E25-13** (0.53 g, 0.87 mmol) was dissolved in dry THF (7.5 mL), and methylmagnesium bromide (1.16 mL, 3.0 M, 3.48 mmol) was added dropwise thereto at -20°C. After the dropwise addition was completed, the reaction mixture was naturally warmed to room temperature and stirred overnight. The reaction mixture was poured into cold ammonium chloride solution (10 mL) and extracted twice with EtOAc. The organic phase was concentrated and subjected to column chromatography (MeOH/DCM = 0 to 2%) to obtain a crude product, which was subjected to preparative column chromatography (preparation method: mobile phase: A: 0.05% ammonia water; B: acetonitrile; chromatographic column: XBridge C18, 19 * 250 mm * 10 μm, flow rate: 20 mL/min, column temperature: 25°C; gradient: 51% to 51%, collected for 8.6 min to 9.6 min) to obtain the title compound **E25** (light yellow solid, 52 mg, yield: 9.6%).

**Step 14:** Preparation of compounds **E25-a** and **E25-b**

Compound **E25** was purified by SFC to obtain compound **E25-a** and compound **E25-b.**

Similar to the synthesis of examples **E1, E2, E3, E5,** and **E25,** examples **E6** to **E24** and examples **E26** to **E66** were synthesized as shown in Table 5 below.

Table 5: Structural formulas and analytical data of examples **E6** to **E66**

| Example | Structural formula | Analytical data |
|---|---|---|
| E6<br>E6-a<br><br>E6-b | 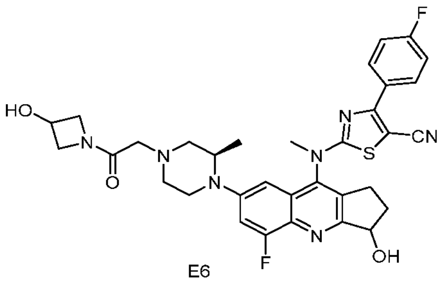<br>E6<br>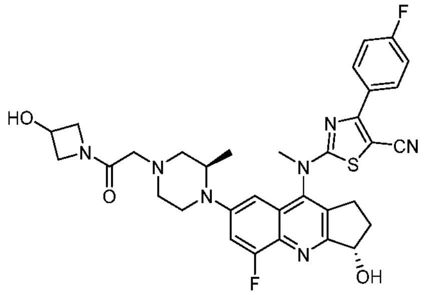<br>E6-a or E6-b<br>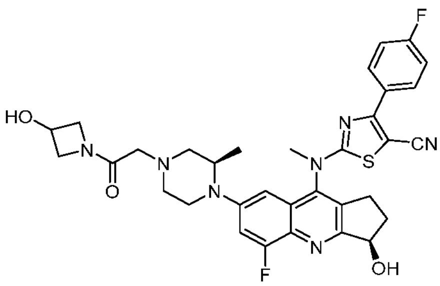<br>E6-b or E6-a | E6:<br>$^1$H NMR (400 MHz, CHLOROFORM-*d*) δ = 8.14 (br s, 2H), 7.24 - 7.05 (m, 3H), 6.62 (br s, 1H), 5.38 (br s, 1H), 4.70 (br s, 1H), 4.49 (br s, 1H), 4.30 (br d, *J* = 6.2 Hz, 1H), 4.17 (br s, 1H), 4.06 (br s, 1H), 3.92 (br s, 1H), 3.64 (br d, *J* = 7.4 Hz, 3H), 3.52 - 3.34 (m, 2H), 3.24 (br s, 1H), 3.12 (br s, 2H), 3.03 (br s, 1H), 2.89 (br s, 2H), 2.62 (br s, 2H), 2.39 (br s, 1H), 2.18 (br s, 1H), 1.24 - 1.12 (m, 3H). MS (ESI) *m/z* $(M+H)^+$ = 646.2.<br>HPLC retention time: 3.571 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.<br>E6-a:<br>$^1$H NMR (400 MHz, CHLOROFORM-*d*) δ = 8.14 (br s, 2H), 7.18 (br t, *J* = 8.4 Hz, 2H), 6.73 (br d, *J* = 11.3 Hz, 1H), 6.20 (s, 1H), 5.45 - 5.31 (m, 1H), 4.46 - 4.28 (m, 2H), 4.21 (br s, 2H), 4.14 (br s, 4H), 3.99 (br t, *J* = 7.8 Hz, 1H), 3.94 - 3.76 (m, 3H), 3.63 (s, 3H), 3.09 (br s, 1H), 2.98 - 2.83 (m, 2H), 2.70 - 2.59 (m, 1H), 2.26 - 1.98 (m, 3H). MS (ESI) *m/z* $(M+1)^+$ = 646.2.<br><br>SFC retention time: 4.424 min.<br><br>Separation conditions: chromatographic column: Chiralcel OD-3 100 mm * 4.6 mm I.D., 3 μm; column temperature: 35°C; mobile phase: $CO_2$-ethanol (0.05% diethylamine); ethanol (0.05% diethylamine): 5% to 40% 4 min, 40% 2.5 min, 5% 1.5 min; 2.8 mL/min.<br>E6-b:<br>$^1$H NMR (400 MHz, CHLOROFORM-*d*) δ = 8.14 (br s, 2H), 7.18 (br t, *J* = 8.4 Hz, 2H), 6.73 (br d, *J* = 11.3 Hz, 1H), 6.20 (s, 1H), 5.45 - 5.31 (m, 1H), 4.46 - 4.28 (m, 2H), 4.21 (br s, 2H), 4.14 (br s, 4H), 3.99 (br t, *J* = 7.8 Hz, 1H), 3.94 - 3.76 (m, 3H), 3.63 (s, 3H), 3.09 (br s, 1H), 2.98 - 2.83 (m, 2H), 2.70 - 2.59 (m, 1H), 2.26 - 1.98 (m, 3H). MS (ESI) *m/z* $(M+1)^+$ = 646.2.<br>SFC retention time: 4.645 min.<br>Separation conditions: chromatographic column: Chiralcel OD-3 100 mm * 4.6 mm I.D., 3 μm; column temperature: 35°C; mobile phase: $CO_2$-ethanol (0.05% diethylamine); ethanol (0.05% diethylamine): 5% to 40% 4 min, 40% 2.5 min, 5% 1.5 min; 2.8 mL/min. |
| | | E7: |

(continued)

| Example | Structural formula | Analytical data |
|---|---|---|
| E7<br>E7-a<br><br>E7-b | 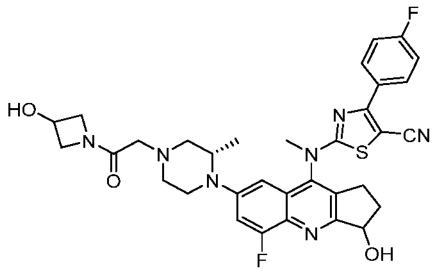<br>E7<br>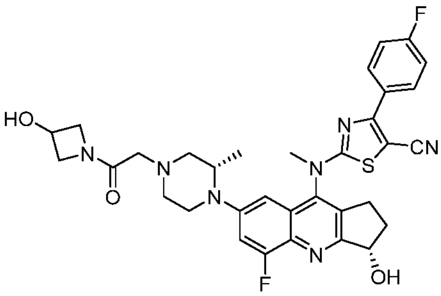<br>E7-a or E7-b<br>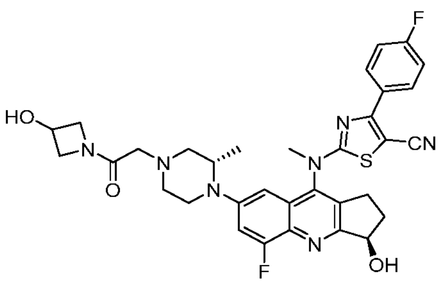<br>E7-b or E7-a | $^1$H NMR (400 MHz, CHLOROFORM-*d*) $\delta$ = 8.14 (br s, 2H), 7.24 - 7.10 (m, 3H), 6.62 (br s, 1H), 5.43 - 5.32 (m, 1H), 4.70 (br s, 1H), 4.49 (br s, 1H), 4.36 - 4.25 (m, 1H), 4.20 - 4.11 (m, 1H), 4.05 (br s, 1H), 3.92 (br d, *J* = 10.0 Hz, 1H), 3.64 (br d, *J* = 7.3 Hz, 3H), 3.54 (br s, 1H), 3.36 (br s, 1H), 3.28 - 3.18 (m, 1H), 3.16 - 3.03 (m, 3H), 2.98 (br s, 1H), 2.94 - 2.75 (m, 3H), 2.65 (br d, *J* = 8.0 Hz, 1H), 2.57 (br d, *J* = 11.3 Hz, 1H), 2.38 (br d, *J* = 10.3 Hz, 1H), 2.18 (br d, *J* = 6.3 Hz, 1H), 1.26 - 1.09 (m, 3H). MS (ESI) *m/z* $(M+1)^+$ = 646.2.<br>HPLC retention time: 3.63 min.<br>Separation conditions: chromatographic column: Ultimate C18 3.0 $\times$ 50 mm, 3 $\mu$m; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min.<br>E7-a:<br>$^1$H NMR (400 MHz, CHLOROFORM-*d*) $\delta$ = 8.13 (br s, 2H), 7.23 - 7.10 (m, 3H), 6.61 (s, 1H), 5.37 (td, *J* = 6.9, 10.5 Hz, 1H), 4.70 (br d, *J* = 2.5 Hz, 1H), 4.48 (br s, 1H), 4.35 - 4.24 (m, 1H), 4.20 - 4.10 (m, 1H), 4.04 (br d, *J* = 9.5 Hz, 1H), 3.96 - 3.87 (m, 1H), 3.64 (d, *J* = 6.8 Hz, 3H), 3.41 - 3.29 (m, 1H), 3.26 - 3.18 (m, 1H), 3.15 - 3.03 (m, 3H), 2.97 (br s, 1H), 2.92 - 2.85 (m, 1H), 2.80 (br t, *J* = 10.7 Hz, 1H), 2.70 - 2.62 (m, 1H), 2.58 - 2.50 (m, 1H), 2.40 - 2.29 (m, 1H), 2.23 - 2.14 (m, 1H), 1.22 - 1.11 (m, 3H). MS (ESI) *m/z* $(M+1)^+$ = 646.2.<br>SFC retention time: 4.758 min.<br>Separation conditions: chromatographic column: Chiralcel OD-3 100 mm * 4.6 mm I.D., 3 $\mu$m; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min.<br>E7-b:<br>$^1$H NMR (400 MHz, CHLOROFORM-*d*) $\delta$ = 8.13 (br s, 2H), 7.24 - 7.12 (m, 3H), 6.61 (br s, 1H), 5.42 - 5.31 (m, 1H), 4.70 (br s, 1H), 4.55 - 4.44 (m, 1H), 4.36 -4.24 (m, 1H), 4.15 (dt, *J* = 4.1, 9.8 Hz, 1H), 4.05 (br d, *J* = 3.1 Hz, 1H), 3.96 - 3.88 (m, 1H), 3.64 (d, *J* = 7.5 Hz, 3H), 3.51 (br s, 1H), 3.34 (br s, 1H), 3.27 - 3.19 (m, 1H), 3.17 - 3.05 (m, 3H), 2.99 (br d, *J* = 11.2 Hz, 1H), 2.94 - 2.75 (m, 3H), 2.69 - 2.54 (m, 2H), 2.45 - 2.32 (m, 1H), 2.25 - 2.13 (m, 1H), 1.22 - 1.14 (m, 3H). MS (ESI) *m/z* $(M+1)^+$ = 646.1.<br>SFC retention time: 5.214 min.<br>Separation conditions: chromatographic column: Chiralcel OD-3 100 mm * 4.6 mm I.D., 3 $\mu$m; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min. |
| | | E8: |

(continued)

| Example | Structural formula | Analytical data |
|---|---|---|
| E8<br>E8-a<br>E8-b | 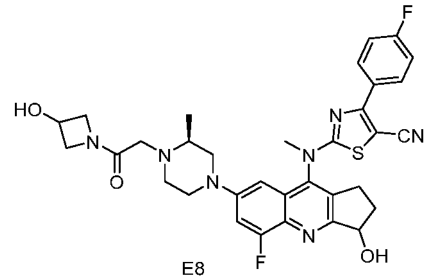<br>E8<br>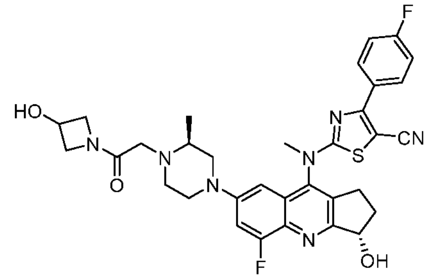<br>E8-a or E8-b<br>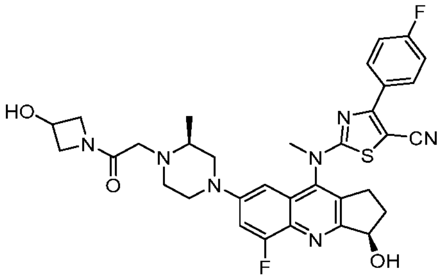<br>E8-b or E8-a | $^1H$ NMR (400 MHz, CHLOROFORM-*d*) δ = 8.13 (br s, 1H), 8.22 - 8.04 (m, 1H), 7.23 - 7.10 (m, 3H), 6.64 (br s, 1H), 5.43 - 5.32 (m, 1H), 4.69 (br s, 1H), 4.54 - 4.40 (m, 1H), 4.28 (br d, *J* = 5.4 Hz, 1H), 4.20 - 4.07 (m, 1H), 3.91 (br d, *J* = 10.8 Hz, 1H), 3.64 (s, 3H), 3.51 (br d, *J* = 8.8 Hz, 3H), 3.20 - 3.08 (m, 2H), 3.05 - 2.96 (m, 2H), 2.93 - 2.80 (m, 3H), 2.65 (br dd, *J* = 4.7, 12.7 Hz, 2H), 2.18 (br dd, *J* = 7.0, 13.7 Hz, 1H), 1.17 (br d, *J* = 5.1 Hz, 3H). MS (ESI) *m/z* $(M+H)^+$ = 646.2.<br>HPLC retention time: 3.63 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min.<br>E8-a:<br>$^1H$ NMR (400 MHz, CHLOROFORM-*d*) δ = 8.14 (br s, 2H), 7.25 - 7.12 (m, 3H), 6.66 (br s, 1H), 5.38 (br s, 1H), 4.69 (br s, 1H), 4.46 (br d, *J* = 8.0 Hz, 1H), 4.29 (br s, 1H), 4.21 - 4.08 (m, 1H), 3.94 (br s, 1H), 3.65 (s, 3H), 3.59 - 3.48 (m, 3H), 3.28 - 3.09 (m, 4H), 2.91 (br dd, *J* = 8.3, 16.3 Hz, 3H), 2.64 (br s, 2H), 2.24 - 2.12 (m, 1H), 1.24 (br s, 3H). MS (ESI) *m/z* $(M+H)^+$ = 646.2.<br>SFC retention time: 4.905 min.<br>Separation conditions: chromatographic column: Chiralcel OD-3 100 mm * 4.6 mm I.D., 3 μm; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min.<br>E8-b:<br>1H NMR (400 MHz, CHLOROFORM-*d*) δ = 8.23 - 8.01 (m, 2H), 7.24 - 7.08 (m, 3H), 6.64 (br s, 1H), 5.46 - 5.24 (m, 1H), 4.68 (br d, *J* = 6.1 Hz, 1H), 4.58 - 4.39 (m, 1H), 4.36 - 4.22 (m, 1H), 4.21 - 4.04 (m, 1H), 3.97 - 3.85 (m, 1H), 3.64 (s, 3H), 3.56 - 3.36 (m, 3H), 3.21 - 2.89 (m, 5H), 2.89 - 2.71 (m, 3H), 2.63 (br s, 2H), 2.19 - 2.12 (m, 2H), 1.15 (dd, *J* = 6.0, 9.8 Hz, 3H). MS (ESI) *m/z* $(M+H)^+$ = 646.2.<br>SFC retention time: 4.600 min.<br>Separation conditions: chromatographic column: Chiralcel OD-3 100 mm * 4.6 mm I.D., 3 μm; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min. |

(continued)

| Example | Structural formula | Analytical data |
|---|---|---|
| E9<br>E9-a<br><br>E9-b | 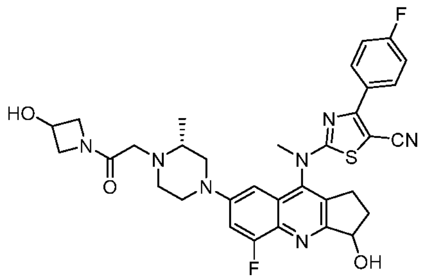<br>E9<br>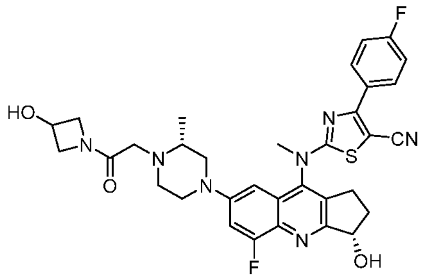<br>E9-a or E9-b<br>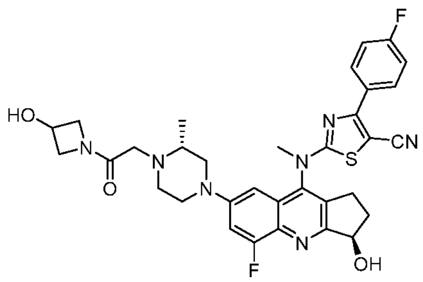<br>E9-b or E9-a | E9:<br>$^1$H NMR (400 MHz, CHLOROFORM-*d*) δ = 8.15 (br s, 2H), 7.26 - 7.15 (m, 3H), 6.66 (br s, 1H), 5.44 - 5.32 (m, 1H), 4.81 - 4.65 (m, 1H), 4.54 - 4.42 (m, 1H), 4.29 (br d, *J* = 6.6 Hz, 1H), 4.21 - 4.11 (m, 1H), 3.94 (br s, 1H), 3.66 (s, 3H), 3.58 - 3.46 (m, 3H), 3.42 - 3.32 (m, 3H), 3.21 - 3.00 (m, 3H), 2.96 - 2.83 (m, 3H), 2.81 - 2.61 (m, 2H), 2.27 - 2.14 (m, 1H), 1.54 - 1.44 (m, 2H), 1.21 (br s, 3H), 1.03 (t, *J* = 7.3 Hz, 4H). MS (ESI) *m/z* $(M+H)^+$ = 646.3.<br>HPLC retention time: 3.507 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.<br>E9-a:<br>$^1$H NMR (400 MHz, CHLOROFORM-*d*) δ = 8.15 (br s, 2H), 7.24 - 7.15 (m, 3H), 6.69 (br s, 1H), 5.45 - 5.32 (m, 1H), 4.71 (br s, 1H), 4.48 (br s, 1H), 4.36 - 4.27 (m, 1H), 4.24 - 4.09 (m, 1H), 3.96 (br d, *J* = 9.9 Hz, 1H), 3.67 (s, 3H), 3.65 - 3.35 (m, 6H), 3.28 - 3.08 (m, 5H), 3.01 - 2.85 (m, 1H), 2.67 (br d, *J* = 13.1 Hz, 2H), 2.28 - 2.15 (m, 1H), 1.30 (br d, *J* = 16.7 Hz, 3H). MS<br>(ESI) *m/z* $(M+H)^+$ = 646.2.<br>SFC retention time: 4.648 min.<br>Separation conditions: chromatographic column: Chiralcel OD-3 100 mm * 4.6 mm I.D., 3 μm; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min.<br>E9-b:<br>$^1$H NMR (400 MHz, CHLOROFORM-*d*) δ = 8.16 (br s, 2H), 7.25 - 7.15 (m, 3H), 6.68 (br s, 1H), 5.47 - 5.30 (m, 1H), 4.70 (br s, 1H), 4.57 - 4.41 (m, 1H), 4.31 (br dd, *J* = 6.5, 10.9 Hz, 1H), 4.22 - 4.07 (m, 1H), 3.94 (br d, *J* = 7.4 Hz, 1H), 3.66 (s, 3H), 3.63 - 3.28 (m, 6H), 3.24 - 3.04 (m, 5H), 2.97 - 2.85 (m, 1H), 2.75 - 2.60 (m, 2H), 2.26 - 2.14 (m, 1H), 1.28 (br s, 3H). MS (ESI) *m/z* $(M+H)^+$ = 646.2.<br>SFC retention time: 5.082 min.<br>Separation conditions: chromatographic column: Chiralcel OD-3 100 mm * 4.6 mm I.D., 3 μm; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min. |

(continued)

| Example | Structural formula | Analytical data |
|---|---|---|
| E10<br>E10-a<br>E10-b | 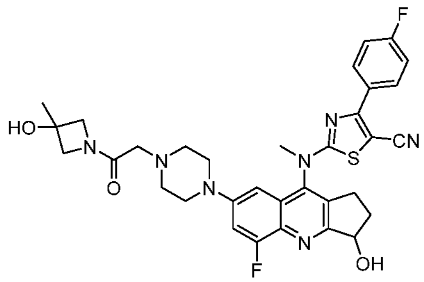<br>E10<br>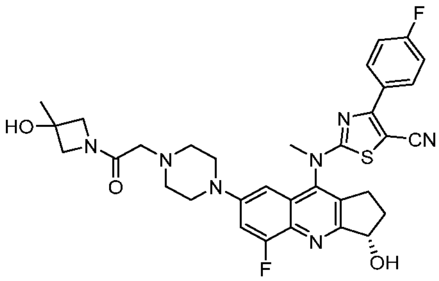<br>E10-a or E10-b | E10:<br>$^1$H NMR (400 MHz, CHLOROFORM-*d*) $\delta$ = 8.13 (br s, 2H), 7.22 - 7.11 (m, 3H), 6.66 (s, 1H), 5.37 (td, *J* = 6.8, 13.4 Hz, 1H), 4.20 (br d, *J* = 9.3 Hz, 1H), 4.10 (br d, *J* = 9.3 Hz, 1H), 4.02 - 3.92 (m, 2H), 3.64 (s, 3H), 3.33 (br s, 4H), 3.23 - 3.01 (m, 3H), 2.95 - 2.84 (m, 1H), 2.78 - 2.62 (m, 5H), 2.17 (dt, *J* = 7.5, 14.0 Hz, 1H), 1.54 (s, 3H). MS (ESI) *m/z* $(M+H)^+$ = 646.2<br>HPLC retention time: 3.491 min.<br>Separation conditions: chromatographic column: Xbridge Shield RP-18, 5 $\mu$m, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.<br>E10-a:<br>$^1$H NMR (400 MHz, METHANOL-$d_4$) $\delta$ = 8.09 (br s, 2H), 7.44 (br d, *J* = 12.0 Hz, 1H), 7.22 (br t, *J* = 8.4 Hz, 2H), 6.77 (s, 1H), 5.29 - 5.20 (m, 1H), 4.18 - 4.06 (m, 2H), 3.92 - 3.81 (m, 2H), 3.67 (d, *J* = 6.3 Hz, 3H), 3.36 (br d, *J* = 5.3 Hz, 4H), 3.18 - 3.02 (m, 3H), 2.99 -2.79 (m, 1H), 2.71 - 2.62 (m, 4H), 2.56 (br dd, *J* = 7.0, 13.1 Hz, 1H), 2.10 (br dd, *J* = 5.4, 13.4 Hz, 1H), 1.46 (d, *J* = 2.0 Hz, 3H). MS (ESI) *m/z* $(M+H)^+$ = 646.2.<br>SFC retention time: 4.507 min.<br>Separation conditions: chromatographic column: Chiralcel OD-3 100 mm * 4.6 mm I.D., 3 $\mu$m; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min.<br>E10-b:<br>$^1$H NMR (400 MHz, METHANOL-$d_4$) $\delta$ = 8.10 (br s, 2H), 7.45 (br d, *J* = 13.8 Hz, 1H), 7.23 (br t, *J* = 8.6 Hz, 2H), 6.76 (s, 1H), 5.30 - 5.18 (m, 1H), 4.21 - 4.07 (m, 2H), 3.91 - 3.80 (m, 2H), 3.67 (d, *J* = 6.4 Hz, 3H), 3.36 (br d, *J* = 5.0 Hz, 4H), 3.20 - 3.05 (m, 3H), 3.00 - 2.79 (m, 1H), 2.66 (br t, *J* = 4.7 Hz, 4H), 2.56 (br dd, *J* = 7.0, 13.1 Hz, 1H), 2.10 (br dd, *J* = 6.2, 12.9 Hz, 1H), 1.46 (d, *J* = 2.0 Hz, 3H). MS (ESI) *m/z* $(M+H)^+$ = 646.2.<br>SFC retention time: 4.846 min.<br>Separation conditions: chromatographic column: Chiralcel OD-3 100 mm * 4.6 mm I.D., 3 $\mu$m; column temperature: 35°C; mobile phase: $CO_2$-ethanol (0.05% diethylamine); ethanol (0.05% diethylamine): 5% to 40% 4 min, 40% 2.5 min, 5% 1.5 min; flow rate: 2.8 mL/min. |

(continued)

| Example | Structural formula | Analytical data |
|---|---|---|
| E11 | 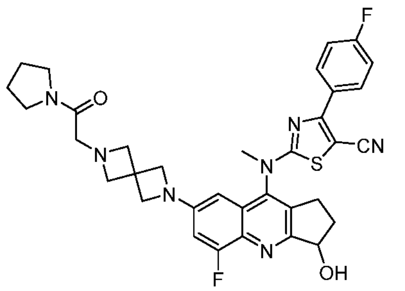 | $^1$H NMR (400 MHz, CHLOROFORM-*d*) δ = 8.31 - 8.01 (m, 2H), 7.23 - 7.08 (m, 2H), 6.70 (br d, *J* = 10.1 Hz, 1H), 6.26 - 6.10 (m, 1H), 5.50 - 5.26 (m, 1H), 4.11 (s, 4H), 3.83 (s, 4H), 3.72 - 3.53 (m, 3H), 3.51 - 3.28 (m, 6H), 3.15 - 2.77 (m, 3H), 2.71 - 2.56 (m, 1H), 2.24 - 2.15 (m, 3H), 1.98 - 1.84 (m, 2H). MS (ESI) *m*/*z* $(M+H)^+$ = 642.3.<br>HPLC retention time: 3.92 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| E12 | 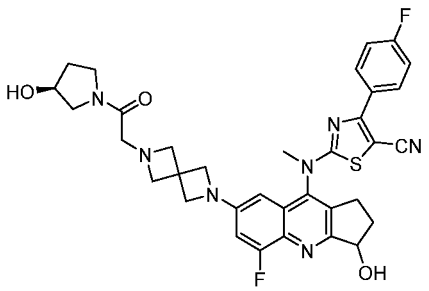 | $^1$H NMR (400 MHz, CHLOROFORM-*d*) δ = 8.21 - 8.09 (m, 2H), 7.24 - 7.11 (m, 2H), 6.70 (br d, *J* = 11.0 Hz, 1H), 6.23 - 6.12 (m, 1H), 5.41 - 5.28 (m, 1H), 5.07 - 4.62 (m, 2H), 4.59 - 4.48 (m, 1H), 4.22 - 4.14 (m, 1H), 4.08 (d, *J* = 3.0 Hz, 3H), 3.72 - 3.57 (m, 8H), 3.57 - 3.36 (m, 3H), 3.35 - 3.16 (m, 2H), 3.16 - 2.96 (m, 1H), 2.95 - 2.77 (m, 1H), 2.64 (br s, 1H), 2.27 - 1.91 (m, 3H). MS (ESI) *m*/*z* $(M+H)^+$ = 658.3.<br>HPLC retention time: 3.64 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min. |
| E13<br>E13-a<br>E13-b | 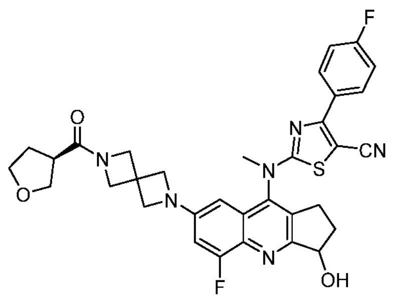<br>E13<br>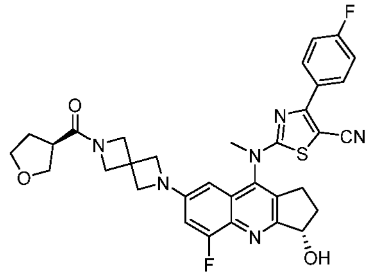<br>E13-a or E13-b | E13:<br>$^1$H NMR (400 MHz, CHLOROFORM-*d*) δ = 8.14 (br s, 2H), 7.18 (t, *J* = 8.7 Hz, 2H), 6.79 - 6.66 (m, 1H), 6.19 (s, 1H), 5.43 - 5.28 (m, 1H), 4.42 - 4.32 (m, 2H), 4.21 (s, 2H), 4.14 (br s, 4H), 3.99 (br t, *J* = 7.3 Hz, 1H), 3.93 - 3.79 (m, 3H), 3.63 (s, 3H), 3.37 - 3.30 (m, 1H), 3.10 (qd, *J* = 8.4, 12.5 Hz, 1H), 2.97 - 2.85 (m, 2H), 2.69 - 2.60 (m, 1H), 2.24 - 2.13 (m, 2H), 2.08 - 2.02 (m, 1H). MS (ESI) *m*/*z* $(M+1)^+$ = 629.1.<br>HPLC retention time: 3.790 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.<br><br>E13-a: |

(continued)

| Example | Structural formula | Analytical data |
|---|---|---|
| | 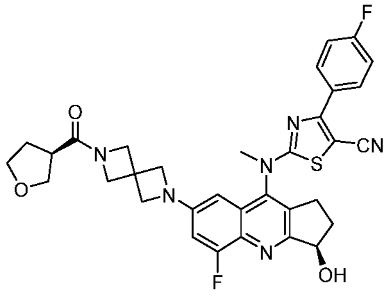<br>E13-b or E13-a | $^1$H NMR (400 MHz, CHLOROFORM-*d*) δ = 8.15 (br s, 2H), 7.18 (t, *J* = 8.5 Hz, 2H), 6.73 (dd, *J* = 2.0, 11.5 Hz, 1H), 6.20 (d, *J* = 1.5 Hz, 1H), 5.37 (td, *J* = 6.4, 12.1 Hz, 1H), 4.43 - 4.31 (m, 2H), 4.26 - 4.18 (m, 2H), 4.15 (br s, 4H), 3.99 (br t, *J* = 8.2 Hz, 1H), 3.95 - 3.76 (m, 3H), 3.63 (s, 3H), 3.10 (br dd, *J* = 3.9, 13.7 Hz, 1H), 2.98 - 2.83 (m, 2H), 2.70 - 2.58 (m, 1H), 2.24 - 2.12 (m, 2H), 2.11 - 1.99 (m, 1H).<br>SFC retention time: 2.125 min.<br>Separation conditions: chromatographic column: Chiralpak AD-3 50 mm * 4.6 mm I.D., 3 μm; column temperature: 35°C; mobile phase: $CO_2$-ethanol (0.05% diethylamine); ethanol (0.05% diethylamine): 5% to 40% 2 min, 40% 1.2 min, 5% 0.8 min; flow rate: 2.8 mL/min.<br>E13-b:<br>$^1$H NMR (400 MHz, CHLOROFORM-*d*) δ = 8.15 (br s, 2H), 7.18 (t, *J* = 8.5 Hz, 2H), 6.73 (br d, *J* = 10.8 Hz, 1H), 6.20 (s, 1H), 5.45 - 5.32 (m, 1H), 4.42 - 4.30 (m, 2H), 4.21 (s, 2H), 4.18 - 4.10 (m, 4H), 3.99 (t, *J* = 8.2 Hz, 1H), 3.95 - 3.76 (m, 3H), 3.63 (s, 3H), 3.09 (br s, 1H), 2.98 - 2.84 (m, 2H), 2.64 (dt, *J* = 7.9, 12.6 Hz, 1H), 2.25 - 1.98 (m, 3H).<br>SFC retention time: 2.373 min.<br>Separation conditions: chromatographic column: Chiralpak AD-3 50 mm * 4.6 mm I.D., 3 μm; column temperature: 35°C; mobile phase: $CO_2$-ethanol (0.05% diethylamine); ethanol (0.05% diethylamine): 5% to 40% 2 min, 40% 1.2 min, 5% 0.8 min; flow rate: 2.8 mL/min. |
| E14<br>E14-a<br><br>E14-b | 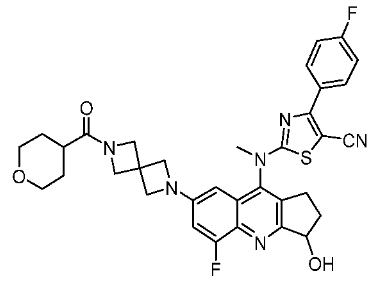<br>E14<br>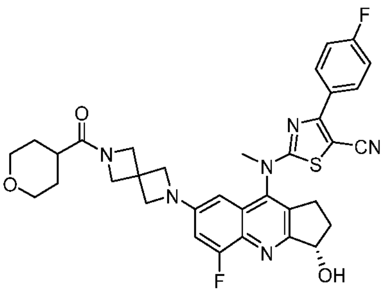<br>E14-a or E14-b | E14:<br>$^1$H NMR (400 MHz, CHLOROFORM-*d*) δ = 8.14 (br s, 2H), 7.17 (t, *J* = 8.5 Hz, 2H), 6.73 (br d, *J* = 10.5 Hz, 1H), 6.19 (s, 1H), 5.38 (td, *J* = 6.7, 12.8 Hz, 1H), 4.37 (s, 2H), 4.20 (s, 2H), 4.17 - 4.07 (m, 4H), 4.01 (br d, *J* = 10.5 Hz, 2H), 3.63 (s, 3H), 3.41 (br t, *J* = 11.3 Hz, 2H), 3.18 - 3.05 (m, 1H), 2.95 - 2.81 (m, 1H), 2.71 - 2.57 (m, 1H), 2.45 - 2.33 (m, 1H), 2.18 (dt, *J* = 7.7, 14.0 Hz, 1H), 1.92 - 1.80 (m, 3H), 1.57 (br d, *J* = 13.1 Hz, 2H). MS (ESI) *m/z* $(M+1)^+$ = 643.2.<br>HPLC retention time: 4.120 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.<br><br>E14-a: |

(continued)

| Example | Structural formula | Analytical data |
|---|---|---|
| | 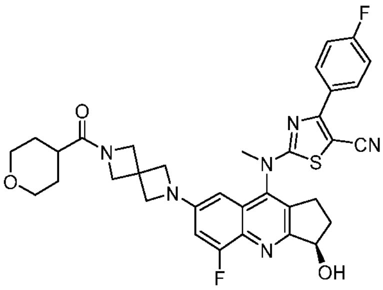<br>E14-b or E14-a | $^1$H NMR (400 MHz, CHLOROFORM-*d*) δ = 8.15 (br s, 2H), 7.18 (t, *J* = 8.7 Hz, 2H), 6.73 (br d, *J* = 11.5 Hz, 1H), 6.19 (s, 1H), 5.42 - 5.30 (m, 1H), 4.37 (s, 2H), 4.21 (s, 2H), 4.18 - 4.09 (m, 4H), 4.02 (br d, *J* = 10.8 Hz, 2H), 3.63 (s, 3H), 3.42 (br t, *J* = 11.4 Hz, 2H), 3.10 (dt, *J* = 4.3, 8.7 Hz, 1H), 2.95 - 2.83 (m, 1H), 2.64 (dt, *J* = 7.9, 12.6 Hz, 1H), 2.40 (br t, *J* = 11.4 Hz, 1H), 2.25 - 2.13 (m, 1H), 1.93 - 1.78 (m, 3H), 1.57 (br d, *J* = 13.1 Hz, 2H).<br>MS (ESI) *m/z* (M+1)$^+$ = 643.2.<br>SFC retention time: 2.031 min.<br>Separation conditions: chromatographic column: (S, S)-Whelk-O1 100 mm * 4.6 mm I.D., 5 μm; column temperature: 35°C; mobile phase: $CO_2$-methanol (0.05% diethylamine); methanol (0.05% diethylamine): 5% to 50%; flow rate: 2.8 mL/min. E14-b:<br>$^1$H NMR (400 MHz, CHLOROFORM-*d*) δ = 8.15 (br s, 2H), 7.18 (t, *J* = 8.5 Hz, 2H), 6.73 (br d, *J* = 11.5 Hz, 1H), 6.19 (s, 1H), 5.41 - 5.32 (m, 1H), 4.37 (s, 2H), 4.21 (s, 2H), 4.17 - 4.09 (m, 4H), 4.02 (br d, *J* = 10.3 Hz, 2H), 3.63 (s, 3H), 3.42 (br t, *J* = 11.4 Hz, 2H), 3.11 (dt, *J* = 4.3, 8.5 Hz, 1H), 2.96 - 2.84 (m, 1H), 2.70 - 2.61 (m, 1H), 2.45 - 2.35 (m, 1H), 2.19 (td, *J* = 7.0, 14.1 Hz, 1H), 1.94 - 1.81 (m, 2H), 1.58 (br d, *J* = 13.6 Hz, 2H). MS (ESI) *m/z* (M+1)$^+$ = 643.1.<br>SFC retention time: 2.428 min.<br>Separation conditions: chromatographic column: (S, S)-Whelk-O1 100 mm * 4.6 mm I.D., 5 μm; column temperature: 35°C; mobile phase: $CO_2$-methanol (0.05% diethylamine); methanol (0.05% diethylamine): 5% to 50%; flow rate: 2.8 mL/min. |
| E15<br>E15-a<br>E15-b | 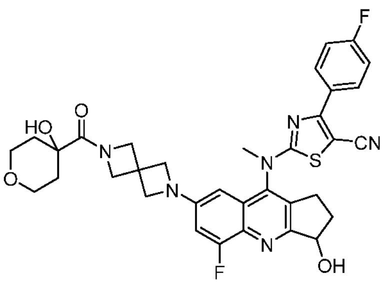<br>E15<br>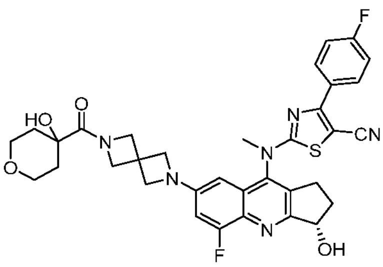<br>E15-a or E15-b | E15:<br>$^1$H NMR (400 MHz, CHLOROFORM-*d*) δ = 8.13 (br s, 2H), 7.17 (t, *J* = 8.7 Hz, 2H), 6.73 (br d, *J* = 11.5 Hz, 1H), 6.19 (s, 1H), 5.42 (br d, *J* = 6.8 Hz, 1H), 4.65 (br s, 2H), 4.27 (br s, 2H), 4.15 (br s, 4H), 3.89 - 3.76 (m, 4H), 3.63 (s, 3H), 3.08 (br s, 1H), 2.95 - 2.82 (m, 1H), 2.65 (dt, *J* = 7.8, 12.8 Hz, 1H), 2.19 (br dd, *J* = 6.0, 13.8 Hz, 2H), 2.13 - 2.04 (m, 3H), 1.50 (br d, *J* = 12.8 Hz, 2H). MS (ESI) *m/z* (M+1)$^+$ = 659.2.<br>HPLC retention time: 3.837 min.<br>Separation conditions: chromatographic column: Shield 5 2.1 * 50 column<br>Xbridge RP-18, μm, mm; temperature: 50°C; mobile phase: water (0.2 mL/1 L ammonia water)-acetonitrile; acetonitrile: 10% to 80% 6 min, 80% 2 min; flow rate: 0.8 mL/min. |

(continued)

| Example | Structural formula | Analytical data |
|---|---|---|
| | 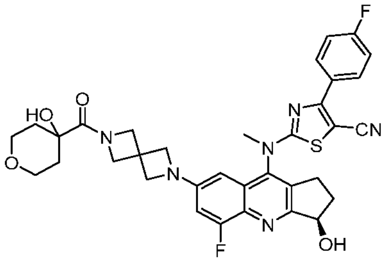 E15-b or E15-a | E15-a: $^1H$ NMR (400 MHz, CHLOROFORM-*d*) $\delta$ = 8.13 (br s, 2H), 7.17 (br t, *J* = 8.7 Hz, 2H), 6.72 (br d, *J* = 11.8 Hz, 1H), 6.19 (s, 1H), 5.40 (br d, *J* = 5.8 Hz, 1H), 4.65 (br s, 2H), 4.27 (br s, 2H), 4.14 (br s, 4H), 3.89 - 3.76 (m, 4H), 3.63 (s, 3H), 3.39 (br s, 1H), 3.09 (br s, 1H), 2.95 - 2.83 (m, 1H), 2.65 (br dd, *J* = 4.6, 12.7 Hz, 1H), 2.18 (br dd, *J* = 6.3, 13.6 Hz, 2H), 2.09 (dt, *J* = 5.1, 12.1 Hz, 3H), 1.50 (br d, *J* = 13.1 Hz, 2H). MS (ESI) *m/z* $(M+1)^+$ = 659.1. SFC retention time: 2.276 min. Separation conditions: chromatographic column: Chiralpak AD-3 150 mm * 4.6 mm I.D., 3 $\mu$m; column temperature: 35°C; mobile phase: $CO_2$-ethanol (0.05% diethylamine); ethanol (0.05% diethylamine): 5% to 40%; flow rate: 2.5 mL/min. E15-b: $^1H$ NMR (400 MHz, CHLOROFORM-*d*) $\delta$ = 8.15 (br s, 2H), 7.18 (br t, *J* = 8.5 Hz, 2H), 6.73 (br d, *J* = 10.3 Hz, 1H), 6.21 (s, 1H), 5.46 - 5.33 (m, 1H), 4.66 (br s, 2H), 4.28 (br s, 2H), 4.15 (br s, 4H), 3.90 - 3.77 (m, 4H), 3.64 (s, 3H), 3.10 (br s, 1H), 2.91 (br d, *J* = 5.5 Hz, 1H), 2.74 - 2.61 (m, 1H), 2.32 - 2.16 (m, 2H), 2.15 - 2.06 (m, 3H), 1.51 (br d, *J* = 13.1 Hz, 2H). MS (ESI) *m/z* $(M+1)^+$ = 659.2. SFC retention time: 3.253 min. Separation conditions: chromatographic column: Chiralpak AD-3 150 mm * 4.6 mm I.D., 3 $\mu$m; column temperature: 35°C; mobile phase: $CO_2$-ethanol (0.05% diethylamine); ethanol (0.05% diethylamine): 5% to 40%; flow rate: 2.5 mL/min. |
| E16 E16-a E16-b | 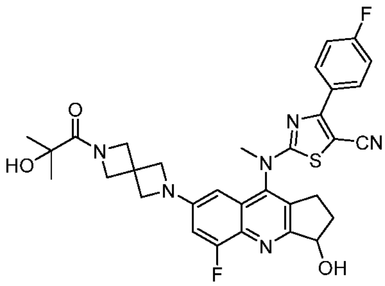 E16 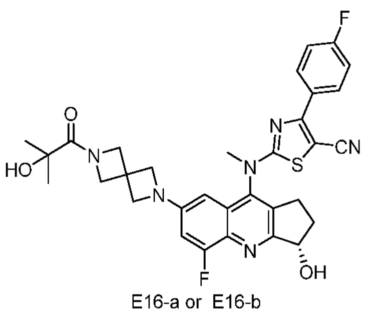 E16-a or E16-b | E16: $^1H$ NMR (400 MHz, CHLOROFORM-*d*) $\delta$ = 8.13 (br d, *J* = 5.8 Hz, 2H), 7.27 (s, 2H), 7.17 (t, *J* = 8.6 Hz, 2H), 6.74 (dd, *J* = 2.1, 11.7 Hz, 1H), 6.19 (d, *J* = 1.9 Hz, 1H), 5.46 - 5.28 (m, 1H), 4.58 (br s, 2H), 4.26 (br s, 2H), 4.15 (br s, 4H), 3.63 (s, 3H), 3.25 (br s, 1H), 3.09 (br s, 1H), 2.95 - 2.80 (m, 1H), 2.71 - 2.58 (m, 1H), 2.25 - 2.12 (m, 1H), 1.42 (s, 7H). MS (ESI) *m/z* $(M+H)^+$ = 617.1. HPLC retention time: 4.018 min. Separation conditions: chromatographic column: Xbridge Shield RP-18, 5 $\mu$m, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. E16-a: $^1H$ NMR (400 MHz, CHLOROFORM-*d*) $\delta$ = 8.15 (br s, 2H), 7.19 (t, *J* = 8.6 Hz, 2H), 6.75 (dd, *J* = 2.0, 11.7 Hz, 1H), 6.21 (d, *J* = 1.7 Hz, 1H), 5.41 (br d, *J* = 7.4 Hz, 1H), 4.59 (br s, 2H), 4.28 (br s, 2H), 4.22 - 4.08 |

(continued)

| Example | Structural formula | Analytical data |
|---|---|---|
| | 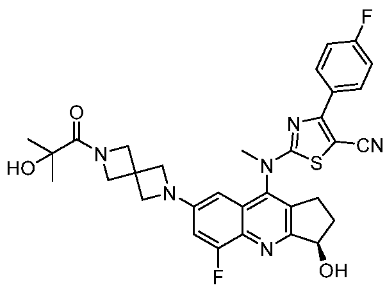<br>E16-b or E16-a | (m, 1H), 4.17 (br s, 3H), 3.64 (s, 3H), 3.28 (br s, 1H), 3.09 (br s, 1H), 3.00 - 2.83 (m, 1H), 2.73 - 2.58 (m, 1H), 2.27 - 2.13 (m, 1H), 1.43 (s, 7H). MS (ESI) *m/z* $(M+H)^+$ = 617.2.<br>SFC retention time: 0.384 min.<br>Separation conditions: chromatographic column: Chiralpak AD-3 50 mm * 4.6 mm I.D., 3 μm; column temperature: 35°C; mobile phase: $CO_2$-ethanol (0.05% diethylamine); ethanol (0.05% diethylamine): 5% to 40%; flow rate: 4 mL/min.<br>E16-b:<br>$^1$H NMR (400 MHz, CHLOROFORM-*d*) δ = 8.13 (br s, 2H), 7.17 (t, *J* = 8.6 Hz, 2H), 6.75 (br d, *J* = 9.7 Hz, 1H), 6.19 (s, 1H), 5.45 (br s, 1H), 4.58 (br s, 2H), 4.26 (br s, 2H), 4.16 (br s, 4H), 3.63 (s, 3H), 3.23 (br s, 1H), 3.09 (br s, 1H), 2.95 - 2.83 (m, 1H), 2.72 - 2.59 (m, 1H), 2.26 - 2.11 (m, 1H), 1.47 - 1.37 (m, 1H), 1.42 (s, 7H). MS (ESI) *m/z* $(M+H)^+$ = 617.1.<br>SFC retention time: 0.813 min.<br>Separation conditions: chromatographic column: Chiralpak AD-3 50 mm * 4.6 mm I.D., 3 μm; column temperature: 35°C; mobile phase: $CO_2$-ethanol (0.05% diethylamine); ethanol (0.05% diethylamine): 5% to 40%; flow rate: 4 mL/min. |
| E17<br>E17-a<br>E17-b | 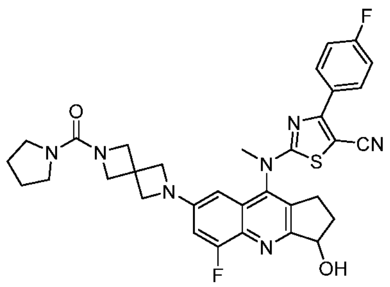<br>E17<br>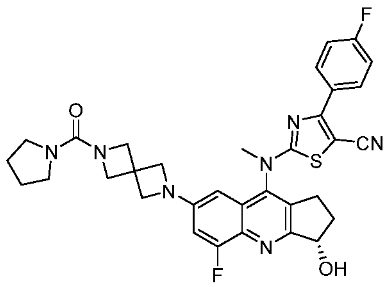<br>E17-a or E17-b | E17:<br>$^1$H NMR (400 MHz, CHLOROFORM-*d*) δ = 8.14 (br d, *J* = 5.4 Hz, 2H), 7.18 (t, *J* = 8.6 Hz, 2H), 6.72 (dd, *J* = 2.3, 11.7 Hz, 1H), 6.17 (s, 1H), 5.41 - 5.30 (m, 1H), 4.16 (s, 4H), 4.10 (d, *J* = 3.5 Hz, 4H), 3.63 (s, 3H), 3.32 (br t, *J* = 6.5 Hz, 5H), 3.10 (dtd, *J* = 4.5, 8.7, 17.2 Hz, 1H), 2.87 (qd, *J* = 8.1, 16.6 Hz, 1H), 2.71 - 2.58 (m, 1H), 2.22 - 2.12 (m, 1H), 1.89 - 1.82 (m, 4H). MS (ESI) *m/z* $(M+H)^+$ = 742.3.<br>HPLC retention time: 4.89 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min, 80% 2 min; flow rate: 1.2 mL/min.<br>E17-a:<br>$^1$H NMR (400 MHz, CHLOROFORM-*d*) δ = 8.14 (br d, *J* = 5.4 Hz, 2H), 7.17 (t, *J* = 8.6 Hz, 2H), 6.71 (dd, *J* = 1.9, 11.8 Hz, 1H), 6.17 (s, 1H), 5.43 - 5.31 (m, 1H), |

(continued)

| Example | Structural formula | Analytical data |
|---|---|---|
| | 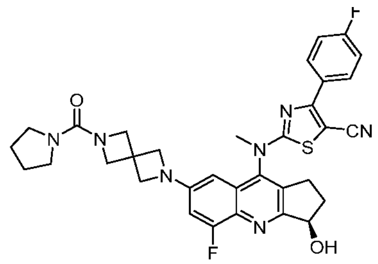<br>E17-b or E17-a | 4.16 (s, 4H), 4.09 (d, *J* = 3.6 Hz, 4H), 3.63 (s, 3H), 3.32 (br t, *J* = 6.4 Hz, 4H), 3.17 - 3.04 (m, 1H), 2.95 - 2.82 (m, 1H), 2.71 - 2.57 (m, 1H), 2.17 (dt, *J* = 7.5, 14.0 Hz, 1H), 1.87 - 1.81 (m, 5H), 1.83 - 1.81 (m, 1H). MS (ESI) *m/z* $(M+H)^+$ = 628.3.<br>SFC retention time: 2.575 min.<br>Separation conditions: chromatographic column: Chiralpak AD-3 150 mm * 4.6 mm I.D., 3 μm; column temperature: 40°C; mobile phase: $CO_2$-ethanol (0.05% diethylamine); ethanol (0.05% diethylamine): 5% to 40%; flow rate: 2.5 mL/min.<br>E17-b:<br>$^1$H NMR (400 MHz, CHLOROFORM-*d*) δ = 8.14 (br d, *J* = 5.8 Hz, 2H), 7.17 (t, *J* = 8.6 Hz, 2H), 6.71 (dd, *J* = 2.1, 11.9 Hz, 1H), 6.17 (s, 1H), 5.41 - 5.29 (m, 1H), 4.16 (s, 4H), 4.09 (d, *J* = 3.7 Hz, 4H), 3.63 (s, 3H), 3.32 (br t, *J* = 6.4 Hz, 4H), 3.09 (dtd, *J* = 4.3, 8.9, 17.4 Hz, 1H), 2.95 - 2.80 (m, 1H), 2.72 - 2.58 (m, 1H), 2.17 (dt, *J* = 7.9, 13.8 Hz, 1H), 1.87 - 1.81 (m, 5H). MS (ESI) *m/z* $(M+H)^+$ = 628.3.<br>SFC retention time: 3.226 min.<br>Separation conditions: chromatographic column: Chiralpak AD-3 150 mm * 4.6 mm I.D., 3 μm; column temperature: 40°C; mobile phase: $CO_2$-ethanol (0.05% diethylamine); ethanol (0.05% diethylamine): 5% to 40%; flow rate: 2.5 mL/min. |
| E18<br><br>E18-a<br><br>E18-b | 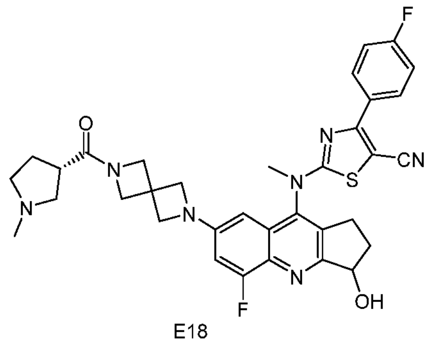<br>E18<br>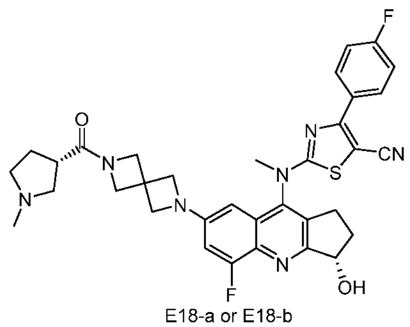<br>E18-a or E18-b | E18:<br>$^1$H NMR (400 MHz, CHLOROFORM-*d*) δ = 8.52 (br s, 1H), 8.15 (br s, 2H), 7.18 (br t, *J* = 8.7 Hz, 2H), 6.72 (br d, *J* = 11.8 Hz, 1H), 6.20 (s, 1H), 5.37 (td, *J* = 6.5, 12.5 Hz, 1H), 4.74 (br s, 1H), 4.36 (br s, 2H), 4.21 (s, 2H), 4.13 (br s, 4H), 3.63 (s, 3H), 3.13 (br d, *J* = 8.0 Hz, 1H), 3.02 (br d, *J* = 7.0 Hz, 2H), 2.95 - 2.83 (m, 1H), 2.69 - 2.59 (m, 3H), 2.50 (s, 3H), 2.18 (br d, *J* = 7.0 Hz, 2H), 2.13 (br d, *J* = 9.0 Hz, 2H). MS (ESI) *m/z* $(M+1)^+$ = 642.2.<br><br>HPLC retention time: 7.26 min.<br>Separation conditions: chromatographic column: Ultimate C18 3.0 × 50 mm, 3 μm; column temperature: 40°C; mobile phase: water (2.75 mL/4 L trifluoroacetic acid)-acetonitrile (2.5 mL/4 L trifluoroacetic acid); acetonitrile: 10% to 80% 6 min,<br>80% 2 min; flow rate: 1.2 mL/min.<br><br>E18-a: |

(continued)

| Example | Structural formula | Analytical data |
|---|---|---|
| | 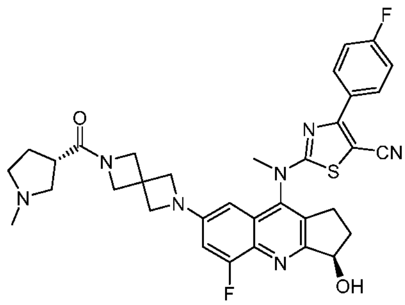<br>E18-b or E18-a | $^1H$ NMR (400 MHz, CHLOROFORM-*d*) δ = 8.15 (br s, 2H), 7.18 (t, *J* = 8.5 Hz, 2H), 6.72 (br d, *J* = 9.8 Hz, 1H), 6.19 (s, 1H), 5.42 - 5.30 (m, 1H), 4.35 (s, 2H), 4.20 (s, 2H), 4.16 - 4.09 (m, 4H), 3.63 (s, 3H), 3.09 (br s, 2H), 3.03 - 2.93 (m, 2H), 2.88 (br dd, *J* = 8.0, 16.8 Hz, 1H), 2.62 (br d, *J* = 4.5 Hz, 2H), 2.54 (q, *J* = 8.4 Hz, 1H), 2.47 (s, 3H), 2.24 - 2.04 (m, 3H). MS (ESI) *m/z* $(M+1)^+$ = 642.0.<br>SFC retention time: 2.556 min.<br>Separation conditions: chromatographic column: Chiralpak AD-3 150 mm * 4.6 mm I.D., 3 μm; column temperature: 40°C; mobile phase: $CO_2$-ethanol (0.05% diethylamine); ethanol (0.05% diethylamine): 5% to 40%; flow rate: 2.5 mL/min.<br>E18-b:<br>$^1H$ NMR (400 MHz, CHLOROFORM-*d*) δ = 8.15 (br s, 2H), 7.18 (br t, *J* = 8.5 Hz, 2H), 6.72 (br d, *J* = 9.3 Hz, 1H), 6.19 (s, 1H), 5.42 - 5.31 (m, 1H), 4.81 (br s, 1H), 4.35 (s, 2H), 4.20 (s, 2H), 4.13 (br s, 4H), 3.63 (s, 3H), 3.07 (br s, 2H), 3.03 - 2.83 (m, 3H), 2.69 - 2.58 (m, 2H), 2.57 - 2.49 (m, 1H), 2.47 (s, 3H), 2.24 - 2.04 (m, 3H).<br>MS (ESI) *m/z* $(M+1)^+$ = 642.1.<br>SFC retention time: 4.937 min.<br>Separation conditions: chromatographic column: Chiralpak AD-3 150 mm * 4.6 mm I.D., 3 μm; column temperature: 40°C; mobile phase: $CO_2$-ethanol (0.05% diethylamine); ethanol (0.05% diethylamine): 5% to 40%; flow rate: 2.5 mL/min. |
| E19<br>E19-P1A<br>E19-P1B<br><br>E19-P2A<br><br>E19-P2B | 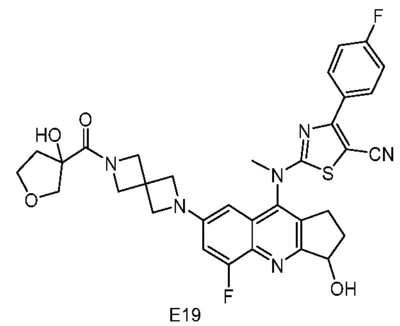<br>E19<br>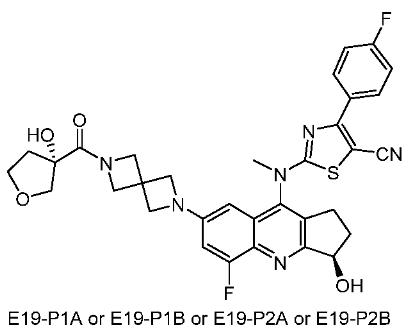<br>E19-P1A or E19-P1B or E19-P2A or E19-P2B | E19:<br>$^1H$ NMR (400 MHz, CHLOROFORM-*d*) δ = 8.14 (br s, 2H), 7.18 (t, *J* = 8.6 Hz, 2H), 6.72 (dd, *J* = 2.2, 11.6 Hz, 1H), 6.19 (s, 1H), 5.45 - 5.28 (m, 1H), 4.68 - 4.47 (m, 2H), 4.29 (br s, 2H), 4.19 - 3.93 (m, 7H), 3.74 (d, *J* = 10.0 Hz, 3.63 3.52 3.30 1H), (s, 3H), (br s, 1H), (br s, 1H), 3.17 - 3.02 (m, 1H), 2.97 - 2.81 (m, 1H), 2.70 - 2.55 (m, 1H), 2.50 - 2.35 (m, 1H), 2.26 - 2.11 (m, 1H), 2.07 - 1.97 (m, 1H), 2.04 (ddd, *J* = 3.6, 6.7, 13.1 Hz, 1H). MS (ESI) *m/z* $(M+H)^+$ = 645.3.<br>HPLC retention time: 3.845 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min. E19-P1 and E19-P2 were obtained by the first resolution:<br><br>Separation conditions: chromatographic column: DAICEL CHIRALPAK AD (250 mm * 30 mm, 10 μm; column temperature: 35°C; mobile phase: $CO_2$-ethanol (0.1% ammonia water); ethanol (0.1% ammonia water): 5% to 40%. |

(continued)

| Example | Structural formula | Analytical data |
|---|---|---|
| | 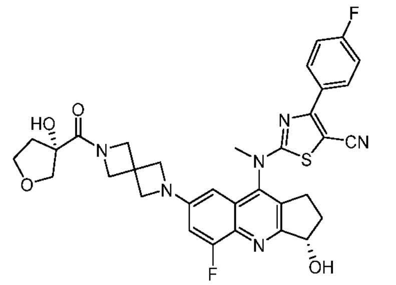<br>E19-P1A or E19-P1B or E19-P2A or E19-P2B<br>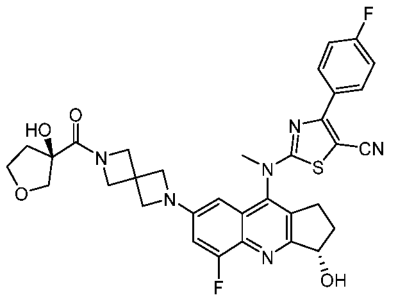<br>E19-P1A or E19-P1B or E19-P2A or E19-P2B<br>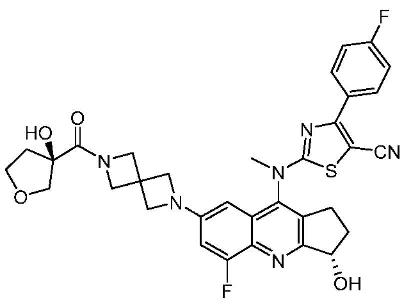<br>E19-P1A or E19-P1B or E19-P2A or E19-P2B | E19-P1A and E19-P1B were obtained by the resolution of E19-P1: SFC retention time: E19-P1A: 2.736 min, E19-P1B: 3.808 min.<br><br>Separation conditions: chromatographic column: Chiralpak IG-3 50 mm * 4.6 mm I.D., 3 μm; column temperature: 35°C; mobile phase: $CO_2$-ethanol (0.05% diethylamine); ethanol (0.05% diethylamine): 5% to 40%; flow rate: 4 mL/min.<br><br>E19-P1A:<br><br>$^1$H NMR (E19-P1A) (400 MHz, CHLOROFORM-*d*) δ = 8.14 (br s, 2H), 7.18 (br t, *J* = 8.6 Hz, 2H), 6.72 (dd, *J* = 2.1, 11.7 Hz, 1H), 6.18 (s, 1H), 5.44 - 5.30 (m, 1H), 5.44 - 5.30 (m, 1H), 4.66 - 4.52 (m, 2H), 4.29 (br s, 2H), 4.18 - 3.90 (m, 8H), 3.74 (d, *J* = 9.9 Hz, 1H), 3.67 - 3.59 (m, 1H), 3.63 (s, 3H), 3.50 (br s, 1H), 3.16 - 3.02 (m, 1H), 2.94 - 2.81 (m, 1H), 2.64 (dt, *J* = 7.8, 12.7 Hz, 1H), 2.41 (td, *J* = 8.8, 13.0 Hz, 1H), 2.24 - 2.11 (m, 1H), 2.04 (ddd, *J* = 3.6, 6.7, 13.3 Hz, 1H). E19-P1B:<br><br>$^1$H NMR (E19-P1B) (400 MHz, CHLOROFORM-*d*) δ = 8.14 (br s, 2H), 7.18 (t, *J* = 8.6 Hz, 2H), 6.72 (dd, *J* = 2.1, 11.6 Hz, 1H), 6.19 (s, 1H), 5.44 - 5.26 (m, 1H), 4.68 - 4.50 (m, 2H), 4.29 (br s, 2H), 4.18 - 3.94 (m, 7H), 3.75 (dd, *J* = 1.8, 9.8 Hz, 1H), 3.63 (s, 3H), 3.55 (br d, *J* = 15.3 Hz, 1H), 3.42 (br s, 1H), 3.16 - 3.02 (m, 1H), 2.96 - 2.81 (m, 1H), 2.71 - 2.57 (m, 1H), 2.47 - 2.35 (m, 1H), 2.17 (dt, *J* = 7.7, 13.8 Hz, 1H), 2.08 - 1.96 (m, 1H).<br><br>E19-P2A and E19-P2B were obtained by the third resolution of E19-P2:<br>SFC retention time: E19-P2A: 6.407 min, E19-P2B: 7.112 min.<br>Separation conditions: chromatographic column: Chiralpak IG-3 50 mm * 4.6 mm I.D., 3 μm; column temperature: 35°C; mobile phase: $CO_2$-ethanol (0.05% diethylamine); ethanol (0.05% diethylamine): 5% to 40%; flow rate: 4 mL/min.<br>E19-P2A:<br>$^1$H NMR (E19-P2A) (400 MHz, CHLOROFORM-*d*) δ = 8.14 (br s, 2H), 7.27 (s, 7H), 7.18 (br t, *J* = 8.6 Hz, 2H), 6.76 - 6.66 (m, 1H), 6.19 (s, 1H), 5.41 - 5.30 (m, 1H), 4.66 - 4.51 (m, 3H), 4.29 (br s, 2H), 4.19 - 3.94 (m, 8H), 3.75 (br d, *J* = 9.7 Hz, 1H), 3.67 - 3.47 (m, 4H), 3.14 - 3.13 (m, 1H), 3.25 - 3.02 (m, 2H), 2.95 - 2.80 (m, 1H), 2.64 (br dd, *J* = 4.8, 12.3 Hz, 1H), 2.47 - 2.31 (m, 1H), 2.26 - 2.10 (m, 1H), 2.03 (ddd, *J* = 3.5, 6.5, 9.7 Hz, 2H).<br>E19-P2B: |

(continued)

| Example | Structural formula | Analytical data |
|---|---|---|
| | | $^1$H NMR (E19-P2B) (400 MHz, CHLOROFORM-*d*) δ = 8.14 (br s, 2H), 7.27 (s, 8H), 7.18 (t, *J* = 8.6 Hz, 2H), 6.72 (dd, *J* = 2.3, 11.7 Hz, 1H), 6.19 (d, *J* = 2.1 Hz, 1H), 5.42 - 5.30 (m, 1H), 4.66 - 4.50 (m, 3H), 4.29 (br s, 2H), 4.19 - 3.93 (m, 8H), 3.74 (d, *J* = 9.8 Hz, 1H), 3.63 (s, 3H), 3.53 (br d, *J* = 17.2 Hz, 1H), 3.35 (br d, *J* = 8.1 Hz, 1H), 3.19 - 3.02 (m, 1H), 2.95 - 2.80 (m, 1H), 2.72 - 2.56 (m, 1H), 2.50 - 2.32 (m, 1H), 2.25 - 2.12 (m, 1H), 2.03 (ddd, *J* = 3.8, 6.9, 13.2 Hz, 1H). |
| E20<br>E20-a<br><br>E20-b<br><br>E20-c<br><br>E20-d | 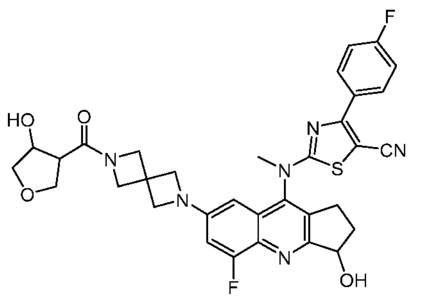<br>E20<br>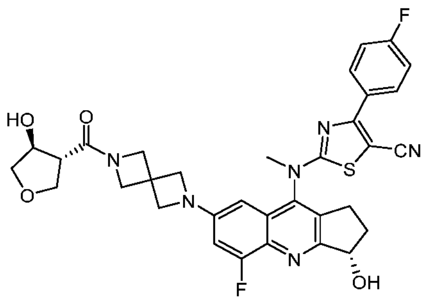<br>E20-a or 20-b or 20-c or 20-d | E20:<br>$^1$H NMR (400 MHz, CHLOROFORM-*d*) δ = 8.14 (br s, 2H), 7.17 (br t, *J* = 8.4 Hz, 2H), 6.70 (br d, *J* = 11.7 Hz, 1H), 6.19 (s, 1H), 5.36 (td, *J* = 6.6, 12.9 Hz, 1H), 4.67 - 4.57 (m, 1H), 4.52 (br d, *J* = 9.3 Hz, 1H), 4.35 (br d, *J* = 9.2 Hz, 1H), 4.28 - 4.16 (m, 3H), 4.16 - 4.09 (m, 4H), 3.90 (dd, *J* = 4.9, 9.5 Hz, 1H), 3.87 - 3.80 (m, 1H), 3.77 (dd, *J* = 2.9, 9.5 Hz, 1H), 3.62 (s, 3H), 3.29 - 3.18 (m, 1H), 3.16 - 3.05 (m, 1H), 2.98 - 2.80 (m, 2H), 2.70 - 2.58 (m, 1H), 2.17 (dt, *J* = 7.7, 13.9 Hz, 1H). MS (ESI) *m/z* $(M+1)^+$ = 645.2.<br>HPLC retention time: 3.697 min.<br>Separation conditions: chromatographic column: Xbridge Shield RP-18, 5 μm, 2.1 * 50 mm; column temperature: 50°C; mobile phase: water (0.2 mL/1 L ammonia water)-acetonitrile; acetonitrile: 10% to 80% 6 min, 80% 2 min; flow rate: 0.8 mL/min.<br>SFC retention time: E20-a: 4.068 min; E20-b: 4.479 min; E20-c: 5.054 min; E20-d: 5.518 min.<br>Separation conditions: chromatographic column: (S,S) Whelk-O1 100 × 4.6 mm I.D., 5.0 μm; column temperature: 40°C; mobile phase: $CO_2$-methanol |

(continued)

| Example | Structural formula | Analytical data |
|---|---|---|
| | 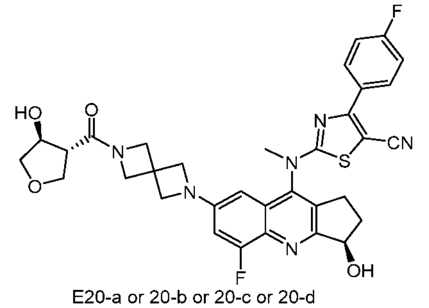<br>E20-a or 20-b or 20-c or 20-d<br>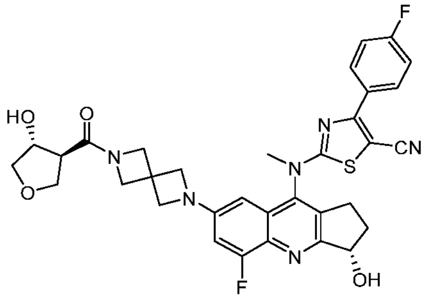<br>E20-a or 20-b or 20-c or 20-d<br>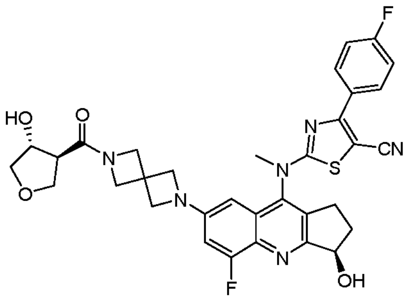<br>E20-a or 20-b or 20-c or 20-d | (0.05% diethylamine); methanol (0.05% diethylamine): 50%; flow rate: 2.5 mL/min.<br>E20-a:<br>$^1$H NMR (E20-a) (400 MHz, CHLOROFORM-*d*) δ = 8.07 (br s, 2H), 7.10 (br t, *J* = 8.5 Hz, 2H), 6.69 - 6.59 (m, 1H), 6.12 (s, 1H), 5.34 - 5.23 (m, 1H), 4.54 (br s, 1H), 4.43 (br d, *J* = 9.4 Hz, 1H), 4.28 (d, *J* = 9.2 Hz, 1H), 4.20 - 4.09 (m, 3H), 4.09 - 4.03 (m, 4H), 3.84 (dd, *J* = 4.9, 9.5 Hz, 1H), 3.79 - 3.73 (m, 1H), 3.70 (dd, *J* = 2.9, 9.6 Hz, 1H), 3.55 (s, 3H), 3.44 (br s, 1H), 3.08 - 2.97 (m, 1H), 2.88 - 2.75 (m, 2H), 2.63 - 2.52 (m, 1H), 2.39 (br s, 1H), 2.10 (dt, *J* = 7.6, 14.1 Hz, 1H). E20-b:<br>$^1$H NMR (E20-b) (400 MHz, CHLOROFORM-*d*) δ = 8.14 (br s, 2H), 7.18 (t, *J* = 8.5 Hz, 2H), 6.72 (br d, *J* = 11.5 Hz, 1H), 6.20 (s, 1H), 5.42 - 5.31 (m, 1H), 4.62 (br s, 1H), 4.50 (br d, *J* = 9.5 Hz, 1H), 4.36 (br d, *J* = 9.0 Hz, 1H), 4.27 - 4.17 (m, 3H), 4.17 - 4.11 (m, 4H), 3.91 (dd, *J* = 4.9, 9.7 Hz, 1H), 3.86 - 3.75 (m, 2H), 3.63 (s, 3H), 3.29 (br s, 1H), 3.10 (dt, *J* = 4.3, 8.8 Hz, 1H), 2.97 - 2.82 (m, 2H), 2.71 - 2.58 (m, 1H), 2.32 - 2.06 (m, 2H).<br>E20-c:<br>$^1$H NMR (E20-c) (400 MHz, CHLOROFORM-*d*) δ = 8.15 (br d, *J* = 6.4 Hz, 2H), 7.19 (t, *J* = 8.6 Hz, 2H), 6.73 (dd, *J* = 2.1, 11.6 Hz, 1H), 6.21 (s, 1H), 5.43 - 5.31 (m, 1H), 4.63 (br s, 1H), 4.52 (br d, *J* = 9.3 Hz, 1H), 4.37 (br d, *J* = 9.5 Hz, 1H), 4.28 - 4.21 (m, 2H), 4.20 (s, 1H), 4.17 - 4.13 (m, 4H), 3.92 (dd, *J* = 4.9, 9.5 Hz, 1H), 3.84 (t, *J* = 8.0 Hz, 1H), 3.79 (br d, *J* = 9.7 Hz, 1H), 3.64 (s, 3H), 3.49 (br s, 1H), 3.17 - 3.06 (m, 1H), 2.96 - 2.84 (m, 2H), 2.70 - 2.61 (m, 1H), 2.40 (br s, 1H), 2.24 - 2.14 (m, 1H).<br>E20-d:<br>$^1$H NMR (E20-d) (400 MHz, CHLOROFORM-*d*) δ = 8.15 (br s, 2H), 7.18 (t, *J* = 8.4 Hz, 2H), 6.73 (br d, *J* = 11.5 Hz, 1H), 6.20 (s, 1H), 5.40 - 5.31 (m, 1H), 4.61 (br s, 1H), 4.50 (br d, *J* = 9.8 Hz, 1H), 4.36 (d, *J* = 9.3 Hz, 1H), 4.27 - 4.16 (m, 3H), 4.15 - 4.10 (m, 4H), 3.91 (dd, *J* = 4.8, 9.5 Hz, 1H), 3.87 - 3.75 (m, 2H), 3.63 (s, 3H), 3.24 (br s, 1H), 3.10 (td, *J* = 4.5, 8.7 Hz, 1H), 2.96 - 2.82 (m, 2H), 2.70 - 2.58 (m, 1H), 2.31 - 2.08 (m, 2H). |
| E21 | 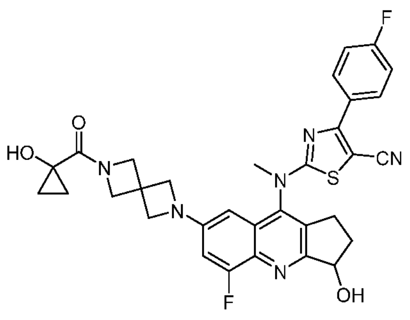 | $^1$H NMR (400 MHz, Chloroform-*d*) δ 8.17 - 7.99 (m, 2H), 7.16 (t, *J* = 8.6 Hz, 2H), 6.80 (d, *J* = 10.9 Hz, 1H), 6.19 (s, 1H), 5.60 (s, 1H), 4.78 - 4.12 (m, 8H), 3.63 (s, 3H), 3.06 (br, 1H), 2.96 - 2.81 (m, 1H), 2.76 - 2.63 (m, 1H), 2.41 (br, 1H), 2.28 - 2.17 (m, 1H), 1.37 - 1.30 (m, 2H), 1.05 - 0.97 (m, 2H). MS (ESI) *m/z* (M+H)$^+$ = 614.8.<br>HPLC retention time: 6.96 min.<br>Separation conditions: chromatographic column: High Performance GOLD 50 g HP C18; mobile phase: [water (10 mM/L ammonium bicarbonate)-acetonitrile]; gradient: 0 to 55% acetonitrile/water; flow rate: 50 mL/min. |

(continued)

| Example | Structural formula | Analytical data |
|---|---|---|
| E22 | 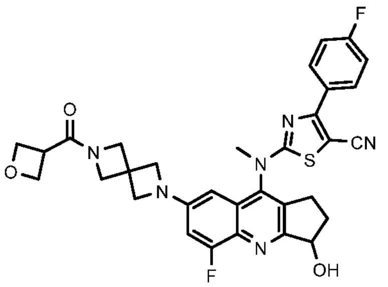 | $^1$H NMR (400 MHz, DMSO-d6) δ 8.14 - 7.80 (m, 2H), 7.47 - 7.21 (m, 2H), 7.03 - 6.87 (m, 1H), 6.27 - 6.10 (m, 1H), 5.13 - 4.99 (m, 1H), 4.67 - 4.49 (m, 3H), 4.22 - 3.97 (m, 8H), 3.63 - 3.48 (m, 4H), 3.17 - 3.07 (m, 1H), 2.94 - 2.75 (m, 1H), 2.01 - 1.83 (m, 1H), 1.62 - 1.45 (m, 1H), 1.37 - 1.24 (m, 1H). MS (ESI) *m/z* (M+H)$^+$ = 614.6.<br>HPLC retention time: 6.90 min.<br>Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic acid); acetonitrile: 5% to 95% 8 min, 95% 7 min; flow rate: 1.0 mL/min. |
| E23<br>E23-a<br>E23-b | 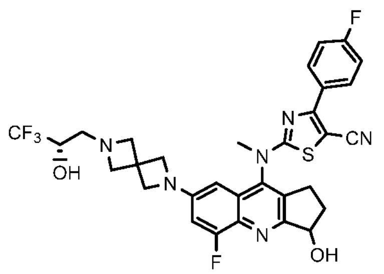<br>E23<br>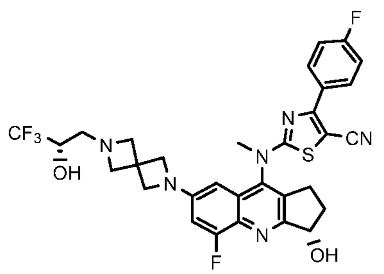<br>E23-a or E23-b<br>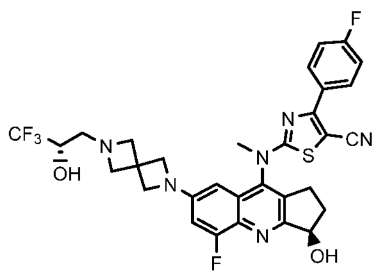<br>E23-a or E23-b | E23:<br>$^1$H NMR (400 MHz, Chloroform-*d*) δ 8.22 - 8.06 (m, 2H), 7.22 - 7.11 (m, 2H), 6.66 (dd, *J* = 11.8, 2.4 Hz, 1H), 6.16 (d, *J* = 2.4 Hz, 1H), 5.42 - 5.27 (m, 1H), 4.12 - 3.96 (m, 5H), 3.76 - 3.68 (m, 4H), 3.61 (s, 3H), 3.16 - 3.02 (m, 1H), 2.94 - 2.79 (m, 3H), 2.69 - 2.54 (m, 1H), 2.23 - 2.10 (m, 1H).<br>MS (ESI) *m/z* (M+H)$^+$ = 643.2.<br>HPLC retention time: 5.24 min.<br>Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min.<br>E23-a:<br>$^1$H NMR (400 MHz, Chloroform-*d*) δ 8.23 - 8.04 (m, 2H), 7.22 - 7.10 (m, 2H), 6.68 (dd, *J* = 11.8, 2.4 Hz, 1H), 6.16 (d, *J* = 2.4 Hz, 1H), 5.40 - 5.30 (m, 1H), 4.13 - 3.96 (m, 5H), 3.77 - 3.65 (m, 4H), 3.61 (s, 3H), 3.17 - 3.01 (m, 1H), 2.92 - 2.79 (m, 3H), 2.69 - 2.54 (m, 1H), 2.22 - 2.13 (m, 1H).<br>MS (ESI) *m/z* (M+H)$^+$ = 642.6.<br>HPLC retention time: 6.65 min. Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min.<br>SFC retention time: 1.695 min.<br>Separation conditions: chromatographic column: Chiralcel OD-3 150 × 4.6 mm I.D., 3 μm; column temperature: 35°C; mobile phase: $CO_2$-methanol (0.05% DEA); methanol (0.05% DEA): 5% to 40%; flow rate: 2.5 mL/min. |

(continued)

| Example | Structural formula | Analytical data |
|---|---|---|
| | | E23-b:<br>$^1$H NMR (400 MHz, Chloroform-*d*) δ 8.23 - 8.02 (m, 2H), 7.22 - 7.11 (m, 2H), 6.69 (dd, *J* = 11.8, 2.2 Hz, 1H), 6.15 (d, *J* = 2.2 Hz, 1H), 5.43 - 5.23 (m, 1H), 4.14 - 3.98 (m, 4H), 3.97 - 3.83 (m, 1H), 3.69 - 3.53 (m, 7H), 3.16 - 3.01 (m, 1H), 2.94 - 2.73 (m, 3H), 2.69 - 2.56 (m, 1H), 2.21 - 2.12 (m, 1H). MS (ESI) *m/z* $(M+H)^+$ = 642.6.<br>HPLC retention time: 6.60 min. Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min.<br>SFC retention time: 1.959 min.<br>Separation conditions: chromatographic column: Chiralcel OD-3 150 × 4.6 mm I.D., 3 μm; column temperature: 35°C; mobile phase: $CO_2$-methanol (0.05% DEA); methanol (0.05% DEA): 5% to 40%; flow rate: 2.5 mL/min. |
| E24<br>E24-a<br>E24-b | 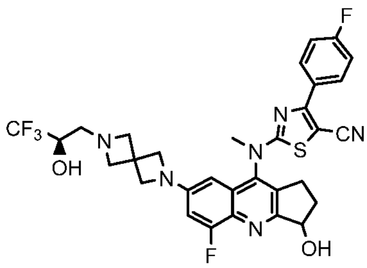<br>E24<br>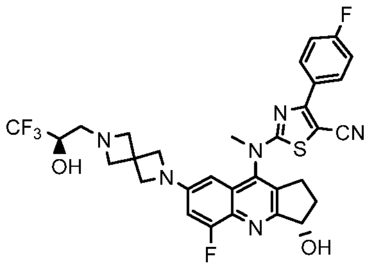<br>E24-a or E24-b<br>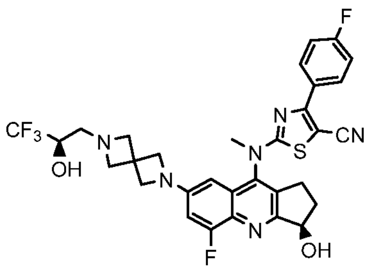<br>E24-a or E24-b | E24:<br>$^1$H NMR (400 MHz, Chloroform-*d*) δ 8.26 - 8.02 (m, 2H), 7.22 - 7.11 (m, 2H), 6.65 (dd, *J* = 11.8, 2.4 Hz, 1H), 6.16 (d, *J* = 2.4 Hz, 1H), 5.45 - 5.25 (m, 1H), 4.15 - 3.95 (m, 5H), 3.83 - 3.66 (m, 4H), 3.62 (s, 3H), 3.17 - 3.00 (m, 1H), 2.94 - 2.78 (m, 3H), 2.70 - 2.54 (m, 1H), 2.25 - 2.08 (m, 1H).<br>MS (ESI) *m/z* $(M+H)^+$ = 643.2.<br>HPLC retention time: 5.25 min.<br>Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min.<br>E24-a:<br>$^1$H NMR (400 MHz, Chloroform-*d*) δ 8.21 - 8.08 (m, 2H), 7.23 - 7.11 (m, 2H), 6.70 (dd, *J* = 11.8, 2.2 Hz, 1H), 6.15 (d, *J* = 2.2 Hz, 1H), 5.41 - 5.27 (m, 1H), 4.14 - 3.99 (m, 4H), 3.97 - 3.82 (m, 1H), 3.67 - 3.53 (m, 7H), 3.16 - 3.01 (m, 1H), 2.94 - 2.73 (m, 3H), 2.71 - 2.56 (m, 1H), 2.21 - 2.11 (m, 1H).<br>MS (ESI) *m/z* $(M+H)^+$ = 642.6.<br>HPLC retention time: 6.60 min. Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min.<br>SFC retention time: 1.748 min. |

(continued)

| Example | Structural formula | Analytical data |
|---|---|---|
| | | Separation conditions: chromatographic column: Chiralcel OD-3 150 × 4.6 mm I.D., 3 μm; column temperature: 35°C; mobile phase: $CO_2$-methanol (0.05% DEA); methanol (0.05% DEA): 5% to 40%; flow rate: 2.5 mL/min.<br>E24-b:<br>$^1$H NMR (400 MHz, Chloroform-*d*) δ 8.21 - 8.07 (m, 2H), 7.22 - 7.11 (m, 2H), 6.70 (dd, *J* = 11.8, 2.2 Hz, 1H), 6.15 (d, *J* = 2.2 Hz, 1H), 5.41 - 5.27 (m, 1H), 4.11 - 4.00 (m, 4H), 3.96 - 3.85 (m, 1H), 3.66 - 3.54 (m, 7H), 3.15 - 3.04 (m, 1H), 2.92 - 2.73 (m, 3H), 2.69 - 2.57 (m, 1H), 2.20 - 2.13 (m, 1H).<br>MS (ESI) *m/z* $(M+H)^+$ = 642.6.<br>HPLC retention time: 6.60 min. Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min.<br>SFC retention time: 1.980 min.<br>Separation conditions: chromatographic column: Chiralcel OD-3 150 × 4.6 mm I.D., 3 μm; column temperature: 35°C; mobile phase: $CO_2$-methanol (0.05% DEA); methanol (0.05% DEA): 5% to 40%; flow rate: 2.5 mL/min. |
| | | E25:<br>$^1$H NMR (400 MHz, Chloroform-*d*) δ 8.05 (m, 2H), 7.14 - 7.10 (m, 2H), 6.66 (s, 1H), 4.56 (m, 2H), 4.26 (m, 6H), 3.69 (s, 3H), 3.23 - 3.96 (m, 2H), 2.88 (s, 3H), 2.38 (s, 2H), 1.80 (s, 3H), 1.41 (s, 6H).<br>MS (ESI) *m/z* $(M+H)^+$ = 628.4.<br>HPLC retention time: 8.72 min. |

(continued)

| Example | Structural formula | Analytical data |
|---|---|---|
| E25<br>E25-a<br><br>E25-b | 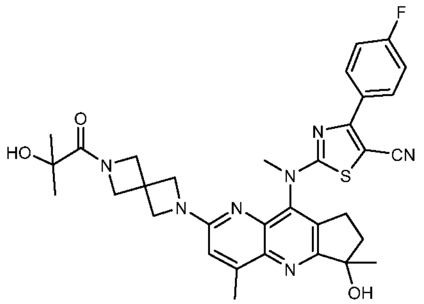<br>E25<br>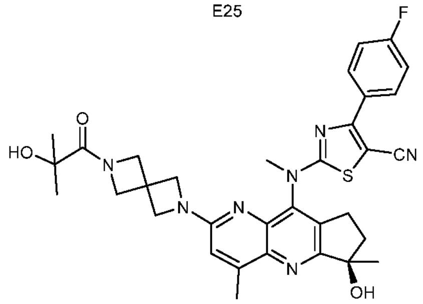<br>E25a or E25b<br>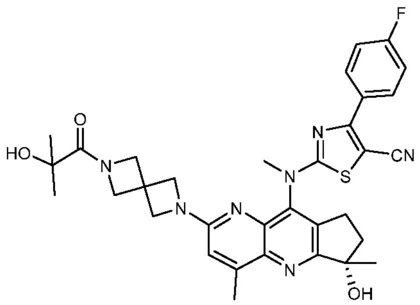<br>E25a or E25b | Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min.<br>E25-a:<br>$^1$H NMR (400 MHz, DMSO-d6) δ 8.02 (brs, 2H), 7.39 (t, *J* = 8.9 Hz, 2H), 6.84 (s, 1H), 5.22 (s, 1H), 5.04 (s, 1H), 4.53 (s, 2H), 4.24 - 4.09 (m, 4H), 4.02 (s, 2H), 3.62 (s, 3H), 3.11 - 2.93 (m, 1H), 2.88 - 2.73 (m, 1H), 2.66 (s, 3H), 2.25 - 2.08 (m, 2H), 1.55 (s, 3H), 1.22 (s, 6H).<br>MS (ESI) *m*/*z* $(M+H)^+$ = 628.3.<br>HPLC retention time: 6.484 min. Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic acid); acetonitrile: 5% to 95% 8 min, 95% 7 min; flow rate: 1.0 mL/min.<br>SFC retention time: 0.482 min.<br>Separation conditions: chromatographic column: ChiralPak AD-3 50 × 4.6 mm I.D., 3 μm; column temperature: 35°C; mobile phase: $CO_2$-ethanol (0.05% DEA); ethanol (0.05% DEA): 40%; flow rate: 3.0 mL/min.<br>E25-b:<br>$^1$H NMR (400 MHz, DMSO-d6) δ 8.02 (brs, 2H), 7.39 (t, *J* = 8.8 Hz, 2H), 6.84 (s, 1H), 5.22 (s, 1H), 5.04 (s, 1H), 4.53 (s, 2H), 4.18 (m, 4H), 4.02 (s, 2H), 3.62 (s, 3H), 3.14 - 2.94 (m, 1H), 2.92 - 2.75 (m, 1H), 2.67 (s, 3H), 2.29 - 2.09 (m, 2H), 1.55 (s, 3H), 1.22 (s, 6H). MS (ESI) *m*/*z* $(M+H)^+$ = 628.3.<br>HPLC retention time: 6.466 min. Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic acid); acetonitrile: 5% to 95% 8 min, 95% 7 min; flow rate: 1.0 mL/min.<br>SFC retention time: 1.049 min.<br>Separation conditions: chromatographic column: ChiralPak AD-3 50 × 4.6 mm I.D., 3 μm; column temperature: 35°C; mobile phase: $CO_2$-ethanol (0.05% DEA); ethanol (0.05% DEA): 40%; flow rate: 3.0 mL/min. |

(continued)

| Example | Structural formula | Analytical data |
|---|---|---|
| E26 | 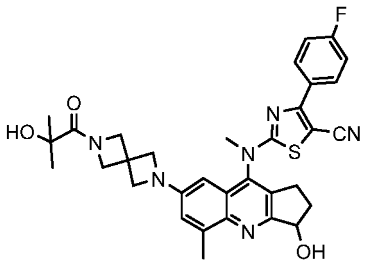 | $^1$H NMR (400 MHz, DMSO-d6) δ 8.08 (s, 2H), 7.41 (s, 2H), 6.97 (s, 1H), 6.25 (s, 1H), 5.51 (t, *J* = 6.0 Hz, 1H), 5.11 - 5.04 (m, 2H), 4.54 (s, 2H), 4.09 - 4.02 (m, 6H), 3.59 (d, *J* = 8.8 Hz, 3H), 3.12 - 2.81 (m, 2H), 2.71 (s, 3H), 2.40 (s, 1H), 1.99 - 1.88 (m, 1H), 1.23 (s, 6H). MS (ESI) *m/z* (M+H)$^+$ = 613.4.<br>HPLC retention time: 8.79 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| E27 | 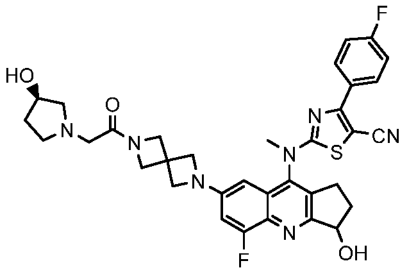 | $^1$H NMR (400 MHz, Chloroform-*d*) δ 8.24 - 8.04 (m, 2H), 7.22 - 7.12 (m, 2H), 6.71 (dd, *J* = 11.6, 2.2 Hz, 1H), 6.18 (d, *J* = 2.2 Hz, 1H), 5.42 - 5.25 (m, 1H), 4.45 - 4.33 (m, 3H), 4.27 - 4.19 (m, 2H), 4.17 - 4.06 (m, 4H), 3.62 (s, 3H), 3.16 - 2.98 (m, 2H), 2.94 - 2.79 (m, 3H), 2.70 - 2.54 (m, 2H), 2.23 - 2.09 (m, 3H), 1.89 - 1.80 (m, 4H).<br>MS (ESI) *m/z* (M+H)$^+$ = 657.8.<br>LCMS retention time: 1.11 min.<br>Separation conditions: chromatographic column: waters acquity CSH C18 3.0 * 30 mm, 1.7 μm; column temperature: 40°C; mobile phase: water (0.01% trifluoroacetic acid)-acetonitrile (0.01% trifluoroacetic acid); acetonitrile: 5% to 95% 0.7 min, 95% 0.8 min, 95% to 5% 0.5 min; flow rate: 1.2 mL/min. |
| E28 | 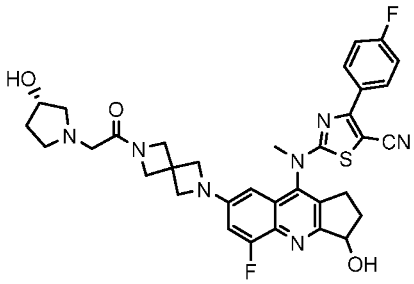 | $^1$H NMR (400 MHz, Chloroform-*d*) δ 8.21 - 8.05 (m, 2H), 7.23 - 7.10 (m, 2H), 6.71 (dd, *J* = 11.6, 2.2 Hz, 1H), 6.18 (d, 2.2 Hz, 1H), 5.43 - 5.27 (m, 1H), 4.47 - 4.31 (m, 3H), 4.27 - 4.19 (m, 2H), 4.18 - 4.05 (m, 4H), 3.62 (s, 3H), 3.16 - 3.01 (m, 2H), 2.94 - 2.79 (m, 3H), 2.69 - 2.56 (m, 2H), 2.22 - 2.10 (m, 3H), 1.88 - 1.79 (m, 4H).<br>MS (ESI) *m/z* (M+H)$^+$ = 657.8.<br>LCMS retention time: 1.11 min.<br>Separation conditions: chromatographic column: waters acquity CSH C18 3.0 * 30 mm, 1.7 μm; column temperature: 40°C; mobile phase: water (0.01% trifluoroacetic acid)-acetonitrile (0.01% trifluoroacetic acid); acetonitrile: 5% to 95% 0.7 min, 95% 0.8 min, 95% to 5% 0.5 min; flow rate: 1.2 mL/min. |

(continued)

| Example | Structural formula | Analytical data |
|---|---|---|
| E29 | 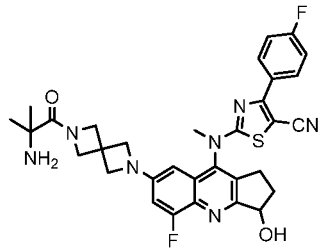 | $^1$H NMR (400 MHz, DMSO-d6) δ 8.29 (s, 1H), 8.05 (s, 2H), 7.40 (t, *J* = 8.8 Hz, 2H), 6.99 (dd, *J* = 12.4, 2.4 Hz, 1H), 6.21 (dd, *J* = 4.8, 2.4 Hz, 1H), 5.10 - 5.04 (m, 1H), 4.59 (s, 2H), 4.22 - 4.01 (m, 8H), 3.59 (d, *J* = 8.4 Hz,, 3H), 2.93 - 2.86 (m, 2H), 2.46 - 2.38(m, 1H), 1.95 - 1.90 (m, 1H), 1.25 (s, 6H).<br>MS (ESI) *m/z* $(M+H)^+$ = 615.80.<br>LCMS retention time: 1.14 min.<br>Separation conditions: chromatographic column: waters acquity CSH C18 3.0 * 30 mm, 1.7 μm; column temperature: 40°C; mobile phase: water (0.01% trifluoroacetic acid)-acetonitrile (0.01% trifluoroacetic acid); acetonitrile: 5% to 95% 0.7 min, 95% 0.8 min, 95% to 5% 0.5 min; flow rate: 1.2 mL/min. |
| E30<br>E30-a<br><br>E30-b | 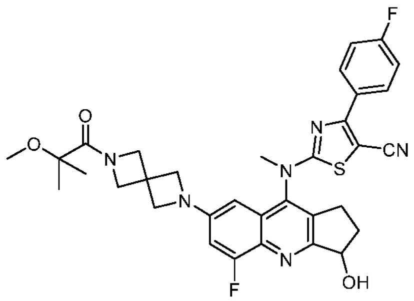<br>E30<br>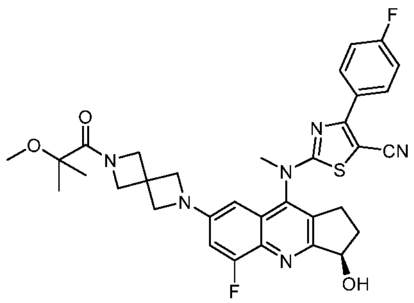<br>E30a or E30b | E30:<br>$^1$H NMR (400 MHz, DMSO-d6) δ 8.05 (s, 2H), 7.42 - 7.38 (m, 2H), 7.09 - 6.90 (m, 1H), 6.28 - 6.14 (m, 1H), 5.70 (d, *J* = 5.6 Hz, 1H), 5.16 - 5.01 (m, 1H), 4.45 (s, 2H), 4.12 - 4.07 (m, 6H), 3.59 (d, *J* = 8.4 Hz, 3H), 3.11 (s, 3H), 3.04 - 2.65 (m, 2H), 2.43 - 2.38 (m, 1H), 1.95 - 1.90 (m, 1H), 1.23 (s, 6H).<br>MS (ESI) *m/z* $(M+H)^+$ = 630.80.<br>LCMS retention time: 1.39 min.<br>Separation conditions: chromatographic column: waters acquity CSH C18 3.0 * 30 mm, 1.7 μm; column temperature: 40°C; mobile phase: water (0.01% trifluoroacetic acid)-acetonitrile (0.01% trifluoroacetic acid); acetonitrile: 5% to 95% 0.7 min, 95% 0.8 min, 95% to 5% 0.5 min; flow rate: 1.2 mL/min.<br><br>E30-a:<br><br>$^1$H NMR (400 MHz, Chloroform-*d*) δ 8.14 (s, 2H), 7.17 (t, *J* = 8.6 Hz, 2H), 6.73 (dd, *J* = 11.7, 2.2 Hz, 1H), 6.19 (d, *J* = 2.2 Hz, 1H), 5.41 (d, *J* = 6.5 Hz, 1H), 4.54 (s, 2H), 4.22 (s, 2H), 4.14 (s, 4H), 3.63 (s, 3H), 3.23 (s, 3H), 3.10 - 3.05 (m, 1H), 2.93 - 2.84 (m, 1H), 2.64 (m, 1H), 2.21 (m, 1H), 1.37 (s, 6H).<br><br>MS (ESI) *m/z* $(M+H)^+$ = 631.20. |

(continued)

| Example | Structural formula | Analytical data |
|---|---|---|
| | 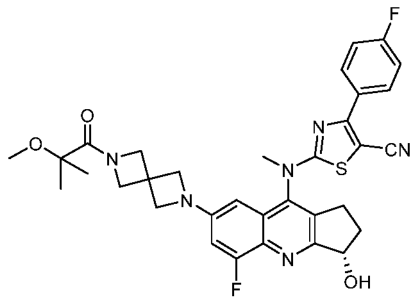 E30a or E30b | HPLC retention time: 6.175 min. Separation conditions: chromatographic column: Waters XBridge 4.6 * 100 mm, 3.5 μm; column temperature: 40°C; mobile phase: [water (10 mM ammonium bicarbonate)-acetonitrile; acetonitrile: 5% to 95% 7 min, 95% 5 min, 95% to 5% 3 min; flow rate: 1.0 mL/min. SFC retention time: 2.955 min. Separation conditions: chromatographic column: ChiralPak AD-3 50 × 4.6 mm I.D., 3 μm; column temperature: 35°C; mobile phase: A: $CO_2$, B: ethanol (0.05% DEA); from 5% to 40% of B in 4 min and from 40% to 5% of B in 0.2 min, then hold 5% of B for 1.8 min; flow rate: 3.0 mL/min. E30-b: $^1$H NMR (400 MHz, Chloroform-*d*) δ 8.14 (t, *J* = 6.9 Hz, 2H), 7.17 (t, *J* = 8.7 Hz, 2H), 6.72 (d, *J* = 11.6 Hz, 1H), 6.28 - 6.10 (m, 1H), 5.43 - 5.36 (m, 1H), 4.54 (s, 2H), 4.22 (s, 2H), 4.13 (s, 4H), 3.63 (s, 3H), 3.22 (s, 3H), 3.11 - 3.08 (m, 1H), 2.91 - 2.84 (m, 1H), 2.66 - 2.61 (m, 1H), 2.23 - 2.16 (m, 1H), 1.37 (s, 6H). MS (ESI) *m/z* $(M+H)^+$ = 631.20. HPLC retention time: 6.160 min. Separation conditions: chromatographic column: Waters XBridge 4.6 * 100 mm, 3.5 μm; column temperature: 40°C; mobile phase: [water (10 mM ammonium bicarbonate)-acetonitrile; acetonitrile: 5% to 95% 7 min, 95% 5 min, 95% to 5% 3 min; flow rate: 1.0 mL/min. SFC retention time: 3.331 min. Separation conditions: chromatographic column: ChiralPak AD-3 50 × 4.6 mm I.D., 3 μm; column temperature: 35°C; mobile phase: A: $CO_2$, B: ethanol (0.05% DEA); from 5% to 40% of B in 4 min and from 40% to 5% of B in 0.2 min, then hold 5% of B for 1.8 min; flow rate: 3.0 mL/min. |
| E31 | 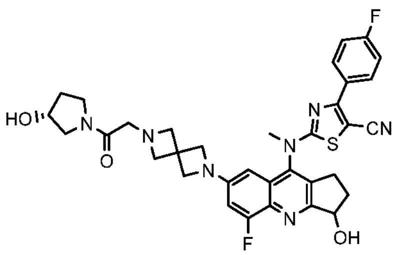 | $^1$H NMR (400 MHz, DMSO-d6) δ 8.21 (s, 1H), 8.05 (s, 2H), 7.40 (t, *J* = 8.9 Hz, 2H), 6.99 - 6.65 (m, 1H), 6.20 - 6.18(m, 1H), 5.15 - 5.04 (m, 1H), 4.29 - 4.21 (m, 1H), 4.05 - 3.98 (m, 4H), 3.60 (d, *J* = 8.4 Hz, 3H), 3.58 (m, 2H), 3.45 - 3.41 (m, 6H), 3.17 (m, 3H), 3.02 - 2.85 (m, 2H), 2.46 - 2.36 (m, 1H), 1.99 - 1.71 (m, 3H). MS (ESI) *m/z* $(M+H)^+$ = 657.80. LCMS retention time: 1.13 min. Separation conditions: chromatographic column: waters acquity CSH C18 3.0 * 30 mm, 1.7 μm; column temperature: 40°C; mobile phase: water (0.01% trifluoroacetic acid)-acetonitrile (0.01% trifluoroacetic acid); acetonitrile: 5% to 95% 0.7 min, 95% 0.8 min, 95% to 5% 0.5 min; flow rate: 1.2 mL/min. |
| | | E32: |

(continued)

| Example | Structural formula | Analytical data |
|---|---|---|
| E32<br>E32-a<br>E32-b | 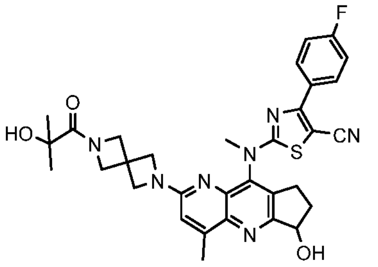<br>E32<br>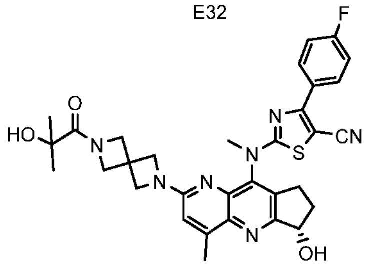<br>E32-a or E32-b<br>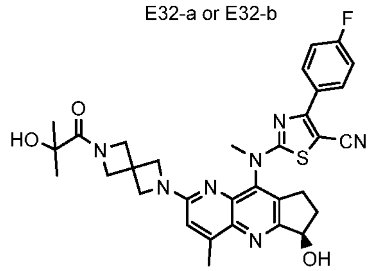<br>E32-a or E32-b | $^1H$ NMR (400 MHz, DMSO-d6) δ 8.01 (brs, 2H), 7.41 - 7.37 (t, *J* = 8.8 Hz, 2H), 6.85 (s, 1H), 5.56 - 5.55 (d, *J* = 5.8 Hz, 1H), 5.07 (m, 1H), 5.05 (s, 1H), 4.53 (s, 2H), 4.19 (m, 4H), 4.02 (s, 2H), 3.61 (s, 3H), 3.02 (m, 1H), 2.84 (m, 1H), 2.65 (s, 3H), 2.33 (m, 1H), 1.95 (m, 1H), 1.22 (s, 6H).<br>MS (ESI) *m/z* $(M+H)^+$ = 614.3.<br>HPLC retention time: 8.20 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min.<br>E32-a:<br>$^1H$ NMR (400 MHz, DMSO-d6) δ 8.03 - 7.98 (m, 2H), 7.39 (t, *J* = 8.7 Hz, 2H), 6.84 (s, 1H), 5.55 - 5.54 (d, *J* = 5.7 Hz, 1H), 5.05 (m, 1H), 5.04 (s, 1H), 4.53 (s, 2H), 4.18 (m, 4H), 4.02 (s, 2H), 3.61 (s, 3H), 3.02 (m, 1H), 2.82 (m, 1H), 2.65 (s, 3H), 2.44 (m, 1H), 1.96 (m, 1H), 1.22 (s, 6H).<br>MS (ESI) *m/z* $(M+H)^+$ = 614.4.<br>HPLC retention time: 8.18 min. Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min.<br>SFC retention time: 7.099 min.<br><br>Separation conditions: chromatographic column: DAICELCHIRALCEL®AS 250 * 25 mm 10 μm; mobile phase: A: n-hexane, B: EtOH (+0.1% 7.0 mol/L ammonia in MEOH); elution gradient: 40% A, 60% B; flow rate: 30 mL/min; column temperature: 25°C; column pressure: 100 bar.<br><br>E32-b:<br>$^1H$ NMR (400 MHz, DMSO-d6) δ 8.03 - 7.98 (m, 2H), 7.39 (t, *J* = 8.7 Hz, 2H), 6.85 (s, 1H), 5.08 (s, 1H), 4.53 (s, 2H), 4.17 (s, 4H), 4.02 (s, 2H), 3.61 (s, 3H), 3.02 (m, 1H), 2.85 (m, 1H), 2.65 (s, 3H), 2.44 (m, 1H), 1.96 (m, 1H), 1.22 (s, 6H).<br>MS (ESI) *m/z* $(M+H)^+$ = 614.5.<br>HPLC retention time: 8.18 min. Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min.<br>SFC retention time: 7.631 min. |

(continued)

| Example | Structural formula | Analytical data |
|---|---|---|
| | | Separation conditions: chromatographic column: DAICELCHIRALCEL®AS 250 * 25 mm 10 μm; mobile phase: A: n-hexane, B: EtOH (+0.1% 7.0 mol/L ammonia in MEOH); elution gradient: 40% A, 60% B; flow rate: 30 mL/min; column temperature: 25°C; column pressure: 100 bar. |
| E33 | 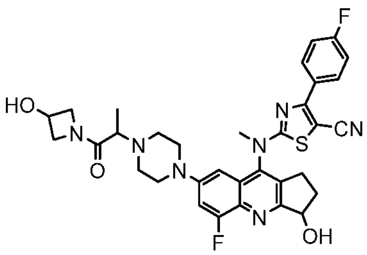 | $^1$H NMR (400 MHz, Chloroform-*d*) δ 8.20 - 8.06 (m, 2H), 7.24 - 7.10 (m, 3H), 6.65 (s, 1H), 5.44 - 5.26 (m, 1H), 4.74 - 4.62 (m, 1H), 4.32 - 4.23 (m, 1H), 3.96 - 3.83 (m, 1H), 3.64 (s, 3H), 3.36 - 3.24 (m, 4H), 3.16 - 2.99 (m, 3H), 2.96 - 2.82 (m, 1H), 2.82 - 2.71 (m, 2H), 2.72 - 2.59 (m, 3H), 2.25 - 2.12 (m, 1H), 1.25 (m, 4H).<br>MS (ESI) *m*/*z* $(M+H)^+$ = 646.2.<br>HPLC retention time: 4.81 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E34 | 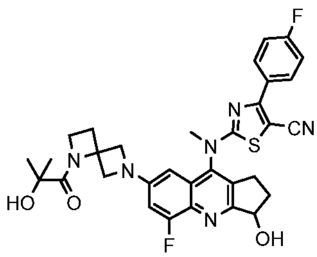 | $^1$H NMR (400 MHz, $CDCl_3$) δ 8.14 (dd, *J* = 7.6, 4.8 Hz, 2H), 7.16 (t, *J* = 8.6 Hz, 2H), 6.73 (dd, *J* = 12.0, 2.2 Hz, 1H), 6.17 (s, 1H), 5.45 - 5.25 (m, 1H), 4.76 (dd, *J* = 12.6, 7.2 Hz, 2H), 4.31 (t, *J* = 7.4 Hz, 2H), 4.03 (dd, *J* = 16.7, 6.8 Hz, 2H), 3.61 (s, 3H), 3.37 (s, 1H), 3.15 - 2.99 (m, 2H), 2.86 (td, *J* = 16.5, 8.0 Hz, 1H), 2.64 (t, *J* = 7.5 Hz, 3H), 2.16 (dt, *J* = 13.8, 8.1 Hz, 1H), 1.39 (s, 6H).<br>MS (ESI): *m*/*z* $[M+H]^+$ = 617.4.<br>HPLC retention time: 9.60 min.<br>Separation conditions: chromatographic column: Sunfire C18 150 * 4.6 mm, 5 μm; column temperature: 40°C; mobile phase: water (1.2 mL/4 L trifluoroacetic acid)-acetonitrile (1.2 mL/4 L trifluoroacetic acid); acetonitrile: 10% to 95% 12 min, 95% 3 min; flow rate: 1.0 mL/min. |
| | | E35:<br>$^1$H NMR (400 MHz, DMSO-d6) δ 8.05 (brs, 2H), 7.62 - 7.15 (m, 2H), 6.97 (d, *J* = 11.3 Hz, 1H), 6.21 (s, 1H), 5.35 (s, 1H), 5.05 (s, 1H), 4.54 (s, 2H), 4.05 (d, *J* = 21.9 Hz, 6H), 3.60 (d, *J* = 7.2 Hz, 3H), 3.16 - 2.70 (m, 2H), 2.27 - 1.98 (m, 2H), 1.55 (s, 3H), 1.23 (s, 6H). MS (ESI): *m*/*z* $[M+H]^+$ = 631.3.<br>HPLC retention time: 9.43 min. |

(continued)

| Example | Structural formula | Analytical data |
|---|---|---|
| E35<br><br>E35-a<br><br>E35-b | 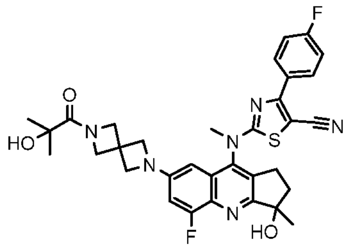<br>E35<br>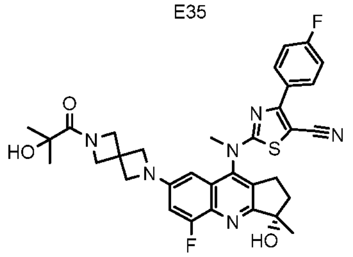<br>E35-a or E35-b<br>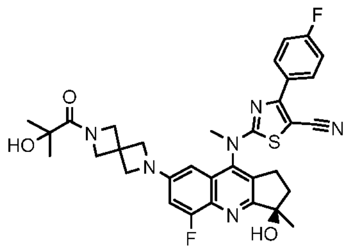<br>E35-a or E35-b | Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min.<br>E35-a:<br>$^1$H NMR (400 MHz, $CDCl_3$) δ 8.13 (dd, *J* = 5.9, 5.5 Hz, 2H), 7.17 (t, *J* = 8.4 Hz, 2H), 6.85 - 6.59 (m, 1H), 6.19 (s, 1H), 4.57 (s, 2H), 4.25 (s, 2H), 4.15 (m, 4H), 3.63 (s, 3H), 3.26 (m, 1H), 3.03 (m, 1H), 2.88 (m, 1H), 2.34 (m, 2H), 1.74 (s, 3H), 1.41 (s, 6H).<br>MS (ESI): *m/z* $[M+H]^+$ = 631.4.<br>HPLC retention time: 10.06 min. Separation conditions: chromatographic column: Agilent Eclipse Plus C18 150 * 4.6 mm, 3.5 μm; column temperature: 30°C; mobile water trifluoroacetic<br>phase: (0.03% acid)-acetonitrile (0.03% trifluoroacetic acid); acetonitrile: 10% 1.8 min, 10% to 95% 10.2 min, 95% 3 min; flow rate: 1.0 mL/min.<br>SFC retention time: 3.989 min.<br>Separation conditions: chromatographic column: DAICELCHIRALCEL®AS 250 * 25 mm 10 μm; mobile phase: A: supercritical $CO_2$, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi.<br>E35-b:<br>$^1$H NMR (400 MHz, $CDCl_3$) δ 8.13 (dd, *J* = 5.9, 5.5 Hz, 2H), 7.17 (t, *J* = 8.4 Hz, 2H), 6.85 - 6.59 (m, 1H), 6.19 (s, 1H), 4.57 (s, 2H), 4.25 (s, 2H), 4.13 (m, 4H), 3.63 (s, 3H), 3.26 (m, 1H), 3.02 (m, 1H), 2.86 (m, 1H), 2.38 (m, 2H), 1.74 (s, 3H), 1.41 (s, 6H). MS (ESI): *m/z* $[M+H]^+$ = 631.4.<br>HPLC retention time: 10.07 min. Separation conditions: chromatographic column: Agilent Eclipse Plus C18 150 * 4.6 mm, 3.5 μm; column temperature: 30°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min.<br>SFC retention time: 4.582 min.<br>Separation conditions: chromatographic column: DAICELCHIRALCEL®AS 250 * 25 mm 10 μm; mobile phase: A: supercritical $CO_2$, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi. |

(continued)

| Example | Structural formula | Analytical data |
|---|---|---|
| E36 | 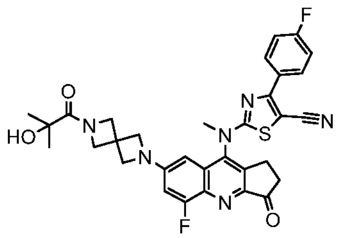 | $^1H$ NMR (400 MHz, DMSO-d6) δ 8.23 - 7.80 (brs, 2H), 7.39 (t, *J* = 7.9 Hz, 2H), 7.16 (d, *J* = 12.1 Hz, 1H), 6.18 (s, 1H), 5.07 (s, 1H), 4.55 (s, 2H), 4.29 - 4.10 (m, 4H), 4.04 (s, 2H), 3.63 (s, 3H), 3.20 (dd, *J* = 12.7, 6.7 Hz, 1H), 3.13 - 2.89 (m, 1H), 2.77 (dd, *J* = 12.7, 8.0 Hz, 2H), 1.23 (s, 6H).<br>MS (ESI): *m/z* $[M+H]^+$ = 615.3.<br>HPLC retention time: 9.37 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| E37 | 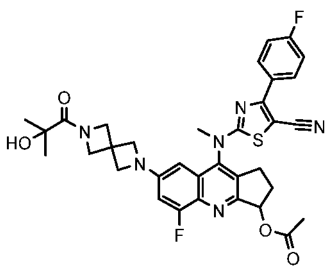 | $^1H$ NMR (400 MHz, $CDCl_3$) δ 8.14 (m, 2H), 7.17 (t, *J* = 8.4 Hz, 2H), 6.74 (d, *J* = 11.5 Hz, 1H), 6.42 - 5.66 (m, 2H), 4.57 (s, 2H), 4.21 (m, 6H), 3.63 (s, 3H), 3.22 - 3.00 (m, 1H), 3.01 - 2.86 (m, 1H), 2.80 - 2.58 (m, 1H), 2.33 - 2.02 (m, 5H), 1.41 (s, 6H).<br>MS (ESI): *m/z* $[M+H]^+$ = 659.3.<br>HPLC retention time: 10.16 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| E38 | 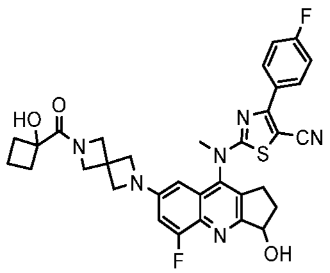 | $^1H$ NMR (400 MHz, $CDCl_3$) δ 8.24 - 8.01 (m, 2H), 7.16 (t, *J* = 8.3 Hz, 2H), 6.72 (d, *J* = 11.5 Hz, 1H), 6.18 (s, 1H), 5.42 (s, 1H), 4.55 (s, 2H), 4.19 (d, *J* = 47.7 Hz, 6H), 3.62 (s, 3H), 3.25 - 2.71 (m, 4H), 2.70 - 2.60 (m, 3H), 2.21 - 2.02 (m, 3H), 1.99 - 1.75 (m, 2H).<br>MS (ESI) *m/z* $(M+H)^+$ = 629.3.<br>HPLC retention time: 9.17 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |

(continued)

| Example | Structural formula | Analytical data |
| --- | --- | --- |
| E39 | 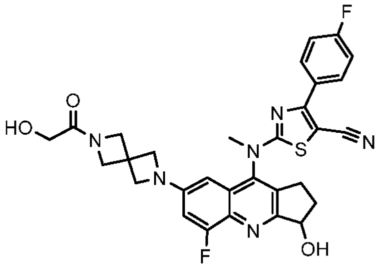 | $^1$H NMR (400 MHz, Methanol-$d_4$) δ 8.01 (s, 2H), 7.14 (t, *J* = 8.6 Hz, 2H), 6.80 (dd, *J* = 12.3, 2.3 Hz, 1H), 6.19 (d, *J* = 2.2 Hz, 1H), 5.20 - 5.04 (m, 1H), 4.36 (s, 2H), 4.12 (d, *J* = 5.7 Hz, 2H), 4.03 (q, *J* = 6.1, 4.1 Hz, 4H), 3.96 (s, 2H), 3.56 (d, *J* = 6.4 Hz, 3H), 2.90 - 2.68 (m, 2H), 2.53 - 2.37 (m, 1H), 2.09 - 1.89 (m, 1H).<br>MS (ESI) *m/z* (M+H)$^+$ = 589.2.<br>HPLC retention time: 5.92 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E40 | 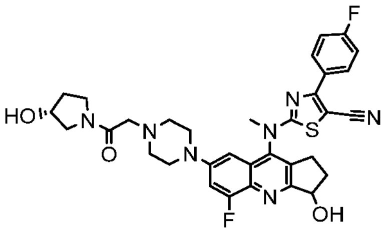 | $^1$H NMR (400 MHz, DMSO-d6) δ 8.21 - 7.89 (m, 2H), 7.44 (dd, *J* = 13.8, 2.6 Hz, 1H), 7.29 - 7.06 (m, 2H), 6.75 (s, 1H), 5.27 - 5.12 (m, 1H), 4.60 (s, 1H), 4.47 - 4.32 (m, 1H), 3.72 - 3.62 (m, 4H), 3.59 - 3.51 (m, 1H), 3.40 - 3.33 (m, 3H), 3.27 - 3.16 (m, 4H), 3.00 - 2.79 (m, 1H), 2.79 - 2.59 (m, 4H), 2.59 - 2.47 (m, 1H), 2.22 - 1.79 (m, 4H).<br>MS (ESI) *m/z* (M+H)$^+$ = 646.2.<br>HPLC retention time: 5.91 min.<br>Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic acid); acetonitrile: 5% to 95% 8 min, 95% 7 min; flow rate: 1.0 mL/min. |
| E41 | 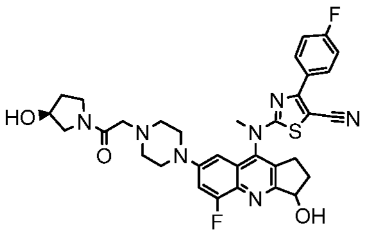 | $^1$H NMR (400 MHz, Methanol-$d_4$) δ 7.99 (s, 2H), 7.35 (dd, *J* = 13.8, 2.6 Hz, 1H), 7.17 - 7.05 (m, 2H), 6.67 (s, 1H), 5.20 - 5.07 (m, 1H), 4.50 (s, 1H), 4.38 - 4.25 (m, 1H), 3.63 - 3.54 (m, 4H), 3.49 - 3.43 (m, 1H), 3.30 - 3.25 (m, 3H), 3.20 - 3.08 (m, 4H), 2.88 - 2.70 (m, 1H), 2.69 - 2.53 (m, 4H), 2.53 - 2.35 (m, 1H), 2.06 - 1.74 (m, 4H).<br>MS (ESI) *m/z* (M+H)$^+$ = 646.2.<br>HPLC retention time: 5.90 min.<br>Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic acid); acetonitrile: 5% to 95% 8 min, 95% 7 min; flow rate: 1.0 mL/min. |

(continued)

| Example | Structural formula | Analytical data |
|---|---|---|
| E42 | 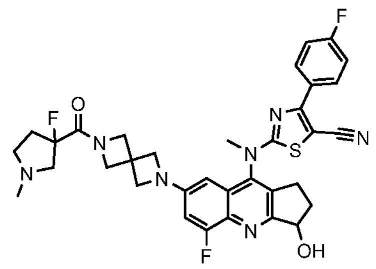 | $^1$H NMR (400 MHz, DMSO-d6) δ 8.16 - 8.05 (m, 2H), 7.43 - 7.40 (m, 2H), 6.98 (dd, *J* = 12.4, 2.4 Hz, 1H), 6.21 - 6.19 (m, 1H), 5.71 (br, 1H), 5.11 - 5.04(m, 1H), 4.47 (d, *J* = 4.0 Hz, 2H), 4.13 - 4.06 (m, 6H), 3.59 (d, *J* = 8.4 Hz, 3H), 2.98 - 2.64 (m, 5H), 2.45 - 2.28 (m, 3H), 2.25 (s, 3H), 2.08 - 1.90 (m, 2H).<br>MS (ESI) *m/z* $(M+H)^+$ = 660.2.<br>HPLC retention time: 6.25 min.<br>Separation conditions: chromatographic column: Waters XBridge 4.6 * 100 mm, 3.5 μm; column temperature: 40°C; mobile phase: [water (10 mM ammonium bicarbonate)-acetonitrile; acetonitrile: 5% to 95% 7 min, 95% 5 min, 95% to 5% 3 min; flow rate: 1.0 mL/min. |
| E43 | 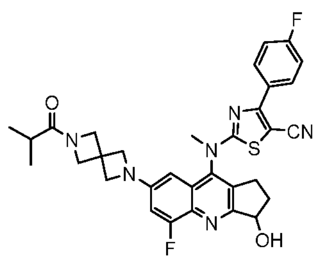 | $^1$H NMR (400 MHz, Methanol-$d_4$) δ 8.01 (s, 2H), 7.13 (t, *J* = 8.6 Hz, 2H), 6.82 (dd, *J* = 12.3, 2.3 Hz, 1H), 6.20 (d, *J* = 2.3 Hz, 1H), 5.13 (d, *J* = 6.2 Hz, 1H), 4.32 (s, 2H), 4.04 (m, 7H), 3.56 (s, 3H), 2.77 (s, 1H), 2.61 - 2.31 (m, 2H), 2.14 - 1.81 (m, 1H), 0.96 (d, *J* = 6.8 Hz, 6H).<br>MS (ESI) *m/z* $(M+H)^+$ = 600.8.<br>HPLC retention time: 6.13 min.<br>Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min. |
| E44 | 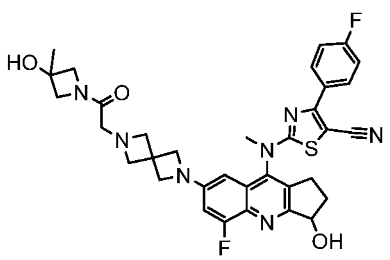 | $^1$H NMR (400 MHz, Methanol-$d_4$) δ 8.11 - 7.87 (m, 2H), 7.23 - 7.02 (m, 2H), 6.79 (dd, *J* = 12.2, 2.4 Hz, 1H), 6.17 (d, *J* = 2.4 Hz, 1H), 5.17 - 5.06 (m, 1H), 4.09 - 4.00 (m, 4H), 4.00 - 3.95 (m, 3H), 3.95 - 3.89 (m, 2H), 3.88 - 3.74 (m, 2H), 3.74 - 3.63 (m, 2H), 3.56 - 3.51 (m, 4H), 2.53 - 2.35 (m, 1H), 2.04 - 1.96 (m, 1H), 1.95 - 1.88 (m, 1H), 1.60 - 1.45 (m, 1H), 1.38 (s, 3H).<br>MS (ESI) *m/z* $(M+H)^+$ = 658.4.<br>HPLC retention time: 6.97 min.<br>Separation conditions: chromatographic column: Waters XBridge 4.6 * 150 mm, 3.5 μm; column temperature: 30°C; mobile phase: [water (10 mM ammonium bicarbonate)-acetonitrile; acetonitrile: 5% to 95% 8 min, 95% 7 min; flow rate: 1.0 mL/min. |

(continued)

| Example | Structural formula | Analytical data |
|---|---|---|
| E45 | 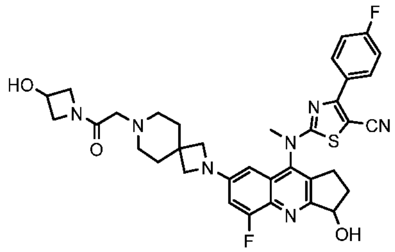 | $^1$H NMR (400 MHz, DMSO-d6) δ 8.05 (s, 2H), 7.39 (d, *J* = 9.0 Hz, 2H), 6.95 (d, *J* = 12.3 Hz, 1H), 6.20 (s, 1H), 5.68 (dd, *J* = 7.9, 5.8 Hz, 2H), 5.07 (dq, *J* = 17.5, 6.1 Hz, 1H), 4.48 - 4.36 (m, 1H), 4.33 (t, *J* = 8.2 Hz, 1H), 4.07 - 3.97 (m, 1H), 3.89 (dd, *J* = 9.3, 4.4 Hz, 1H), 3.72 - 3.51 (m, 8H), 2.97 - 2.80 (m, 3H), 2.35 (s, 5H), 1.92 (s, 1H), 1.73 (s, 4H).<br>MS (ESI) *m/z* $(M+H)^+$ = 672.0.<br>HPLC retention time: 4.05 min.<br>Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min. |
| E46 | 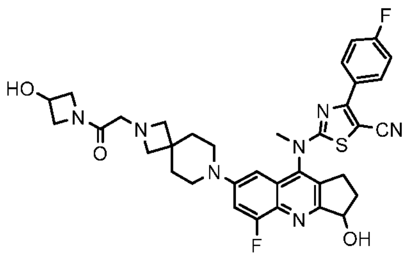 | $^1$H NMR (400 MHz, DMSO-d6) δ 8.20 - 7.85 (m, 2H), 7.67 - 7.46 (m, 1H), 7.40 (m, 2H), 6.69 (dd, *J* = 4.3, 2.1 Hz, 1H), 5.70 (t, *J* = 6.1 Hz, 2H), 5.17 - 4.99 (m, 1H), 4.50 - 4.36 (m, 1H), 4.33 - 4.19 (m, 1H), 4.00 (dd, *J* = 9.7, 6.9 Hz, 1H), 3.84 (dd, *J* = 8.9, 4.4 Hz, 1H), 3.69 - 3.47 (m, 4H), 3.25 (s, 3H), 3.19 - 2.73 (m, 8H), 2.46 - 2.35 (m, 1H), 2.06 - 1.86 (m, 2H), 1.74 (s, 4H).<br>MS (ESI): *m/z* $[M+H]^+$ = 672.4.<br>HPLC retention time: 7.57 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| E47 | 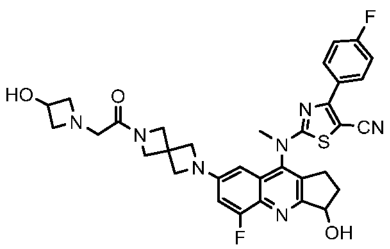 | $^1$H NMR (400 MHz, DMSO-d6) δ 8.05 (s, 2H), 7.39 (d, *J* = 9.0 Hz, 2H), 6.98 (dd, *J* = 12.4, 2.3 Hz, 1H), 6.21 (dd, *J* = 4.9, 2.3 Hz, 1H), 5.70 (s, 1H), 5.26 (s, 1H), 5.17 - 4.95 (m, 1H), 4.29 (s, 2H), 4.15 (t, *J* = 6.1 Hz, 1H), 4.12 - 4.03 (m, 4H), 4.00 (s, 2H), 3.59 (d, *J* = 8.4 Hz, 3H), 3.56 - 3.49 (m, 3H), 3.00 (s, 2H), 2.80 - 2.72 (m, 2H), 2.45 - 2.35 (m, 2H), 1.99 - 1.84 (m, 1H).<br>MS (ESI) *m/z* $(M+H)^+$ = 643.8.<br>HPLC retention time: 4.04 min.<br>Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min. |

(continued)

| Example | Structural formula | Analytical data |
|---|---|---|
| E48 | 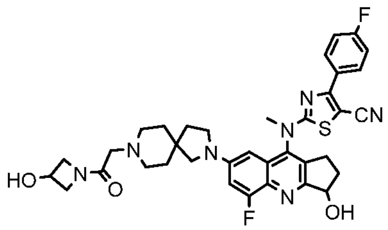 | $^{1}$H NMR (400 MHz, DMSO-d6) $\delta$ 8.06 (s, 2H), 7.40 (s, 2H), 7.13 (d, *J* = 13.2 Hz, 1H), 6.23 (t, *J* = 3.0 Hz, 1H), 5.66 (d, *J* = 6.1 Hz, 2H), 5.17 - 4.95 (m, 1H), 4.47 - 4.38 (m, 2H), 4.38 - 4.28 (m, 1H), 4.08 - 3.96 (m, 1H), 3.95 - 3.85 (m, 1H), 3.61 - 3.54 (m, 3H), 3.40 - 3.27 (m, 4H), 3.24 - 3.10 (m, 2H), 2.97 - 2.87 (m, 2H), 2.44 - 2.29 (m, 5H), 1.98 - 1.88 (m, 1H), 1.84 (t, *J* = 7.0 Hz, 2H), 1.61 - 1.47 (m, 4H). MS (ESI) *m/z* (M+H)$^+$ = 686.0.<br>HPLC retention time: 4.16 min.<br>Separation conditions: chromatographic column: waters XSelect CSH C18 4.6 * 100 mm, 3.5 $\mu$m; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min. |
| E49 | 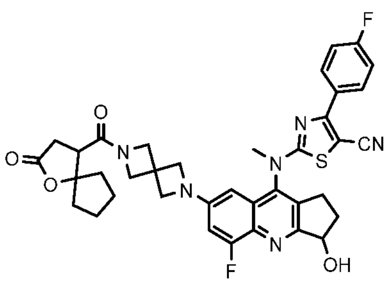 | $^{1}$H NMR (400 MHz, DMSO-d6) $\delta$ 8.05 (s, 2H), 7.40 (s, 2H), 6.99 (dd, *J* = 12.4, 2.3 Hz, 1H), 6.21 (dd, *J* = 4.9, 2.3 Hz, 1H), 5.71 (dd, *J* = 5.5, 1.7 Hz, 1H), 5.12 - 5.03 (m, 1H), 4.42 (d, *J* = 9.4 Hz, 1H), 4.32 (d, *J* = 9.3 Hz, 1H), 4.09 (dt, *J* = 16.0, 6.5 Hz, 6H), 3.59 (d, *J* = 8.4 Hz, 3H), 3.23 (dd, *J* = 8.2, 4.3 Hz, 1H), 3.07 - 2.73 (m, 3H), 2.56 (s, 1H), 2.46 - 2.35 (m, 2H), 1.95 - 1.90 (m, 2H), 1.72 - 1.61 (m, 6H).<br>MS (ESI) *m/z* (M+H)$^+$ = 697.6.<br>HPLC retention time: 7.01 min.<br>Separation conditions: chromatographic column: waters XSelect CSH C18 4.6 * 100 mm, 3.5 $\mu$m; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E50 | 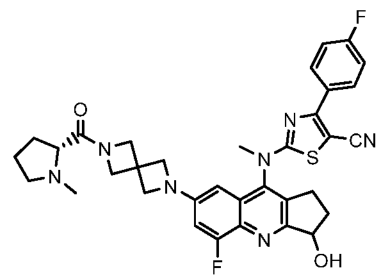 | $^{1}$H NMR (400 MHz, DMSO-*d6*) $\delta$ 8.19 - 8.05 (m, 2H), 7.41 (s, 2H), 6.98 (d, *J* = 12.4 Hz, 1H), 6.21 (d, *J* = 4.8 Hz, 1H), 5.72 (s, 1H), 5.12 - 5.04 (m, 1H), 4.38 (s, 2H), 4.15 - 3.98 (m, 6H), 3.59 (d, *J* = 8.4 Hz, 3H), 3.03 - 2.67 (m, 4H), 2.44 - 2.35 (m, 1H), 2.20 (s, 3H), 2.15 - 2.13 (m, 1H), 1.97 - 1.91 (m, 2H), 1.73 - 1.67(m, 3H). MS (ESI) *m/z* (M+H)$^+$ = 642.5.<br>HPLC retention time: 5.01 min.<br>Separation conditions: chromatographic column: waters XSelect CSH C18 4.6 * 100 mm, 3.5 $\mu$m; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |

(continued)

| Example | Structural formula | Analytical data |
|---|---|---|
| E51 | 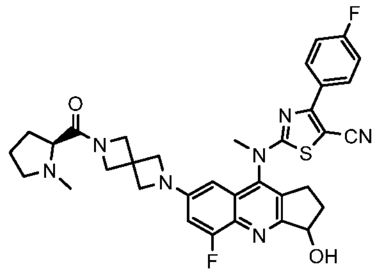 | $^1$H NMR (400 MHz, DMSO-*d6*) δ 8.17 - 8.06 (m, 2H), 7.41 (s, 2H), 6.98 (dd, *J* = 12.4, 2.4 Hz, 1H), 6.30 - 6.13 (m, 1H), 5.72 (s, 1H), 5.12 - 5.04 (m, 1H), 4.38 (s, 2H), 4.20 - 3.97 (m, 6H), 3.59 (d, *J* = 8.4 Hz, 3H), 2.97 - 2.77 (m, 4H), 2.46 - 2.36 (m, 1H), 2.21 (s, 3H), 2.19 - 2.15 (m, 1H), 2.03 - 1.86 (m, 2H), 1.74 - 1.71 (m, 3H).<br>MS (ESI) *m/z* $(M+H)^+$ = 642.5.<br>HPLC retention time: 5.00 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E52 | 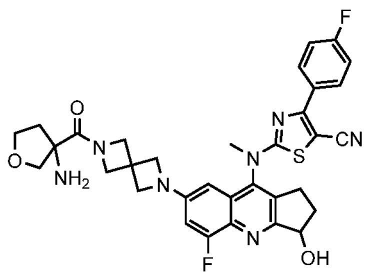 | $^1$H NMR (400 MHz, DMSO-*d6*) δ 8.06 (s, 2H), 7.42 (d, *J* = 9.1 Hz, 2H), 6.99 (dd, *J* = 12.4, 2.3 Hz, 1H), 6.21 (dd, *J* = 5.0, 2.3 Hz, 1H), 5.71 (d, *J* = 5.6 Hz, 1H), 5.11 - 5.04 (m, 1H), 4.56 (s, 2H), 4.13 - 4.03 (m, 6H), 3.89 - 3.81 (m, 2H), 3.75 (dt, *J* = 8.2, 4.2 Hz, 1H), 3.59 (d, *J* = 8.4 Hz, 3H), 3.44 (d, *J* = 8.5 Hz, 1H), 2.92 - 2.85 (m, 1H), 2.44 - 2.39 (m, 1H), 2.27 - 2.21 (m, 1H), 1.95 - 1.90 (m, 1H), 1.73 - 1.68 (m, 1H), 1.23 - 1.11 (m, 1H), 0.93 - 0.76 (m, 1H).<br>MS (ESI) *m/z* $(M+H)^+$ = 644.2.<br>HPLC retention time: 5.36 min.<br>Separation conditions: chromatographic column: Waters XBridge 4.6 * 100 mm, 3.5 μm; column temperature: 40°C; mobile phase: [water (10 mM ammonium bicarbonate)-acetonitrile; acetonitrile: 5% to 95% 7 min, 95% 5 min, 95% to 5% 3 min; flow rate: 1.0 mL/min. |
| E53 | 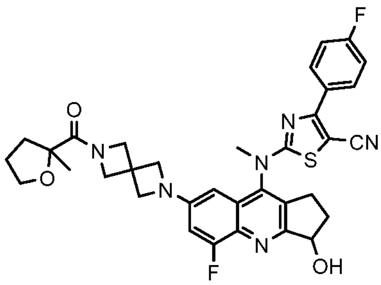 | $^1$H NMR (400 MHz, DMSO-d6) δ 8.05 (s, 2H), 7.40 (t, *J* = 7.9 Hz, 2H), 6.97 (dd, *J* = 12.5, 2.3 Hz, 1H), 6.20 (dd, *J* = 4.7, 2.3 Hz, 1H), 5.70 (s, 1H), 5.11 - 5.03 (m, 1H), 4.52 - 4.40 (m, 2H), 4.12 - 4.00 (m, 6H), 3.81 (qd, *J* = 5.1, 3.2 Hz, 1H), 3.72 - 3.66 (m, 1H), 3.59 (d, *J* = 8.5 Hz, 3H), 3.06 - 2.69 (m, 2H), 2.44 - 2.39 (m, 1H), 2.29 - 2.23 (m, 1H), 1.94 - 1.79 (m, 2H), 1.77 - 1.70 (m, 1H), 1.62 - 1.56 (m, 1H), 1.27 (s, 3H). MS (ESI) *m/z* $(M+H)^+$ = 643.4.<br>HPLC retention time: 6.51 min.<br>Separation conditions: chromatographic column: Waters XBridge 4.6 * 100 mm, 3.5 μm; column temperature: 40°C; mobile phase: [water (10 mM ammonium bicarbonate)-acetonitrile; acetonitrile: 5% to 95% 7 min, 95% 5 min, 95% to 5% 3 min; flow rate: 1.0 mL/min. |

(continued)

| Example | Structural formula | Analytical data |
|---|---|---|
| E54<br>E54-a<br><br>E54-b | 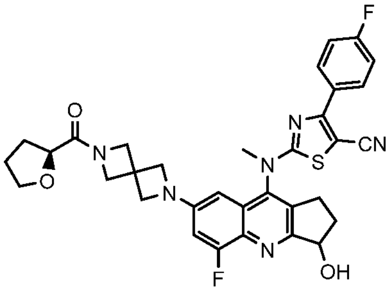<br>E54<br>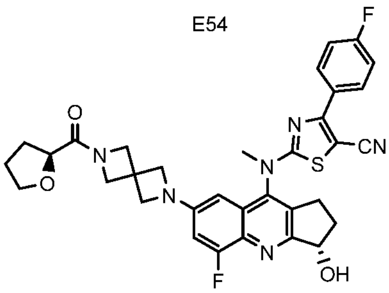<br>E54-a or E54-b<br>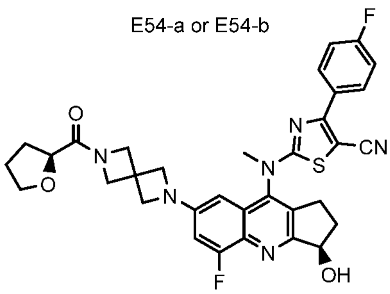<br>E54-a or E54-b | E54:<br>$^1H$ NMR (400 MHz, DMSO-d6) δ 8.05 (s, 2H), 7.41 (s, 2H), 6.98 (dd, *J* = 12.4, 2.4 Hz, 1H), 6.21 (dd, *J* = 4.5, 2.3 Hz, 1H), 5.71 (dd, *J* = 5.5, 1.3 Hz, 1H), 5.15 - 5.02 (m, 1H), 4.45 - 4.36 (m, 2H), 4.30 (dd, *J* = 7.8, 5.9 Hz, 1H), 4.14 - 4.02 (m, 6H), 3.78 - 3.69 (m, 2H), 3.59 (d, *J* = 8.5 Hz, 3H), 3.05 - 2.67 (m, 2H), 2.44 - 2.38 (m, 1H), 2.04 - 1.97 (m, 1H), 1.96 - 1.88 (m, 2H), 1.84 - 1.76 (m, 2H).<br>MS (ESI) *m/z* $(M+H)^+$ = 629.1.<br>HPLC retention time: 6.64 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min.<br>E54-a:<br>$^1H$ NMR (400 MHz, DMSO-d6) δ 8.05 (s, 2H), 7.41 (s, 2H), 6.98 (dd, *J* = 12.4, 2.4 Hz, 1H), 6.21 (dd, *J* = 4.5, 2.3 Hz, 1H), 5.71 (dd, *J* = 5.5, 1.3 Hz, 1H), 5.15 - 5.02 (m, 1H), 4.45 - 4.36 (m, 2H), 4.30 (dd, *J* = 7.8, 5.9 Hz, 1H), 4.14 - 4.02 (m, 6H), 3.78 - 3.69 (m, 2H), 3.59 (d, *J* = 8.5 Hz, 3H), 3.06 - 2.67 (m, 2H), 2.44 - 2.38 (m, 1H), 2.04 - 1.97 (m, 1H), 1.96 - 1.88 (m, 2H), 1.84 - 1.76 (m, 2H).<br>MS (ESI) *m/z* $(M+H)^+$ = 629.2.<br>HPLC retention time: 6.69 min. Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min.<br>SFC retention time: 3.398 min.<br>Separation conditions: chromatographic column: Chiralpak AD-3 150 × 4.6 mm I.D., 3 μm; column temperature: 35°C; mobile phase: $CO_2$-ethanol (0.05% DEA); ethanol (0.05% DEA): 5% to 40%; flow rate: 2.5 mL/min.<br>E54-b:<br>$^1H$ NMR (400 MHz, DMSO-d6) δ 8.05 (s, 2H), 7.40 (s, 2H), 6.98 (dd, *J* = 12.4, 2.3 Hz, 1H), 6.21 (dd, *J* = 5.1, 2.2 Hz, 1H), 5.71 (dd, *J* = 5.7, 1.3 Hz, 1H), 5.13 - 5.02 (m, 1H), 4.40 (s, 2H), 4.30 (dd, *J* = 7.9, 5.9 Hz, 1H), 4.08 (dd, *J* = 15.0, 10.1 Hz, 6H), 3.77 - 3.69 (m, 2H), 3.59 (d, *J* = 8.4 Hz, 3H), 3.03 - 2.69 (m, 2H), 2.44 - 2.38 (m, 1H), 2.04 - 1.98 (m, 1H), 1.92 (dt, *J* = 12.0, 6.3 Hz, 2H), 1.84 - 1.77 (m, 2H).<br>MS (ESI) *m/z* $(M+H)^+$ = 629.2. |

(continued)

| Example | Structural formula | Analytical data |
|---|---|---|
| | | HPLC retention time: 6.68 min. Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. SFC retention time: 4.636 min. Separation conditions: chromatographic column: Chiralpak AD-3 150 × 4.6 mm I.D., 3 μm; column temperature: 35°C; mobile phase: $CO_2$-ethanol (0.05% DEA); ethanol (0.05% DEA): 5% to 40%; flow rate: 2.5 mL/min. |
| E55 | 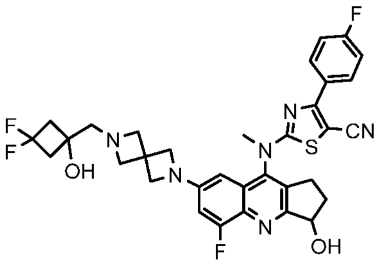 | $^1$H NMR (400 MHz, DMSO-d6) δ 8.21 - 7.89 (m, 2H), 7.40 (t, *J* = 7.2 Hz, 2H), 7.06 - 6.84 (m, 1H), 6.19 (d, *J* = 2.0 Hz, 1H), 5.69 (d, *J* = 5.5 Hz, 1H), 5.25 (s, 1H), 5.15 - 4.90 (m, 1H), 4.14 - 3.88 (m, 4H), 3.59 (d, *J* = 8.2 Hz, 4H), 3.36 (s, 3H), 3.09 - 2.81 (m, 2H), 2.65 - 2.53 (m, 2H), 2.52 - 2.27 (m, 5H), 2.13 - 1.62 (m, 1H). MS (ESI): *m/z* $[M+H]^+$ = 651.3. HPLC retention time: 8.29 min. Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| E56 | 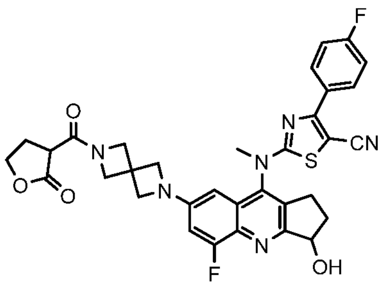 | $^1$H NMR (400 MHz, Chloroform-*d*) δ 8.12 (s, 2H), 7.16 (t, *J* = 8.6 Hz, 2H), 6.78 - 6.72 (m, 1H), 6.22 - 6.17 (m, 1H), 5.43 (s, 1H), 4.87 (d, *J* = 9.3 Hz, 1H), 4.52 (td, *J* = 8.5, 5.8 Hz, 1H), 4.44 - 4.19 (m, 5H), 4.19 - 4.14 (m, 3H), 3.63 (s, 3H), 3.43 (dd, *J* = 9.2, 6.8 Hz, 1H), 3.08 (s, 1H), 2.94 - 2.82 (m, 2H), 2.68 - 2.59 (m, 1H), 2.44 - 2.22 (m, 2H), 2.19 (dd, *J*= 13.9, 6.7 Hz, 1H). MS (ESI) *m/z* $(M+H)^+$ = 642.8. HPLC retention time: 5.71 min. Separation conditions: chromatographic column: waters XSelect CSH C18 4.6 * 100 mm, 3.5 μm; column temperature: 40°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.2 mL/min. |

(continued)

| Example | Structural formula | Analytical data |
|---|---|---|
| E57 | 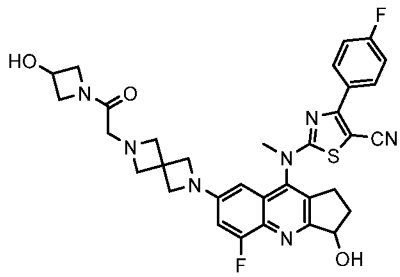 | $^1$H NMR (400 MHz, DMSO-*d6*) δ 8.05 (s, 2H), 7.42 (d, *J* = 8.6 Hz, 2H), 7.03 - 6.94 (m, 1H), 6.19 (dd, *J* = 5.0, 2.2 Hz, 1H), 5.71 (dd, *J* = 11.2, 5.7 Hz, 2H), 5.11 - 5.04 (m, 1H), 4.46 - 4.41 (d, *J* = 8.2 Hz, 1H), 4.25 (t, *J* = 8.0 Hz, 1H), 4.07 - 3.97 (m, 5H), 3.82 (dd, *J* = 9.1, 4.4 Hz, 1H), 3.60 - 3.54 (m, 5H), 3.16 (t, *J* = 8.6 Hz, 2H), 2.93 - 2.84 (m, 1H), 2.45 - 2.37 (m, 2H), 1.95 - 1.88 (m, 1H), 1.59 - 1.53 (m, 1H), 1.31 (q, *J* = 7.4 Hz, 1H), 0.94 (t, *J* = 7.3 Hz, 1H).<br>MS (ESI) *m/z* $(M+H)^+$ = 644.4.<br>HPLC retention time: 6.04 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E58 | 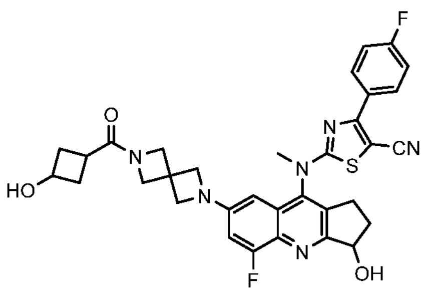 | $^1$H NMR (400 MHz, DMSO-*d6*) δ 8.05 (s, 2H), 7.40 (s, 2H), 7.02 - 6.95 (m, 1H), 6.23 - 6.18 (m, 1H), 5.71 (d, *J* = 5.4 Hz, 1H), 5.11 - 5.04 (m, 2H), 4.22 (s, 2H), 4.10 - 4.00 (m, 6H), 3.92 (q, *J* = 7.5 Hz, 1H), 3.59 (d, *J*= 8.4 Hz, 3H), 2.43 - 2.38 (m, 2H), 2.28 - 2.12 (m, 3H), 1.96 - 1.83 (m, 4H).<br>MS (ESI) *m/z* $(M+H)^+$ = 629.4.<br>HPLC retention time: 6.37 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E59 | 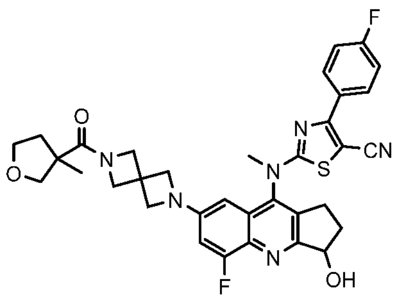 | $^1$H NMR (400 MHz, DMSO-*d6*) δ 8.05 (s, 2H), 7.41 (s, 2H), 6.99 (dd, *J* = 12.5, 2.4 Hz, 1H), 6.21 (dd, *J* = 4.8, 2.2 Hz, 1H), 5.71 (dd, *J* = 5.6, 1.4 Hz, 1H), 5.12 - 5.04 (m, 1H), 4.44 (s, 2H), 4.14 - 4.02 (m, 6H), 3.88 (d, *J* = 8.5 Hz, 1H), 3.76 - 3.70 (m, 2H), 3.59 (d, *J* = 8.4 Hz, 3H), 3.40 (d, *J* = 8.7 Hz, 1H), 3.07 - 2.88 (d, *J* = 7.9 Hz, 2H), 2.45 - 2.37 (m, 1H), 2.22 (dt, *J* = 12.7, 8.3 Hz, 1H), 1.92 (dq,J= 13.6, 6.9 Hz, 1H), 1.66 (ddd, *J* = 12.1, 7.0, 5.1 Hz, 1H), 1.20 (s, 3H).<br>MS (ESI) *m/z* $(M+H)^+$ = 643.4.<br>HPLC retention time: 6.98 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |

(continued)

| Example | Structural formula | Analytical data |
|---|---|---|
| E60 | 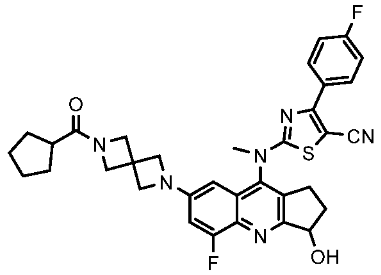 | $^1$H NMR (400 MHz, DMSO-*d6*) δ 8.05 (s, 2H), 7.40 (s, 2H), 6.99 (dd, *J* = 12.5, 2.4 Hz, 1H), 6.21 (dd, *J* = 5.1, 2.2 Hz, 1H), 5.71 (d, *J* = 5.5 Hz, 1H), 5.14 - 5.00 (m, 1H), 4.32 - 3.98 (m, 8H), 3.59 (d, *J*= 8.3 Hz, 3H), 3.10 - 2.69 (m, 2H), 2.57 (t, *J* = 7.2 Hz, 1H), 2.45 - 2.35 (m, 1H), 1.92 (dd, *J* = 13.4, 6.7 Hz, 1H), 1.71 (d, *J*= 9.5 Hz, 2H), 1.62 - 1.46 (m, 6H).<br>MS (ESI) *m/z* (M+H)$^+$ = 627.2.<br>HPLC retention time: 7.59 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E61 | 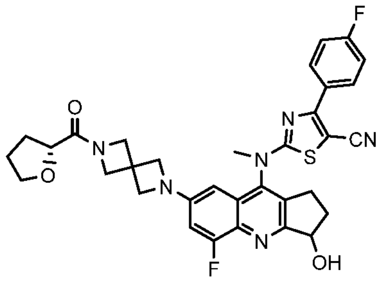 | $^1$H NMR (400 MHz, Chloroform-*d*) δ 8.11 (s, 2H), 7.16 (t, J= 8.6 Hz, 2H), 6.77 (d, *J* = 11.2 Hz, 1H), 6.22 - 6.10 (m, 1H), 5.49 (s, 1H), 4.60 - 4.47 (m, 2H), 4.43 - 4.36 (m, 1H), 4.26 - 4.10 (m, 6H), 3.89 - 3.82 (m, 2H), 3.63 (s, 3H), 3.07 (s, 1H), 2.92 - 2.84 (m, 1H), 2.67 (q, *J* = 4.5 Hz, 1H), 2.20 - 2.11 (m, 3H), 1.94 - 1.89 (m, 2H).<br>MS (ESI) *m/z* (M+H)$^+$ = 629.2.<br>HPLC retention time: 6.79 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |
| E62 | 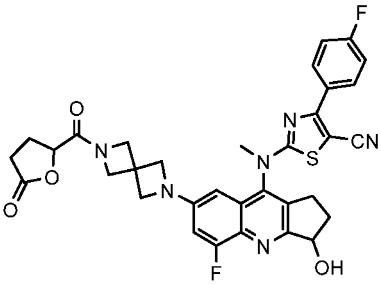 | $^1$H NMR (400 MHz, Chloroform-*d*) δ 8.06 (s, 2H), 7.15 (t, *J* = 8.6 Hz, 2H), 6.84 (d, *J* = 11.6 Hz, 1H), 6.20 (s, 1H), 5.79 - 5.52 (m, 1H), 4.98 - 4.84 (m, 1H), 4.60 (q, *J* = 10.4 Hz, 2H), 4.28 (q, *J* = 11.3 Hz, 2H), 4.20 (s, 4H), 3.63 (s, 3H), 3.13 - 2.99 (m, 1H), 2.95 - 2.83 (m, 1H), 2.73 (dd, *J* = 16.5, 8.3 Hz, 1H), 2.60 - 2.47 (m, 4H), 2.29 - 2.21 (m, 1H).<br>MS (ESI) *m/z* (M+H)$^+$ = 643.4.<br>HPLC retention time: 6.47 min.<br>Separation conditions: chromatographic column: waters XSelect CSH C18 4.6 * 100 mm, 3.5 μm; column temperature: 50°C; mobile phase: water (0.01% trifluoroacetic acid)-acetonitrile (0.01% trifluoroacetic acid); acetonitrile: 5% to 95% 7 min, 95% 5 min, 95% to 5% 3 min; flow rate: 1.0 mL/min. |

(continued)

| Example | Structural formula | Analytical data |
|---|---|---|
| E63 | 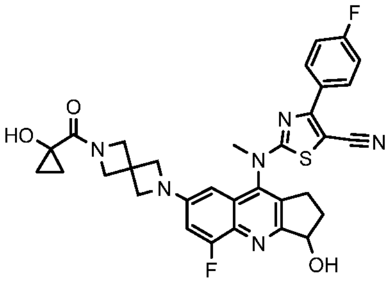 | $^1$H NMR (400 MHz, Chloroform-*d*) δ 8.13 - 8.03 (m, 2H), 7.16 (t, *J* = 8.6 Hz, 2H), 6.80 (d, *J* = 11.0 Hz, 1H), 6.19 (s, 1H), 5.60 (s, 1H), 4.67 (s, 2H), 4.40 - 4.21 (m, 2H), 4.17 (s, 4H), 3.63 (s, 3H), 3.06 (s, 1H), 2.88 (dt, *J* = 16.9, 8.1 Hz, 1H), 2.70 (dt, *J* = 8.5, 4.2 Hz, 1H), 2.24 (dt, *J* = 14.0, 7.6 Hz, 1H), 1.36 - 1.31 (m, 2H), 1.04 - 0.97 (m, 2H).<br>MS (ESI) *m/z* $(M+H)^+$ = 614.8.<br>HPLC retention time: 6.96 min.<br>Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic acid); acetonitrile: 5% to 95% 8 min, 95% 7 min; flow rate: 1.0 mL/min. |
| E64 | 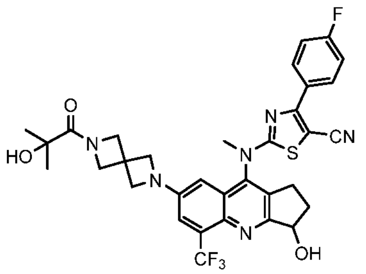 | $^1$H NMR (400 MHz, DMSO-*d6*) δ 8.05 (s, 2H), 7.39 (d, *J* = 2.3 Hz, 3H), 6.64 (s, 1H), 5.64 (s, 1H), 5.24 - 4.86 (m, 2H), 4.54 (s, 2H), 4.14 (d, *J* = 8.5 Hz, 4H), 4.03 (s, 2H), 3.60 (d, *J* = 9.4 Hz, 3H), 3.12 - 2.65 (m, 2H), 2.46 - 2.37 (m, 1H), 2.02 - 1.90 (m, 1H), 1.23 (s, 6H).<br>MS (ESI) *m/z* $(M+H)^+$ = 667.2.<br>HPLC retention time: 9.934 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |
| E65 | 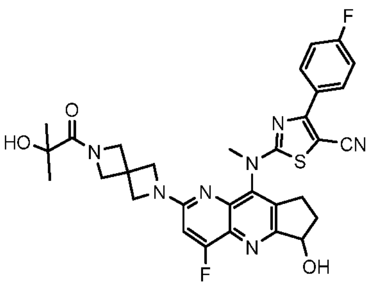 | $^1$H NMR (400 MHz, $CDCl_3$) δ 8.13 - 8.04 (m, 2H), 8.01 (s, 1H), 7.13 (t, *J* = 8.6 Hz, 2H), 6.45 (d, *J* = 10.8 Hz, 1H), 5.35 (m, 2H), 4.55 (s, 2H), 4.24 (s, 6H), 3.64 (s, 3H), 3.18 (m, 2H), 2.96 - 2.87 (m, 1H), 2.65 (m, *J* = 7.4 Hz, 1H), 1.40 (s, 6H).<br>MS (ESI) *m/z* $(M+H)^+$ = 618.3.<br>HPLC retention time: 8.84 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min. |

(continued)

| Example | Structural formula | Analytical data |
|---|---|---|
| E66<br>E66-a<br>E66-b | 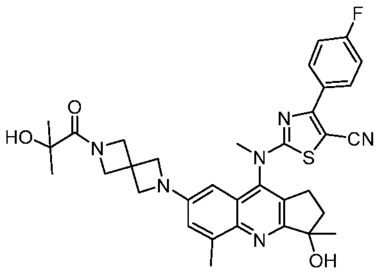<br>E66<br>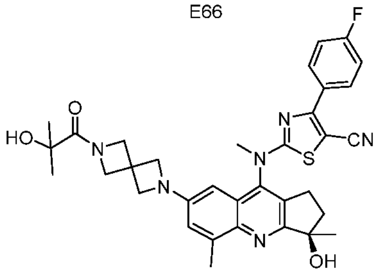<br>E66a or E66b<br>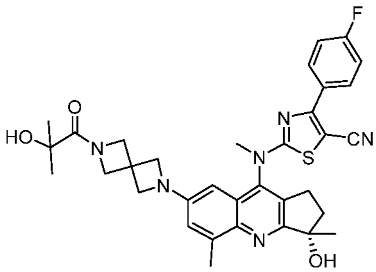<br>E66a or E66b | E66:<br>$^1$H NMR (400 MHz, DMSO-*d6*) δ 8.07 (brs, 2H), 7.41 (t, *J* = 8.6 Hz, 2H), 6.97 (s, 1H), 6.25 (s, 1H), 5.20 (d, *J* = 9.4 Hz, 1H), 5.07 (s, 1H), 4.54 (brs, 2H), 4.09 - 4.03 (m, 6H), 3.61 (d, *J* = 8.3 Hz, 3H), 3.10 - 2.73 (m, 2H), 2.72 (s, 3H), 2.22 - 2.07 (m, 2H), 1.56 (d, *J* = 2.8 Hz, 3H), 1.23 (s, 6H).<br>MS (ESI) *m*/*z* (M+H)$^+$ = 627.5.<br>HPLC retention time: 10.728 min.<br>Separation conditions: chromatographic column: Sunfire C18 150 * 4.6 mm, 5 μm; column temperature: 40°C; mobile phase: water (0.03% trifluoroacetic acid)-acetonitrile (0.03% trifluoroacetic acid); acetonitrile: 10% 1.8 min, 10% to 95% 10.2 min, 95% 3 min; flow rate: 1.0 mL/min.<br>E66-a:<br>$^1$H NMR (400 MHz, DMSO-d6) δ 8.08 (brs, 2H), 7.41 (t, *J* = 8.6 Hz, 2H), 6.96 (s, 1H), 6.25 (s, 1H), 5.19 (d, *J* = 9.6 Hz, 1H), 5.06 (s, 1H), 4.54 (brs, 2H), 4.14 - 3.96 (m, 6H), 3.60 (d, *J* = 8.3 Hz, 3H), 3.10 - 2.75 (m, 2H), 2.72 (s, 3H), 2.25 - 2.01 (m, 2H), 1.56 (d, *J* = 2.8 Hz, 3H), 1.23 (s, 6H).<br>MS (ESI) *m*/*z* (M+H)$^+$ = 627.0.<br>HPLC retention time: 7.426 min. Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic |
| | | acid); acetonitrile: 5% to 95% 8 min, 95% 7 min; flow rate: 1.0 mL/min. SFC retention time: 2.221 min. Separation conditions: chromatographic column: Chiralcel OX-3 100 × 4.6 mm I.D., 3 μm; column temperature: 35°C; mobile phase: $CO_2$-ethanol (0.05% DEA); ethanol (0.05% DEA): 40%; flow rate: 2.5 mL/min.<br>E66-b:<br>$^1$H NMR (400 MHz, DMSO-d6) δ 8.08 (brs, 2H), 7.41 (t, *J* = 8.6 Hz, 2H), 6.96 (s, 1H), 6.25 (s, 1H), 5.19 (d, *J* = 9.6 Hz, 1H), 5.06 (s, 1H), 4.54 (brs, 2H), 4.15 - 3.92 (m, 6H), 3.60 (d, *J* = 8.3 Hz, 3H), 3.11 - 2.76 (m, 2H), 2.72 (s, 3H), 2.25 - 2.05 (m, 2H), 1.56 (d, *J* = 2.8 Hz, 3H), 1.23 (s, 6H).<br>MS (ESI) *m*/*z* (M+H)$^+$ = 627.0.<br>HPLC retention time: 7.393 min. Separation conditions: chromatographic column: Agilent ZORBAX Extend-C18 4.6 * 150 mm, 3.5 μm; column temperature: 30°C; mobile phase: water (0.1% trifluoroacetic acid)-acetonitrile (0.1% trifluoroacetic acid); acetonitrile: 5% to 95% 8 min, 95% 7 min; flow rate: 1.0 mL/min.<br>SFC retention time: 3.003 min. |

(continued)

| Example | Structural formula | Analytical data |
|---|---|---|
| | | Separation conditions: chromatographic column: Chiralcel OX-3 100 × 4.6 mm I.D., 3 μm; column temperature: 35°C; mobile phase: $CO_2$-ethanol (0.05% DEA); ethanol (0.05% DEA): 40%; flow rate: 2.5 mL/min. |

**Synthesis of example F1**

Similar to the synthesis of example **A1**, the following compound **F1-1** was synthesized. MS (ESI) *m/z* $(M+H)^+$ = 575.0.

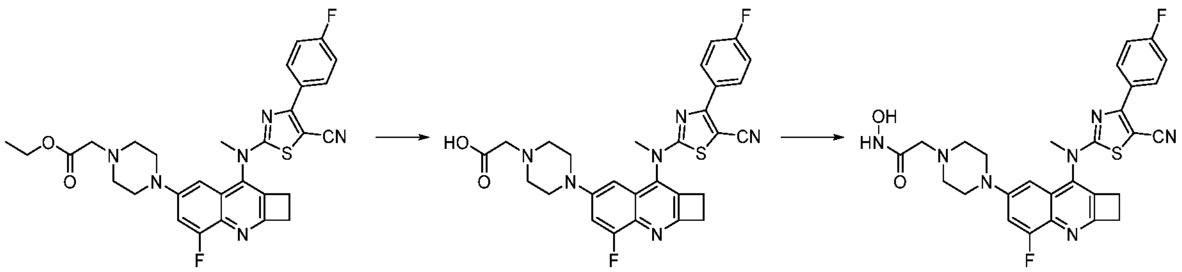

F1-1 F1-2 F1

**Step 1:** Preparation of compound **F1-2**

Compound **F1-1** (57.0 mg, 0.1 mmol) and lithium hydroxide (50.0 mg) were dissolved in a mixed solution of tetrahydrofuran (3.0 mL) and water (1.0 mL), and the reaction mixture was stirred at 50°C for 1.0 hour. After the reaction was completed, the crude product was purified by reversed phase column chromatography [water (10.0 mM/L ammonium bicarbonate solution)-acetonitrile: 20% to 60%] to obtain compound F1-2. MS (ESI) *m/z* $(M+H)^+$ = 547.0.

**Step 2:** Preparation of compound **F1**

To a solution of compound **F1-2** (30.0 mg, 0.055 mmol) and 1*H*-benzotriazol-1-yloxytripyrrolidinyl hexafluorophosphate (43.0 mg, 0.082 mmol) in A A-dimethylformamide (1.0 mL) was added *N,N*-diisopropylethylamine (14.0 mg, 0.11 mmol), and the reaction mixture was stirred at room temperature (25°C) for 15 minutes. The system was then added with hydroxylamine hydrochloride (7.7 mg, 0.11 mmol) and stirred for another 1.0 hour. After the reaction was completed, the crude product was purified by preparative high performance liquid chromatography (separation conditions: chromatographic column: Welch Xtimate® C18 21.2 × 250 mm; column temperature: 25°C; mobile phase: [water (10.0 mM/L ammonium bicarbonate solution)-acetonitrile]; percentage of acetonitrile in the mobile phase: 50 to 80% 9 min; flow rate: 30.0 mL/min) to obtain compound **F1**.

1H NMR (400 MHz, DMSO-*d6*) δ 10.38 (s, 1H), 8.72 (s, 1H), 8.04 - 7.90 (m, 2H), 7.44 (dd, *J* = 14.2, 2.6 Hz, 1H), 7.38 - 7.29 (m, 2H), 6.73 (d, *J* = 2.5 Hz, 1H), 3.56 (s, 3H), 3.48 (t, *J* = 5.1 Hz, 2H), 3.21 (d, *J* = 5.3 Hz, 4H), 3.19 - 3.12 (m, 3H), 2.86 (s, 2H), 2.51 (d, *J* = 5.0 Hz, 4H). MS (ESI) *m/z* $(M+H)^+$ = 562.0. HPLC purity: 98.8%; retention time: 5.56 min.

Separation conditions: chromatographic column: Waters XBridge 4.6 * 100 mm, 3.5 μm; column temperature: 40°C; mobile phase: water (10 mM $NH_4HCO_3$)-acetonitrile; acetonitrile: 5% to 95% 7 min; 95% 8 min; flow rate: 1.2 mL/min.

**Synthesis of example F2**

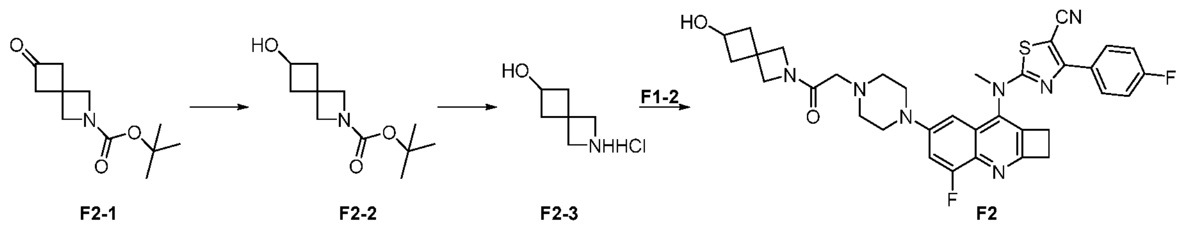


F2-1 F2-2 F2-3 F2

**Step 1:** Preparation of compound **F2-2**

To a solution of compound **F2-1** (1 g, 4.74 mmol) in methanol (10 mL) was added sodium borohydride (540 mg, 14.22 mmol) in batches, and the reaction mixture was stirred at room temperature (25°C) for 2 hours. After the reaction was completed, the reaction mixture was added with water to quench, and extracted with ethyl acetate (80 mL × 3). The organic phases were combined, washed with saturated sodium chloride (200 mL × 2), dried over anhydrous sodium sulfate, and filtered. The filtrate was concentrated under reduced pressure to obtain compound **F2-2,** which was directly used in the next reaction step without further purification. MS (ESI) *m/z* $(M+H)^+$-56 = 158.0

**Step 2:** Preparation of compound **F2-3**

Compound **F2-2** (1 g, 4.74 mmol) was dissolved in dichloromethane (15 mL), then a solution of hydrochloride acid in dioxane (2 mL) was added thereto, and the reaction mixture was stirred at room temperature (25°C) for 6 hours. The reaction mixture was concentrated under reduced pressure to obtain compound **F2-3,** which was directly used in the next reaction step without further purification. MS (ESI) *m/z* $(M+H)^+$ = 114.0.

**Step 3:** Preparation of compound **F2**

A solution of compound **F2-3** (50 mg, 0.0915 mmol), compound F1-2 (51 mg, 0.457 mmol), 1*H*-benzotriazol-1-yloxytripyrrolidinyl hexafluorophosphate (52 mg, 0.137 mmol), and *N,N*-diisopropylethylamine (35.3 mg, 0.274 mmol) in *N,N*-dimethylformamide (1 mL) was stirred at room temperature (25°C) for 2 hours. The reaction crude product was purified by preparative high performance liquid chromatography (separation conditions: chromatographic column: Welch Xtimate C18 250 × 21.2 mm; column temperature: 25°C; mobile phase: water (10 mM/L $NH_4HCO_3$)-acetonitrile; acetonitrile: 20% to 40% 12 min; flow rate: 30 mL/min) to obtain compound **F2.**

$^1$H NMR (400 MHz, DMSO-*d6*) δ 8.04 (dd, *J* = 8.6, 5.5 Hz, 2H), 7.51 (d, *J* = 14.2 Hz, 1H), 7.41 (t, *J* = 8.9 Hz, 2H), 6.8 (s, 1H), 5.02 (d, *J* = 6.2 Hz, 1H), 4.11 (d, *J* = 19.8 Hz, 2H), 3.94 (dd, *J* = 15.0, 7.9 Hz, 1H), 3.78 (d, *J* = 19.8 Hz, 2H), 3.63 (s, 3H), 3.59 - 3.50 (m, 2H), 3.29 - 3.25 (m, 4H), 3.23 (s, 2H), 2.97 (s, 2H), 2.54 (s, 2H), 2.36 (s, 4H), 1.93 (t, *J* = 9.0 Hz, 2H). MS (ESI) *m/z* $(M+H)^+$ = 642.0. HPLC 100%. Retention time: 5.672 min.

Separation conditions: chromatographic column: Waters XBridge 4.6 * 100 mm, 3.5 μm; column temperature: 40°C; mobile phase: A: water (10 mM ammonium bicarbonate), B: acetonitrile; acetonitrile: 5% to 95% 7 min; flow rate: 1.2 mL/min.

Similar to the synthesis of example **F2,** the following examples **F3** to **F5** were synthesized as shown in Table **6** below.

**Table 6: Structural formulas and analytical data of examples F3 to F5**

| Example | Structural formula | Analytical data |
|---|---|---|
| F3 | 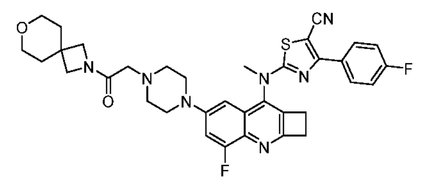 | $^1H$ NMR (400 MHz, DMSO-*d6*) δ 8.04 (dd, *J* = 8.9, 5.6 Hz, 2H), 7.51 (dd, *J* = 14.3, 2.5 Hz, 1H), 7.46 - 7.35 (m, 2H), 6.80 (d, *J* = 2.5 Hz, 1H), 3.90 (s, 2H), 3.63 (s, 3H), 3.58 (s, 2H), 3.55 (t, *J* = 5.3 Hz, 2H), 3.48 (q, *J* = 4.5 Hz, 4H), 3.27 (t, *J* = 4.9 Hz, 4H), 3.23 (d, *J* = 5.5 Hz, 2H), 3.02 (s, 2H), 2.56 (d, *J* = 5.0 Hz, 4H), 1.64 (t, *J* = 5.2 Hz, 4H). MS (ESI) *m*/z $(M+H)^+$ = 656.0.<br>HPLC retention time: 6.168 min.<br>Separation conditions: chromatographic column: Waters XBridge 4.6 * 100 mm, 3.5 μm; column temperature: 40°C; mobile phase: A: water (10 mM ammonium bicarbonate), B: acetonitrile; acetonitrile: 5% to 95% 7 min; flow rate: 1.2 mL/min. |
| F4 | 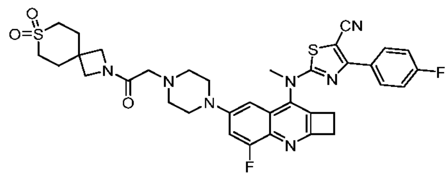 | $^1H$ NMR (400 MHz, DMSO-*d6*) δ 7.98 (dd, *J* = 8.6, 5.4 Hz, 2H), 7.47 (d, *J* = 13.9 Hz, 1H), 7.34 (t, *J* = 8.7 Hz, 2H), 6.76 (s, 1H), 3.87 (s, 2H), 3.61 (s, 2H), 3.57 (s, 3H), 3.48 (t, *J* = 5.2 Hz, 2H), 3.24 - 3.10 (m, 6H), 3.00 (dq, *J* = 14.6, 8.7, 7.8 Hz, 6H), 2.56 (d, *J* = 36.3 Hz, 2H), 2.33 (d, *J* = 37.7 Hz, 2H), 2.08 (t, *J* = 5.8 Hz, 4H). MS (ESI) *m*/*z* $(M+H)^+$ = 704.2.<br>HPLC 100%. Retention time: 5.826 min.<br>Separation conditions: chromatographic column: Waters XBridge 4.6 * 100 mm, 3.5 μm; column temperature: 40°C; mobile phase: A: water (10 mM ammonium bicarbonate), B: acetonitrile; acetonitrile: 5% to 95% 7 min; flow rate: 1.2 mL/min. |
| F5 | 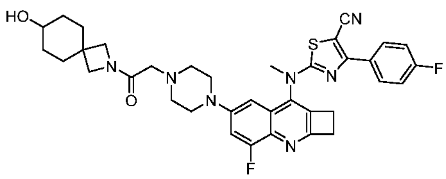 | $^1H$ NMR (400 MHz, DMSO-*d6*) δ 7.95 (s, 2H), 7.52 - 7.40 (m, 1H), 7.35 (d, *J* = 8.8 Hz, 2H), 6.73 (s, 1H), 4.41 (d, *J* = 4.0 Hz, 1H), 3.79 - 3.69 (m, 2H), 3.56 (s, 3H), 3.51 - 3.45 (m, 2H), 3.41 (d, *J* = 13.6 Hz, 2H), 3.34 (t, *J* = 11.7 Hz, 1H), 3.18 (dd, *J* = 19.3, 6.6 Hz, 6H), 2.94 (d, *J* = 5.2 Hz, 2H), 2.49 (s, 4H), 1.69 (d, *J* = 16.9 Hz, 2H), 1.54 (s, 2H), 1.34 (t, *J* = 12.0 Hz, 2H), 1.16 (s, 2H). MS (ESI) *m*/*z* $(M+H)^+$ = 670.2.<br>HPLC 100%. Retention time: 6.173 min.<br>Separation conditions: chromatographic column: Waters XBridge 4.6 * 100 mm, 3.5 μm; column temperature: 40°C; mobile phase: A: water (10 mM ammonium bicarbonate), B: acetonitrile; acetonitrile: 5% to 95% 7 min; flow rate: 1.2 mL/min. |

**Example 4: Bioactivity assay**

1. Compound preparation and processing

Compound stock solution

All compounds were prepared as a 10 mM DMSO stock solution. The positive control compound GLPG1690 was prepared as a 10 mM DMSO stock solution.

Compound storage

The stock solution dissolved in DMSO was aliquoted and stored at -20°C.

2. Experimental preparation

Preparation of 1x assay buffer:

Solutions at pH 8.5 and pH 7.5 were prepared respectively with the following formulations: 50 mM Tris, 10 mM $CaCl_2$, 5 mM $MgCl_2$, 0.02% Brij-35 (w/v).

Preparation of 10x compound diluent:

All compounds, including the positive control compound, were diluted 5-fold from 10 mM to 2 mM with DMSO in the first step. The mixture was then diluted to 100 μM with 1x assay buffer.

Preparation of 100 U/mL choline oxidase stock solution:

1 volume of 5x reaction buffer and 4 volumes of double distilled water were mixed thoroughly to prepare 1x reaction buffer. Choline oxidase was dissolved in 1x reaction buffer. The mixture was aliquoted into small amounts and frozen at -20°C.

Preparation of 200 U/mL HRP (horseradish peroxidase) stock solution:

1 volume of 5x reaction buffer and 4 volumes of double distilled water were mixed thoroughly to prepare 1x reaction buffer. HRP was dissolved in 1x reaction buffer. The mixture was aliquoted into small amounts and frozen at -20°C.

Preparation of 10 mM Amplex® UltraRed reagent (detection reagent) stock solution:

Amplex UltraRed reagent and DMSO were equilibrated to room temperature. 1 tube of Amplex UltraRed reagent (1 mg/tube) was dissolved in 340 μL of DMSO.

3. Separate detection of compounds with 1x assay buffer at two pH values:

a) 5 μL of 10x compound diluent was added to a 384-well plate (Greiner 781209) using a motorized pipette (see the "experimental preparation" step). 5 μL of 1x assay buffer containing 5% DMSO was added to both negative and positive control wells. The plate was centrifuged at 300 rpm for 1 minute at room temperature.
b) 2x ATX (4 ng/μL) was prepared with 1x assay buffer, and 25 μL of 2x ATX was added to the plate using a motorized pipette. 25 μL of 1x assay buffer was added to the negative control wells and 25 μL of 2x ATX was added to the positive control wells. The plate was centrifuged at 300 rpm for 1 minute at room temperature.
The plate was incubated at room temperature for 10 minutes.
c) 2.5x working solution containing Amplex UltraRed reagent (125 μM), HRP (2.5 U/mL), choline oxidase (1.25 U/mL), and 16:0 Lyso PC (75 μM) was prepared with 1x assay buffer. 20 μL of 2.5x working solution was added to all reaction wells using a motorized pipette. The plate was centrifuged at 300 rpm for 1 minute at room temperature.
d) The plate was detected by SpectraMax with the following procedures: fluorescence mode; excitation at 550 nm; emission at 590 nm; kinetics; read once every 2 minutes; read continuously for 60 minutes.

4. Data analysis:

The calculation formula of % inhibition (*i.e.*, % inhibition rate) was as follows:

$$\%\ \text{inhibition} = (\overline{\text{V0max}} - \text{V0cmp}d)/(\overline{V0max} - \overline{V0min}) \times 100$$

V0: X-axis is taken as 0 to 30 minutes, Y-axis is taken as fluorescence readings, and the slope corresponding

to the straight line fitted by the software GraphPad Prism 8 is the initial response rate.

$\overline{V0max}$: Average V0 of the positive control wells in the plate.

$\overline{V0min}$: Average V0 of the negative control wells in the plate.

5. Calculation of $IC_{50}$ and dose-response curve of compounds:

The $IC_{50}$ of the compounds was calculated from a nonlinear regression line (doseinitial response rate) fitted by % inhibition and log value of compound concentration using the software GraphPad Prism 8.

$$Y = Bottom + (Top - Bottom) / (1 + 10 ^\wedge ((LogIC_{50} - X) * Hill\ Slope))$$

X: Log value of inhibitor concentration

Y: % inhibition

The biological data results are shown in Table 7.

Table 7

| Compound No. | $IC_{50}$ (nM) pH 8.5 | $IC_{50}$ (nM) pH 7.5 |
|---|---|---|
| GLPG1690 | 305.27 | 1754.50 |
| A1 | 27.70 | 137.70 |
| A2 | 96.72 | 623.8 |
| A3 | 27.79 | 213.80 |
| A4 | 52.06 | 173.70 |
| A5 | 32.75 | 112.10 |
| A6 | 44.70 | 317.70 |
| A7 | 22.21 | 85.06 |
| A8 | 63.96 | 883.4 |
| A9 | 86.83 | 699.1 |
| A10 | 60.73 | 558.60 |
| A11 | 144.70 | 1176.00 |
| A12 | 22.34 | 219.00 |
| A14 | 227.70 | ND |
| A15 | 133.80 | 655.9 |
| A16 | 75.71 | 282.4 |
| A17 | 48.72 | ND |
| A18 | 61.13 | 227.70 |
| A19 | 52.72 | 413.30 |
| A20 | 84.03 | 645.00 |
| A21 | 23.74 | 125.2 |
| A22 | 258.50 | ND |
| A23 | 75.30 | ND |
| A24 | 181.30 | ND |
| A25 | 75.04 | 540.60 |
| A26 | 26.60 | ND |

(continued)

| Compound No. | $IC_{50}$ (nM) pH 8.5 | $IC_{50}$ (nM) pH 7.5 |
|---|---|---|
| A27 | 111.20 | ND |
| A28 | 226.1 | ND |
| A29 | 110.7 | ND |
| A30 | 22.11 | ND |
| A31 | 26.85 | ND |
| A32 | 94.29 | ND |
| A33 | 44.62 | ND |
| A34 | 446.3 | ND |
| A35 | 66.83 | ND |
| A36 | 304.6 | ND |
| B1 | 111.7 | ND |
| B2 | 58.8 | 141.30 |
| B3 | 41.24 | 170.8 |
| B4 | 66.52 | 611.2 |
| C1 | 36.26 | 63.55 |
| C2 | 23.75 | 41.62 |
| C3 | 22.68 | 31.96 |
| C4 | 27.37 | 38.62 |
| C5 | 24.53 | 94.06 |
| C6 | 196.20 | 1228 |
| C7 | 192.80 | 1142 |
| C8 | 27.53 | 67.32 |
| C9 | 22.96 | 78.55 |
| C10 | 18.58 | 33.59 |
| C11 | 61.93 | 131.80 |
| C12 | 52.61 | 82.15 |
| C14 | 83.12 | 333.90 |
| C15 | 60.58 | 231.50 |
| C16 | 25.59 | 77.53 |
| C17 | 25.85 | ND |
| C18 | 15.15 | ND |
| C20 | 24.25 | ND |
| C21 | 49.13 | ND |
| C22 | 73.05 | ND |
| D1 | 47.6 | ND |
| E1-1 | 21.26 | ND |
| E1-2 | 18.17 | ND |
| E2 | 3.46 | ND |

(continued)

| Compound No. | $IC_{50}$ (nM) pH 8.5 | $IC_{50}$ (nM) pH 7.5 |
|---|---|---|
| E3 | 30.69 | ND |
| E4 | 24.48 | ND |
| E5 | 21.77 | ND |
| E5-a | 22.68 | ND |
| E5-b | 23.68 | ND |
| E6-a | 18.34 | ND |
| E6-b | 13.98 | ND |
| E7 | 15.42 | ND |
| E7-a | 15.25 | ND |
| E7-b | 18.86 | ND |
| E8 | 15.36 | ND |
| E8-a | 22.5 | ND |
| E8-b | 19.42 | ND |
| E9 | 19.64 | ND |
| E9-a | 21.47 | ND |
| E9-b | 18.36 | ND |
| E10 | 16.2 | ND |
| E10-a | 17.92 | ND |
| E10-b | 14.37 | ND |
| E11 | 35.7 | ND |
| E12 | 97.6 | ND |
| E13 | 23.49 | ND |
| E13-a | 19.76 | ND |
| E13-b | 20.22 | ND |
| E14 | 17.32 | ND |
| E14-a | 16.61 | ND |
| E14-b | 14.68 | ND |
| E15 | 13.3 | ND |
| E15-a | 14.92 | ND |
| E15-b | 18.5 | ND |
| E16 | 16.76 | ND |
| E16-a | 20.06 | ND |
| E16-b | 23.03 | ND |
| E17 | 22.57 | ND |
| E17-a | 22.43 | ND |
| E17-b | 21.49 | ND |
| E18 | 21.35 | ND |
| E18-a | 23.42 | ND |

(continued)

| Compound No. | $IC_{50}$ (nM) pH 8.5 | $IC_{50}$ (nM) pH 7.5 |
|---|---|---|
| E18-b | 37.07 | ND |
| E19 | 20.35 | ND |
| E19-1a | 22.85 | ND |
| E19-1b | 27.16 | ND |
| E19-2a | 26.69 | ND |
| E19-2b | 333.7 | ND |
| E20 | 23.52 | ND |
| E20-a | 18.06 | ND |
| E20-b | 17.04 | ND |
| E20-c | 25.71 | ND |
| E20-d | 29.25 | ND |
| F1 | 44.88 | ND |
| F2 | 65.20 | ND |
| F3 | 60.63 | ND |
| F4 | 19.56 | ND |
| F5 | 19.91 | ND |

**Example 5: Bioactivity assay**

Compound preparation and processing

Compound stock solution

All compounds, including the positive control GLPG1690, were prepared as a 10 mM DMSO stock solution.

Compound storage

The stock solution dissolved in DMSO was aliquoted and stored at -20°C.

Experimental preparation

Aliquot and freezing of human plasma/murine plasma:

The collected plasma was centrifuged at 4000 rpm for 15 minutes at 4°C. The supernatant was aliquoted into a 96-well plate (25 μL per well), sealed, and frozen at -80°C for further use.

Preparation of 2.5 μM 50X LPA 17:0 (lysophosphatidic acid 17:0, Sigma 857127P) stock solution (internal standard):

LPA 17:0 powder was dissolved in a mixed solution of water: isopropanol: formic acid (1:1:1) to a final concentration of 2.5 μM. The mixture was aliquoted and frozen at -20°C.

Preparation of 0.1% FA stock solution:

1 volume of formic acid was added to 1000X methanol, mixed well, and stored at 4°C.

Experimental steps:

a) The 96-well plate containing frozen plasma was removed from the -80°C freezer and thawed on ice at room temperature.

b) 6 μL of 50X LPA 17:0 (internal standard) and 264 μL of 0.1% FA were added to a clean well of the 96-well plate; the final concentration of LPA 17:0 (internal standard) was 50 nM.
c) 6X compound was 1:3 serially diluted to a total of 8 concentrations, and 5 μL of the compound per well was added to the 96-well plate containing plasma. The maximum final concentration of the compound was 10 μM, and the final concentration of DMSO was 0.1%. The plate was centrifuged at 300 rpm for 30 seconds. The DMSO control sample reacted for 0 hours was used as the minimum value (Min), and the DMSO control sample reacted for 2 hours was used as the maximum value (Max).
d) The DMSO sample reacted for 0 hours was rapidly transferred to 0.1% FA solution containing the internal standard in step b) to terminate the reaction and precipitate plasma proteins.
e) The 96-well plate containing plasma and compounds was rapidly incubated in a 37°C incubator for 2 hours. After the incubation, 6 μL of 10X LPA 17:0 and 264 μL of 0.1% FA were added to each well to terminate the reaction and precipitate plasma proteins.
f) All the samples were mixed well with a pipette, and centrifuged at 4000 rpm for 10 minutes at 4°C. 150 μL of the supernatant was added to a 96-well U-bottom plate (Thermo 267245), sealed with silica gel, and then loaded. The contents of LPA 18:1 and LPA 18:2 in the samples were detected by LC-MS/MS system.

Data analysis:

LPA 18:1 (or 18:2) relative content = LPA 18:1 (or 18:2) peak area/LPA 17:0 peak area

Percentage reduction (%) in plasma LPA 18:1 (or 18:2) = (Max - X)/(Max - Min) * 100

Max: LPA 18:1 (or 18:2) relative content of the DMSO sample reacted for 2 hours
Min: LPA 18:1 (or 18:2) relative content of the DMSO sample reacted for 0 hours
X: LPA 18:1 (or 18:2) relative content of the sample after compound treatment for 2 hours

Dose-response curve of compounds and calculation of $IC_{50}$:

The $IC_{50}$ of the compounds was calculated by fitting a nonlinear regression through percentage reduction in plasma LPA 18:1 (or 18:2) and log value of compound concentration using the software GraphPad Prism 8.

$$Y = Bottom + (Top - Bottom) / (1 + 10 ^\wedge ((LogIC_{50} - X) * Hill\ Slope))$$

X: Log value of inhibitor concentration
Y: Percentage reduction in plasma LPA 18:1 (or 18:2)

The results are shown in Table 8.

Table 8

| Compound No. | Human plasma *ex vivo* assay $IC_{50}$ (18:1 LPA) nM | Human plasma *ex vivo* assay $IC_{50}$ (18:2 LPA) nM |
|---|---|---|
| GLPG1690 | 465.3 | 464.2 |
| A1 | 183.2 | 195.5 |
| A2 | 85.03 | 73.54 |
| A3 | 109.1 | 102.7 |
| A4 | 159.6 | 175.6 |
| A5 | 199.8 | 239.3 |

(continued)

| Compound No. | Human plasma *ex vivo* assay $IC_{50}$ (18:1 LPA) nM | Human plasma *ex vivo* assay $IC_{50}$ (18:2 LPA) nM |
|---|---|---|
| A6 | 182.5 | 184.3 |
| A7 | 74.2 | 72.74 |
| A8 | 248.3 | 271.3 |
| A9 | 350.6 | 322.7 |
| A10 | 192.1 | 157.2 |
| A11 | 299.1 | 359 |
| A12 | 64.52 | 91.61 |
| A13 | 193.7 | 206.3 |
| A14 | 304.4 | 337.7 |
| A16 | 286.3 | 340.6 |
| A17 | 164.3 | 172.4 |
| A18 | 161.8 | 192.3 |
| A19 | 118.4 | 132.3 |
| A20 | 170.2 | 203.4 |
| A21 | 70.18 | 55.88 |
| A22 | 848.5 | 460.4 |
| A24 | 466.2 | 485.7 |
| A25 | 250.6 | 268.7 |
| A36 | 340.6 | 179.5 |
| B1 | 175.9 | 169.3 |
| B2 | 176.7 | 201.1 |
| B3 | 170.3 | 164.8 |
| B4 | 218.9 | 252.6 |
| C1 | 521.1 | 438.2 |
| C2 | 402.1 | 365 |
| C3 | 140.8 | 134.9 |
| C4 | 113.7 | 89.22 |
| C5 | 137 | 147.5 |
| C6 | 232.1 | 264.1 |
| C7 | 354.1 | 400.8 |
| C8 | 168.5 | 190.7 |
| C9 | 215.5 | 233.6 |
| C10 | 147.2 | 134.5 |
| C12 | 292 | 346.6 |
| C14 | 309.9 | 378.6 |
| C15 | 285 | 326.5 |
| C16 | 305.2 | 294.3 |

(continued)

| Compound No. | Human plasma *ex vivo* assay $IC_{50}$ (18:1 LPA) nM | Human plasma *ex vivo* assay $IC_{50}$ (18:2 LPA) nM |
|---|---|---|
| C17 | 318.2 | 318.5 |
| C20 | 677.4 | 362.2 |
| C24 | 232 | 221.1 |
| C26 | 588 | 434.9 |
| C28 | 545 | 574.1 |
| C29 | 251.1 | 219.6 |
| E5-a | 294.4 | 305.5 |
| E6-b | 386.8 | 431.1 |
| E7 | 149.4 | 326.7 |
| E7-a | 369 | 351.3 |
| E8 | 175.2 | 160 |
| E8-a | 279.9 | 323.9 |
| E9 | 160.4 | 152.5 |
| E9-a | 463.5 | 391.8 |
| E9-b | 208.9 | 199.1 |
| E10 | 138.8 | 269.9 |
| E10-a | 383.6 | 430.3 |
| E10-b | 263.2 | 260.7 |
| E13-a | 384.8 | 384.8 |
| E14 | 281.1 | 651.4 |
| E15 | 265.6 | 271.1 |
| E15-a | 391.6 | 388.5 |
| E16 | 410.3 | 435.5 |
| E16-a | 381 | 383 |
| E18 | 250.2 | 259.8 |
| E19-1a | 206.6 | 361.5 |
| E19-1b | 217.5 | 479.3 |
| E19-2a | 206.9 | 202.8 |
| E19-2b | 113.3 | 310.3 |
| E20-1a | 154.7 | 168.5 |
| E20-1b | 307.6 | 279.1 |
| E20-2b | 112.2 | 289.7 |
| E25 | 379.3 | 457 |
| E25-a | 372.5 | 366.4 |
| E26 | 516 | 592 |
| E27 | 287 | 314.1 |
| E28 | 297.6 | 271.3 |

(continued)

| Compound No. | Human plasma *ex vivo* assay $IC_{50}$ (18:1 LPA) nM | Human plasma *ex vivo* assay $IC_{50}$ (18:2 LPA) nM |
|---|---|---|
| E29 | 526.4 | 521.8 |
| E30 | 165.2 | 149.7 |
| E31 | 222.3 | 164.1 |
| E32 | 202 | 184.9 |
| E32-a | 323.4 | 330.8 |
| E32-b | 373.2 | 489.3 |
| E33 | 323.3 | 154.5 |
| E35 | 301.4 | 392.9 |
| E35-a | 388.1 | 283.7 |
| E35-b | 457.1 | 406.7 |
| E36 | 370.8 | 308.2 |
| E39 | 317 | 397.5 |
| E40 | 303.5 | 336.5 |
| E41 | 305 | 295 |
| E42 | 411.8 | 468.9 |
| E43 | 654 | 737 |
| E44 | 293.4 | 295.8 |
| E45 | 583.5 | 355.9 |
| E47 | 437 | 409 |
| E48 | 740.1 | 671.2 |
| E49 | 494 | 576.1 |
| E50 | 582.7 | 545.7 |
| E51 | 347.5 | 850.4 |
| E52 | 669.9 | 422.9 |
| E54-a | 725.1 | 482.8 |
| E56 | 499 | 440 |
| E57 | 183.3 | 171.4 |
| E58 | 263.1 | 271.3 |
| E59 | 240 | 405 |
| E60 | 461.6 | 448.4 |
| E61 | 583.2 | 839.3 |
| E62 | 331.7 | 576.1 |
| E65 | 368.9 | 495.7 |

**Example 6: Pharmacokinetic test in mice**

Dosage preparation: The dosing solution was prepared on the day of dosing. 2 mg of compound was weighed and dissolved in 5% DMSO + 10% solutol + 85% saline to prepare a solution for intravenous administration at a concentration of 0.2 mg/mL. 2 mg of compound was weighed and dissolved in 25% PEG 200 + 75% (0.5% MC) to obtain

a solution for oral administration at a concentration of 1 mg/mL.

Six healthy male naive ICR mice, weighing 25 to 30 g, were divided into two groups (intravenous and oral groups) with three in each group, and administered in a single dose. After 3 days of acclimatization, the mice were fasted overnight (10 to 12 hours) the night before the experiment, but had free access to water during the experiment, and the diet was resumed 4 hours after administration. After intravenous and oral administration, the timer was started. Blood was collected via the orbital venous plexus at the scheduled time points (IV&PO: 0.083, 0.25, 0.5, 1, 2, 4, 6, 8, and 24 hours). 40 μL of whole blood was collected at each point and added to a 1.5 mL EP tube containing sodium heparin. The collected whole blood was vibrated twice on a vortex mixer, mixed well, placed on wet ice, and centrifuged at 8000 rpm for 5 minutes at 4°C within 1 hour. The supernatant plasma was stored in a -80°C freezer until processing for analysis.

Table 9: Pharmacokinetics in mice

| Compound No. | Route of administration | $C_0$ (IV) or $C_{max}$ (PO) (ng/mL) | CL (mL/min/kg, IV) | $AUC_i$ (ng * hr/mL) | $T_{1/2}$ (hr) | $T_{max}$ (hr, PO) | Bioavailability (%) |
|---|---|---|---|---|---|---|---|
| C1 | IV - 1 mpk | 3141 | 2.92 | 5701 | 2.15 | / | / |
| | PO - 5 mpk | 1903 | / | 7392 | 2.08 | 1.0 | 26 |
| C8 | IV - 1 mpk | 1799 | 8.0 | 2084 | 1.23 | / | / |
| | PO - 5 mpk | 2923 | / | 7936 | 2.4 | 0.25 | 76.2 |
| C16 | IV - 1 mpk | 1518 | 6.21 | 2684 | 1.59 | / | / |
| | PO - 5 mpk | 1833 | / | 9550 | 1.99 | 0.5 | 71.2 |
| C17 | PO - 5 mpk | 4285 | / | 16933 | 2.13 | 0.5 | / |
| C18 | IV - 1 mpk | 1936 | 4.59 | 3701 | 1.82 | / | / |
| | PO - 5 mpk | 3333 | / | 11038 | 2.79 | 0.58 | 59.7 |
| C26 | PO - 5 mpk | 8392 | / | 123974 | 5.13 | 4.08 | / |
| C27 | PO - 5 mpk | 2006 | / | 27648 | 3.98 | 6.67 | / |
| C28 | IV - 1 mpk | 2113 | 1.21 | 14100 | 3.63 | / | / |
| | PO - 5 mpk | 10195 | / | 124565 | 2.82 | 3.0 | 176 |
| C29 | IV - 1 mpk | 2603 | 1.39 | 12650 | 2.73 | / | / |
| | PO - 5 mpk | 3639 | / | 36491 | 1.89 | 0.67 | 57.7 |
| C31 | PO - 5 mpk | 3482 | / | 32319 | 3.77 | 0.42 | / |
| C32 | PO - 5 mpk | 2664 | / | 21379 | 2.56 | 0.5 | / |
| E5-a | IV - 1 mpk | 1882 | 8.54 | 1986 | 0.95 | / | / |
| | PO - 10 mpk | 7763 | / | 44380 | 2.34 | 0.5 | 223 |
| E16-a | IV - 1 mpk | 3553 | 3.07 | 5539 | 1.87 | / | / |
| | PO - 5 mpk | 3007 | / | 16975 | 2.22 | 0.33 | 61.3 |
| E25 | PO - 5 mpk | 2827 | / | 23936 | 2.66 | 0.58 | / |
| E26 | PO - 5 mpk | 4429 | / | 31314 | 2.16 | 1.75 | / |
| E32 | PO - 5 mpk | 2040 | / | 8893 | 1.51 | 1.58 | / |
| E35-b | IV - 1 mpk | 2916 | 2.51 | 6770 | 2.51 | / | / |
| | PO - 5 mpk | 3140 | / | 14725 | 2.05 | 1.58 | 43.5 |

## Claims

**1.** A compound of formula (I), an optical isomer thereof, or a pharmaceutically acceptable salt thereof,

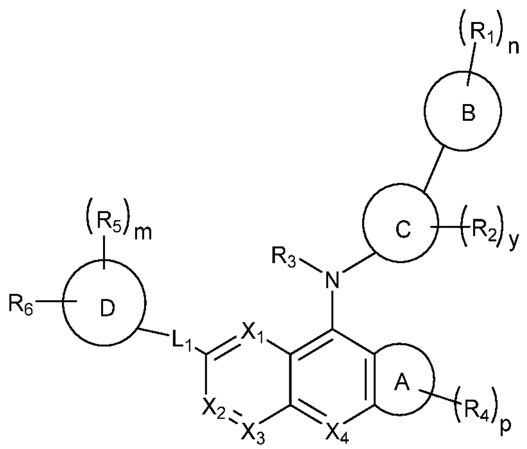

(I)

,

wherein

ring A is selected from cycloalkyl, heterocyclyl, and heteroaryl;
ring B is selected from cycloalkyl, heterocyclyl, aryl, and heteroaryl;
ring C is selected from aryl and heteroaryl;
ring D is selected from aryl, heteroaryl, cycloalkyl, and heterocyclyl;
$X_1$ is selected from $C(R_{7a})$ and N;
$X_2$ is selected from $C(R_{7b})$ and N;
$X_3$ is selected from $C(R_{7c})$ and N;
$X_4$ is selected from $C(R_{7d})$ and N;
$L_1$ is selected from a single bond, NRs, O, S, and $C_{1-6}$ alkyl;
each $R_1$ is independently selected from H, halogen, alkyl, haloalkyl, alkoxy, OH, hydroxyalkyl, cyano, amino, nitro, carboxyl, aldehyde, cycloalkyl, heterocyclyl, aryl, and heteroaryl;
each $R_2$ is independently selected from H, halogen, alkyl, haloalkyl, alkoxy, cyano, amino, nitro, carboxyl, aldehyde, OH, hydroxyalkyl, cycloalkyl, heterocyclyl, aryl, and heteroaryl;
$R_3$ is selected from H, alkyl, cycloalkyl, and heterocyclyl, wherein the alkyl, cycloalkyl, and heterocyclyl are each independently and optionally substituted by one or more than one substituent selected from halogen, alkyl, alkoxy, cyano, amino, nitro, OH, hydroxyalkyl, carboxyl, cycloalkyl, heterocyclyl, aryl, and heteroaryl;
each $R_4$ is independently selected from H, halogen, alkyl, haloalkyl, heteroalkyl, cyano, amino, nitro, OH, hydroxyalkyl, cycloalkyl, heterocyclyl, $-COOR_9$, aryl, and heteroaryl, wherein the alkyl, heteroalkyl, cycloalkyl, heterocyclyl, aryl, and heteroaryl are each independently and optionally substituted by one or more than one substituent selected from halogen, alkyl, alkoxy, cyano, amino, nitro, OH, hydroxyalkyl, cycloalkyl, heterocyclyl, aryl, and heteroaryl;
each $R_5$ is independently selected from H, halogen, alkyl, haloalkyl, alkoxy, cyano, amino, nitro, carboxyl, aldehyde, OH, hydroxyalkyl, oxo, cycloalkyl, heterocyclyl, aryl, and heteroaryl, wherein the alkyl, alkoxy, cycloalkyl, heterocyclyl, aryl, and heteroaryl are each independently and optionally substituted by one or more than one substituent selected from halogen, alkyl, alkoxy, cyano, amino, nitro, carboxyl, OH, hydroxyalkyl, cycloalkyl, heterocyclyl, aryl, and heteroaryl;
$R_6$ is $-M-L_2-R_a$;
M is selected from a single bond or alkyl, wherein the alkyl is optionally substituted by one or more than one substituent selected from halogen, alkyl, alkoxy, cyano, amino, nitro, carboxyl, OH, hydroxyalkyl, cycloalkyl, heterocyclyl, aryl, and heteroaryl;
$L_2$ is selected from a single bond, -C(=O)-, -C(=O)O-, $-C(=O)NR_b-$, $-NR_bC(=O)-$, $- NR_bC(=O)O-$, -O-, -OC(=O)-, -C(=O)-C(=O)-, $-C(=O)-C(=O)NR_b-$, $-NR_b-$, $-S(=O)_2-$, $- S(=O)_2NR_b-$, and $-NR_bS(=O)_2-$;
$R_a$ is selected from H, $-S(O)_2R_c$, alkyl, $-NR_bR_c$, cycloalkyl, heterocyclyl, aryl, and heteroaryl, wherein the alkyl, cycloalkyl, heterocyclyl, aryl, and heteroaryl are each independently and optionally substituted by one or more than one substituent selected from halogen, alkyl, alkoxy, oxo, cyano, amino, nitro, carboxyl, OH, hydroxyalkyl, cycloalkyl, heterocyclyl, aryl, and heteroaryl;
$R_b$ is selected from H, OH, alkyl, haloalkyl, hydroxyalkyl, and cycloalkyl;
$R_c$ is selected from H and alkyl;

$R_{7a}$, $R_{7b}$, $R_{7c}$, and $R_{7d}$ are each independently selected from H, halogen, alkyl, haloalkyl, alkoxy, cyano, amino, nitro, OH, hydroxyalkyl, cycloalkyl, heterocyclyl, aryl, and heteroaryl;
$R_8$ is selected from H, alkyl, haloalkyl, hydroxyalkyl, and cycloalkyl;
$R_9$ is selected from H, alkyl, haloalkyl, hydroxyalkyl, and cycloalkyl;
n is 0, 1, 2, 3, or 4;
y is 0, 1, 2, or 3;
m is 0, 1, 2, 3, or 4;
p is 0, 1, 2, or 3.

**2.** A compound of formula (II), an optical isomer thereof, or a pharmaceutically acceptable salt thereof,

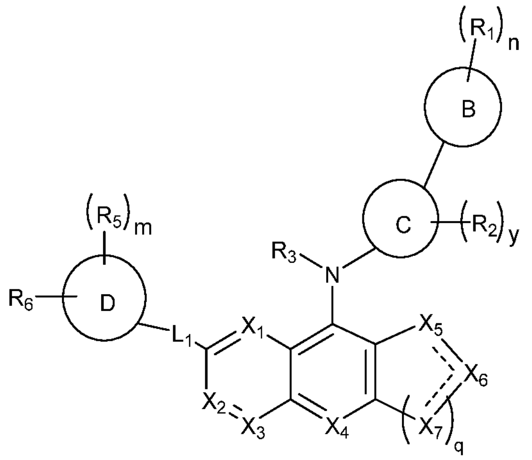

(II)

,

wherein

q is 0, 1, 2, or 3;
$X_5$ is selected from O, S, $N(R_{4a})$, $C(R_{4a})_2$, and C=O;
$X_6$ is selected from O, S, $N(R_{4b})$, $C(R_{4b})_2$, and C=O;
each $X_7$ is independently selected from $N(R_{4c})$, $C(R_{4c})_2$, and C=O;

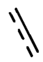

represents a double bond or a single bond;
and, when

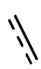

between $X_5$ and $X_6$ represents a double bond, $X_5$ is selected from N and $C(R_{4a})$, and $X_6$ is selected from N and $C(R_{4b})$;
and, $X_6$ is not attached to two double bonds simultaneously;
$R_{4a}$, $R_{4b}$, and $R_{4c}$ are each independently selected from H, halogen, alkyl, haloalkyl, heteroalkyl, cyano, amino, nitro, OH, hydroxyalkyl, cycloalkyl, heterocyclyl, -COOR[9], aryl, and heteroaryl, wherein the alkyl, heteroalkyl, cycloalkyl, heterocyclyl, aryl, and heteroaryl are each independently and optionally substituted by one or more than one substituent selected from halogen, alkyl, alkoxy, cyano, amino, nitro, OH, hydroxyalkyl, cycloalkyl, heterocyclyl, aryl, and heteroaryl;
ring B, ring C, ring D, $R_1$, $R_2$, $R_3$, $R_5$, $R_6$, $X_1$, $X_2$, $X_3$, $X_4$, n, m, and y are as defined in claim 1.

**3.** A compound of formula (III), an optical isomer thereof, or a pharmaceutically acceptable salt thereof,

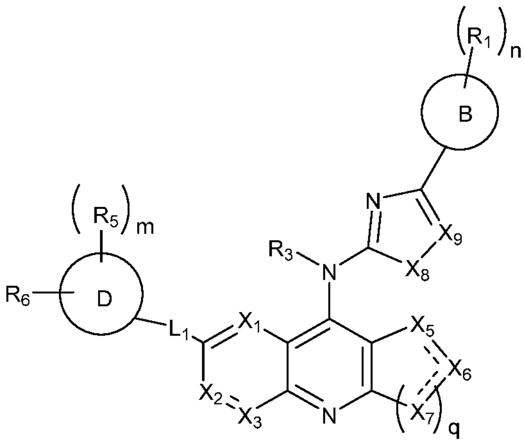

(III)

,

wherein

each $X_8$ is independently selected from O, S, $N(R_{2a})$, -N=CH-, -CH=N-, and -CH=CH;
each $X_9$ is independently selected from $C(R_{2b})$ and N;
$R_{2a}$ and $R_{2b}$ are each independently selected from H, halogen, alkyl, haloalkyl, alkoxy, cyano, amino, nitro, carboxyl, aldehyde, OH, hydroxyalkyl, cycloalkyl, heterocyclyl, aryl, and heteroaryl;
ring B, ring D, $R_1$, $R_3$, $R_5$, $R_6$, $X_1$, $X_2$, $X_3$, $X_4$, n, and m are as defined in claim 1;
$X_5$, $X_6$, $X_7$, q, and

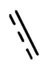

are as defined in claim 2.

4. The compound, the optical isomer thereof, or the pharmaceutically acceptable salt thereof according to claim 1, wherein ring A is selected from $C_{4-8}$ cycloalkyl, 4- to 8-membered heterocyclyl, and 5- to 6-membered heteroaryl.

5. The compound, the optical isomer thereof, or the pharmaceutically acceptable salt thereof according to claim 1, wherein ring A is selected from $C_{4-6}$ cycloalkyl, 5- to 6-membered heterocyclyl, and 5- to 6-membered heteroaryl.

6. The compound, the optical isomer thereof, or the pharmaceutically acceptable salt thereof according to claim 1, wherein ring A is selected from cyclobutyl, cyclopentyl, tetrahydrofuranyl, pyrrolidinyl, cyclopentanone, dihydrofuran-2(3H)-one, pyrrolidin-2-one, imidazolyl, pyrazolyl, oxazolyl, isoxazolyl, and 1,2,3-oxadiazolyl.

7. The compound, the optical isomer thereof, or the pharmaceutically acceptable salt thereof according to claim 1, wherein each $R_4$ is independently selected from H, OH, $C_{1-6}$ alkyl, and $C_{1-6}$ heteroalkyl, wherein the $C_{1-6}$ alkyl and $C_{1-6}$ heteroalkyl are optionally substituted by one or more than one of OH, amino, and halogen.

8. The compound, the optical isomer thereof, or the pharmaceutically acceptable salt thereof according to claim 2, wherein $R_{4a}$, $R_{4b}$, and $R_{4c}$ are each independently selected from H, OH, $C_{1-6}$ alkyl, and $C_{1-6}$ heteroalkyl, wherein the $C_{1-6}$ alkyl and $C_{1-6}$ heteroalkyl are optionally substituted by one or more than one of OH, amino, and halogen.

9. The compound, the optical isomer thereof, or the pharmaceutically acceptable salt thereof according to claim 1, wherein structural moiety

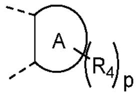

is selected from

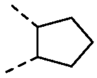,

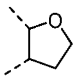, 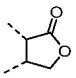, 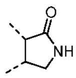, 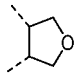, 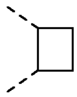, 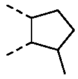, 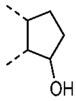, 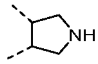, 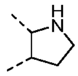, 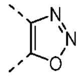, 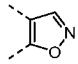, 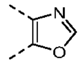,

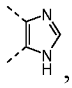, 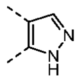, 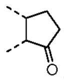, 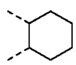, 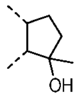, 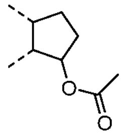, and 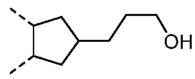.

**10.** The compound, the optical isomer thereof, or the pharmaceutically acceptable salt thereof according to any one of claims 1 to 3, wherein ring B is selected from phenyl, 5- to 6-membered heteroaryl, $C_{3-6}$ cycloalkyl, 5- to 6-membered heterocyclyl, benzo-5- to 6-membered heterocyclyl, and 5- to 9-membered bicycloalkyl.

**11.** The compound, the optical isomer thereof, or the pharmaceutically acceptable salt thereof according to claim 10, wherein ring B is selected from phenyl, pyridyl, cyclohexyl, tetrahydro-2H-pyranyl, benzo[d][1,3]dioxazolyl, and bicyclo[1.1.1]pentyl.

**12.** The compound, the optical isomer thereof, or the pharmaceutically acceptable salt thereof according to any one of claims 1 to 3, wherein structural moiety

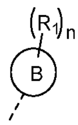

is selected from

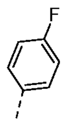, 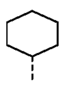, 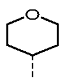, 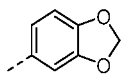, 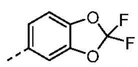, and 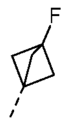.

**13.** The compound, the optical isomer thereof, or the pharmaceutically acceptable salt thereof according to any one of claims 1 to 3, wherein ring D is selected from phenyl, 5- to 6-membered heteroaryl, $C_{3-6}$ cycloalkyl, and 6- to 10-membered heterocyclyl.

**14.** The compound, the optical isomer thereof, or the pharmaceutically acceptable salt thereof according to claim 13, wherein ring D is selected from piperazinyl, 2,6-diazaspiro[3.3]heptyl, 2,7-diazaspiro[4.4]nonyl, 2,8-diazaspiro[4.5]decyl, 2,7-diazaspiro[3.5]nonyl, 2,5-diazabicyclo[2.2.1]heptyl, and octahydropyrrolo[3,4-c]pyrrolyl.

**15.** The compound, the optical isomer thereof, or the pharmaceutically acceptable salt thereof according to claim 14, wherein ring D is selected from

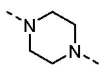, 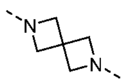,

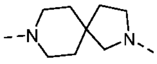, 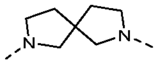, 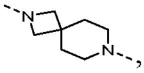, 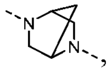, and 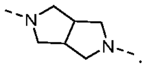.

**16.** The compound, the optical isomer thereof, or the pharmaceutically acceptable salt thereof according to any one of claims 1 to 3, wherein $R_6$ is selected from -$C_{1-3}$ alkyl-C(=O)-3- to 9-membered heterocyclyl, -$C_{1-3}$ alkyl-C(=O)-3- to 9-membered heterocyclyl-$C_{1-6}$ alkyl, - C(=O)-3- to 9-membered heterocyclyl, -C(=O)-$C_{3-9}$ cycloalkyl, -C(=O)-$C_{1-6}$ alkyl, -$C_{1-3}$ alkyl-C(=O)-NH-$C_{1-6}$ alkyl, -S(=O)$_2$-$C_{1-3}$ alkyl, and -$C_{1-3}$ alkyl-C(=O)NH-OH, wherein the -$C_{1-3}$ alkyl-C(=O)-3- to 9-membered heterocyclyl, -$C_{1-3}$ alkyl-C(=O)-3- to 9-membered heterocyclyl-$C_{1-6}$ alkyl, -C(=O)-5- to 6-membered heterocyclyl, -C(=O)-$C_{3-9}$ cycloalkyl,C(=O)-$C_{1-6}$ alkyl, -$C_{1-3}$ alkyl-C(=O)-NH-$C_{1-6}$ alkyl, -S(=O)$_2$-$C_{1-3}$ alkyl, or -$C_{1-3}$ alkyl-C(=O)NH-OH is optionally substituted by 1, 2, or 3 OH, amino, methyl, ethyl, hydroxymethyl, trifluoromethyl, methoxy, or halogens.

**17.** The compound, the optical isomer thereof, or the pharmaceutically acceptable salt thereof according to any one of claims 1 to 3, wherein $R_6$ is selected from

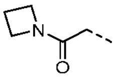, 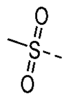,

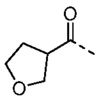, 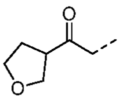, 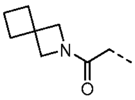, 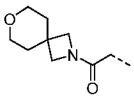, 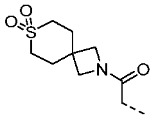, 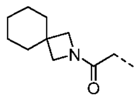, 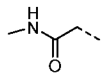, 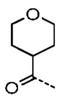, 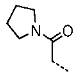,

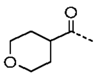, 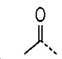, 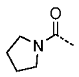, 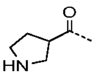, 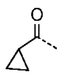, 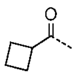, 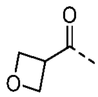, 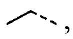, 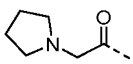, 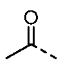, 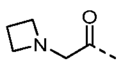,

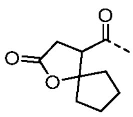, 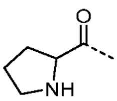, 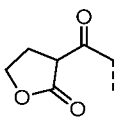, 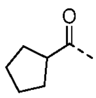, 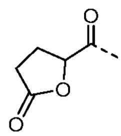, and 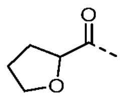,

wherein

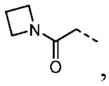, 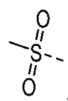,

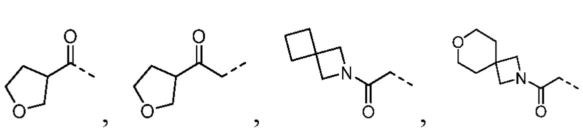, 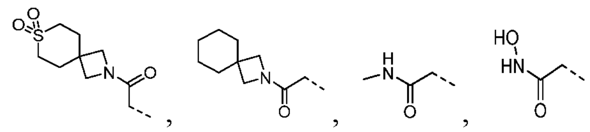,

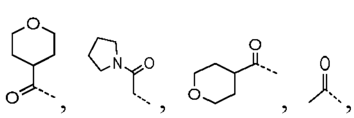, 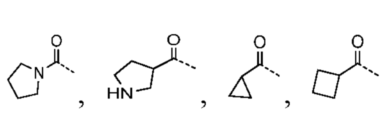, 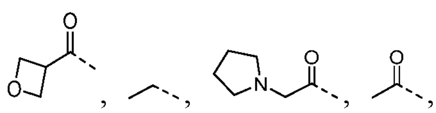,

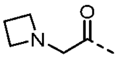, 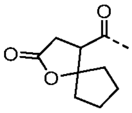, 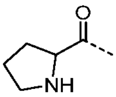, 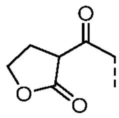, 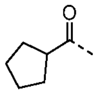, 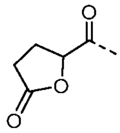, or 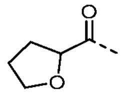

is optionally substituted by 1, 2, or 3 OH, methyl, ethyl, hydroxymethyl, or halogens.

**18.** The compound, the optical isomer thereof, or the pharmaceutically acceptable salt thereof according to claim 17, wherein $R_6$ is selected from

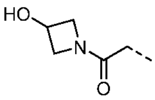, 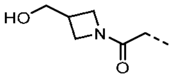,

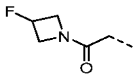, 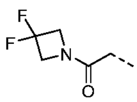, 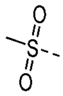, 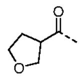, 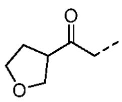, 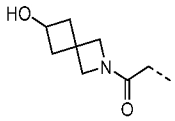, 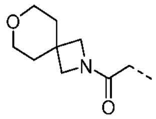, 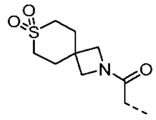,

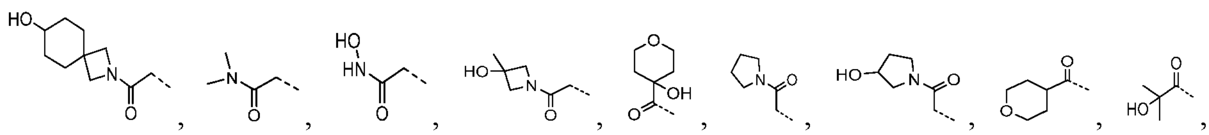

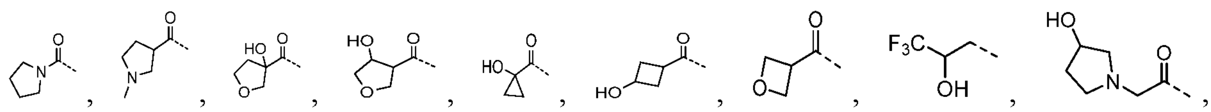

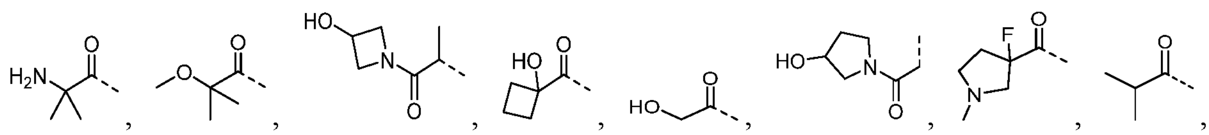

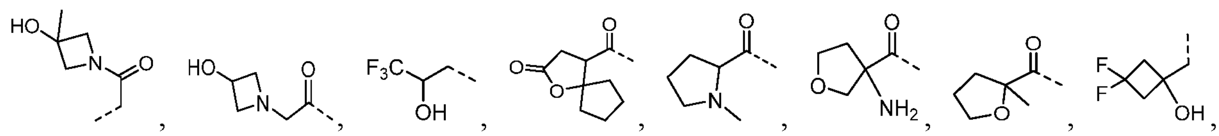

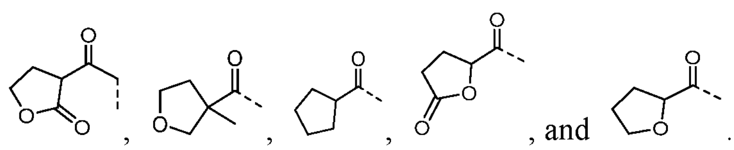

**19.** The compound, the optical isomer thereof, or the pharmaceutically acceptable salt thereof according to any one of claims 1 to 3, wherein structural moiety

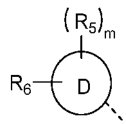

is selected from

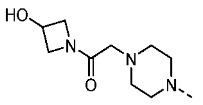, 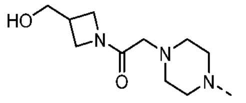, 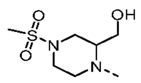, 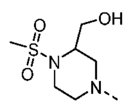, 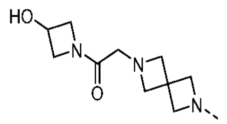,

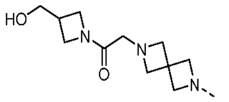, 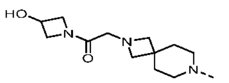, 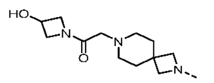, 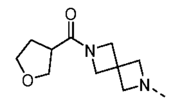, 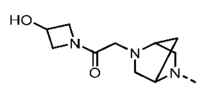,

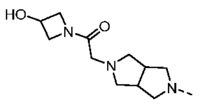, 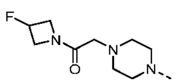, 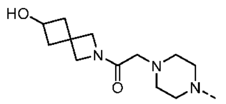, 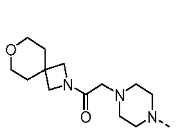, 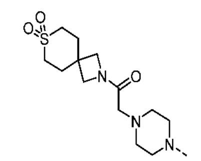,

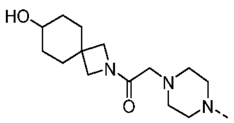, 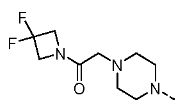, 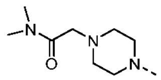, 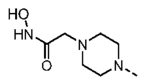, 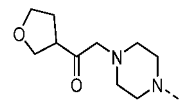, 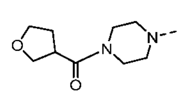,

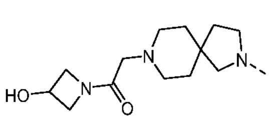, 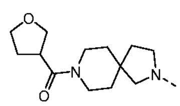, 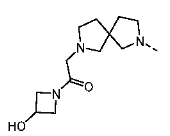, 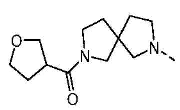, 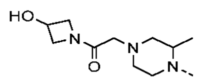,

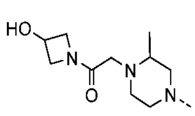, 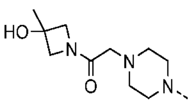, 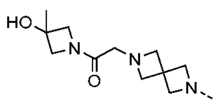, 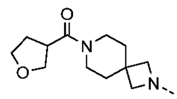, 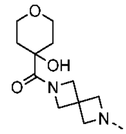, 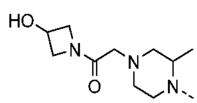,

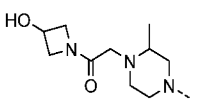, 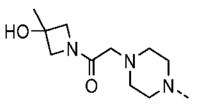, 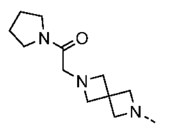, 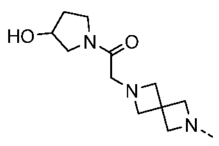, 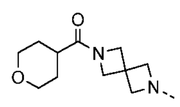,

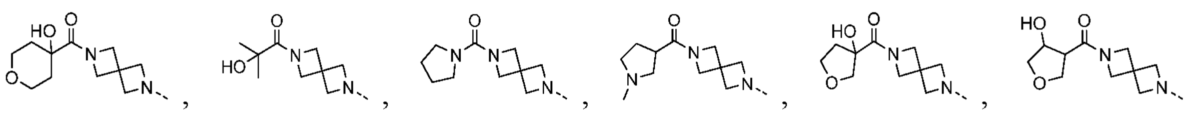
, , , , , ,

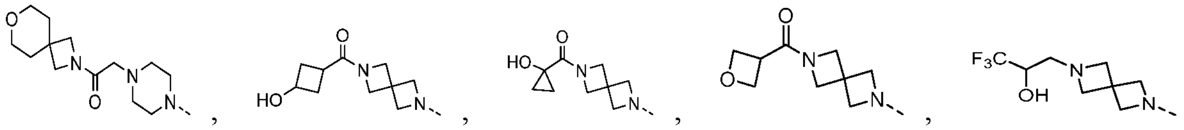
, , , , ,

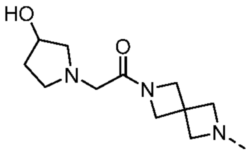, 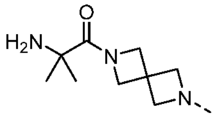, 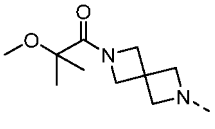, 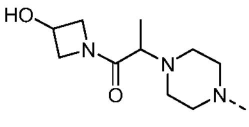, 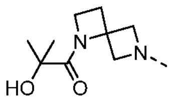,

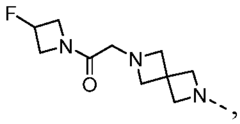, 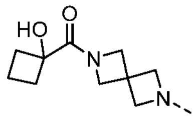, 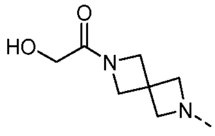, 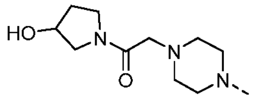, 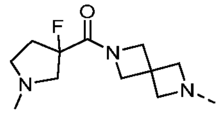,

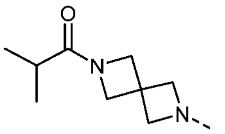, 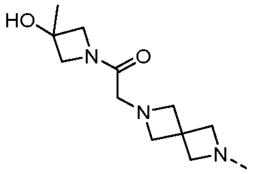, 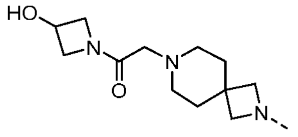, 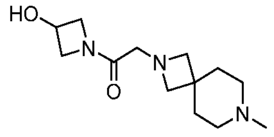,

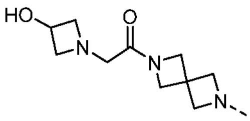, 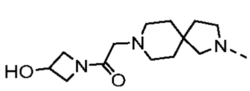, 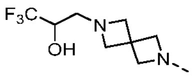, 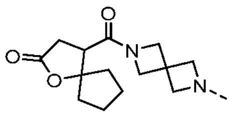,

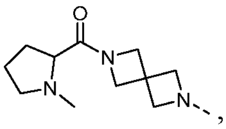, 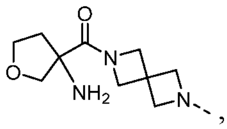, 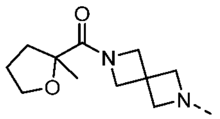, 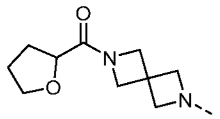, 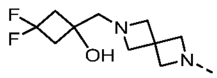,

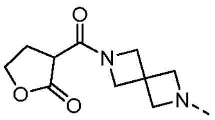, 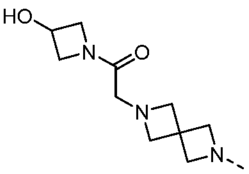, 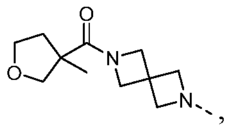, 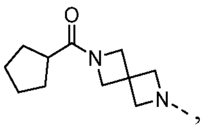, 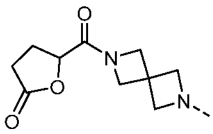,

and

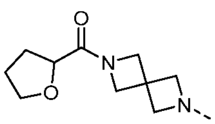.

**20.** The compound, the optical isomer thereof, or the pharmaceutically acceptable salt thereof according to any one of claims 1 to 3, wherein $R_3$ is selected from H, $C_{1-6}$ alkyl, $C_{3-6}$ cycloalkyl, and 4- to 6-membered heterocyclyl, wherein the $C_{1-6}$ alkyl, $C_{3-6}$ cycloalkyl, or 4- to 6-membered heterocyclyl is optionally substituted by 1, 2, or 3 halogens, cyano, amino, or $C_{1-6}$ alkyl.

**21.** The compound, the optical isomer thereof, or the pharmaceutically acceptable salt thereof according to any one of claims 1 to 3, wherein $R_3$ is selected from H, methyl, ethyl,

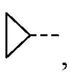, 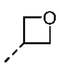, 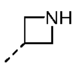, 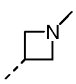, and 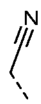.

22. The compound, the optical isomer thereof, or the pharmaceutically acceptable salt thereof according to claim 1 or 2, wherein ring C is selected from 5- to 6-membered heteroaryl, wherein the heteroaryl comprises 1 to 3 heteroatoms selected from N atom, O atom, or S atom.

23. The compound, the optical isomer thereof, or the pharmaceutically acceptable salt thereof according to claim 1, wherein $R_{7a}$, $R_{7b}$, $R_{7c}$, and $R_{7d}$ are each independently selected from H, halogen, $C_{1-6}$ alkyl, $C_{1-6}$ alkoxy, cyano, amino, nitro, OH, $C_{3-6}$ cycloalkyl, 5- to 6-membered heterocyclyl, phenyl, and 5- to 6-membered heteroaryl, wherein the $C_{1-6}$ alkyl is optionally substituted by 1, 2, or 3 halogens.

24. The compound, the optical isomer thereof, or the pharmaceutically acceptable salt thereof according to claim 1, 7, or 23, wherein structural moiety

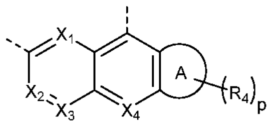

is selected from

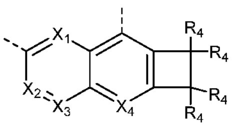 , 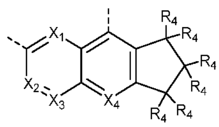 , 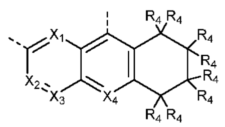 , 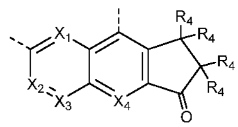 ,

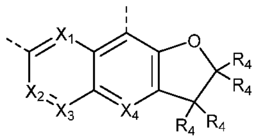 , 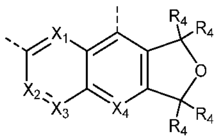 , 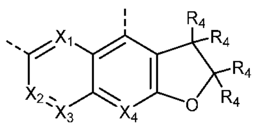 , 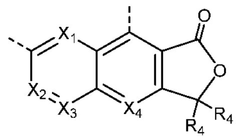 ,

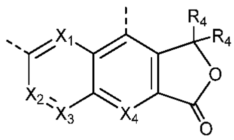 , 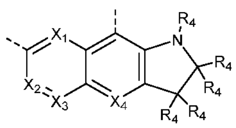 , 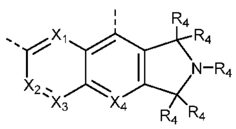 , 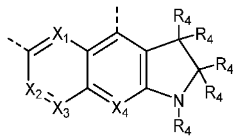 ,

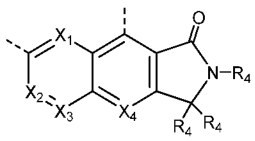 , 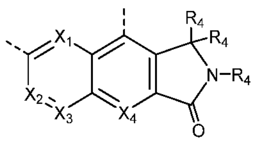 , 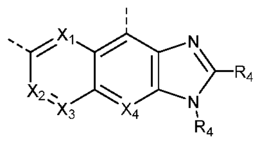 , 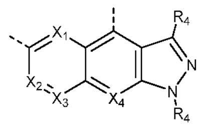 ,

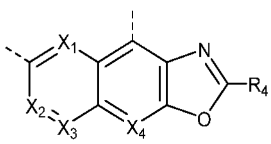 , 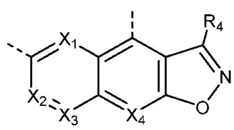 , and 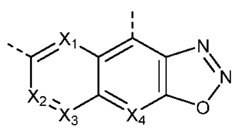 .

25. The compound, the optical isomer thereof, or the pharmaceutically acceptable salt thereof according to claim 1 or 7, wherein structural moiety

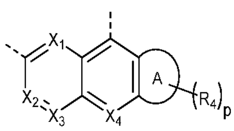

is selected from

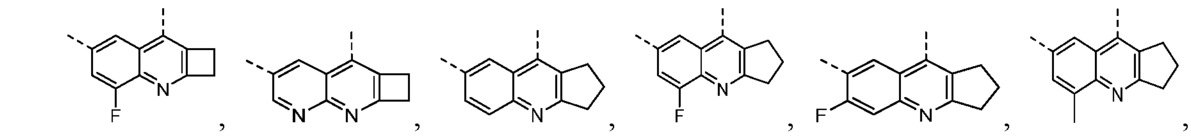, , , , , ,

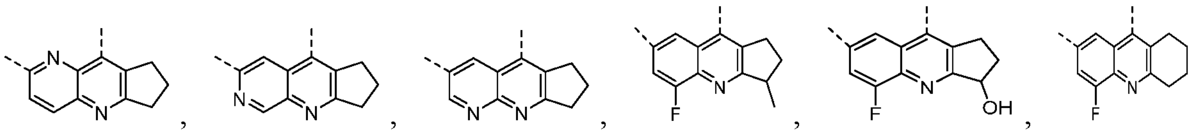, , , , , ,

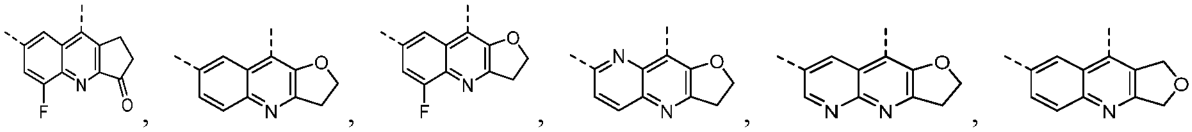, , , , , ,

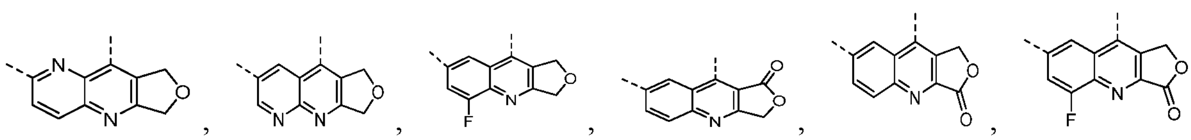, , , , , ,

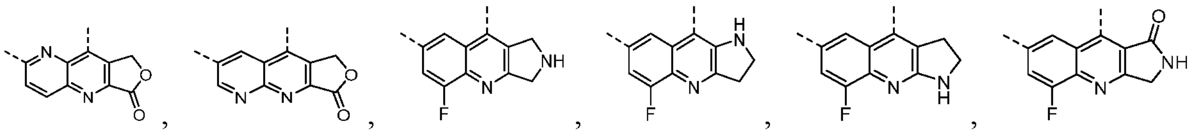, , , , , ,

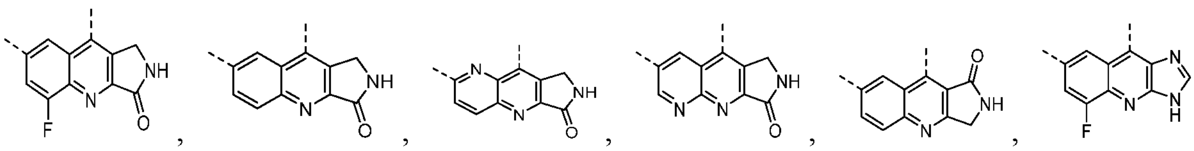, , , , , ,

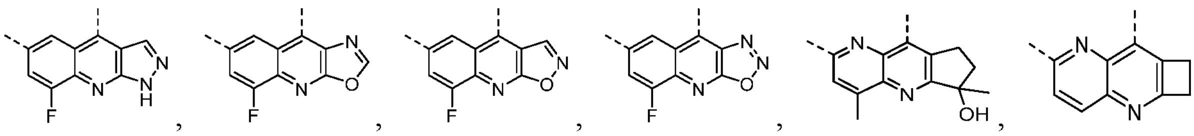, , , , , ,

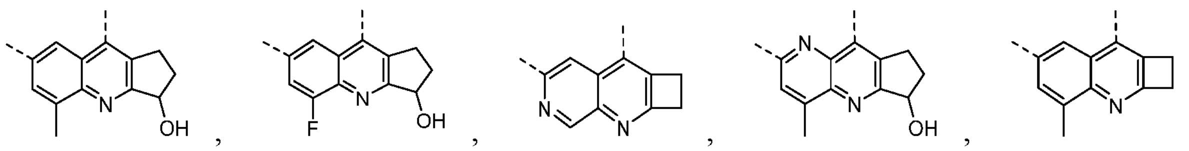, , , , ,

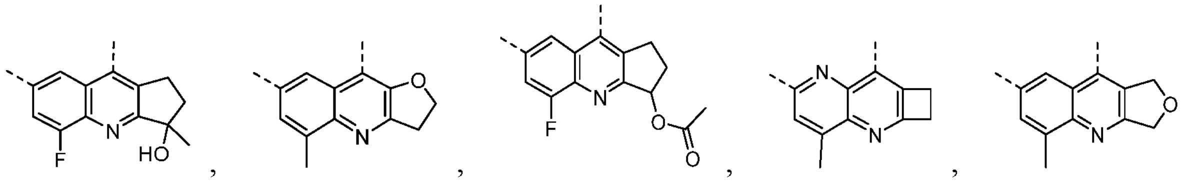, , , , ,

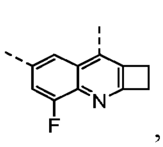, 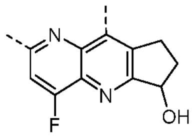, 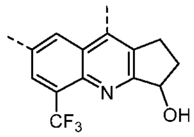, 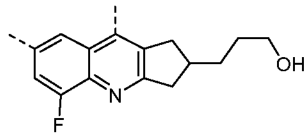, and 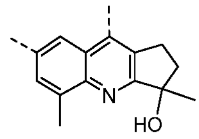.

**26.** A compound of the following formulas, an optical isomer thereof, or a pharmaceutically acceptable salt thereof, selected from:

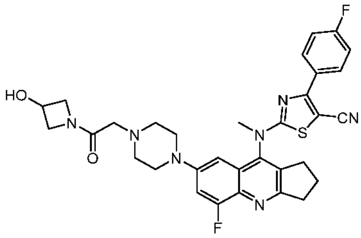,

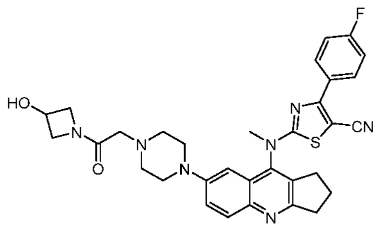,

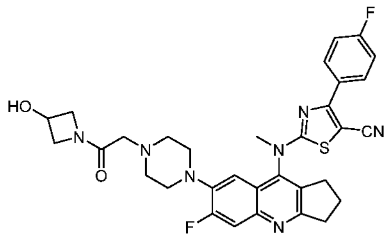,

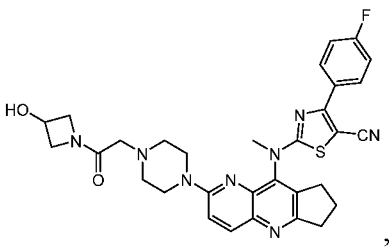,

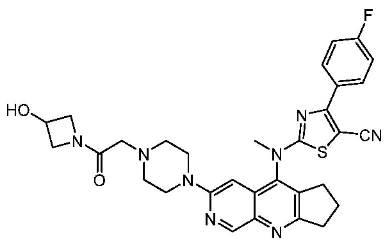,

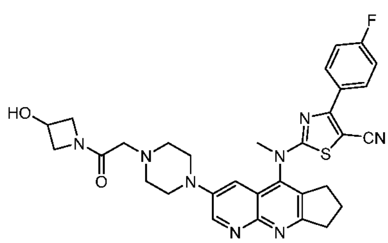,

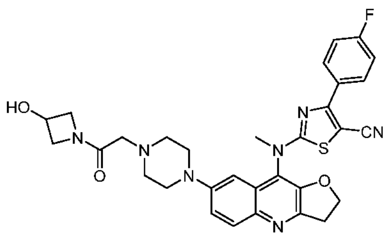,

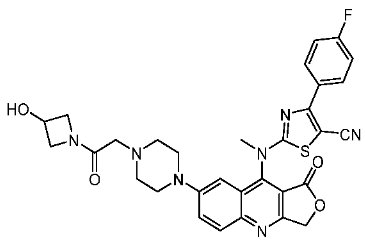,

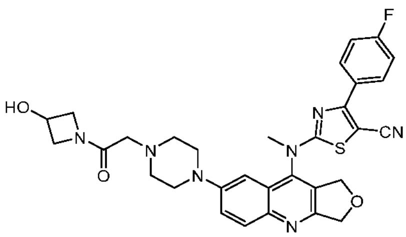,

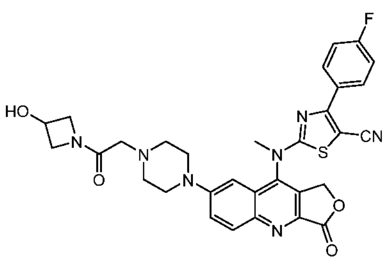,

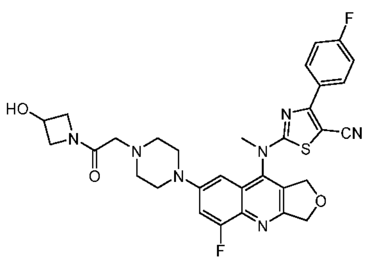,

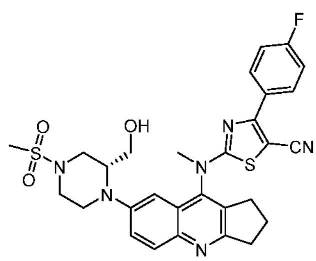,

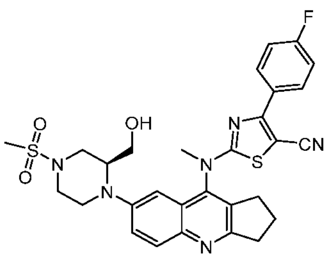,

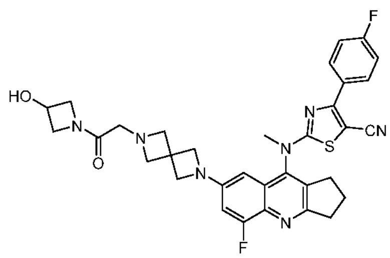,

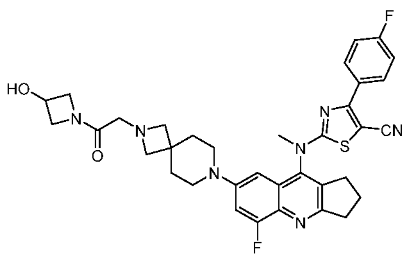,

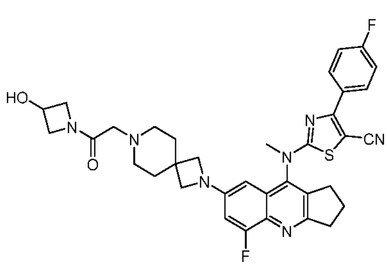,
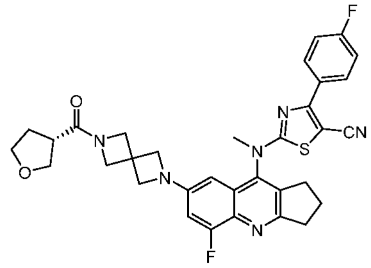,
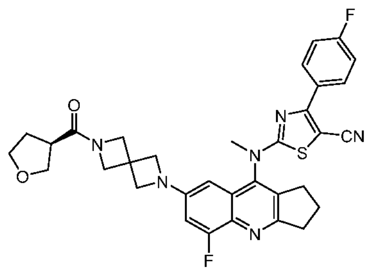,

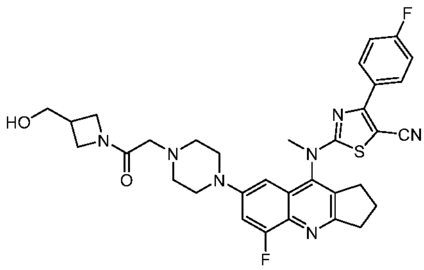,
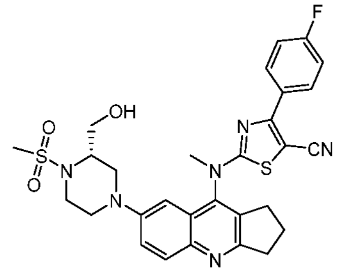,
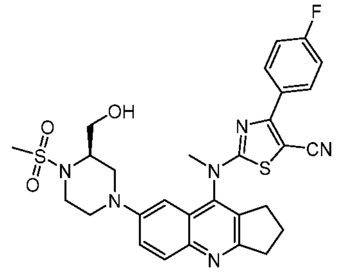,

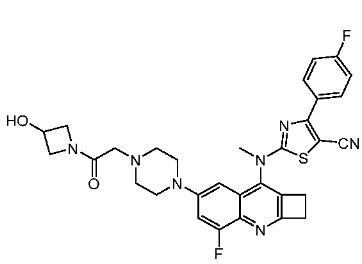,
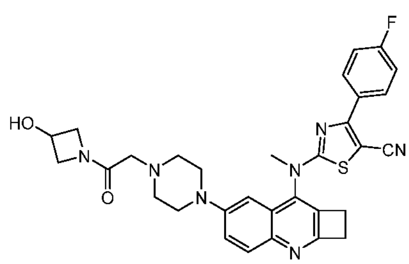,
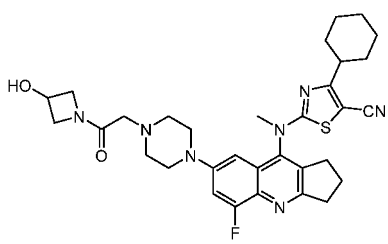,

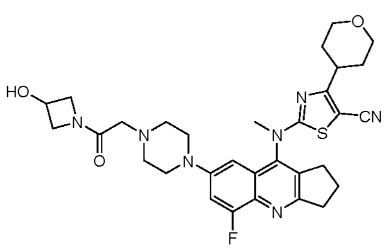,
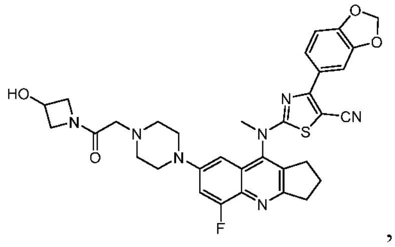,
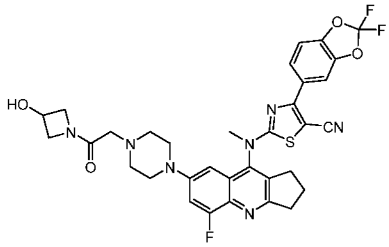,

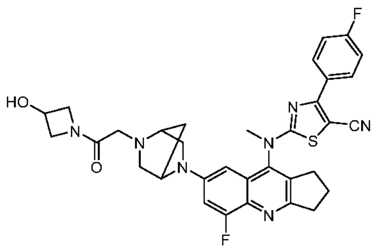,
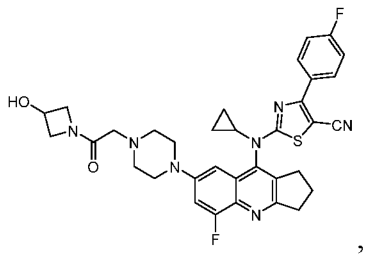,
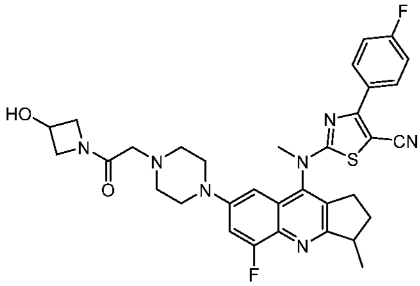,

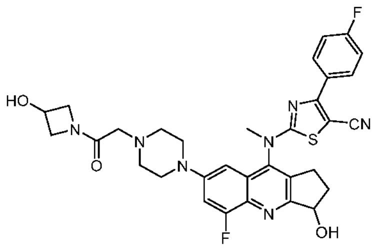,
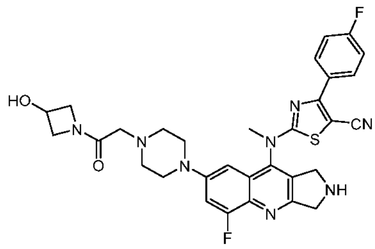,
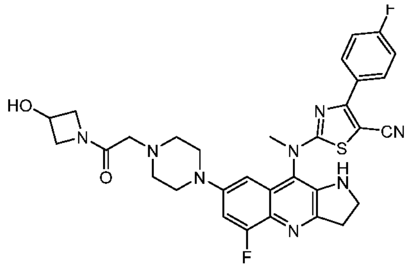,
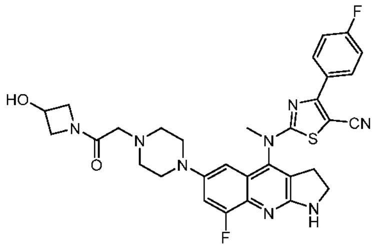,
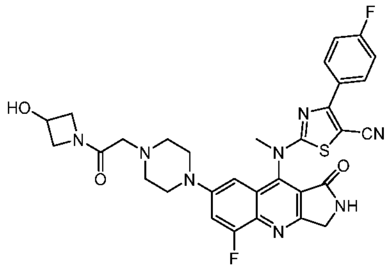,
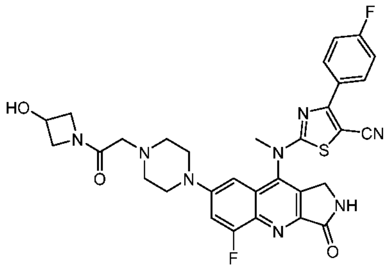,
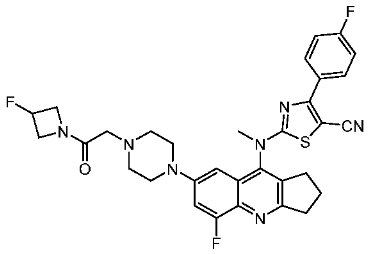,
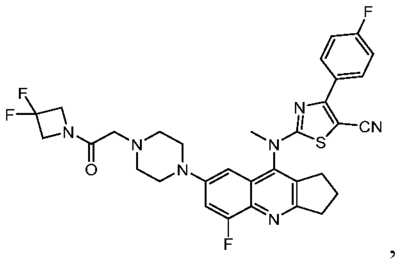,
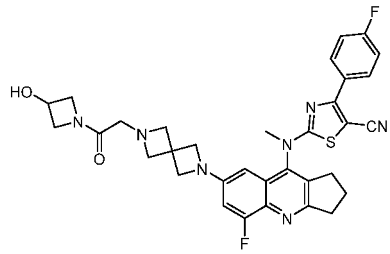,
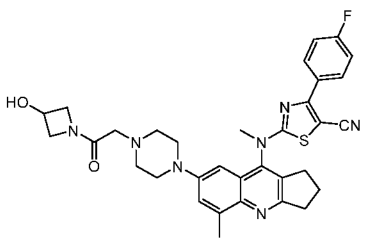,
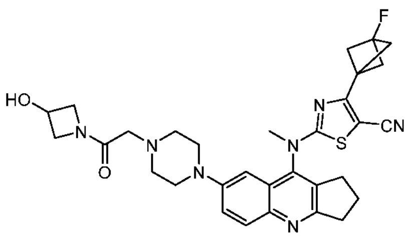,
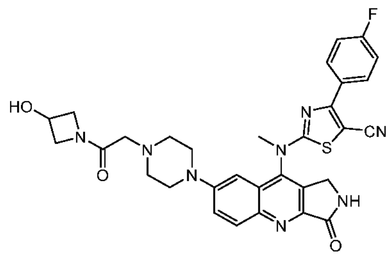,
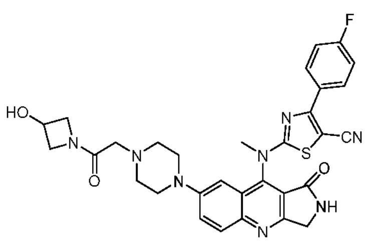,
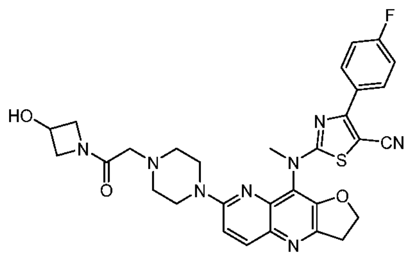,
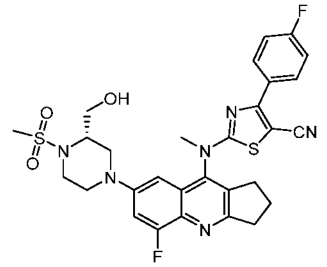,

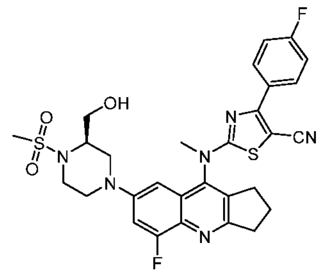 ,

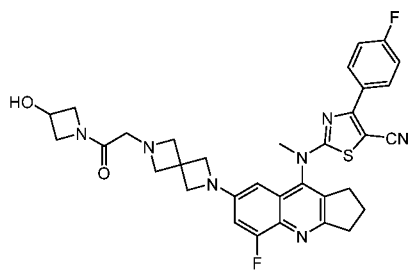 ,

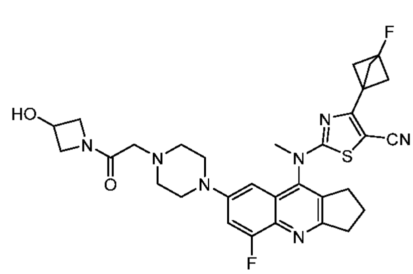 ,

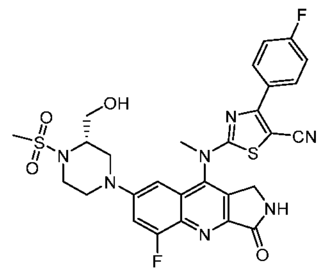 ,

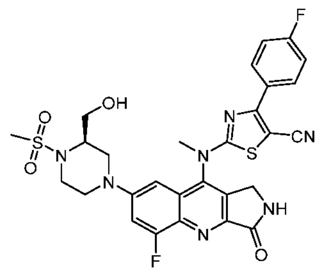 ,

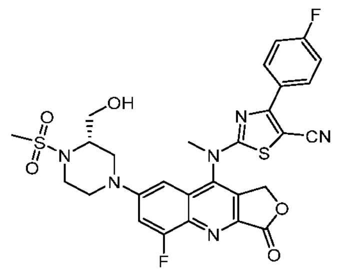 ,

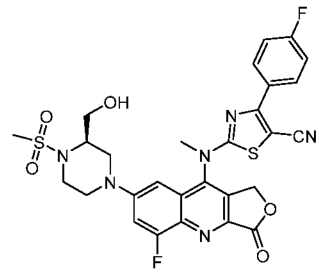 ,

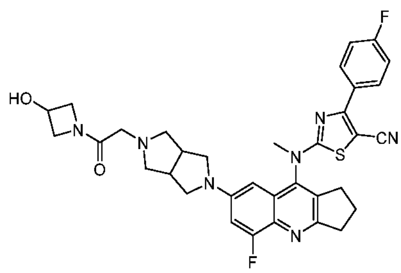 ,

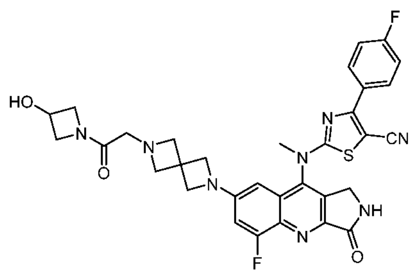 ,

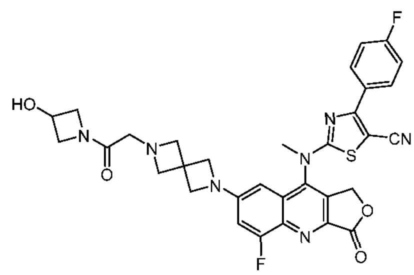 ,

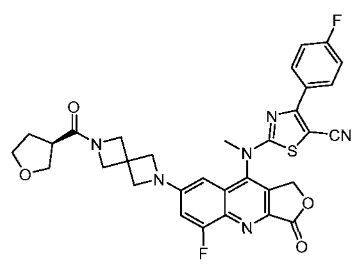 ,

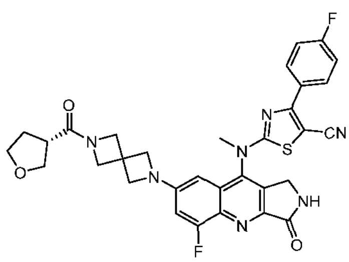 ,

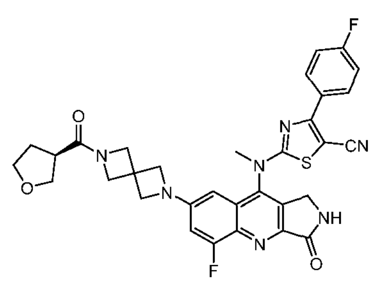 ,

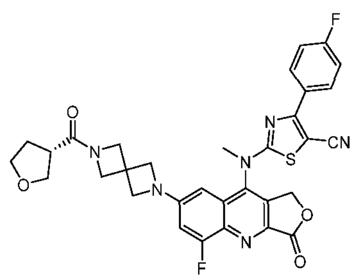 ,

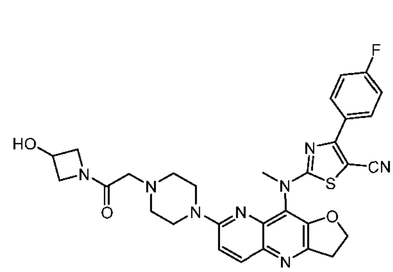 ,

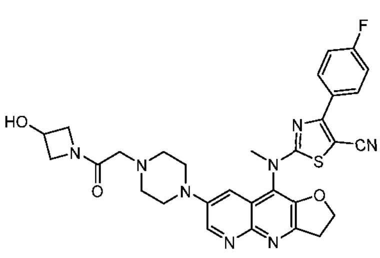 , 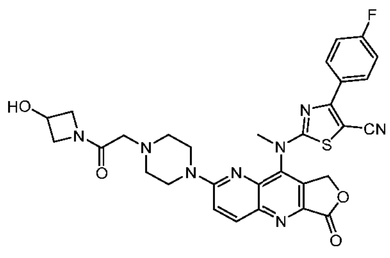 , 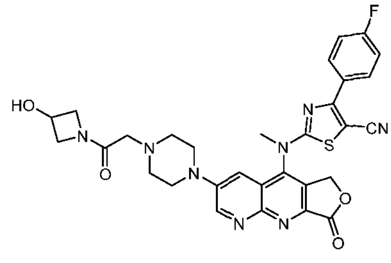 ,

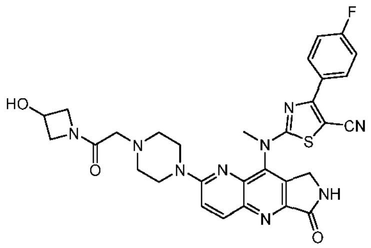 , 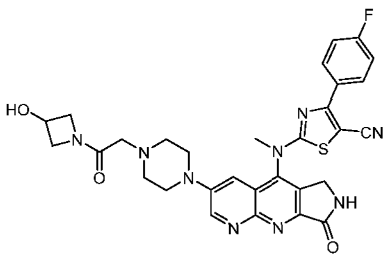 , 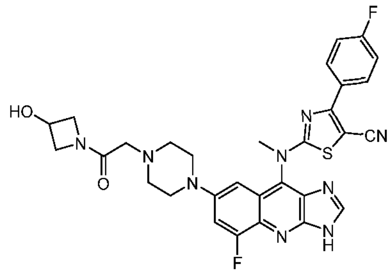 ,

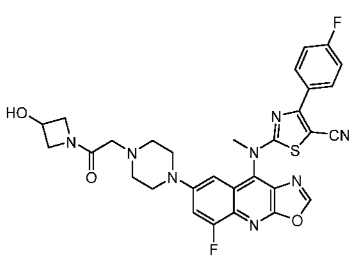 , 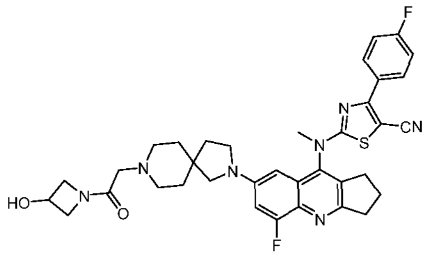 , 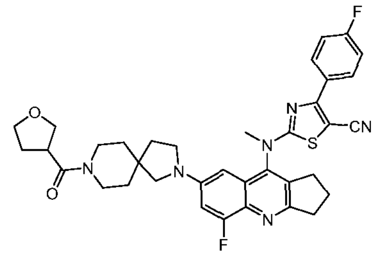 ,

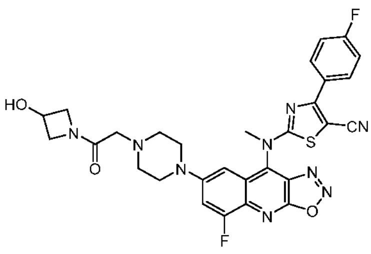 , 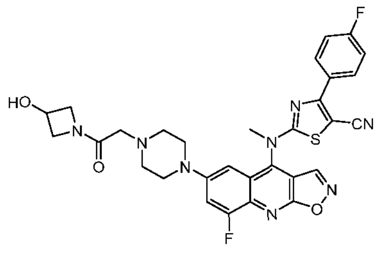 , 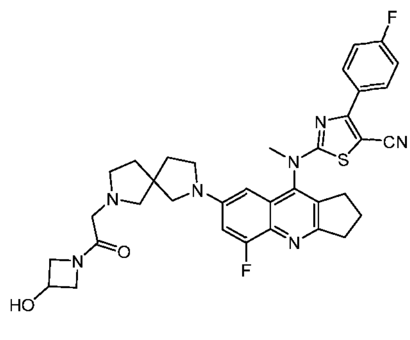 ,

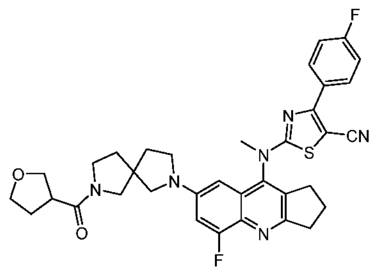 , 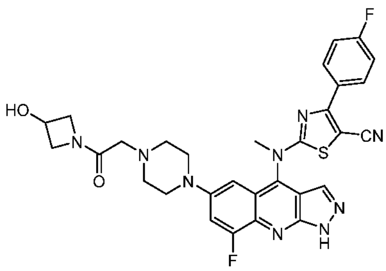 , 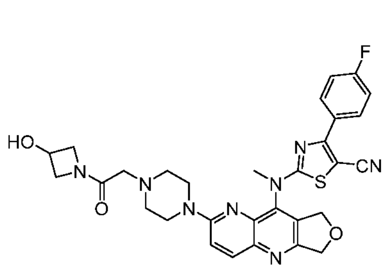 ,

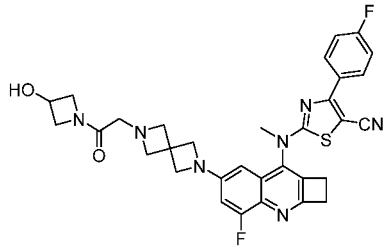
,
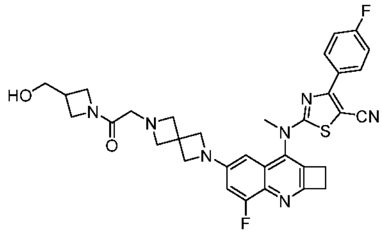
,
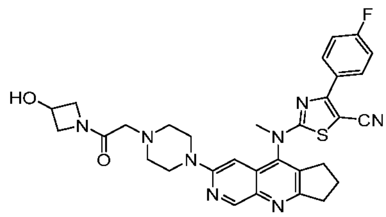
,

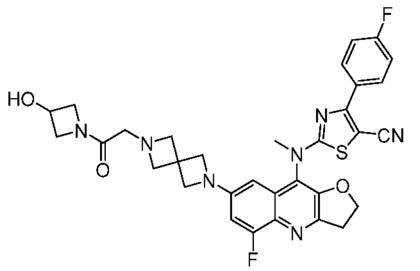
,
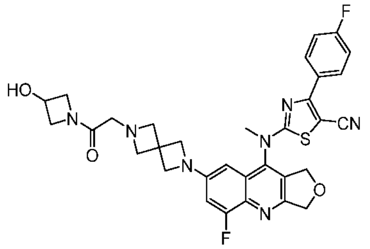
,
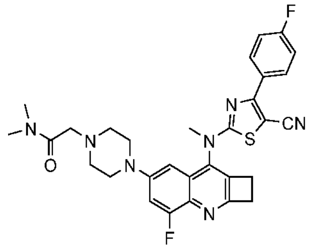
,

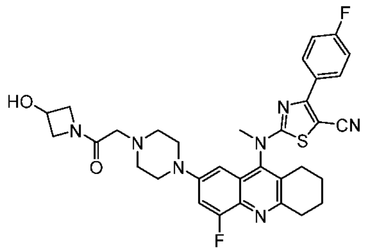
,
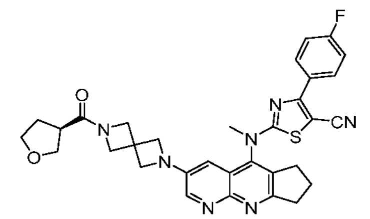
,
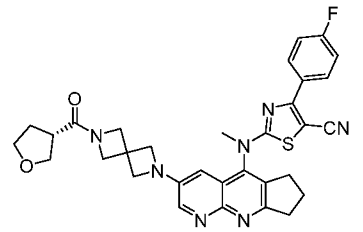
,

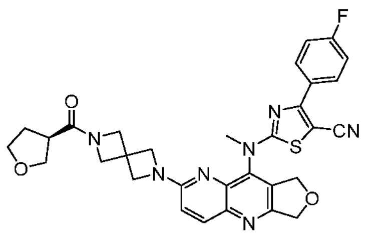
,
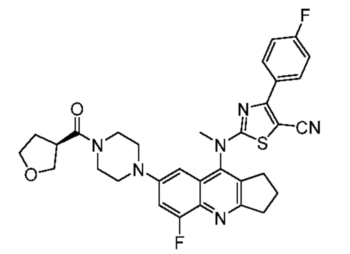
,
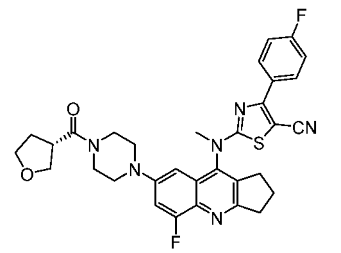
,

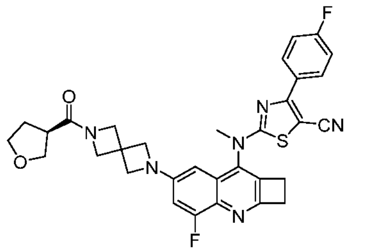
,
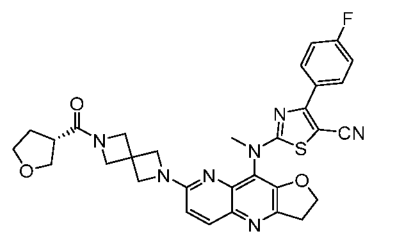
,
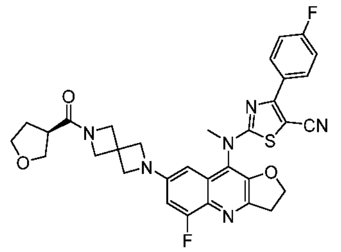
,

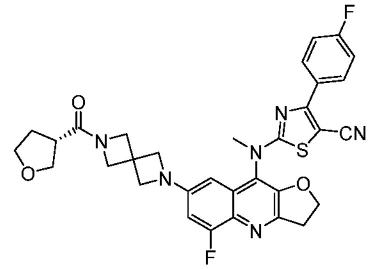, 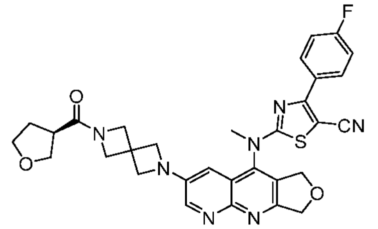, 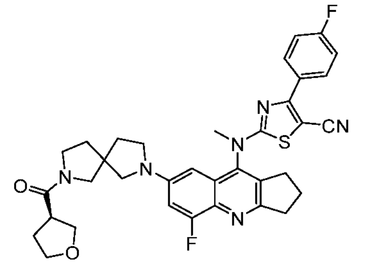,

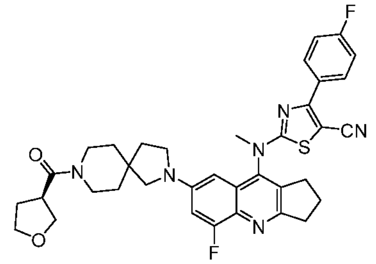, 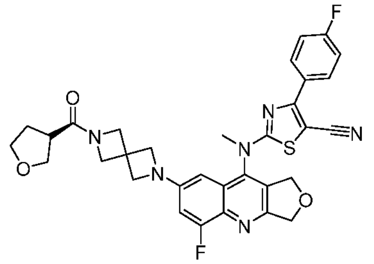, 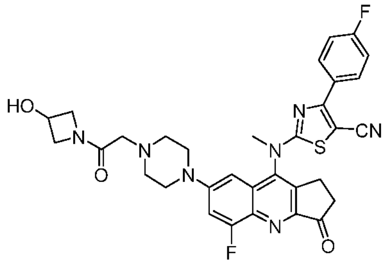,

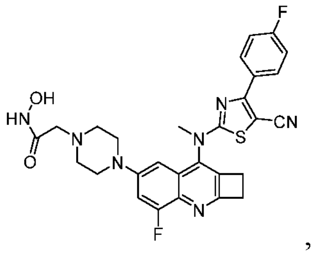, 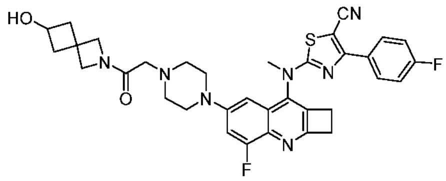, 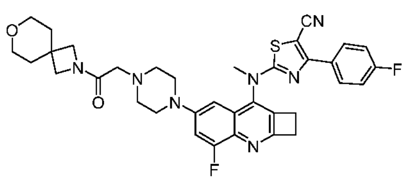,

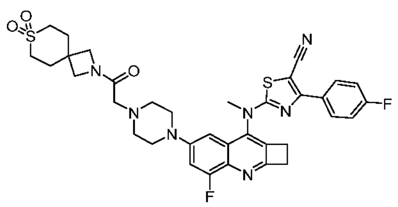, 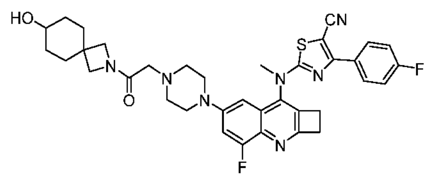, 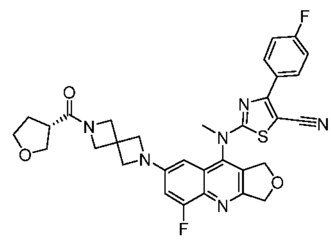,

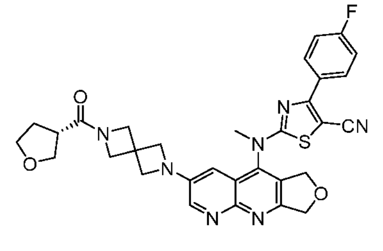, 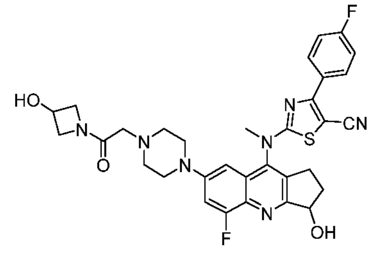, 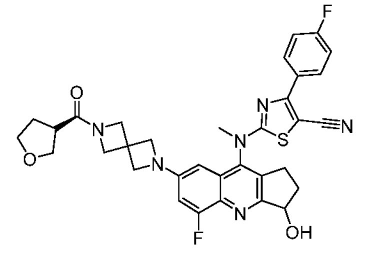,

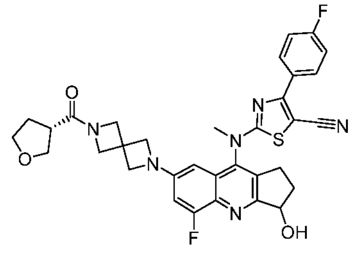, 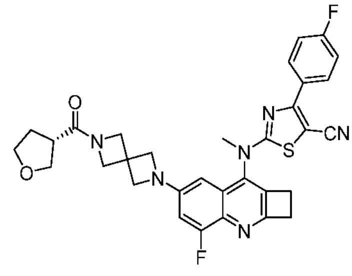, 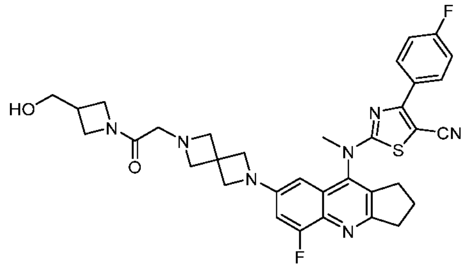,

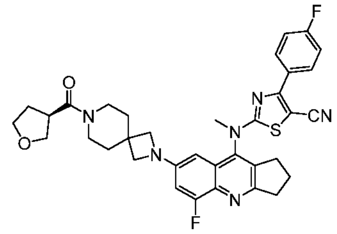 ,

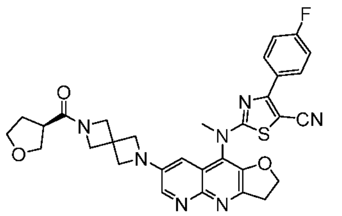 ,

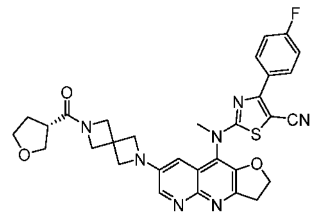 ,

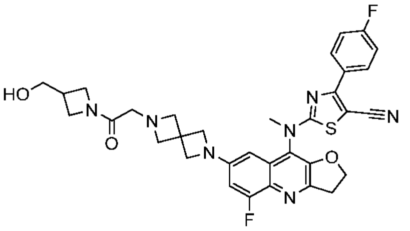 ,

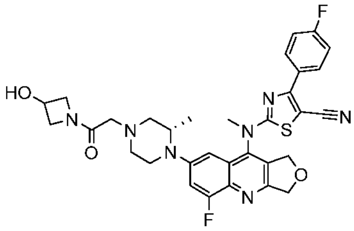 ,

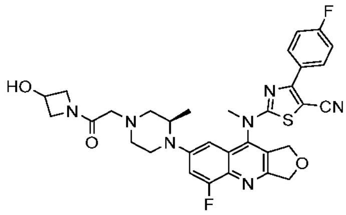 ,

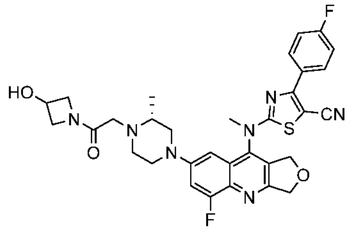 ,

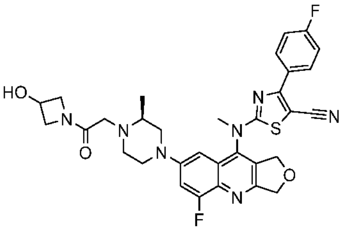 ,

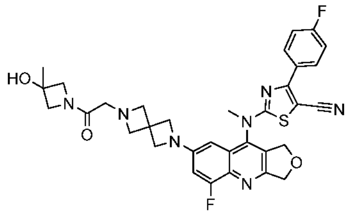 ,

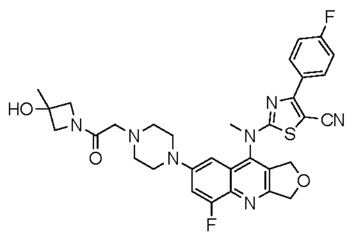 ,

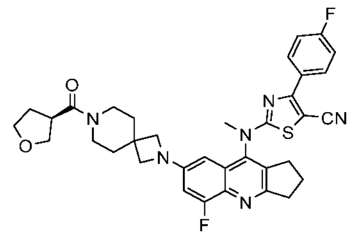 ,

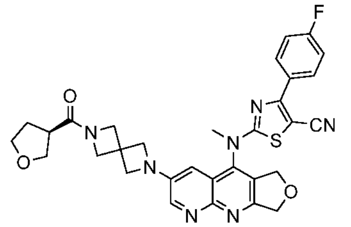 ,

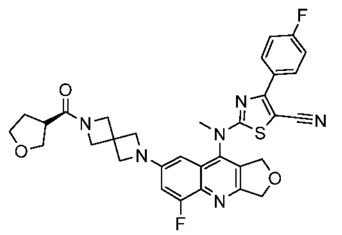 ,

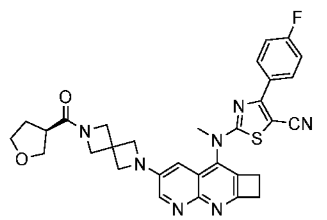 ,

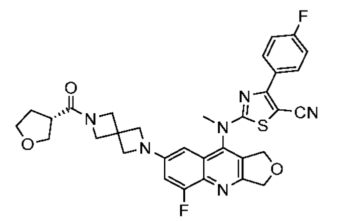 ,

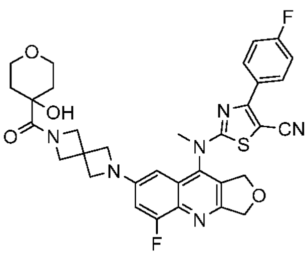 ,

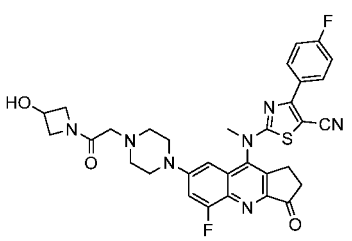 ,

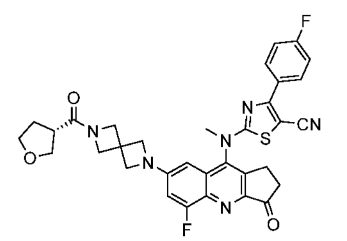 ,

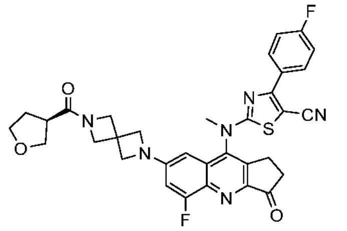 , 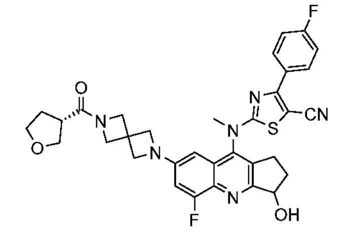 , 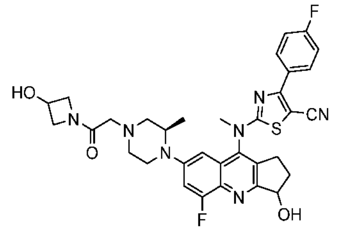 ,

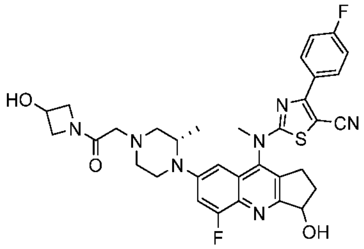 , 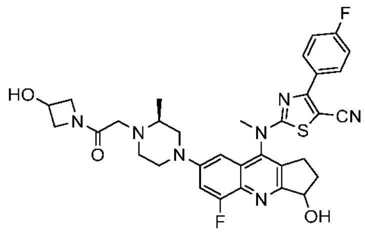 , 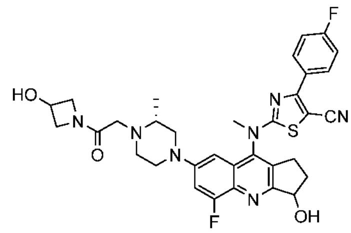 ,

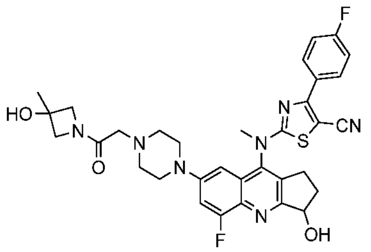 , 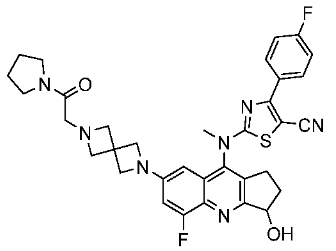 , 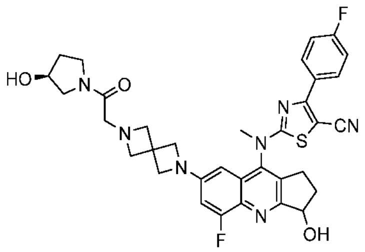 ,

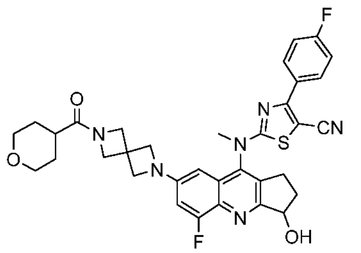 , 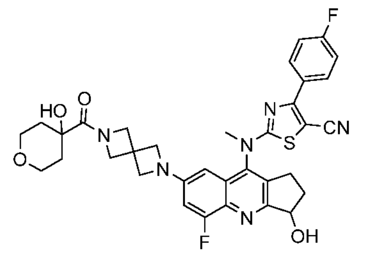 , 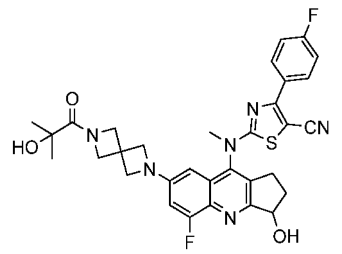 ,

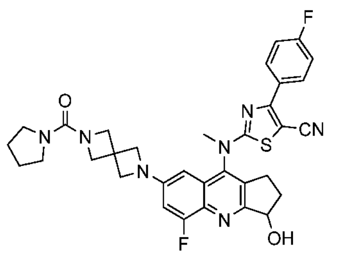 , 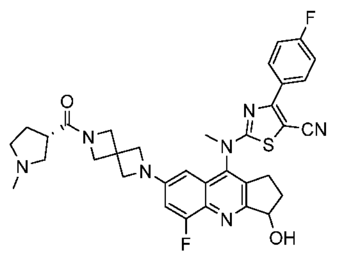 , 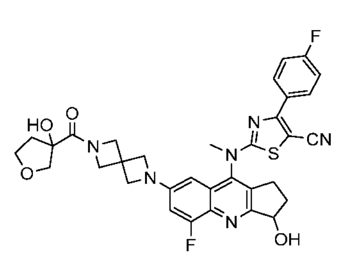 ,

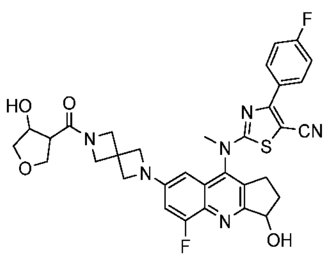 , 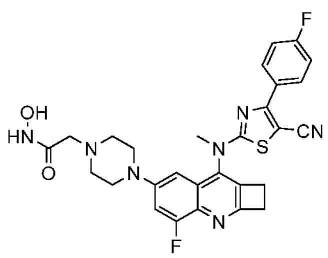 , 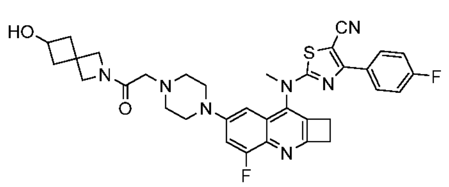 ,

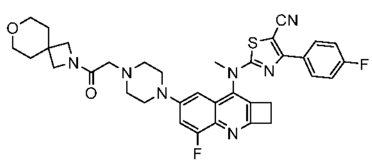,
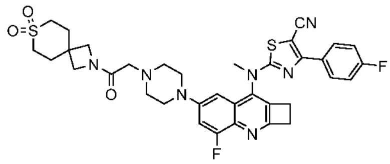,
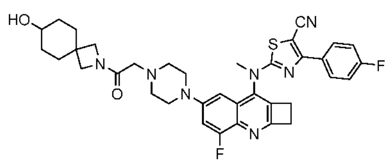,

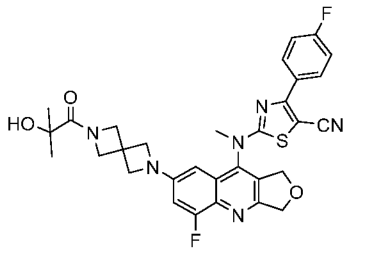,
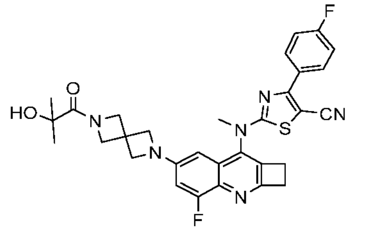,
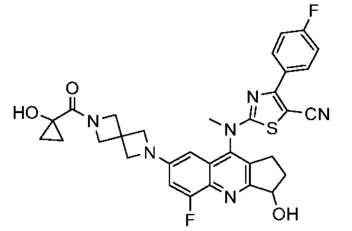,

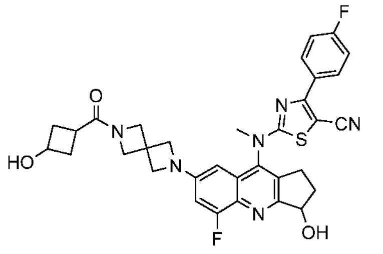,
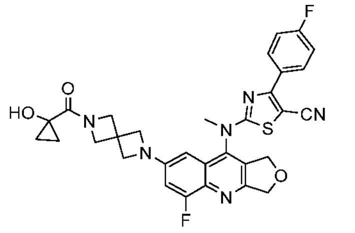,
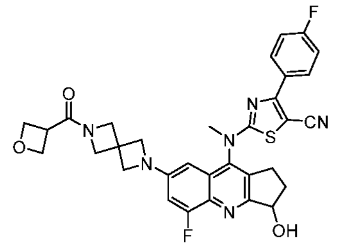,

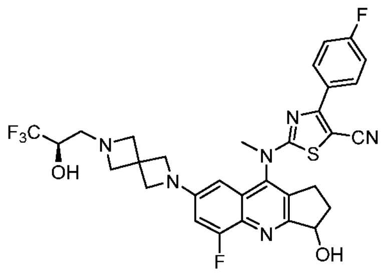,
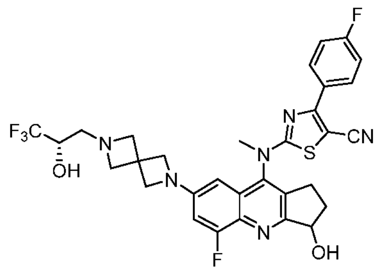,
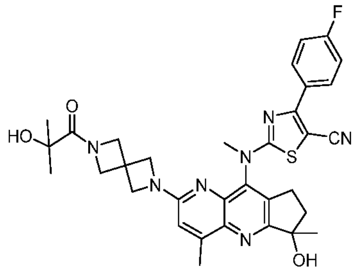,

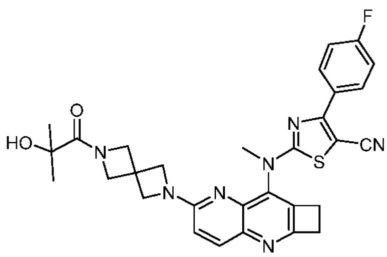,
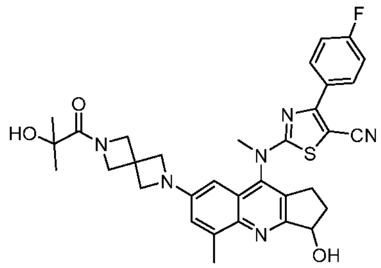,
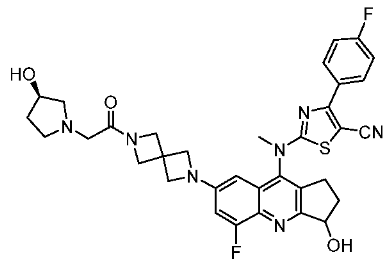,

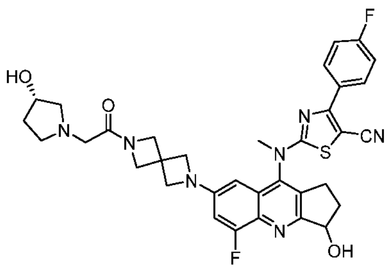,
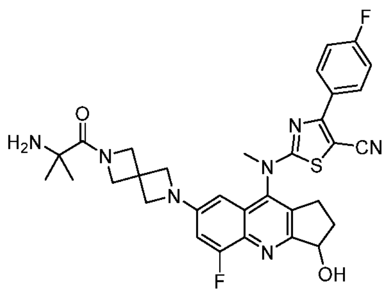,
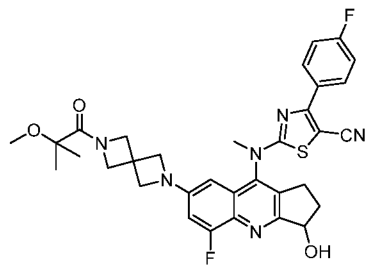,

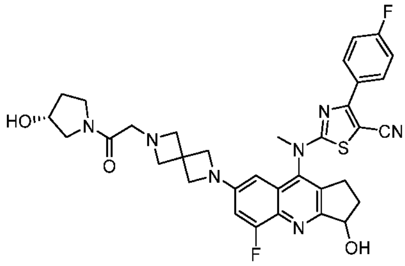,
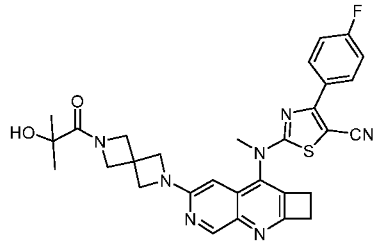,
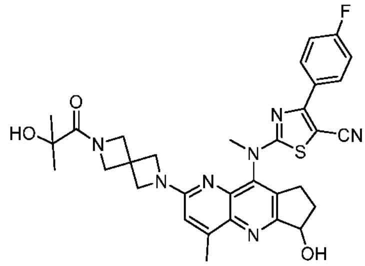,

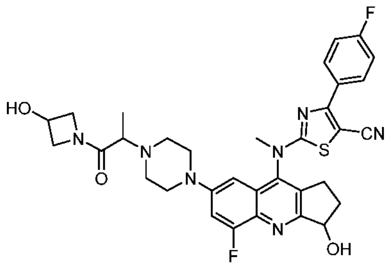,
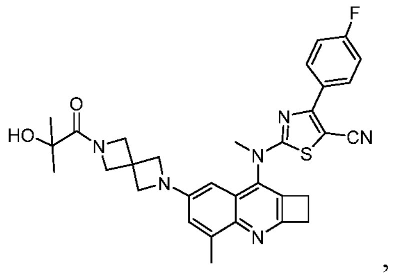,
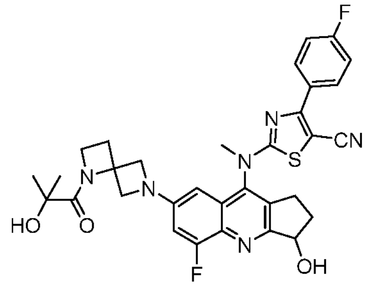,

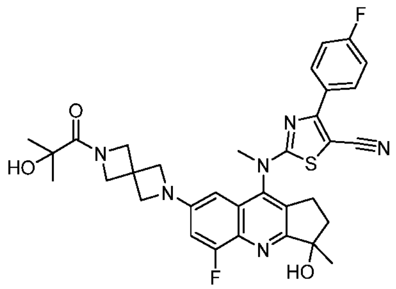,
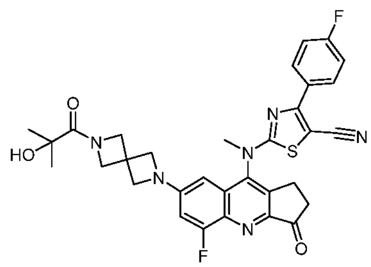,
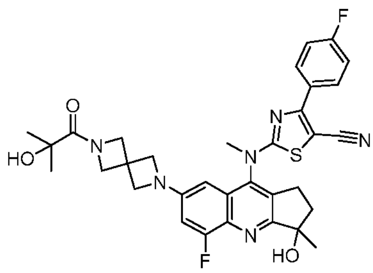,

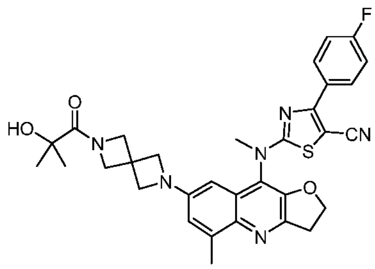,
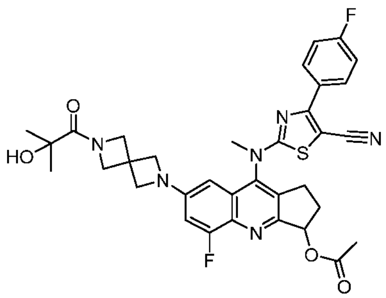,
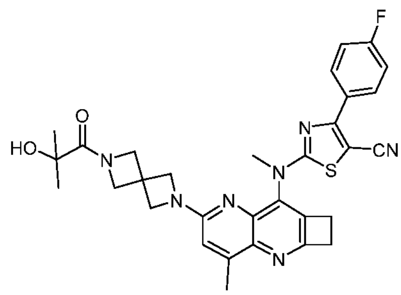,

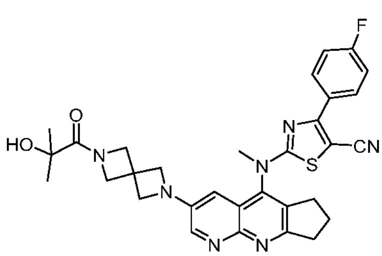,
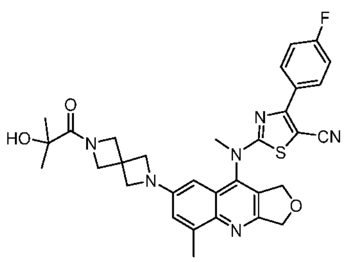,
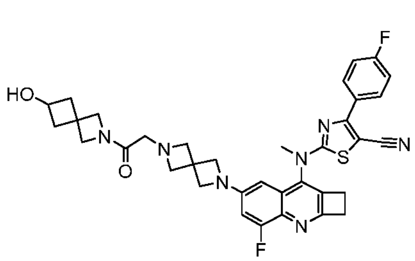,

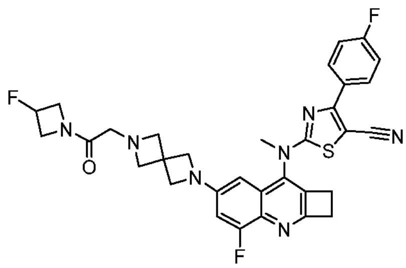 ,

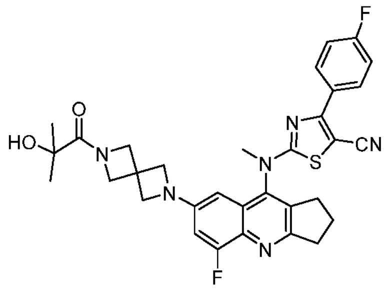 ,

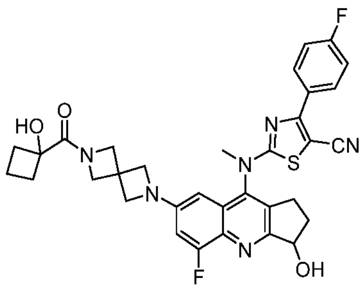 ,

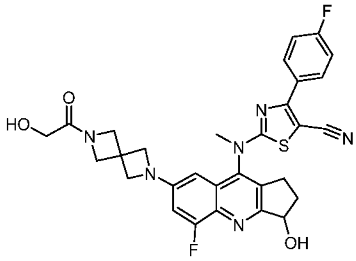 ,

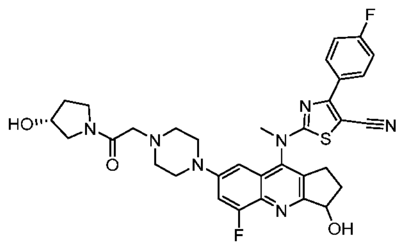 ,

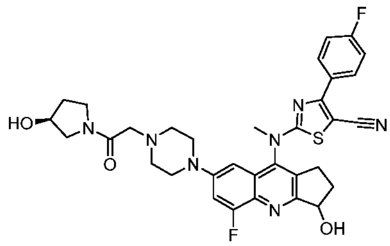 ,

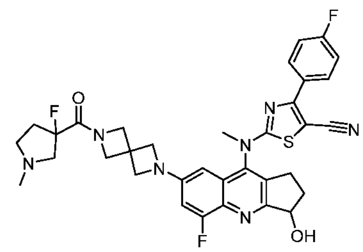 ,

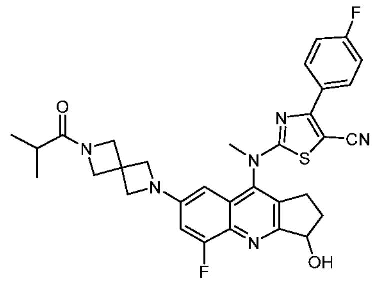 ,

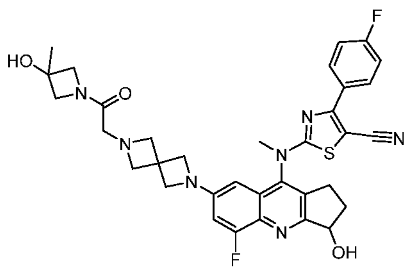 ,

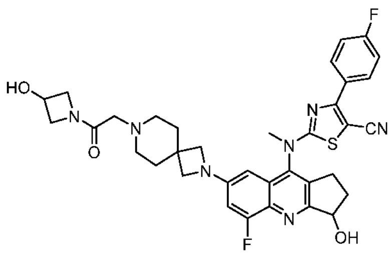 ,

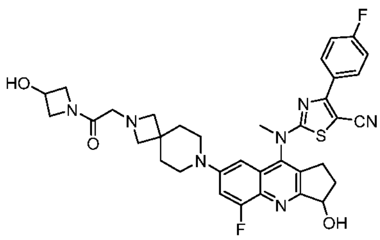 ,

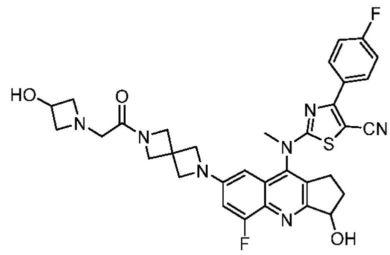 ,

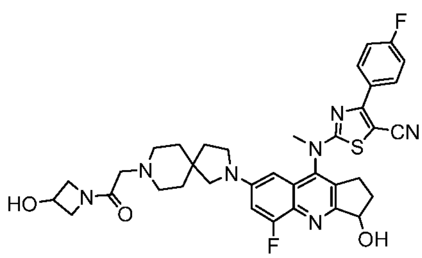 ,

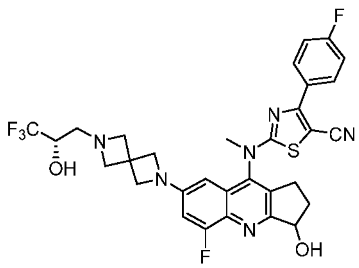 ,

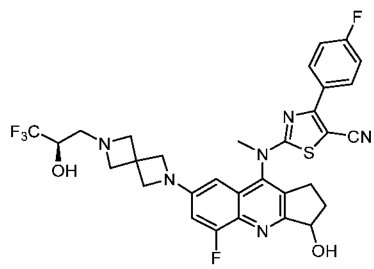 ,

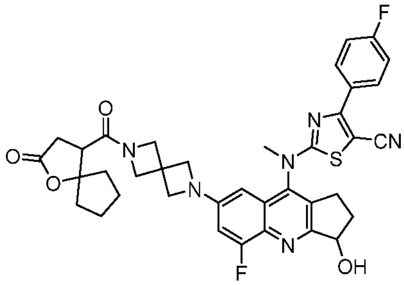,
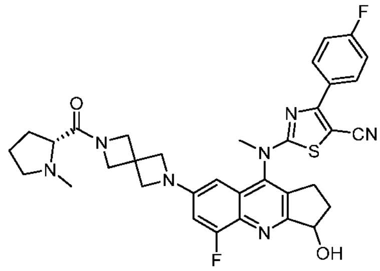,
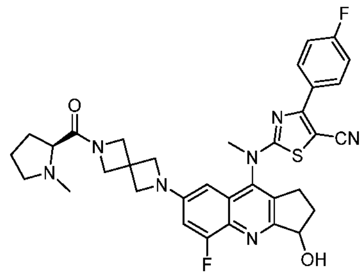,

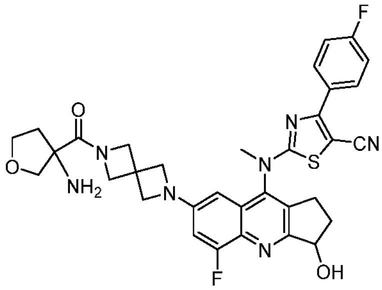,
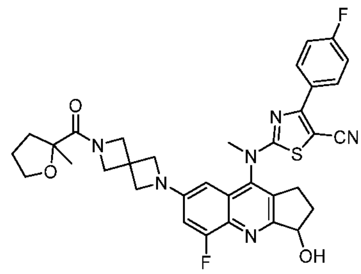,
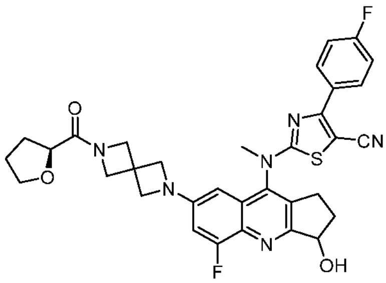,

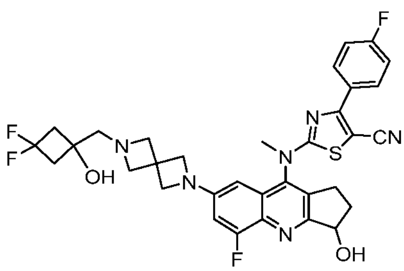,
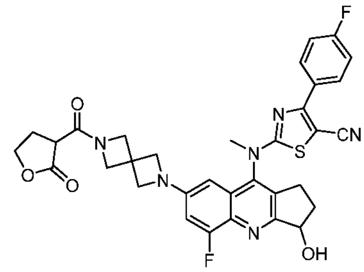,
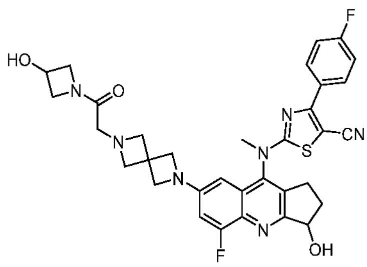,

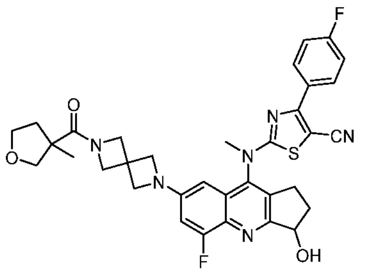,
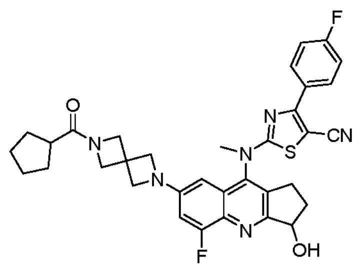,
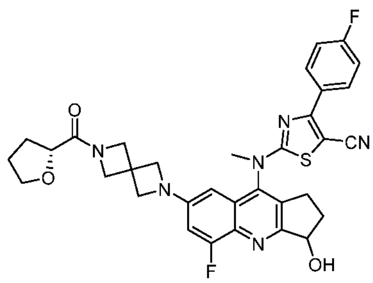,

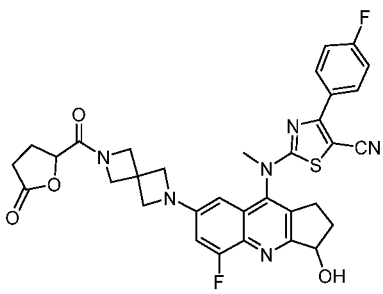,
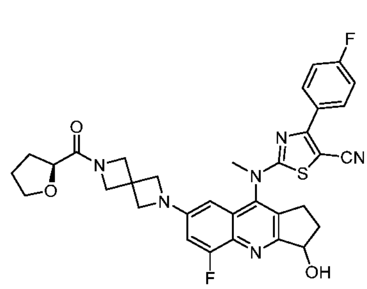,
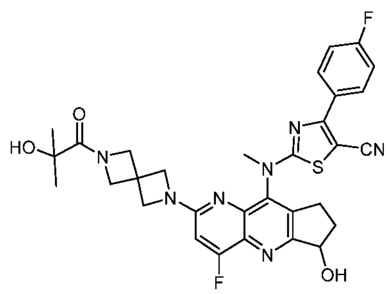,

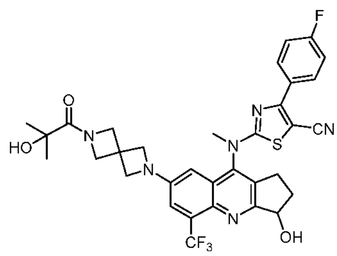,
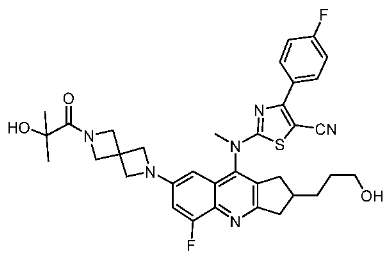, and
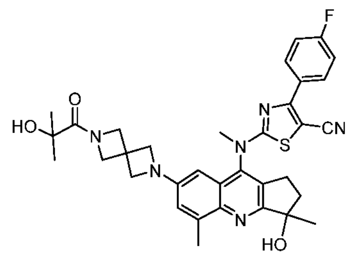.

**27.** A compound of the following formulas, an optical isomer thereof, or a pharmaceutically acceptable salt thereof, selected from:

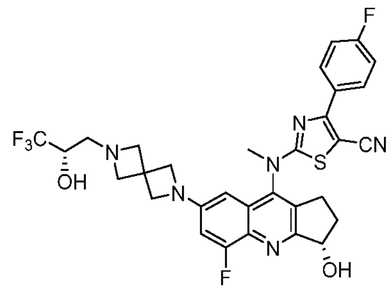,
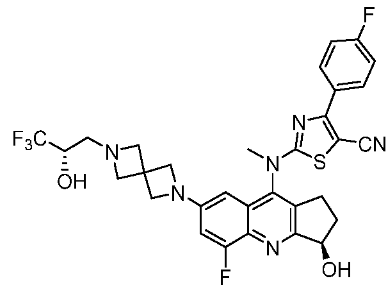,
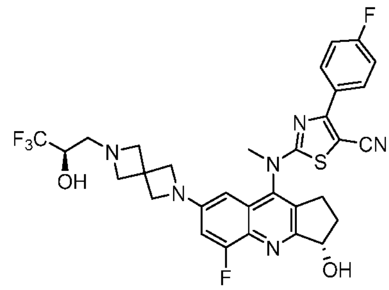,

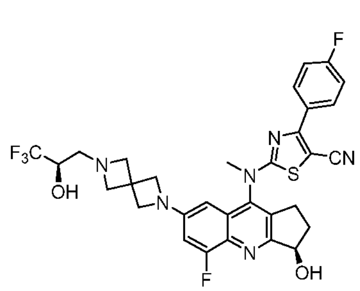,
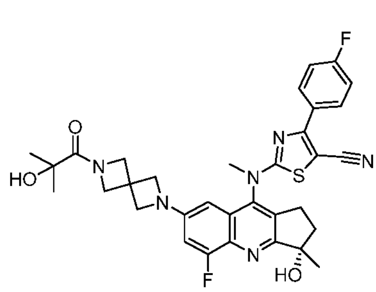,
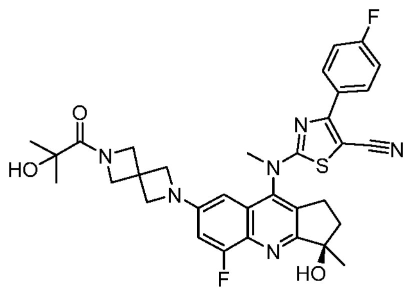,

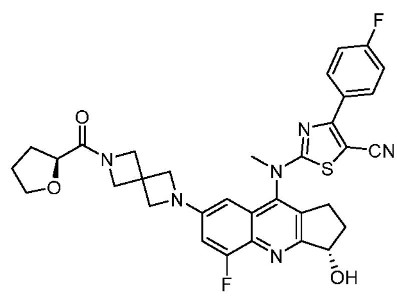,
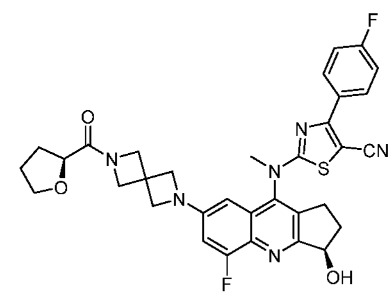,
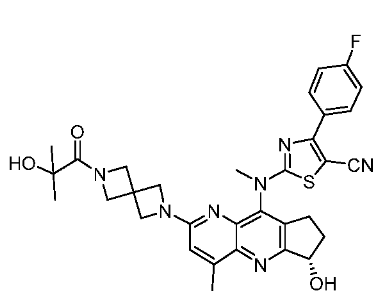,

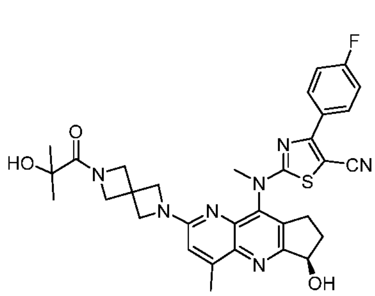,
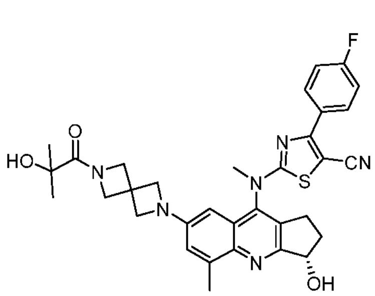,
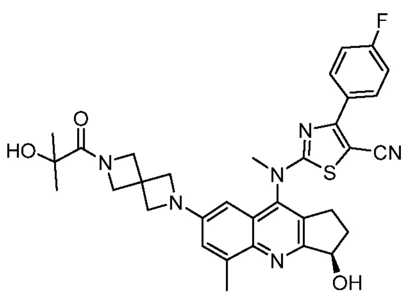,

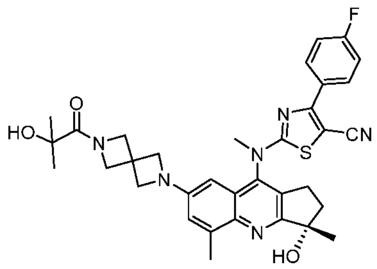, 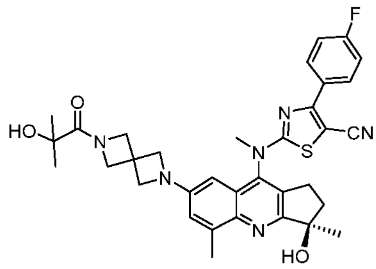, 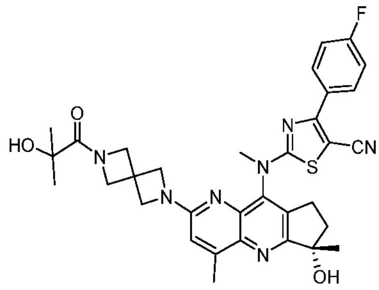,

and

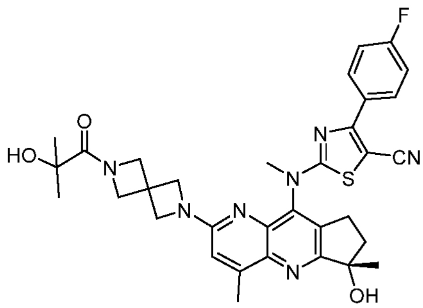.

**28.** A use of the compound, the optical isomer thereof, or the pharmaceutically acceptable salt thereof according to any one of claims 1 to 27 in the manufacture of a medicament for treating a disease related to ATX.

**29.** The disease related to ATX according to claim 28, which is selected from cancer, metabolic disease, renal disease, liver disease, fibrotic disease, inflammatory disease, pain, autoimmune disease, respiratory disease, cardiovascular disease, neurodegenerative disease, myelodysplastic syndrome, obesity, dermatological disorder, and/or disease related to abnormal angiogenesis.

**30.** The fibrotic disease according to claim 29, which is selected from pulmonary fibrosis, renal fibrosis, and hepatic fibrosis.

**31.** The pulmonary fibrosis according to claim 30, which is selected from idiopathic pulmonary fibrosis and non-idiopathic pulmonary fibrosis.

**32.** The liver disease according to claim 29, which is selected from non-alcoholic steatohepatitis.

**33.** The inflammatory disease according to claim 29, which is selected from enteritis and osteoarthritis.

**34.** The autoimmune disease according to claim 29, which is selected from rheumatoid arthritis and multiple sclerosis.

**35.** The respiratory disease according to claim 29, which is selected from interstitial lung disease, asthma, and COPD.

**36.** The cardiovascular disease according to claim 29, which is selected from vascular injury, atherosclerosis, and coagulation.

**INTERNATIONAL SEARCH REPORT**

International application No. **PCT/CN2022/134258**

**A. CLASSIFICATION OF SUBJECT MATTER**

C07D417/14(2006.01)i;A61K31/4353(2006.01)i;A61P11/00(2006.01)i

According to International Patent Classification (IPC) or to both national classification and IPC

**B. FIELDS SEARCHED**

Minimum documentation searched (classification system followed by classification symbols)

C07D417/-; A61K31/-; A61P11/-

Documentation searched other than minimum documentation to the extent that such documents are included in the fields searched

Electronic data base consulted during the international search (name of data base and, where practicable, search terms used)

CNPAT, STN: 结构式, 三并环, 噻唑, 哌嗪, ATX, 癌症, triple ring, thiazole, piperazine, cancer

**C. DOCUMENTS CONSIDERED TO BE RELEVANT**

| Category* | Citation of document, with indication, where appropriate, of the relevant passages | Relevant to claim No. |
|---|---|---|
| A | CN 113493453 A (JIANGSU HENGRUI MEDICINE CO., LTD. et al.) 12 October 2021 (2021-10-12)<br>claims 1-8 | 1-36 |
| A | CN 109384803 A (GUANGZHOU HENOVCOM BIOSCIENCE CO., LTD.) 26 February 2019 (2019-02-26)<br>claims 1-10 | 1-36 |
| A | CN 110526929 A (GUANGZHOU HENOVCOM BIOSCIENCE CO., LTD.) 03 December 2019 (2019-12-03)<br>claims 1-37 | 1-36 |
| A | CN 111620865 A (SUZHOU XINNUOWEI PHARMACEUTICAL TECHNOLOGY CO., LTD.) 04 September 2020 (2020-09-04)<br>claims 1-30 | 1-36 |
| A | CN 112778334 A (GUANGZHOU HENOVCOM BIOSCIENCE CO., LTD.) 11 May 2021 (2021-05-11)<br>claims 1-15 | 1-36 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 February 2023** | **20 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

INTERNATIONAL SEARCH REPORT

International application No.
PCT/CN2022/134258

C. DOCUMENTS CONSIDERED TO BE RELEVANT

| Category* | Citation of document, with indication, where appropriate, of the relevant passages | Relevant to claim No. |
|---|---|---|
| A | WO 2014202458 A1 (GALAPAGOS NV) 24 December 2014 (2014-12-24) claims 1-21 | 1-36 |
| A | WO 2020177729 A1 (JIANGSU HENGRUI MEDICINE CO., LTD. et al.) 10 September 2020 (2020-09-10) claims 1-31 | 1-36 |
| A | WO 2020244539 A1 (JIANGSU HENGRUI MEDICINE CO., LTD. et al.) 10 December 2020 (2020-12-10) claims 1-32 | 1-36 |

Form PCT/ISA/210 (second sheet) (July 2022)

**INTERNATIONAL SEARCH REPORT**
**Information on patent family members**

International application No.
**PCT/CN2022/134258**

| Patent document cited in search report | | | Publication date (day/month/year) | Patent family member(s) | | | Publication date (day/month/year) |
|---|---|---|---|---|---|---|---|
| CN | 113493453 | A | 12 October 2021 | None | | | |
| CN | 109384803 | A | 26 February 2019 | WO | 2019029620 | A1 | 14 February 2019 |
| CN | 110526929 | A | 03 December 2019 | EP | 3805234 | A1 | 14 April 2021 |
| | | | | EP | 3805234 | A4 | 09 March 2022 |
| | | | | WO | 2019223721 | A1 | 28 November 2019 |
| | | | | JP | 2021524475 | A | 13 September 2021 |
| | | | | CA | 3108809 | A1 | 28 November 2019 |
| | | | | US | 2022088008 | A1 | 24 March 2022 |
| | | | | AU | 2019272377 | A1 | 21 January 2021 |
| | | | | AU | 2019272377 | B2 | 10 March 2022 |
| CN | 111620865 | A | 04 September 2020 | AU | 2019220064 | A1 | 01 October 2020 |
| | | | | WO | 2019158107 | A1 | 22 August 2019 |
| | | | | US | 2020369676 | A1 | 26 November 2020 |
| | | | | JP | 2021513551 | A | 27 May 2021 |
| | | | | CA | 3091049 | A1 | 22 August 2019 |
| | | | | EP | 3753943 | A1 | 23 December 2020 |
| | | | | EP | 3753943 | A4 | 23 June 2021 |
| | | | | KR | 20200120930 | A | 22 October 2020 |
| CN | 112778334 | A | 11 May 2021 | WO | 2021088957 | A1 | 14 May 2021 |
| WO | 2014202458 | A1 | 24 December 2014 | US | 2016214986 | A1 | 28 July 2016 |
| | | | | US | 9796719 | B2 | 24 October 2017 |
| | | | | US | 2018127425 | A1 | 10 May 2018 |
| | | | | EP | 3010922 | A1 | 27 April 2016 |
| | | | | EP | 3010922 | B1 | 15 March 2017 |
| | | | | JP | 2016525072 | A | 22 August 2016 |
| | | | | JP | 6435323 | B2 | 05 December 2018 |
| WO | 2020177729 | A1 | 10 September 2020 | TW | 202100512 | A | 01 January 2021 |
| WO | 2020244539 | A1 | 10 December 2020 | TW | 202110831 | A | 16 March 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111416045
- CN 202211205107
- CN 202211457401


### Non-patent literature cited in the description


- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- **ALLINGER N. L. ; ELIEL E. L.** Topics in Stereochemistry. Wiley Interscience, 1971, vol. 6